# EUROPEAN PATENT APPLICATION

(11) **EP 4 530 351 A2**
(43) Date of publication of application: **02.04.2025**
(21) Application number: 24218431.5
(22) Date of filing: 09.04.2020
(51) Int. Cl.: C12N 15/11

(54) **COMPOSITIONS AND METHODS FOR IMPROVED GENE EDITING**

(30) Priority: 12.04.2019 US 201962833404 P
(62) Divisional of application: 20718311.2
(71) Applicant: Astrazeneca AB, 151 85 Södertälje (SE)
(72) Inventor: MARESCA, Marcello, Södertälje (SE); LI, Songyuan, Södertälje (SE)
(74) Representative: AstraZeneca Intellectual Property

(57) **Abstract**

The present disclosure provides methods of introducing site-specific mutations in a target cell and methods of determining efficacy of enzymes capable of introducing site-specific mutations. The present disclosure also provides methods of providing a bi-allelic sequence integration, methods of integrating of a sequence of interest into a locus in a genome of a cell, and methods of introducing a stable episomal vector in a cell. The present disclosure further provides methods of generating a human cell that is resistant to diphtheria toxin.

## Description

### FIELD OF THE INVENTION

The present disclosure provides methods of introducing site-specific mutations in a target cell and methods of determining efficacy of enzymes capable of introducing site-specific mutations. The present disclosure also provides methods of providing a bi-allelic sequence integration, methods of integrating of a sequence of interest into a locus in a genome of a cell, and methods of introducing a stable episomal vector in a cell. The present disclosure further provides methods of generating a human cell that is resistant to diphtheria toxin.

### BACKGROUND

Targeted nucleic acid modification by programmable, site-specific nucleases such as, e.g., zinc-finger nucleases (ZFNs), transcription activator-like effector nucleases (TALENs) and the RNA-guided Cas9, is a highly promising approach for the study of gene function and also has great potential for providing new therapeutics for genetic diseases. Typically, the programmable nuclease generates a double-stranded break (DSB) at the target sequence. The DSB can then be repaired with mutations via the non-homologous end joining (NHEJ) pathway, or the DNA around the cleavage site can be replaced with a simultaneously-introduced template via the homology-directed repair (HDR) pathway. For an overview of targeted nucleic acid modifications, *see,* e.g., Humbert et al., Crit Rev Biochem Mol Biol (2012) 47:264-281; Perez-Pinera et al., Curr Opin Chem Biol (2012) 16:268-277; and Pan et al., Mol Biotechnol (2013) 55:54-62.

Drawbacks of relying upon NHEJ and HDR include, e.g., the low efficiency of HDR and undesired off-target activity by NHEJ. The low efficiency of HDR poses a particular challenge for selection of precise, on-target modifications (*see,* e.g., Humbert et al., Crit Rev Biochem Mol Biol (2012) 47:264-281; Peng et al., FEBS J (2016) 283:1218-1231; Liu et al., J Biol Chem (2017) 292:5624-5633). Various efforts towards biasing HDR over NHEJ include, for example, generating one or more single-stranded nicks in the target DNA rather than a DSB (*see,* e.g., Richardson et al., Nature Biotechnol (2016) 34:339-344; Kocher et al., Mol Ther (2017) 25:2585-2598). However, there remains a need in the field for improved selection of HDR events, for example, when biallelic integration or gene silencing is desired, which is typically achieved with an HDR template.

While HDR is less error-prone compared with NHEJ, HDR is still prone to generation of undesirable modifications that compete with the targeted modification. Thus, base editing has recently emerged as a powerful, precise gene editing technology that facilitates single base pair substitutions at a specific location in the genome. Compared with HDR-based methods for site-specific modifications, base editing provides a more efficient way to introduce single nucleotide mutations, overcoming some of the limitations associated with HDR. Base editing involves a site-specific modification of a single DNA base, along with manipulation of the native DNA repair machinery to avoid faithful repair of the modified base. Base editors are typically chimeric proteins including a DNA targeting module and a catalytic domain capable of deaminating, e.g., a cytidine base to thymine or adenine base to guanine. For example, the DNA targeting module may be based on a catalytically inactive Cas9 (dCas9) or Cas9 nickase variant (Cas9n), guided by a guide RNA molecule (sgRNA or gRNA). The catalytic domain may be a cytidine deaminase or an adenine deaminase. There is no need to generate a DSB to edit DNA bases, limiting the generation of insertions and deletions (indels) at target and off-target sites. Thus, base editing does not rely on the cellular HDR machinery and is therefore more efficient than HDR and results in fewer imprecise modifications by NHEJ. Engineered base editing systems are described in, e.g., Gaudelli et al., Nature (2017) 551:464-471; Rees et al., Nature Comm (2017) 8:15790; Billon et al., Mol Cell (2017) 67:1068-1079; and Zafra et al., Nat Biotechnol (2018) 36:888-893. For an overview of base editing, *see,* e.g., Hess et al., Mol Cell (2017) 68:26-43; Eid et al., Biochem J (2018) 475:1955-1964; and Komor et al., ACS Chem Biol (2018) 13:383-388.

Because many genetic diseases may be attributed to a specific nucleotide change a specific location in the genome (for example, a C to T change in a specific codon of a gene associated with a disease), base editing may serve as a promising therapeutic approach to treating genetic disorders based on a single nucleotide variant. However, despite the improvement over traditional CRISPR/Cas9 editing, base editing efficiency remains low to moderate and additionally suffers from inconsistency across the genome. Thus, there remains a need in the field for an improved base editing system with higher efficiency.

Various publications are cited herein, the disclosures of which are incorporated by reference herein in their entireties.

### SUMMARY OF THE INVENTION

In some embodiments, the present disclosure provides a method of introducing a site-specific mutation in a target polynucleotide in a target cell in a population of cells, the method comprising: (a) introducing into the population of cells: (i) a base-editing enzyme; (ii) a first guide polynucleotide that (1) hybridizes to a gene encoding a cytotoxic agent (CA) receptor, and (2) forms a first complex with the base-editing enzyme, wherein the base-editing enzyme of the first complex provides a mutation in the gene encoding the CA receptor, and wherein the mutation in the gene encoding the CA receptor forms a CA-resistant cell in the population of cells; and (iii) a second guide polynucleotide that (1) hybridizes with the target polynucleotide, and (2) forms a second complex with the base-editing enzyme, wherein the base-editing enzyme of the second complex provides a mutation in the target polynucleotide; (b) contacting the population of cells with the CA; and (c) selecting the CA-resistant cell from the population of cells, thereby enriching for the target cell comprising the mutation in the target polynucleotide.

In some embodiments, the present disclosure provides a method of determining efficacy of a base-editing enzyme in a population of cells, the method comprising: (a) introducing into the population of cells: (i) a base-editing enzyme; (ii) a first guide polynucleotide that (1) hybridizes to a gene encoding a cytotoxic agent (CA) receptor, and (2) forms a first complex with the base-editing enzyme, wherein the base-editing enzyme of the first complex introduces a mutation in the gene encoding the CA receptor, and wherein the mutation in the gene encoding the CA receptor forms a CA-resistant cell in the population of cells; and (iii) a second guide polynucleotide that (1) hybridizes with the target polynucleotide, and (2) forms a second complex with the base-editing enzyme, wherein the base-editing enzyme of the second complex introduces a mutation in the target polynucleotide; (b) contacting the population of cells with the CA to isolate CA-resistant cells; and (c) determining the efficacy of the base-editing enzyme by determining the ratio of the CA-resistant cells to the total population of cells.

In some embodiments, the base-editing enzyme comprises a DNA-targeting domain and a DNA-editing domain.

In some embodiments, the DNA-targeting domain comprises Cas9. In some embodiments, the Cas9 comprises a mutation in a catalytic domain. In some embodiments, the base-editing enzyme comprises a catalytically inactive Cas9 and a DNA-editing domain. In some embodiments, the base-editing enzyme comprises a Cas9 capable of generating single-stranded DNA breaks (nCas9) and a DNA-editing domain. In some embodiments, the nCas9 comprises a mutation at amino acid residue D10 or H840 relative to wild-type Cas9 (numbering relative to SEQ ID NO: 3). In some embodiments, the Cas9 is at least 90% identical to SEQ ID NO: 3 or 4.

In some embodiments, the DNA-editing domain comprises a deaminase. In some embodiments, the deaminase is cytidine deaminase or adenosine deaminase. In some embodiments, the deaminase is cytidine deaminase. In some embodiments, the deaminase is adenosine deaminase. In some embodiments, the deaminase is an apolipoprotein B mRNA-editing complex (APOBEC) deaminase, an activation-induced cytidine deaminase (AID), an ACF1/ASE deaminase, an ADAT deaminase, or an ADAR deaminase. In some embodiments, the deaminase is an apolipoprotein B mRNA-editing complex (APOBEC) family deaminase. In some embodiments, the deaminase is APOBEC1.

In some embodiments, the base-editing enzyme further comprises a DNA glycosylase inhibitor domain. In some embodiments, the DNA glycosylase inhibitor is uracil DNA glycosylase inhibitor (UGI). In some embodiments, the base-editing enzyme comprises nCas9 and cytidine deaminase. In some embodiments, the base-editing enzyme comprises nCas9 and adenosine deaminase. In some embodiments, the base-editing enzyme comprises a polypeptide sequence at least 90% identical to SEQ ID NO: 6. In some embodiments, the base-editing enzyme is BE3.

In some embodiments, the first and/or second guide polynucleotide is an RNA polynucleotide. In some embodiments, the first and/or second guide polynucleotide further comprises a tracrRNA sequence.

In some embodiments, the population of cells are human cells.

In some embodiments, the mutation in the gene encoding the CA receptor is a cytidine (C) to thymine (T) point mutation. In some embodiments, the mutation in the gene encoding the CA receptor is an adenine (A) to guanine (G) point mutation.

In some embodiments, the CA is diphtheria toxin. In some embodiments, the cytotoxic agent (CA) receptor is a receptor for diphtheria toxin. In some embodiments, the CA receptor is a heparin binding EGF like growth factor (HB-EGF). In some embodiments, the HB-EGF comprises the polypeptide sequence of SEQ ID NO: 8.

In some embodiments, the base-editing enzyme of the first complex provides a mutation in one of more of amino acids 107 to 148 in HB-EGF. In some embodiments, the base-editing enzyme of the first complex provides a mutation in one of more of amino acids 138 to 144 in HB-EGF. In some embodiments, the base-editing enzyme of the first complex provides a mutation in amino acid 141 in HB-EGF. In some embodiments, the base-editing enzyme of the first complex provides a GLU141 to LYS 141 mutation in the amino acid sequence of HB-EGF.

In some embodiments, the base-editing enzyme of the first complex provides a mutation in a region of HB-EGF that binds diphtheria toxin. In some embodiments, the base-editing enzyme of the first complex provides a mutation in HB-EGF which makes the target cell resistant to diphtheria toxin. In some embodiments, the mutation in the target polynucleotide is a cytidine (C) to thymine (T) point mutation in the target polynucleotide. In some embodiments, the mutation in the target polynucleotide is an adenine (A) to guanine (G) point mutation in the target polynucleotide.

In some embodiments, the base-editing enzyme is introduced into the population of cells as a polynucleotide encoding the base-editing enzyme. In some embodiments, the polynucleotide encoding the base-editing enzyme, the first guide polynucleotide of (ii), and the second guide polynucleotide of (iii) are on a single vector. In some embodiments, the polynucleotide encoding the base-editing enzyme, the first guide polynucleotide of (ii), and the second guide polynucleotide of (iii) are on one or more vectors. In some embodiments, the vector is a viral vector. In some embodiments, the viral vector is an adenovirus, a lentivirus, or an adeno-associated virus.

In some embodiments, the present disclosure provides a method of providing a bi-allelic integration of a sequence of interest (SOI) into a toxin sensitive gene (TSG) locus in a genome of a cell, the method comprising: (a) introducing into a population of cells: (i) a nuclease capable of generating a double-stranded break; (ii) a guide polynucleotide that forms a complex with the nuclease and is capable of hybridizing with the TSG locus; and (iii) a donor polynucleotide comprising: (1) a 5' homology arm, a 3' homology arm, and a mutation in a native coding sequence of the TSG, wherein the mutation confers resistance to the toxin; and (2) the SOI; wherein introduction of (i), (ii), and (iii) results in integration of the donor polynucleotide in the TSG locus; (b) contacting the population of cells with the toxin; and (c) selecting one or more cells resistant to the toxin, wherein the one or more cells resistant to the toxin comprise the bi-allelic integration of the SOI.

In some embodiments, the donor polynucleotide is integrated by homology-directed repair (HDR). In some embodiments, the donor polynucleotide is integrated by Non-Homologous End Joining (NHEJ).

In some embodiments, the TSG locus comprises an intron and an exon. In some embodiments, the donor polynucleotide further comprises a splicing acceptor sequence. In some embodiments, the nuclease capable of generating a double-stranded break generates a break in the intron. In some embodiments, the mutation in the native coding sequence of the TSG is in an exon of the TSG locus.

In some embodiments, the present disclosure provides a method of integrating a sequence of interest (SOI) into a target locus in a genome of a cell, the method comprising: (a) introducing into a population of cells: (i) a nuclease capable of generating a double-stranded break; (ii) a guide polynucleotide that forms a complex with the nuclease and is capable of hybridizing with a toxin sensitive gene (TSG) locus in the genome of the cell, wherein the TSG is an essential gene; and (iii) a donor polynucleotide comprising: (1) a functional TSG gene comprising a mutation in a native coding sequence of the TSG, wherein the mutation confers resistance to the toxin, (2) the SOI, and (3) a sequence for genome integration at the target locus; wherein introduction of (i), (ii), and (iii) results in: inactivation of the TSG in the genome of the cell by the nuclease, and integration of the donor polynucleotide in the target locus; (b) contacting the population of cells with the toxin; and (c) selecting one or more cells resistant to the toxin, wherein the one or more cells resistant to the toxin comprise the SOI integrated in the target locus.

In some embodiments, the sequence for genome integration is obtained from a transposon or a retroviral vector.

In some embodiments, the functional TSG of the donor polynucleotide or the episomal vector is resistant to inactivation by the nuclease. In some embodiments, the mutation in the native coding sequence of the TSG removes a protospacer adjacent motif from the native coding sequence. In some embodiments, the guide polynucleotide is not capable of hybridizing to the functional TSG of the donor polynucleotide or the episomal vector.

In some embodiments, the nuclease capable of generating a double-stranded break is Cas9. In some embodiments, the Cas9 is capable of generating cohesive ends. In some embodiments, the Cas9 comprises a polypeptide sequence of SEQ ID NO: 3 or 4.

In some embodiments, the guide polynucleotide is an RNA polynucleotide. In some embodiments, the guide polynucleotide further comprises a tracrRNA sequence.

In some embodiments, the donor polynucleotide is a vector. In some embodiments, the mutation in the native coding sequence of the TSG is a substitution mutation, an insertion, or a deletion. In some embodiments, the mutation in the native coding sequence of the TSG is a mutation in a toxin-binding region of a protein encoded by the TSG. In some embodiments, the TSG locus comprises a gene encoding heparin binding EGF-like growth factor (HB-EGF). In some embodiments, the TSG encodes HB-EGF (SEQ ID NO: 8).

In some embodiments, the mutation in the native coding sequence of the TSG is a mutation in one or more of amino acids 107 to 148 in HB-EGF (SEQ ID NO: 8). In some embodiments, the mutation in the native coding sequence of the TSG is a mutation in one or more of amino acids 138 to 144 in HB-EGF (SEQ ID NO: 8). In some embodiments, the mutation in the native coding sequence of the TSG is a mutation in amino acid 141 in HB-EGF (SEQ ID NO: 8). In some embodiments, the mutation in the native coding sequence of the TSG is a mutation of GLU141 to LYS141 in HB-EGF (SEQ ID NO: 8).

In some embodiments, the toxin is diphtheria toxin. In some embodiments, the mutation in the native coding sequence of the TSG makes the cell resistant to diphtheria toxin. In some embodiments, the toxin is an antibody-drug conjugate, wherein the TSG encodes a receptor for the antibody-drug conjugate.

In some embodiments, the present disclosure provides a method of providing resistance to diphtheria toxin in a human cell, the method comprising introducing into the cell: (i) a base-editing enzyme; and (ii) a guide polynucleotide targeting a heparin-binding EGF-like growth factor (HB-EGF) receptor in the human cell, wherein the base-editing enzyme forms a complex with the guide polynucleotide, and wherein the base-editing enzyme is targeted to the HB-EGF and provides a site-specific mutation in the HB-EGF, thereby providing resistance to diphtheria toxin in the human cell.

In some embodiments, the base-editing enzyme comprises a DNA-targeting domain and a DNA-editing domain.

In some embodiments, the DNA-targeting domain comprises Cas9. In some embodiments, the Cas9 comprises a mutation in a catalytic domain. In some embodiments, the base-editing enzyme comprises a catalytically inactive Cas9 and a DNA-editing domain. In some embodiments, the base-editing enzyme comprises a Cas9 capable of generating single-stranded DNA breaks (nCas9) and a DNA-editing domain. In some embodiments, the nCas9 comprises a mutation at amino acid residue D10 or H840 relative to wild-type Cas9 (numbering relative to SEQ ID NO: 3). In some embodiments, the Cas9 is at least 90% identical to SEQ ID NO: 3 or 4.

In some embodiments, the DNA-editing domain comprises a deaminase. In some embodiments, the deaminase is selected from cytidine deaminase and adenosine deaminase. In some embodiments, the deaminase is cytidine deaminase. In some embodiments, the deaminase is adenosine deaminase. In some embodiments, the deaminase is selected from an apolipoprotein B mRNA-editing complex (APOBEC) deaminase, an activation-induced cytidine deaminase (AID), an ACF1/ASE deaminase, an ADAT deaminase, and a TadA deaminase. In some embodiments, the deaminase is an apolipoprotein B mRNA-editing complex (APOBEC) family deaminase. In some embodiments, the cytidine deaminase is APOBEC1. In some embodiments, the base-editing enzyme further comprises a DNA glycosylase inhibitor domain. In some embodiments, the DNA glycosylase inhibitor is uracil DNA glycosylase inhibitor (UGI).

In some embodiments, the base-editing enzyme comprises nCas9 and a cytidine deaminase. In some embodiments, the base-editing enzyme comprises nCas9 and an adenosine deaminase. In some embodiments, the base-editing enzyme comprises a polypeptide sequence at least 90% identical to SEQ ID NO: 6. In some embodiments, the base-editing enzyme is BE3.

In some embodiments, the guide polynucleotide is an RNA polynucleotide. In some embodiments, the guide polynucleotide further comprises a tracrRNA sequence.

In some embodiments, the site-specific mutation is in one or more of amino acids 107 to 148 in the HB-EGF (SEQ ID NO: 8). In some embodiments, the site-specific mutation is in one or more of amino acids 138 to 144 in the HB-EGF (SEQ ID NO: 8). In some embodiments, the site-specific mutation is in amino acid 141 in the HB-EGF (SEQ ID NO: 8). In some embodiments, the site-specific mutation is a GLU141 to LYS141 mutation in the HB-EGF (SEQ ID NO: 8). In some embodiments, the site-specific mutation is in a region of the HB-EGF that binds diphtheria toxin.

In some embodiments, the present disclosure provides a method of integrating and enriching a sequence of interest (SOI) into a target locus in a genome of a cell, the method comprising: (a) introducing into a population of cells: (i) a nuclease capable of generating a double-stranded break; (ii) a guide polynucleotide that forms a complex with the nuclease and is capable of hybridizing with an essential gene (ExG) locus in the genome of the cell; and (iii) a donor polynucleotide comprising: (1) a functional ExG gene comprising a mutation in a native coding sequence of the ExG, wherein the mutation confers resistance to inactivation by the guide polynucleotide, (2) the SOI, and (3) a sequence for genome integration at the target locus; wherein introduction of (i), (ii), and (iii) results in inactivation of the ExG in the genome of the cell by the nuclease, and integration of the donor polynucleotide in the target locus; (b) cultivating the cells; and (c) selecting one or more surviving cells, wherein the one or more surviving cells comprise the SOI integrated at the target locus.

In some embodiments, the present disclosure provides method of introducing a stable episomal vector into a cell, the method comprising: (a) introducing into a population of cells: (i) a nuclease capable of generating a double-stranded break; (ii) a guide polynucleotide that forms a complex with the nuclease and is capable of hybridizing with an essential gene (ExG) locus in the genome of the cell; wherein introduction of (i) and (ii) results in inactivation of the ExG in the genome of the cell by the nuclease; and (iii) an episomal vector comprising: (1) a functional ExG comprising a mutation in a native coding sequence of the ExG, wherein the mutation confers resistance to the inactivation by the nuclease; (2) an autonomous DNA replication sequence; (b) cultivating the cells; and (c) selecting one or more surviving cells, wherein the one or more surviving cells comprise the episomal vector.

In some embodiments, mutation in the native coding sequence of the ExG removes a protospacer adjacent motif from the native coding sequence. In some embodiments, the guide polynucleotide is not capable of hybridizing to the functional ExG of the donor polynucleotide or the episomal vector.

In some embodiments, the nuclease capable of generating a double-stranded break is Cas9. In some embodiments, the Cas9 is capable of generating cohesive ends. In some embodiments, the Cas9 comprises a polypeptide sequence of SEQ ID NO: 3 or 4.

In some embodiments, the guide polynucleotide is an RNA polynucleotide. In some embodiments, the guide polynucleotide further comprises a tracrRNA sequence.

In some embodiments, the donor polynucleotide is a vector. In some embodiments, the mutation in the native coding sequence of the ExG is a substitution mutation, an insertion, or a deletion.

In some embodiments, the sequence for genome integration is obtained from a transposon or a retroviral vector. In some embodiments, the episomal vector is an artificial chromosome or a plasmid.

In some embodiments, more than one guide polynucleotide is introduced into the population of cells, wherein each guide polynucleotide forms a complex with the nuclease, and wherein each guide polynucleotide hybridizes to a different region of the ExG.

In some embodiments, the method further comprises introducing the nuclease of (a)(i) and the guide polynucleotide of (a)(ii) into the surviving cells to enrich for surviving cells comprising the SOI integrated at the target locus. In some embodiments, the method further comprises introducing the nuclease of (a)(i) and the guide polynucleotide of (a)(ii) into the surviving cells to enrich for surviving cells comprising the episomal vector. In some embodiments, the nuclease of (a)(i) and the guide polynucleotide of (a)(ii) are introduced into the surviving cells for multiple rounds of enrichment.

### BRIEF DESCRIPTION OF THE DRAWINGS

FIG. 1A shows an exemplary cell that has a target site and a selection site subjected to base-editing. Without a selection strategy, only a low percentage of the resulting population of cells have the desired "edited" site. With a co-targeting and selection strategy as provided herein, a majority of the resulting population of cells have the desired "edited" site.
FIG. 1B shows selection of a guide RNA for targeting HB-EGF by tiling through the EGF-like domain of HB-EGF and determining the guide RNA that resulted in diphtheria toxin resistance.
FIG. 1C shows a comparison of the editing efficiency of PCSK9 and BFP in various cell lines with (Control) and without (Enriched) the diphtheria toxin selection strategy. The population of cells with PCSK9 or BFP edited was increased significantly after diphtheria toxin selection.

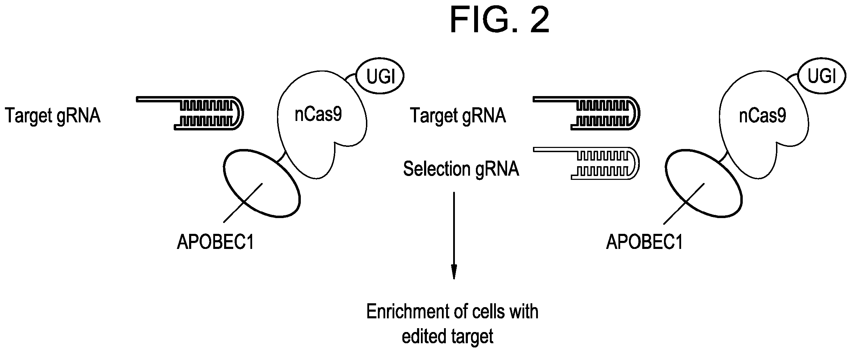

FIG. 2 shows the BE3 base editor, which includes nCas9, APOBEC1, and UGI. BE3 can complex with the target gRNA and the selection gRNA. Utilizing both the target and selection gRNAs results in enrichment of cells with edited target.
FIG. 3A is described by Slonczewski, JL and Foster, JW, "Chapter 25. Microbial Pathogenesis." Microbiology: An Evolving Science. New York: W.W. Norton, 2011. FIG. 3A shows the mechanism by which diphtheria toxin causes cell death.
FIG. 3B is described by Mitamura et al., J Biol Chem 270:1015-1019 (1995). FIG. 3B is a sequence alignment of the polypeptide sequences of human (hHB-EGF) and mouse (mHBEGF) HB-EGF proteins.
FIGS. 4A and 4B show selection of guide RNA for targeting HB-EGF in HEK293 and HCT116 cells, respectively, by tiling through the EGF-like domain of HB-EGF and determining the guide RNA that resulted in diphtheria toxin resistance. FIG. 4C shows the design of the various gRNAs in FIGS. 4A and 4B.
FIG. 5A shows the sequence of gRNA 16 (underlined).
FIGS. 5B and 5C show the editing efficiency at three different locations in HB-EGF using gRNA 16 in HCT116 and HEK293 cells, respectively.
FIG. 5D shows the amino acid mutation patterns of all surviving HEK293 cells in diphtheria toxin selection. The mutation occurring in the highest percentage (44.13%) of cells encode only one amino acid change, i.e., the substitution of glutamate at position 141 to lysine.
FIG. 6 is described by Louie et al., Molecular Cell 1(1):67-78 (1997) and shows a structure of HB-EGF. The E141 residue is targeted by gRNA 16 shown in FIG. 5.
FIGS. 7A and 7B show the editing efficiency at the PCSK9 target site to generate a stop codon, with (Enriched) and without diphtheria selection (Control) in HCT116 cells and HEK293 cells, respectively. Editing efficiency increased with diphtheria selection. FIG. 7C shows the sequence of the gRNA targeting pCKS9 (underlined).
FIG. 7D shows the editing efficiency at the DPM2, EGFR, EMX1 and Yas85 target sites to generate stop codons or introduce SNPs, with (Enriched) and without diphtheria selection (Control) in HEK293 cells, respectively. Editing efficiency increased with diphtheria selection. FIG. 7E shows the sequence of the gRNA targeting DPM2, EGFR, EMX1 and Yas85.
FIG. 8A shows the percentage of indels generated at the PCSK9 target site in HEK293 and HCT116 cells, with (Control) and without (Enriched) diphtheria toxin selection. The sequence of gRNA is the same as the one described in FIG. 7C. FIG. 8B shows the percentage of indels generated at DPM2, EMX1 and Yas85 target sites in HEK293 cells, with (Control) and without (Enriched) diphtheria toxin selection. The sequences of the gRNAs are shown in FIG. 7E. Using diphtheria toxin selection increased the percentage of indels (editing efficiency) dramatically.

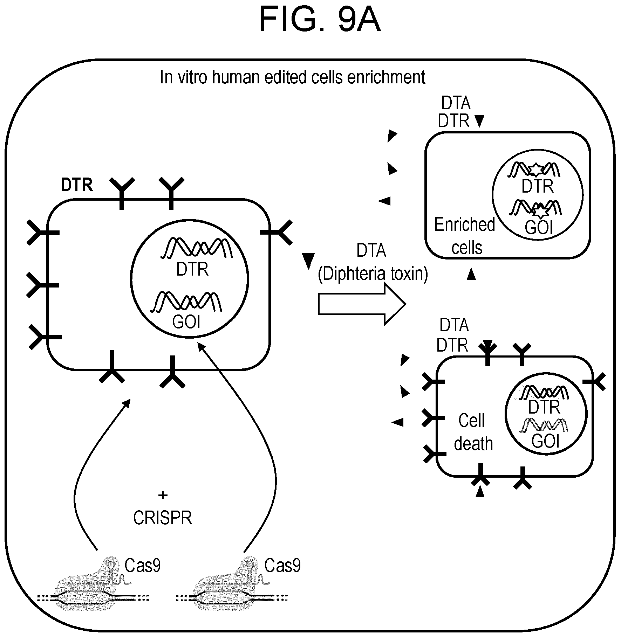

FIG. 9A illustrates an embodiment of the methods provided herein. CRISPR-Cas9 complexes targeting the diphtheria toxin receptor (DTR) and the gene of interest to be edited (GOI) are introduced into the cell, which expresses the DTR on the cell surface. Cells are then exposed to diphtheria toxin (DTA). The cells in which the CRISPR-Cas9 complexes were successfully introduced have edited DTR and the desired edited GOI (indicated by the star). These cells do not express the DTR and survive the DTA treatment. Cells which did not undergo editing express the DTR and die upon DTA treatment.

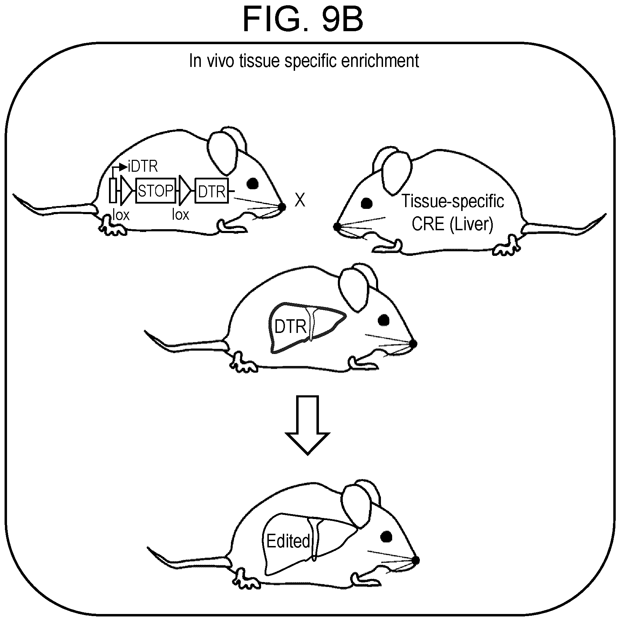

FIG. 9B illustrates a mouse with a humanized liver that is sensitive to diphtheria toxin, which can then be edited and enriched using the selection methods provided herein.
FIG. 10A illustrates an exemplary method for bi-allelic integration of a gene of interest (GOI). In FIG. 10A, the wild-type HB-EGF is cut at an intron by a CRISPR-Cas9 complex. An HDR template that includes a splicing acceptor sequence, an HB-EGF with a diphtheria toxin-resistant mutation, and the GOI is also introduced. Diphtheria toxin selection results in cells that have the diphtheria toxin-resistant mutation and the GOI.
FIGS. 10B and 10C show the results of the GOI insertion (knock-in) after diphtheria toxin selection. The T2A self-cleavage peptide (T2A) with mCherry was tested as GOI. Cells with successful insertions would translate mCherry together with the mutated HB-EGF gene, and the cells would show mCherry fluorescence. After diphtheria toxin selection, almost all cells transfected with Cas9, gRNA SaW10, and mCherry HDR template are mCherry positive (FIG. 10B), and the expression of mCherry is homogenous across the whole population (FIG. 10C).
FIGS. 10D, 10E and 10F show the strategy and PCR analysis results of GOI knock-in cells generated by the method described in FIG. 10A.

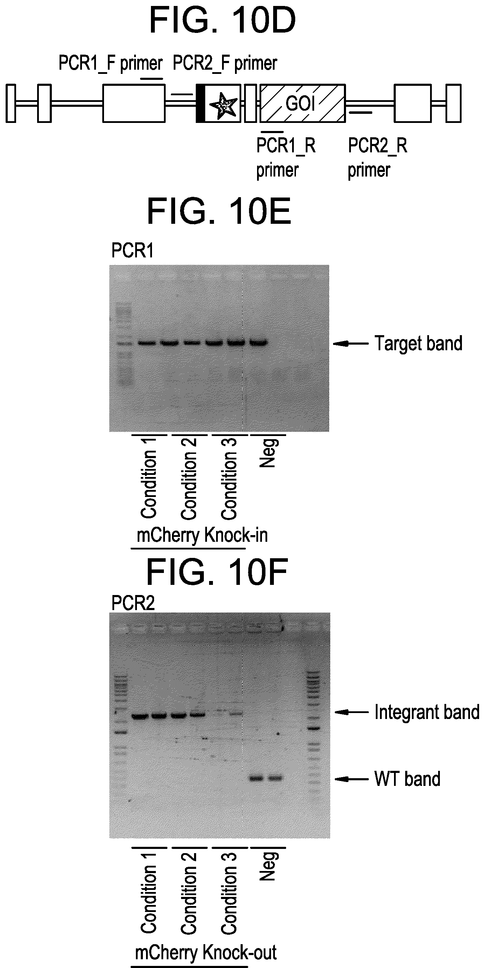

FIG. 10D shows the PCR analysis strategy. PCR1 amplifies the junction region with forward primer (PCR1_F primer) binding a sequence in the genome and reverse primer (PCR1_R primer) binding a sequence in the GOI. Only cells with GOI integrated would show a positive band, as indicated in FIG. 10E. PCR2 amplifies the insertion region with forward primer (PCR2_F primer) binding a sequence in the 5' end of the insertion and reverse primer (PCR2_R primer) binding a sequence at the 3' end of the insertion. Amplification only occurs if all alleles in the cells were inserted sucessfully with the GOI, and the amplified product would be shown as a single integrant band, as indicated in FIG. 10F. If any wild type allele exists, a WT band would be shown, as indicated in FIG. 10F. FIG. 10E shows that insertions are successfully achieved with this method, and FIG. 10F shows that no wild-type alleles exist in the tested cells, indicating a bi-allelic integration. "Condition 1," "Condition 2," and "Condition 3" correspond to different weight ratios of Cas9 plasmid, gRNA plasmid and knock-in plasmid described in Table 2. "Neg" corresponds to Negtive control 1 described in Table 2.

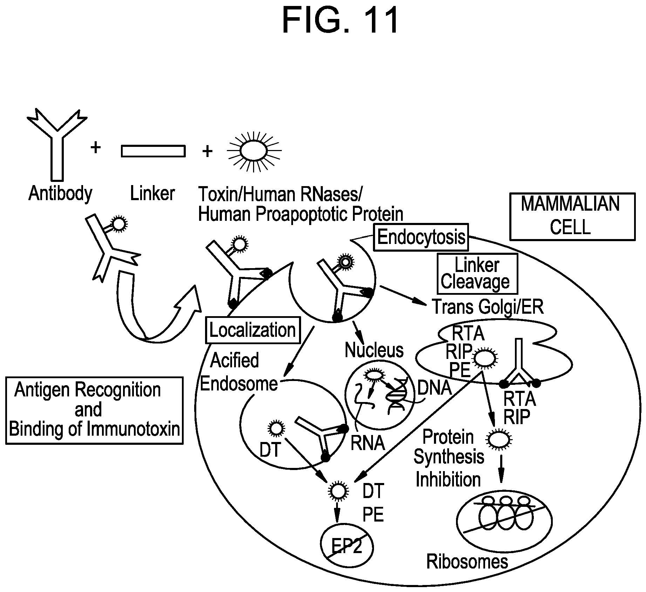

FIG. 11 is described by Grawunder and Barth (Eds.), Next Generation Antibody Drug Conjugates (ADCs) and Immunotoxins, Springer, 2017; doi:10.1007/978-3-319-46877-8. FIG. 11 shows examples of antibody-drug conjugates (ADCs) described herein. In embodiments of the methods provided herein, an ADC is the cytotoxic agent, and the receptor for the antibody of the ADC is the receptor.

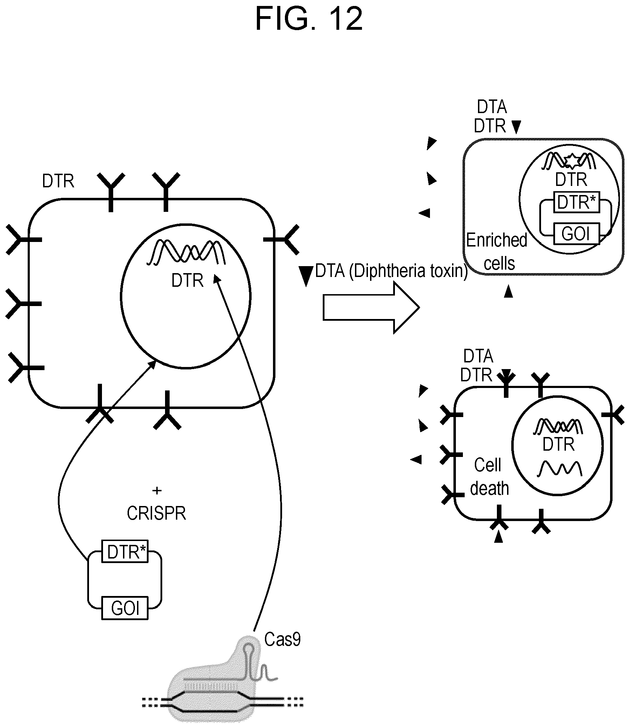

FIG. 12 illustrates an exemplary method for selection of cells with a vector comprising a gene of interest (GOI). A CRISPR-Cas9 complex targets the diphtheria toxin receptor (DTR) and creates a knock-out of the DTR that results in cell death. A vector having a DTR that is resistant to the toxin and resistant to Cas9 cleavage (denoted as DTR*) and the GOI is also introduced into the cell. Selection by diphtheria toxin results in cell death for the cells that either do not have edited DTR or do not have the vector. Surviving cells that have the edited genomic DTR and the vector with DTR* and the GOI. The vector can be an episomal vector or integrated as a plasmid, a transposon, or a retroviral vector.

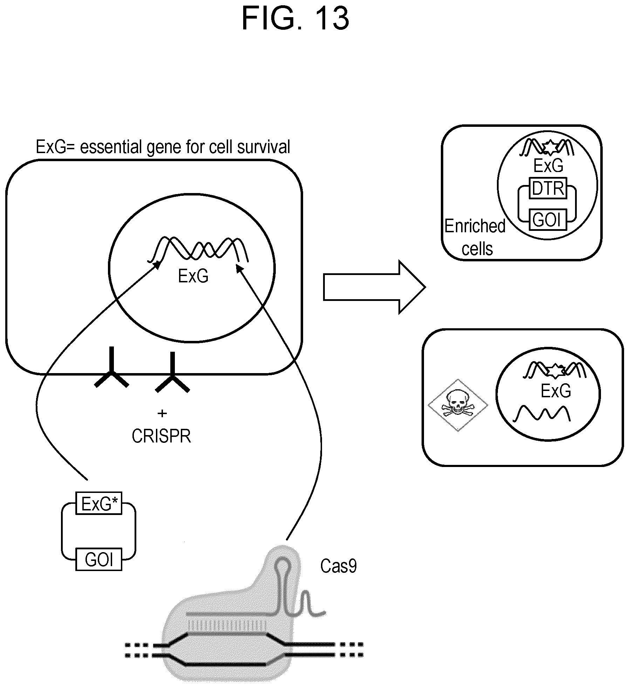

FIG. 13 illustrates an exemplary method for selection of cells with a vector comprising a gene of interest (GOI). A CRISPR-Cas9 complex targets an essential gene (ExG) and creates a knock-out of the ExG that results in cell death. A vector having an ExG that is resistant to Cas9 cleavage (denoted as ExG*) and the GOI is also introduced into the cell. Surviving cells have the edited genomic ExG and the vector with ExG* and the GOI. The vector can be an episomal vector or integrated as a plasmid, a transposon, or a retroviral vector.

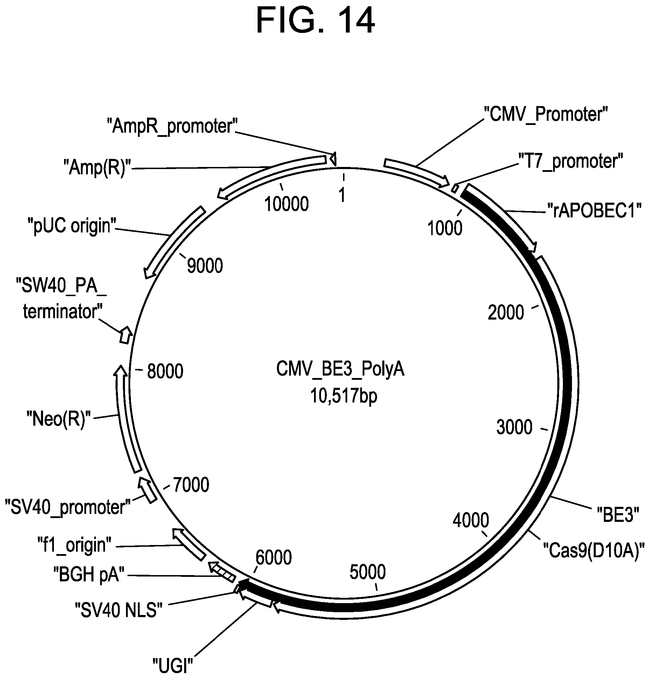

FIGS. 14-22 show maps of the plasmids described in the Examples.
FIG. 14 shows a plasmid expressing the BE3 base editing enzyme used in Example 3.

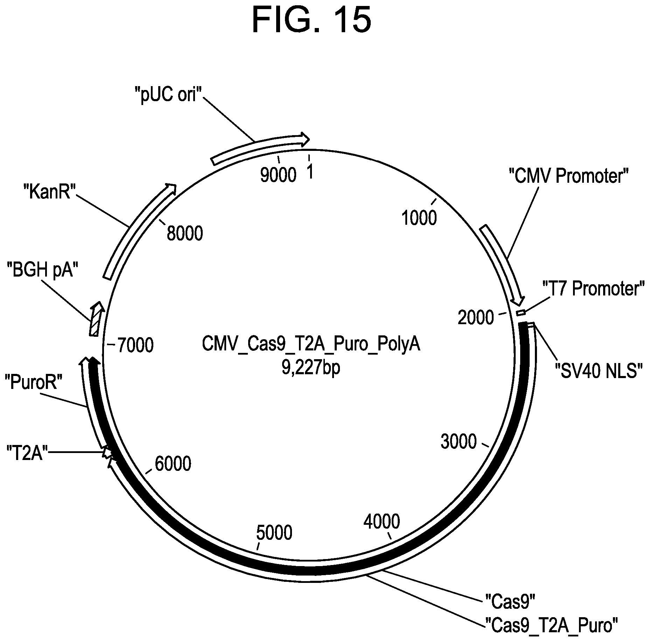

FIG. 15 shows a plasmid expressing Cas9 used in Example 3.

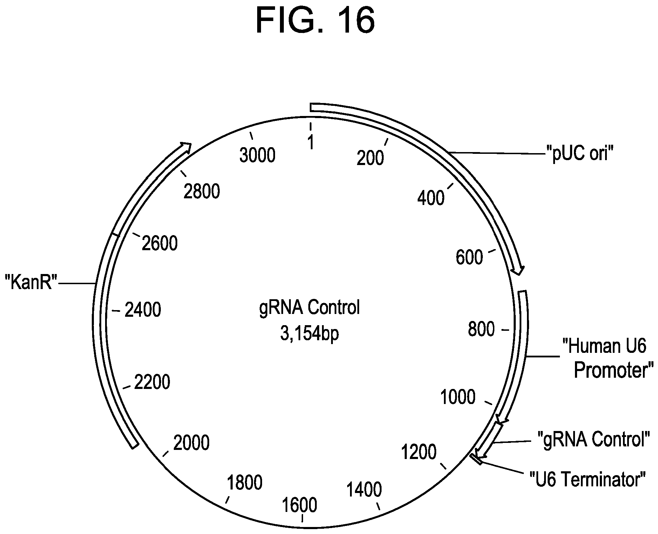

FIG. 16 shows a plasmid expressing a control gRNA used in Example 3.

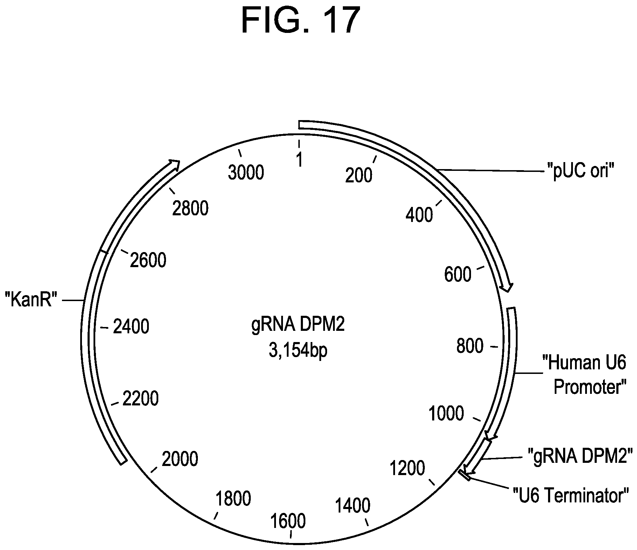

FIG. 17 shows a plasmid expressing a gRNA for DPM2 used in Example 3.

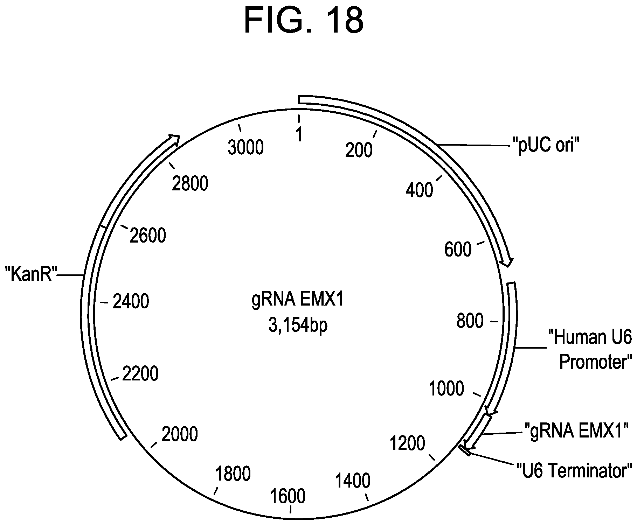

FIG. 18 shows a plasmid expressing a gRNA for EMX1 used in Example 3.

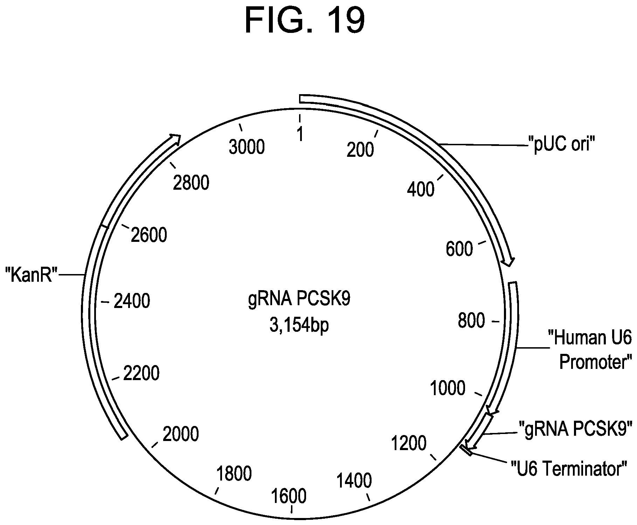

FIG. 19 shows a plasmid expressing a gRNA for PCSK9 used in Example 3.

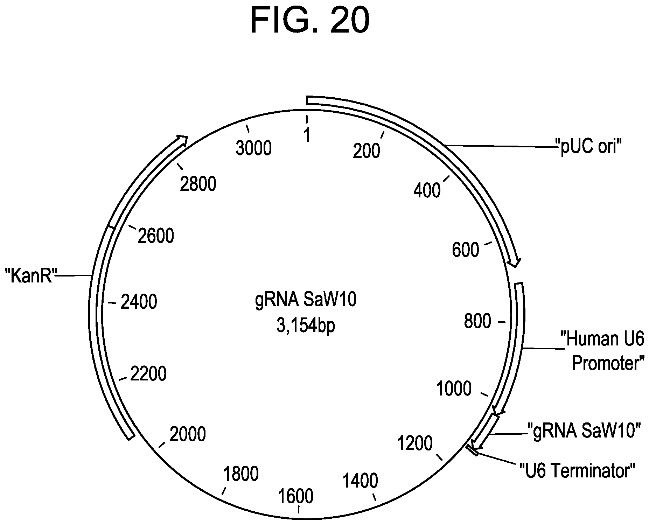

FIG. 20 shows a plasmid expressing a gRNA for SaW10 used in Example 4.

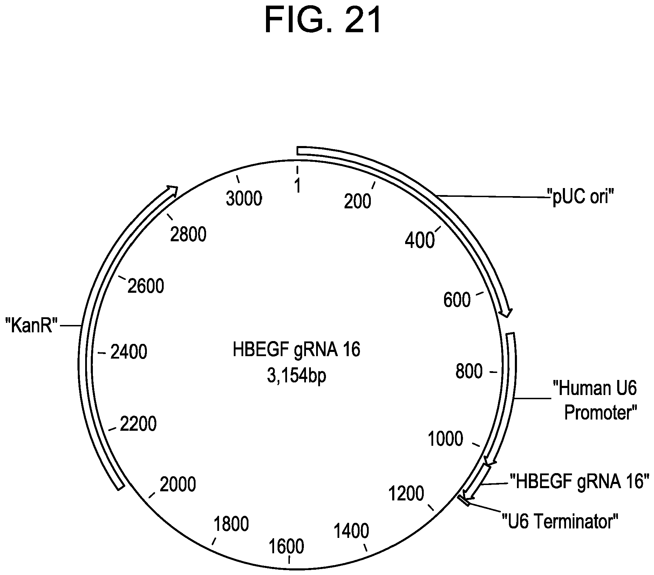

FIG. 21 shows a plasmid expressing a gRNA for HB-EGF gRNA 16 used in Example 3.

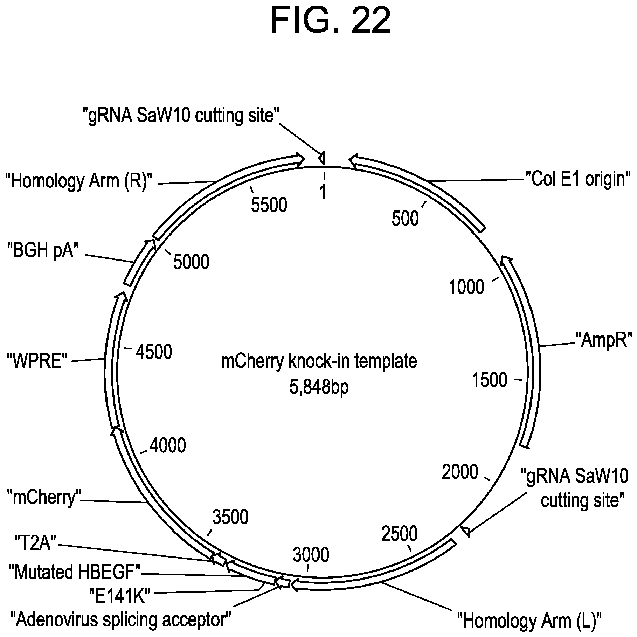

FIG. 22 shows a donor plasmid for inserting mCherry into a site of interest used in Example 4.

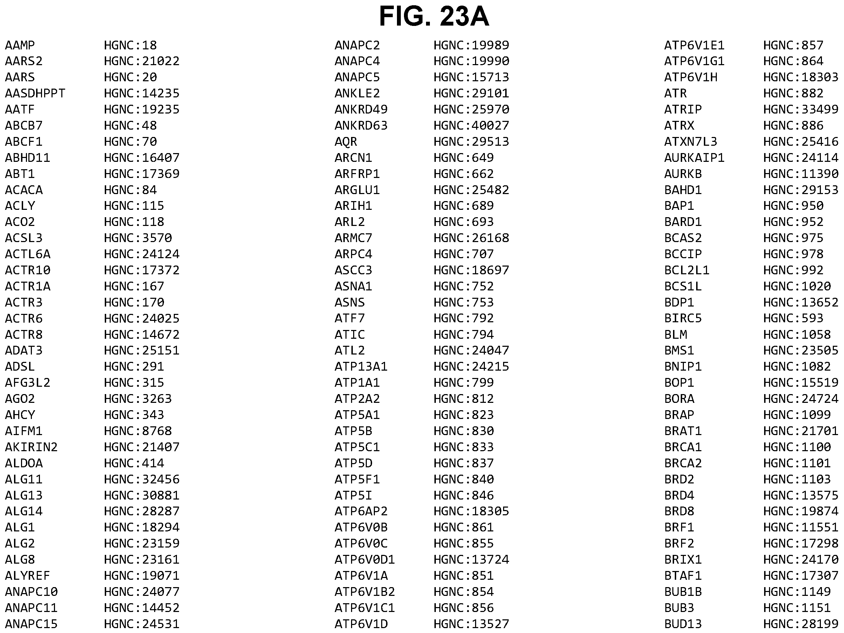

FIGS. 23A-23O shows a list of essential genes as described herein and in Hart et al., Cell 163:1515-1526 (2015), along with each gene's accession number.

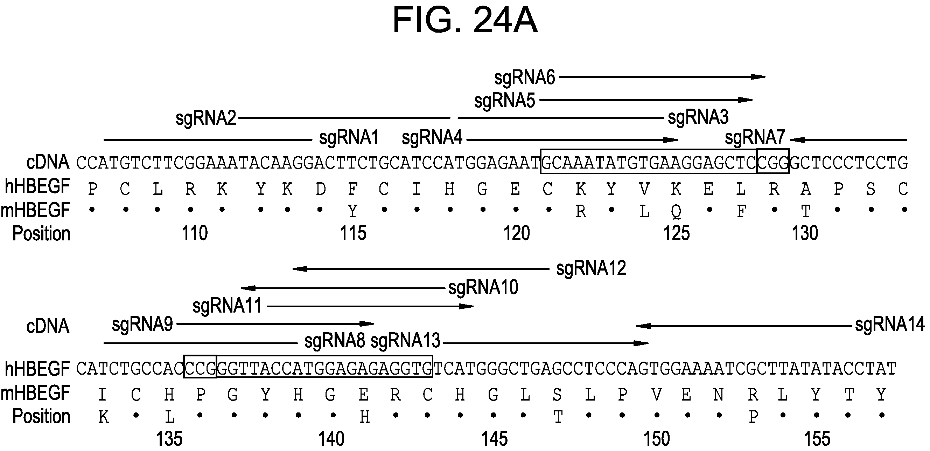

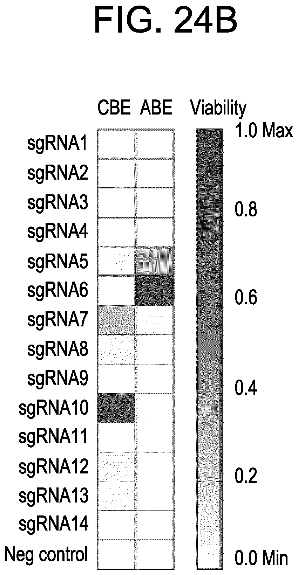

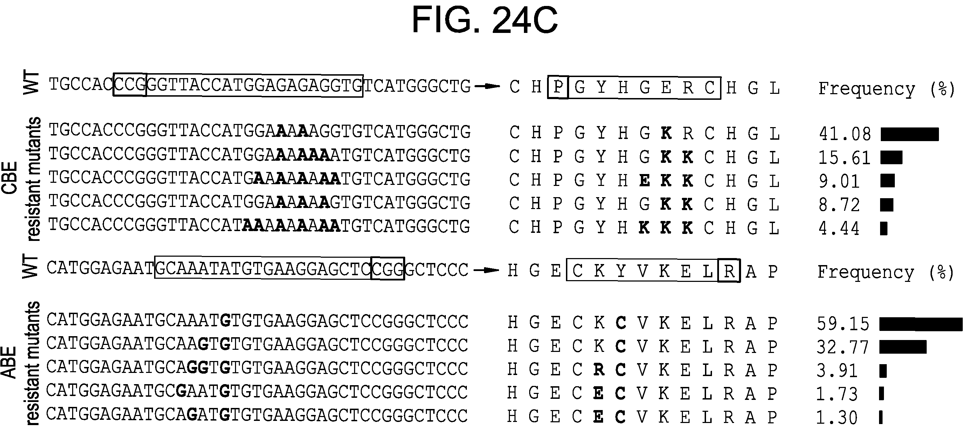

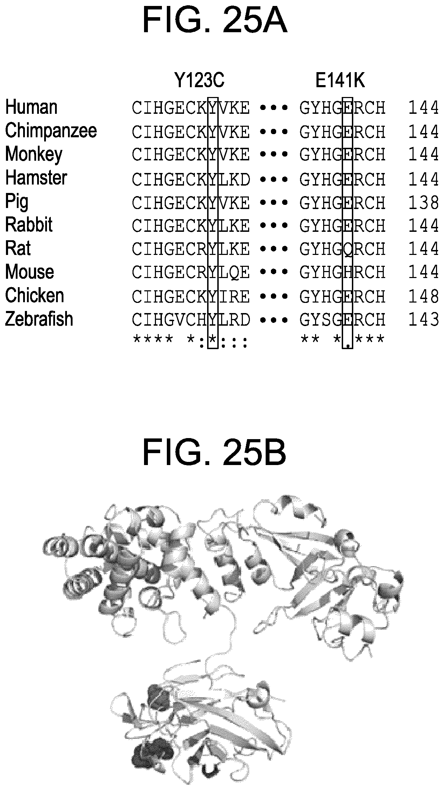

FIGS. 24A-24C and FIGS. 25A-25D relate to Example 6. FIG. 24A shows a schematic representation of sgRNA sites targeted by CBE3 or ABE7.10 to screen for DT-resistant mutations. cDNA and hHBEGF show the DNA sequence encoding the EGF-like domain of human HBEGF protein and its corresponding sequence of amino acids, respectively. mHBEGF shows the aligned amino acids sequence of mouse HBEGF homolog. Matched amino acids in mHBEGF are shown as dot, while the unmatched ones are annotated. The position of amino acids in human HBEGF protein are shown below mHBEGF. Highlighted sgRNAs were chosen to introduce resistant mutations with CBE3 and ABE7.10, respectively. FIG. 24B shows the viability of cells after DT selection for each combination of base editors and sgRNAs. HEK293 cells were transfected with CBE3 or ABE7.10 together with each individual sgRNA followed by DT treatment. The cell viability of re-growing cells were quantified by AlarmarBlue assay. FIG. 24C shows the frequency of resistant alleles in DT resistant cells after CBE or ABE editing. HEK293 cells were first transfected with either plasmids encoding CBE and sgRNA10 or plasmids encoding ABE and sgRNA5, and then selected with DT starting from 72 hours after transfection. Surviving cells were harvested and analyzed by NGS. The frequency of each allele was analyzed following Komor's method. Values represent average (n=3) independent biological replicates.

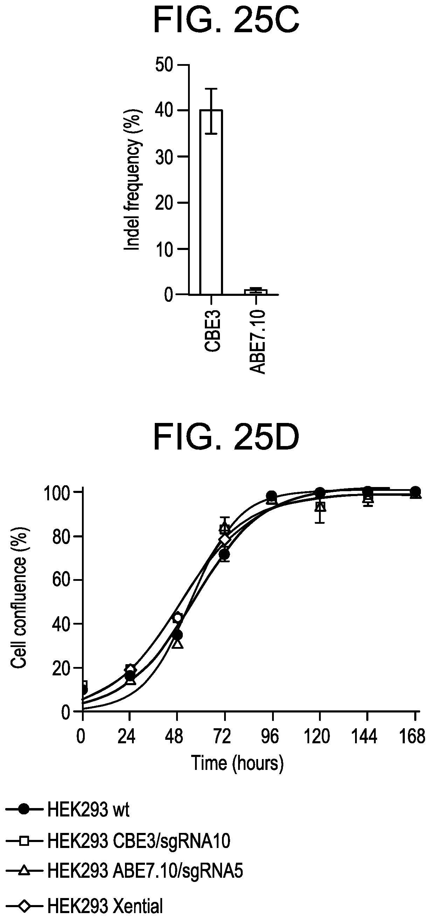

FIG. 25A shows an alignment of HBEGF homologs from different species. FIG. 25B shows an HBEGF protein structure with resistant amino acid substitutions highlighted. The "upper" highlighted amino acid is the resistant substitution introduced by the CBE3/sgRNA10 pair, and the "lower" highlighted amino acid is the resistant substitution introduced by the ABE7.10/sgRNA5 pair. FIG. 25C shows the indel frequencies observed in DT-resistant populations generated with the CBE3/sgRNA10 pair or the ABE7.10/sgRNA5 pair. FIG. 25D shows the cell proliferation curves of HEK293 wildtype cells (HEK293 wt) and DT-resistant cells generated by CBE3/sgRNA10 (HEK293 CBE3/sgRNA10), ABE7.10/sgRNA5 (HEK293 ABE7.10/sgRNA5), and pHMEJ Xential (HEK293 Xential), respectively. Cell proliferation was measured in 96-well plates and quantified by IncuCyte S3 Live Cell Analysis System (Essen BioScience).

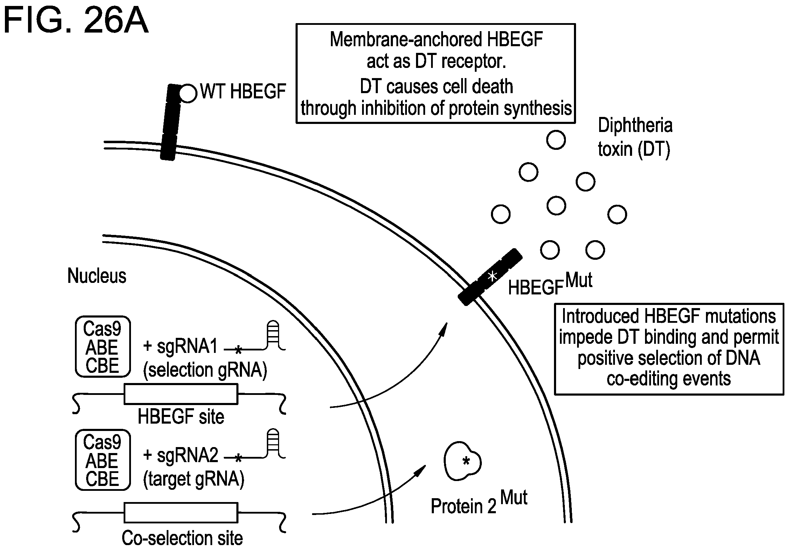

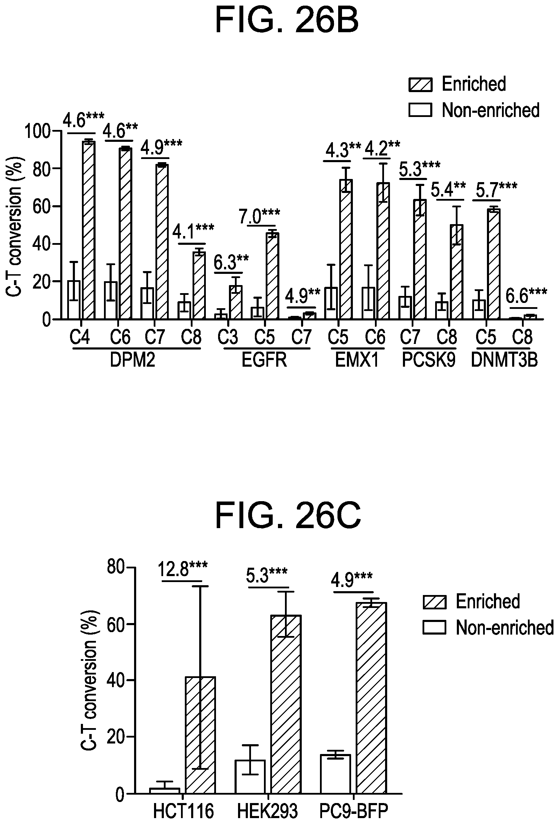

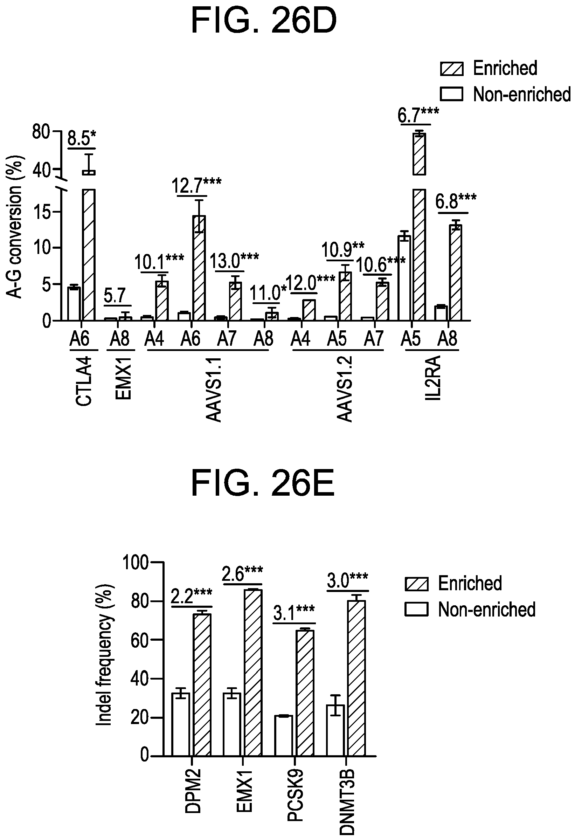

FIGS. 26A-26E relate to Example 7. FIG. 26A shows a schematic representation of the DT-HBEGF co-selection strategy. FIG. 26B shows results of co-selection of cytidine base editing events. HEK293 cells were co-transfected with CBE3, sgRNA10 and a sgRNA targeting the second genomic locus, and were cultivated with (enriched) or without (non-enriched) DT selection starting from 72 hours after transfection. Genomic DNA were harvested when cells became confluent, and the C-T conversion percentage was analyzed by NGS. FIG. 26C shows results of CBE co-selection in different cell lines. CBE3/sgRNA targeting PCSK9, CBE3/sgRNA targeting PCSK9, CBE3/sgRNA targeting BFP were transfected into HCT1 16, HEK293 and PC9-BFP cells, respectively. Genomic DNA was extracted from cells selected or unselected with DT (20ng/mL) and analyzed by Amplicon-Seq. FIG. 26D shows results of co-selection of adenosine base editing events. HEK293 cells were transfected with ABE7.10, sgRNA5 and a sgRNA targeting the second genomic locus, and were cultivated with (enriched) or without (non-enriched) DT selection starting from 72 hours after transfection until confluent. Genomic DNA were harvested from these cells, and the A-G conversion percentage was analyzed by NGS. FIG. 26E shows the results of co-selection with SpCas9 editing events. HEK293 cells were co-transfected with SpCas9, sgRNA10 and a sgRNA targeting the second genomic locus, and were cultivated with (enriched) or without (non-enriched) DT selection starting from 72 hours after transfection until confluent. Genomic DNA were harvested from these cells and the indel frequency was analyzed by NGS. Values and error bars reflect mean ± s.d. of n=3 independent biological replicates. Relative fold-changes are indicated in the graphs. *P < 0.05, **P< 0.01, ***P<0.001, Student's paired t-test.

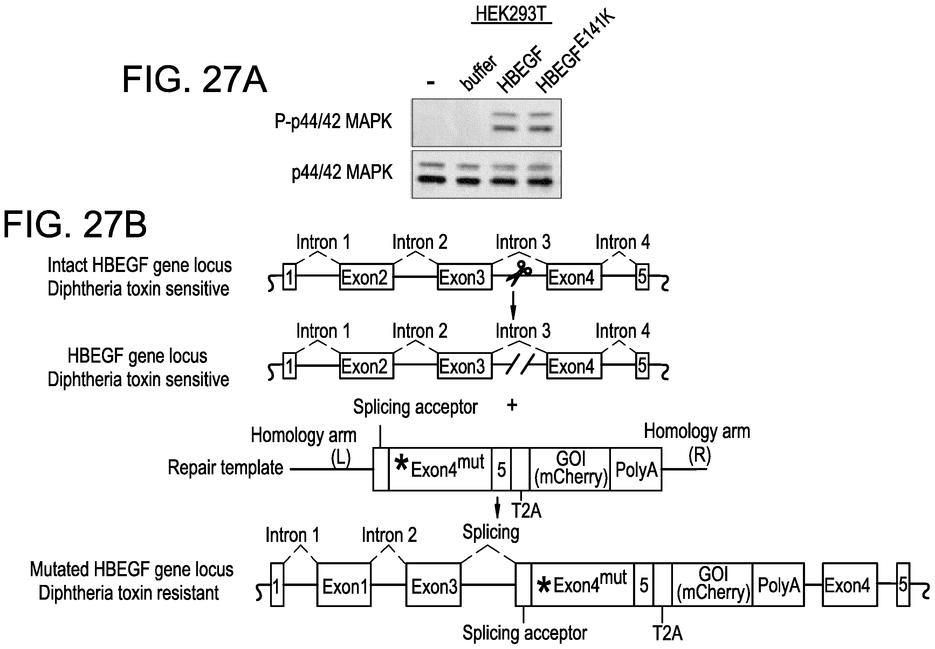

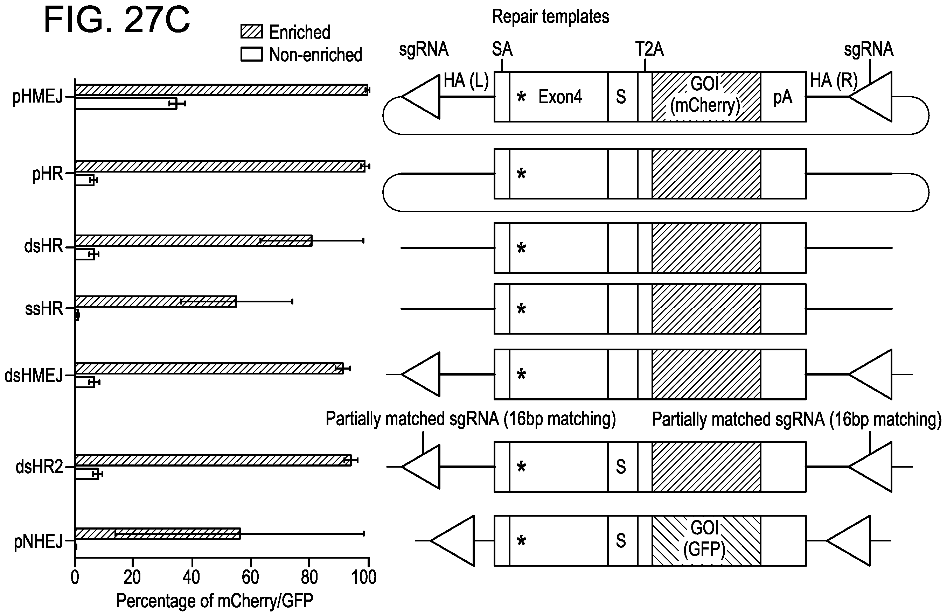

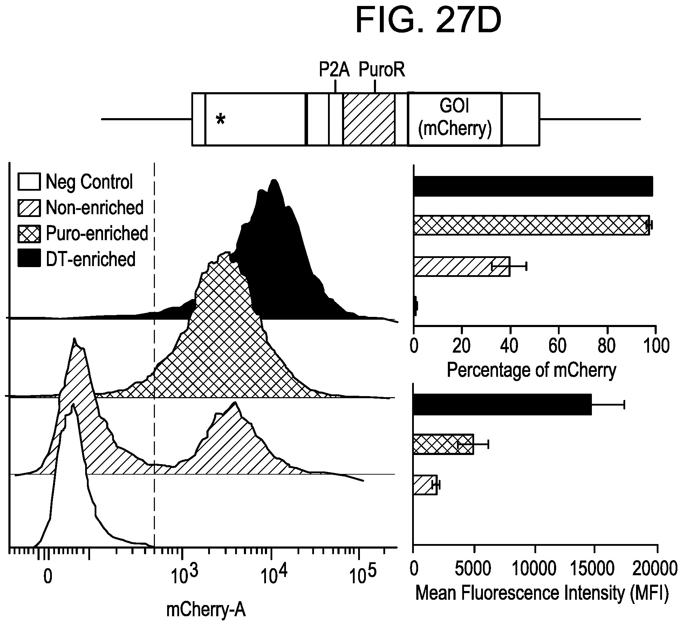

FIGS. 27A-27E relate to Example 8. FIG. 27A shows a Western blot analysis of p44/42 MAPK and Phospho-p44/42 MAPK in cells treated with wild-type HBEGF and HBEGFE141K. Phosphorylation of p44/42 MAPK represents one major downstream signaling of EGFR activation. Values and error bars reflect mean ± s.d. of n=3 independent biological replicates. FIG. 27B shows a schematic description of the knock-in enrichment strategy. FIG. 27C shows results of the knock-in efficiency of various templates and their corresponding designs. HEK293 cells were co-transfected with SpCas9, sgRNAIn3, and each repair template, followed by cultivation with (enriched) or without (non-enriched) DT selection starting from 72h after transfection. The percentage of mCherry/GFP of each sample was analyzed by flow cytometry. Repair templates were provided in forms of plasmid (pHMEJ, pHR or pNHEJ), double-strand DNA (dsHDR, dsHMEJ, dsHR2), or single-strand DNA (ssHR). These templates were designed to be incorporated into the targeted site through either homology-mediated end joining (pHMEJ and dsHMEJ), homology recombination (pHR, dsHR, ssHR, dsHR2), or non-homologous end joining (pNHEJ). FIG. 27D shows a comparison of puromycin and DT enriched knock-in populations. The upper panel shows the design of the repair template used in the experiment. A puromycin resistant gene and a mCherry gene are fused to the mutated *HBEGF* gene in the repair template and are expected to be co-transcribed and co-translated. The lower-left panel shows the mCherry histogram of edited HEK293 cell populations without or with different treatments. HEK239 cells were transfected with SpCas9, sgRNAIn3, and the repair template, followed by cultivation (non-enriched) or the selection with DT (DT-enriched) or puromycin (Puro-enriched) starting from 72 hours after transfection. Neg Control represents cells transfected with control sgRNA without any target loci in human genome instead of sgRNAIn3. Cells were analyzed by flow cytometry. The lower-right panel shows corresponding knock-in efficiencies and mean fluorescence intensities of each population. FIG. 27E shows the results of PCR analyses of each population of cells obtained from the experiments summarized in FIGS. 27C and 27D. The upper panel shows the design of two PCR analyses. PCR1 is designed to confirm the insertion. The forward primer and the reverse primer were designed to binds flanking genomic regions and insertion regions, respectively. A target band will be amplified if cells contain the correct insertion. PCR2 is designed to detect wild-type cells in the population. The forward and reverse primer were designed to bind the left and right flanking genomic regions of the insertion site, respectively. The middle panel shows the PCR analyses of genomic DNA of cells obtained in the experiment summarized in FIG. 27C with the pHMEJ template. The bottom panel shows the PCR analyses of genomic DNA of cells obtained in the experiment summarized in FIG. 27D. In both analyses, Neg Control represent cells transfected with control sgRNA instead of sgRNAIn3. Values and error bars reflect mean ± s.d. of n=3 independent biological replicates.

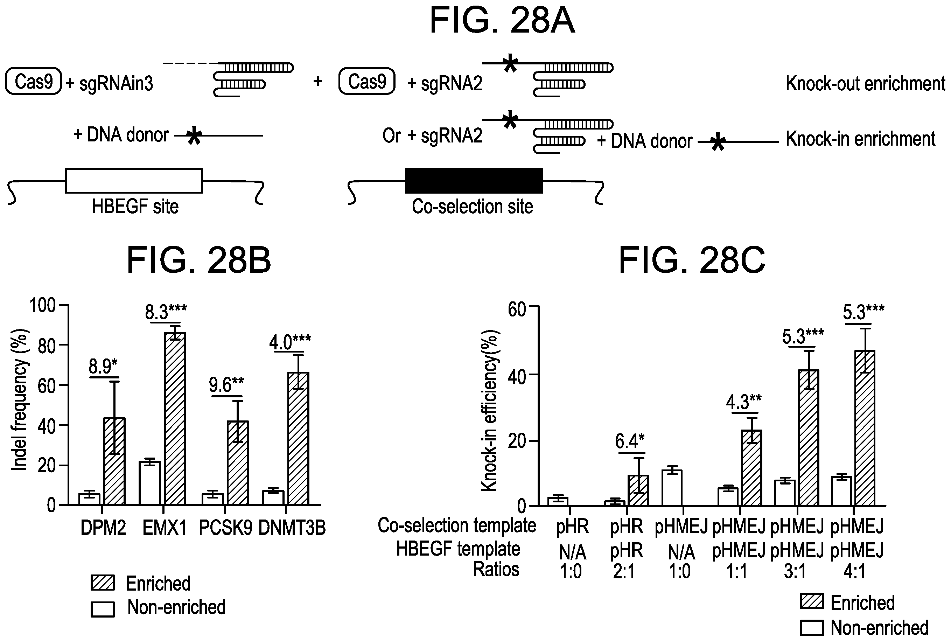

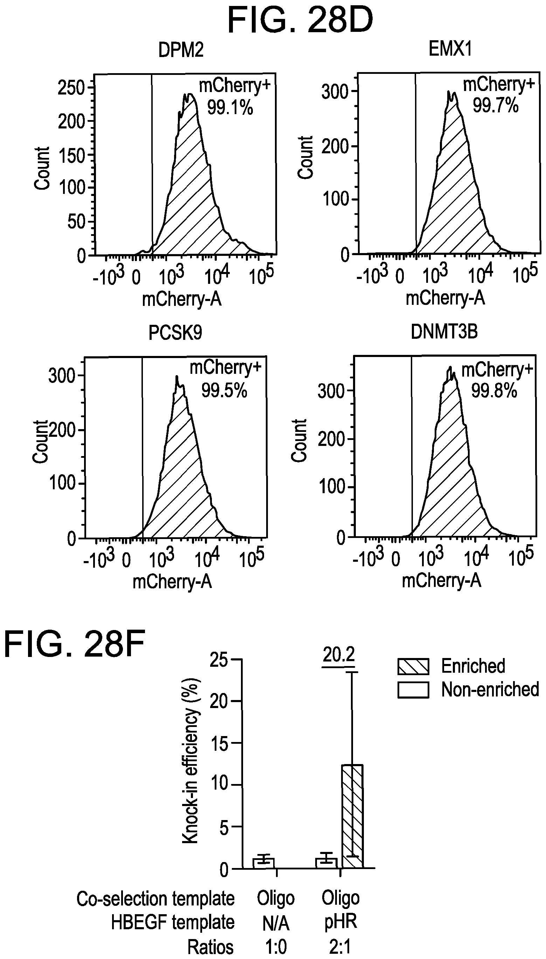

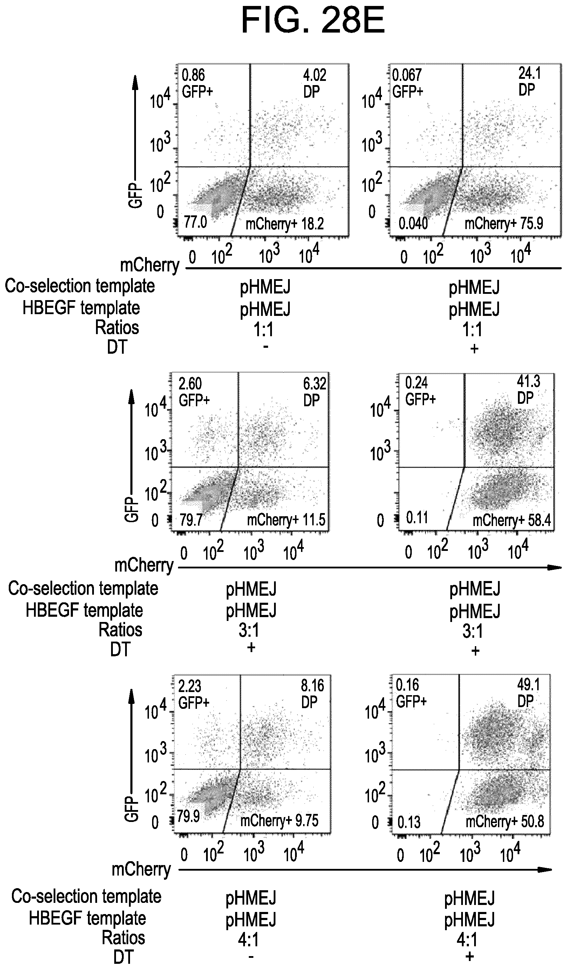

FIGS. 28A-28F relate to Example 9. FIG. 28A shows an experimental strategy of co-selecting knock-out and knock-in events with precise knock-in at HBEGF locus. FIG. 28B shows the results of co-selection of SpCas9 indels in HEK293 cells. Cells were co-transfected with SpCas9, sgRNAIn3, the pHMEJ repair template for HBEGF locus, and a sgRNA targeting a second genomic locus. Cells were then cultivated with (enriched) or without DT (non-enriched) selection starting from 72 hours after transfection until confluent. Genomic DNA were extracted from harvested cells and analyzed by NGS. FIG. 28C shows results of co-selection of knock-in events at a second locus, HIST2BC, in HEK293 cells. Cells were co-transfected with SpCas9, sgRNAs and repair templates for both HBEGF and HIST2BC locus. Both pHR and pHMEJ templates were applied. Different ratios of the amount of sgRNA and template for HBEGF locus to that for HIST2BC locus were applied. N/A indicates no corresponding component was used. Cells were cultivated with (enriched) or without (non-enriched) DT selection starting from 72 hours after transfection and analyzed by flow cytometry. Values and error bars reflect mean ± s.d. of n=3 independent biological replicates. Relative fold-changes are indicated in the graphs. *P < 0.05, **P< 0.01, ***P<0.001, Student's paired t-test. FIG. 28D shows representative histograms indicating that Xential surviving populations co-selected for knock-out events maintained mCherry expression. Each target sgRNA was co-transfected with SpCas9, sgRNAIn3, and pHMEJ targeting HBEGF locus into HEK293 cells. FIG. 28E shows representative scatter plots indicating that of Xential surviving populations co-selected for knock-in events maintained mCherry expression. pHMEJ and sgRNA targeting HIST2BC locus was co-transfected with SpCas9, sgRNAIn3, and pHMEJ targeting HBEGF locus into HEK293 cells at different weight ratiors. DT selected and unselected cells were analyzed by flow cytometry. FIG. 28F shows the results of Xential co-selection of oligo knock-in events. Oligo template and sgRNA targeting CD34 locus was transfected or co-transfected with SpCas9, sgRNAIn3, and pHMEJ targeting HBEGF locus into HEK293 cells, respectively. Genomic DNA was extracted from selected and unselected cells and analyzed by Amplicon-Seq.

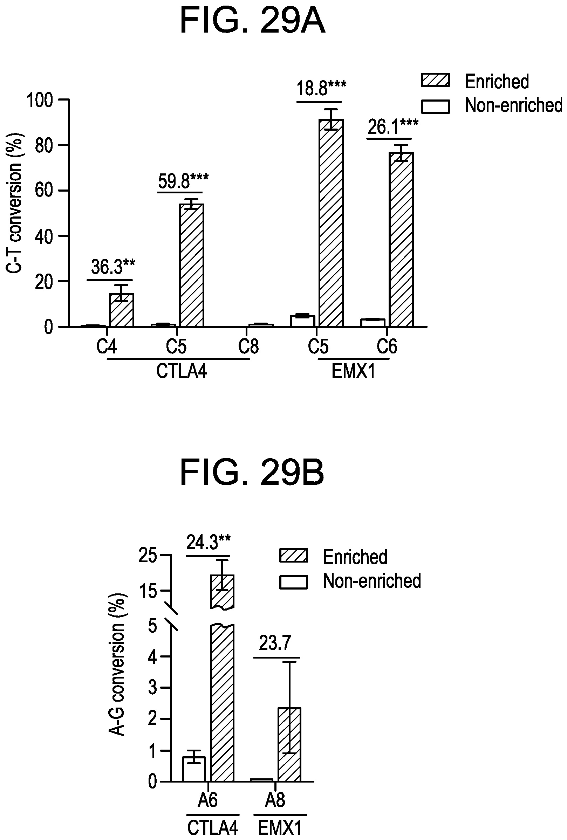

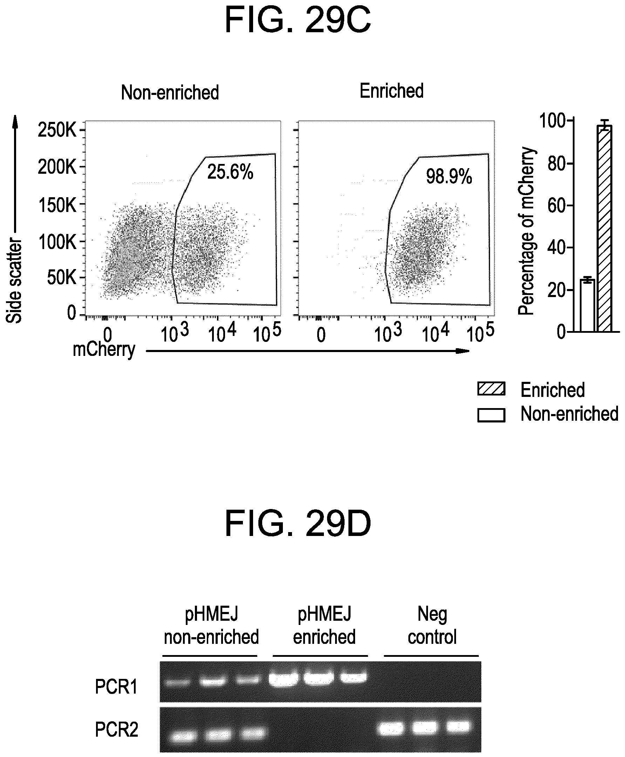

FIGS. 29A-29D relate to Example 10. FIG. 29A shows the results of co-selection of CBE editing events. iPSCs were co-transfected with CBE3, sgRNA10, and a sgRNA targeting a second genomic locus and were cultivated with (Enriched) or without DT selection (Non-enriched) starting from 72 hours after transfection until confluent. Afterwards, genomic DNA were extracted from these cells and analyzed by NGS. FIG. 29B shows the results of co-selection of ABE editing events. iPSCs were co-transfected with ABE7.10, sgRNA5, and a sgRNA targeting a second genomic locus and were cultivated with (Enriched) or without DT selection (Non-enriched) starting from 72 hours after transfection until confluent. Afterwards, genomic DNA were extracted from these cells and analyzed by NGS. FIG. 29C shows the results of enrichment of knock-in events at HBEGF locus. iPSCs were co-transfected with SpCas9, sgRNAIn3, and the pHMEJ template for HBEGF locus and were cultivated with (Enriched) or without DT selection (Non-enriched) starting from 72 hours after transfection. Afterwards, cells were analyzed by flow cytometry. The left panel shows the flow cytometry scatter plots for non-enriched and enriched samples, and the right panel shows the quantitative frequencies of knock-in cells. Values and error bars reflect mean ± s.d. of n=3 independent biological replicates. Relative fold-changes are indicated in the graphs. *P < 0.05, **P< 0.01, ***P<0.001, Student's paired t-test. FIG. 29D shows the results of PCR analyses of iPSCs with Xential knock-in. PCR analyses were performed as described in Example 9 to discriminate between successful knock-in into *HBEGF* intron 3 (PCR1) and wild-type sequence (PCR2). Genomic DNA of cells obtained in experiment FIG. 29C was used as PCR template. Neg Control represent cells transfected with control sgRNA instead of sgRNAIn3.

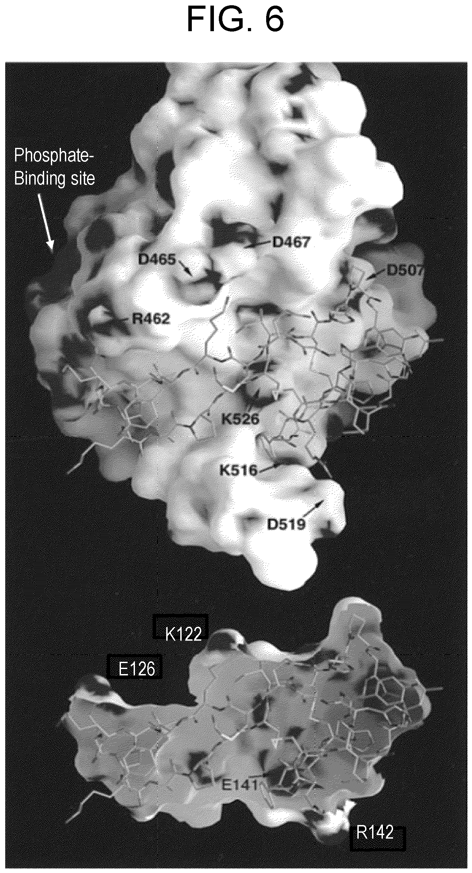

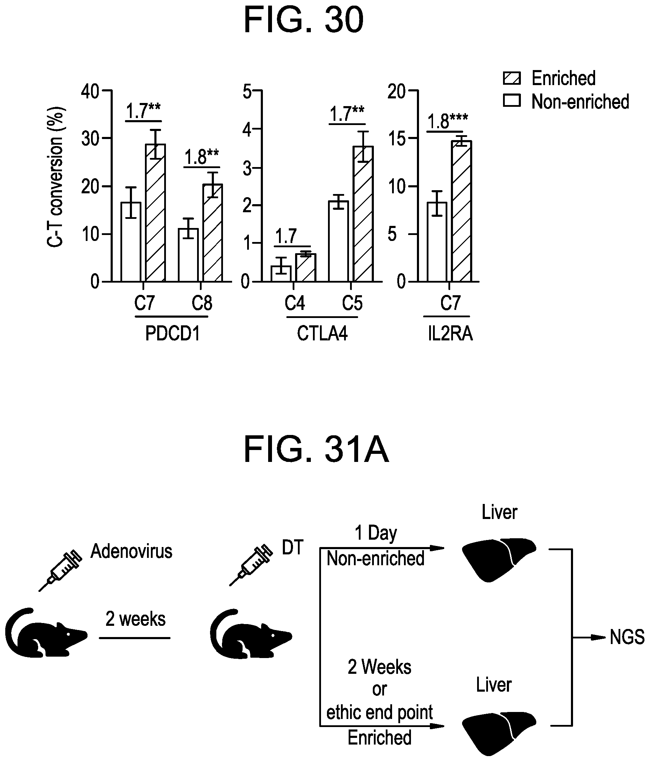

FIG. 30 relates to Example 11. FIG. 6 shows the results of co-selection of CBE editing events in primary T cells. Total CD4+ primary T cells were isolated from human blood and were electroporated with CBE3 proteins, synthetic sgRNA10, and a synthetic sgRNA targeting a second genomic locus. These primary T cells were then cultivated with (Enriched) or without DT selection (Non-enriched) for 9 days starting from 24h after electroporation. Afterwards, genomic DNA was extracted from these cells and analyzed by NGS. Values and error bars reflect mean ± s.d. of n=3 independent biological replicates. Relative fold-changes are indicated in the graphs. *P < 0.05, **P< 0.01, ***P<0.001, Student's paired t-test.

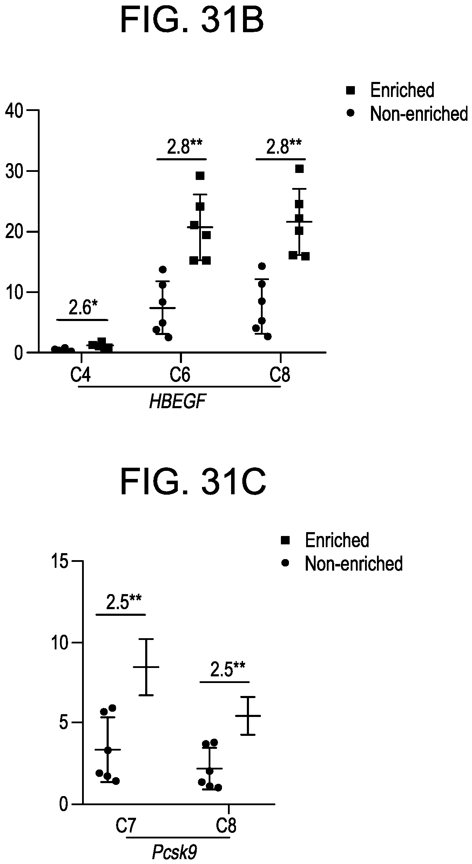

FIGS. 31A-31C relate to Example 12. FIG. 31A shows a schematic representation of the in vivo co-enrichment experiment design. The adenovirus applied was designed to introduce CBE, sgRNA10, and a sgRNA targeting Pcsk9. Upon reaching the end-point of the experiment, mice were terminated and genomic DNA from mice liver were extracted and analyzed by NGS. FIG. 31B shows the results of enrichment of CBE editing at HBEGF locus. FIG. 31C shows the results of co-selection of CBE editing events at Pcsk9 locus. Values and error bars reflect mean ± s.d. of n=3 independent biological replicates. Relative fold-changes are indicated in the graphs. *P < 0.05, **P< 0.01, Student's paired t-test.

### DETAILED DESCRIPTION OF THE INVENTION

The present disclosure provides methods of introducing site-specific mutations in a target cell and methods of determining efficacy of enzymes capable of introducing site-specific mutations. The present disclosure also provides methods of providing a bi-allelic sequence integration, methods of integrating of a sequence of interest into a locus in a genome of a cell, and methods of introducing a stable episomal vector in a cell. The present disclosure further provides methods of generating a human cell that is resistant to diphtheria toxin.

### Definitions

As used herein, "a" or "an" may mean one or more. As used herein in the specification and claims, when used in conjunction with the word "comprising," the words "a" or "an" may mean one or more than one. As used herein, "another" or "a further" may mean at least a second or more.

Throughout this application, the term "about" is used to indicate that a value includes the inherent variation of error for the method/device being employed to determine the value, or the variation that exists among the study subjects. Typically, the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability, depending on the situation.

The use of the term "or" in the claims is used to mean "and/or" unless explicitly indicated to refer only to alternatives or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or openended and do not exclude additional, unrecited, elements or method steps. It is contemplated that any embodiment discussed in this specification can be implemented with respect to any method, system, host cells, expression vectors, and/or composition of the present disclosure. Furthermore, compositions, systems, host cells, and/or vectors of the present disclosure can be used to achieve methods and proteins of the present disclosure.

The use of the term "for example" and its corresponding abbreviation "e.g." (whether italicized or not) means that the specific terms recited are representative examples and embodiments of the disclosure that are not intended to be limited to the specific examples referenced or cited unless explicitly stated otherwise.

A "nucleic acid," "nucleic acid molecule," "nucleotide," "nucleotide sequence," "oligonucleotide," or "polynucleotide" means a polymeric compound including covalently linked nucleotides. The term "nucleic acid" includes ribonucleic acid (RNA) and deoxyribonucleic acid (DNA), both of which may be single- or double-stranded. DNA includes, but is not limited to, complementary DNA (cDNA), genomic DNA, plasmid or vector DNA, and synthetic DNA. In some embodiments, the disclosure provides a polynucleotide encoding any one of the polypeptides disclosed herein, *e.g.,* is directed to a polynucleotide encoding a Cas protein or a variant thereof.

A "gene" refers to an assembly of nucleotides that encode a polypeptide, and includes cDNA and genomic DNA nucleic acid molecules. "Gene" also refers to a nucleic acid fragment that can act as a regulatory sequence preceding (5' non-coding sequences) and following (3' non-coding sequences) the coding sequence.

A nucleic acid molecule is "hybridizable" or "hybridized" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are known and exemplified in Sambrook et al., Molecular Cloning: A Laboratory Manual, Second Edition, Cold Spring Harbor Laboratory Press, Cold Spring Harbor (1989), particularly Chapter 11 and Table 11.1 therein. The conditions of temperature and ionic strength determine the "stringency" of the hybridization. Stringency conditions can be adjusted to screen for moderately similar fragments, such as homologous sequences from distantly related organisms, to highly similar fragments, such as genes that duplicate functional enzymes from closely related organisms. For preliminary screening for homologous nucleic acids, low stringency hybridization conditions, corresponding to a Tₘ of 55°C., can be used, e.g., 5XSSC, 0.1% SDS, 0.25% milk, and no formamide; or 30% formamide, 5XSSC, 0.5% SDS. Moderate stringency hybridization conditions correspond to a higher Tₘ, e.g., 40% formamide, with 5X or 6XSCC. High stringency hybridization conditions correspond to the highest Tₘ, e.g., 50% formamide, 5X or 6XSCC. Hybridization requires that the two nucleic acids contain complementary sequences, although depending on the stringency of the hybridization, mismatches between bases are possible.

The term "complementary" is used to describe the relationship between nucleotide bases that are capable of hybridizing to one another. For example, with respect to DNA, adenosine is complementary to thymine and cytosine is complementary to guanine. Accordingly, the present disclosure also includes isolated nucleic acid fragments that are complementary to the complete sequences as disclosed or used herein as well as those substantially similar nucleic acid sequences.

A DNA "coding sequence" is a double-stranded DNA sequence that is transcribed and translated into a polypeptide in a cell in vitro or in vivo when placed under the control of appropriate regulatory sequences. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, polyadenylation recognition sequences, RNA processing site, effector binding site and stem-loop structure. The boundaries of the coding sequence are determined by a start codon at the 5' (amino) terminus and a translation stop codon at the 3' (carboxyl) terminus. A coding sequence can include, but is not limited to, prokaryotic sequences, cDNA from mRNA, genomic DNA sequences, and even synthetic DNA sequences. If the coding sequence is intended for expression in a eukaryotic cell, a polyadenylation signal and transcription termination sequence will usually be located 3' to the coding sequence.

A "native coding sequence" typically refers to a wild-type sequence in a genome; "native coding sequence" can also refer to a sequence that is substantially similar to the wild-type sequence, e.g., having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence similarity with the wild-type sequence.

"Open reading frame" is abbreviated ORF and means a length of nucleic acid sequence, either DNA, cDNA or RNA, that includes a translation start signal or initiation codon such as an ATG or AUG, and a termination codon and can be potentially translated into a polypeptide sequence.

The term "homologous recombination" refers to the insertion of a foreign DNA sequence into another DNA molecule, e.g., insertion of a vector in a chromosome. In some cases, the vector targets a specific chromosomal site for homologous recombination. For specific homologous recombination, the vector typically contains sufficiently long regions of homology to sequences of the chromosome to allow complementary binding and incorporation of the vector into the chromosome. Longer regions of homology, and greater degrees of sequence similarity, may increase the efficiency of homologous recombination.

Methods known in the art may be used to propagate a polynucleotide according to the disclosure herein. Once a suitable host system and growth conditions are established, recombinant expression vectors can be propagated and prepared in quantity. As described herein, the expression vectors which can be used include, but are not limited to, the following vectors or their derivatives: human or animal viruses such as vaccinia virus or adenovirus; insect viruses such as baculovirus; yeast vectors; bacteriophage vectors (e.g., lambda), and plasmid and cosmid DNA vectors.

As used herein, "operably linked" means that a polynucleotide of interest, e.g., a polynucleotide encoding a Cas9 protein, is linked to the regulatory element in a manner that allows for expression of the polynucleotide sequence. In some embodiments, the regulatory element is a promoter. In some embodiments, polynucleotide of interest is operably linked to a promoter on an expression vector.

As used herein, "promoter," "promoter sequence," or "promoter region" refers to a DNA regulatory region/sequence capable of binding RNA polymerase and involved in initiating transcription of a downstream coding or non-coding sequence. In some examples of the present disclosure, the promoter sequence includes the transcription initiation site and extends upstream to include the minimum number of bases or elements used to initiate transcription at levels detectable above background. In some embodiments, the promoter sequence includes a transcription initiation site, as well as protein binding domains responsible for the binding of RNA polymerase. Eukaryotic promoters will often, but not always, contain "TATA" boxes and "CAT" boxes. Various promoters, including inducible promoters, may be used to drive the various vectors of the present disclosure.

A "vector" is any means for the cloning of and/or transfer of a nucleic acid into a host cell. A vector may be a replicon to which another DNA segment may be attached so as to bring about the replication of the attached segment. A "replicon" is any genetic element (e.g., plasmid, phage, cosmid, chromosome, virus) that functions as an autonomous unit of DNA replication *in vivo,* i.e., capable of replication under its own control. In some embodiments of the present disclosure the vector is an episomal vector, i.e., a non-integrated extrachromosomal plasmid capable of autonomous replication. In some embodiments, the episomal vector includes an autonomous DNA replication sequence, i.e., a sequence that enables the vector to replicate, typically including an origin of replication (OriP). In some embodiments, the autonomous DNA replication sequence is a scaffold/matrix attachment region (S/MAR). In some embodiments, the autonomous DNA replication sequence is a viral OriP. The episomal vector may be removed or lost from a population of cells after a number of cellular generations, e.g., by asymmetric partitioning. In some embodiments, the episomal vector is a stable episomal vector and remains in the cell, i.e., is not lost from the cell. In some embodiments, the episomal vector is an artificial chromosome or a plasmid. In some embodiments, the episomal vector comprises an autonomous DNA replication sequence. Examples of episomal vectors used in genome engineering and gene therapy are derived from the *Papovaviridae* viral family, including simian virus 40 (SV40) and BK virus; the *Herpesviridae* viral family, including bovine papilloma virus 1 (BPV-1), Kaposi's sarcoma-associated herpesvirus (KSHV), and Epstein-Barr virus (EBV); and the S/MAR region of the human interferon β gene. In some embodiments, the episomal vector is an artificial chromosome. In some embodiments, the episomal vector is a mini chromosome. Episomal vectors are further described in, e.g., Van Craenenbroeck et al., Eur J Biochem 267:5665-5678 (2000), and Lufino et al., Mol Ther 16(9): 1525-1538 (2008).

The term "vector" includes both viral and non-viral means for introducing the nucleic acid into a cell *in vitro*, *ex vivo*, or *in vivo.* A large number of vectors known in the art may be used to manipulate nucleic acids, incorporate response elements and promoters into genes, etc. Possible vectors include, for example, plasmids or modified viruses including, for example, bacteriophages such as lambda derivatives, or plasmids such as PBR322 or pUC plasmid derivatives, or the Bluescript vector. For example, the insertion of the DNA fragments corresponding to response elements and promoters into a suitable vector can be accomplished by ligating the appropriate DNA fragments into a chosen vector that has complementary cohesive termini. Alternatively, the ends of the DNA molecules may be enzymatically modified, or any site may be produced by ligating nucleotide sequences (linkers) into the DNA termini. Such vectors may be engineered to contain selectable marker genes that provide for the selection of cells that have incorporated the marker into the cellular genome. Such markers allow identification and/or selection of host cells that incorporate and express the proteins encoded by the marker.

Viral vectors, and particularly retroviral vectors, have been used in a wide variety of gene delivery applications in cells, as well as living animal subjects. Viral vectors that can be used include, but are not limited, to retrovirus, adenovirus adeno-associated virus, pox, baculovirus, vaccinia, herpes simplex, Epstein-Barr, adenovirus, geminivirus, and caulimovirus vectors. Retroviral vectors have emerged as a tool for gene therapy by facilitating genomic insertion of a desired sequence. Retroviral genomes (e.g., murine leukemia virus (MLV), feline leukemia virus (FLV), or any virus belonging to the *Retroviridae* viral family) include long terminal repeat (LTR) sequences flanking viral genes. Upon viral infection of a host, the LTRs are recognized by integrase, which integrates viral genome into the host genome. A retroviral vector for targeted gene insertion does not have any of the viral genes, and instead has the desired sequence to be inserted between the LTRs. The LTRs are recognized by integrase and integrates the desired sequence into the genome of the host cell. Further details on retroviral vectors can be found in, e.g., Kurian et al., Mol Pathol 53(4):173-176; and Vargas et al., J Transl Med 14:288 (2016).

Non-viral vectors include, but are not limited to, plasmids, liposomes, electrically charged lipids (cytofectins), DNA-protein complexes, and biopolymers. In addition to a nucleic acid, a vector may also include one or more regulatory regions, and/or selectable markers useful in selecting, measuring, and monitoring nucleic acid transfer results (transfer to which tissues, duration of expression, etc.).

Transposons and transposable elements may be included on a vector. Transposons are mobile genetic elements that include flanking repeat sequences recognized by a transposase, which then excise the transposon from its locus at the genome and insert it at another genomic locus (commonly referred to as a "cut-and-paste" mechanism). Transposons have been adapted for genome engineering by flanking a desired sequence to be inserted with the repeat sequences recognizable by transposase. The repeat sequences may be collectively referred to as "transposon sequence." In some embodiments, the transposon sequence and a desired sequence to be inserted are included on a vector, the transposon sequence is recognized by transposase, and the desired sequence can then be integrated into the genome by the transposase. Transposons are described in, e.g., Pray, Nature Education 1(1):204, (2008); Vargas et al., J Transl Med 14:288 (2016); and VandenDriessche et al., Blood 114(8):1461-1468 (2009). Non-limiting examples of transposon sequences include the *sleeping beauty* (*SB*)*, piggyBac* (*PB*)*,* and *Tol2* transposons.

Vectors may be introduced into the desired host cells by known methods, including, but not limited to, transfection, transduction, cell fusion, and lipofection. Vectors can include various regulatory elements including promoters. In some embodiments, vector designs can be based on constructs designed by Mali et al., Nature Methods 10:957-63 (2013). In some embodiments, the present disclosure provides an expression vector including any of the polynucleotides described herein, e.g., an expression vector including polynucleotides encoding a Cas protein or variant thereof. In some embodiments, the present disclosure provides an expression vector including polynucleotides encoding a Cas9 protein or variant thereof.

The term "plasmid" refers to an extra chromosomal element often carrying a gene that is not part of the central metabolism of the cell, and usually in the form of circular double-stranded DNA molecules. Such elements may be autonomously replicating sequences, genome integrating sequences, phage or nucleotide sequences, linear, circular, or supercoiled, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

"Transfection" as used herein means the introduction of an exogenous nucleic acid molecule, including a vector, into a cell. A "transfected" cell includes an exogenous nucleic acid molecule inside the cell and a "transformed" cell is one in which the exogenous nucleic acid molecule within the cell induces a phenotypic change in the cell. The transfected nucleic acid molecule can be integrated into the host cell's genomic DNA and/or can be maintained by the cell, temporarily or for a prolonged period of time, extra-chromosomally. Host cells or organisms that express exogenous nucleic acid molecules or fragments are referred to as "recombinant," "transformed," or "transgenic" organisms. In some embodiments, the present disclosure provides a host cell including any of the expression vectors described herein, *e.g.,* an expression vector including a polynucleotide encoding a Cas protein or variant thereof. In some embodiments, the present disclosure provides a host cell including an expression vector including a polynucleotide encoding a Cas9 protein or variant thereof.

The term "host cell" refers to a cell into which a recombinant expression vector has been introduced. The term "host cell" refers not only to the cell in which the expression vector is introduced (the "parent" cell), but also to the progeny of such a cell. Because modifications may occur in succeeding generations, for example, due to mutation or environmental influences, the progeny may not be identical to the parent cell, but are still included within the scope of the term "host cell."

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

The start of the protein or polypeptide is known as the "N-terminus" (or amino-terminus, NH₂-terminus, N-terminal end or amine-terminus), referring to the free amine (-NH₂) group of the first amino acid residue of the protein or polypeptide. The end of the protein or polypeptide is known as the "C-terminus" (or carboxy-terminus, carboxyl-terminus, C-terminal end, or COOH-terminus), referring to the free carboxyl group (-COOH) of the last amino acid residue of the protein or peptide.

An "amino acid" as used herein refers to a compound including both a carboxyl (-COOH) and amino (-NH₂) group. "Amino acid" refers to both natural and unnatural, i.e., synthetic, amino acids. Natural amino acids, with their three-letter and single-letter abbreviations, include: Alanine (Ala; A); Arginine (Arg, R); Asparagine (Asn; N); Aspartic acid (Asp; D); Cysteine (Cys; C); Glutamine (Gln; Q); Glutamic acid (Glu; E); Glycine (Gly; G); Histidine (His; H); Isoleucine (Ile; I); Leucine (Leu; L); Lysine (Lys; K); Methionine (Met; M); Phenylalanine (Phe; F); Proline (Pro; P); Serine (Ser; S); Threonine (Thr; T); Tryptophan (Trp; W); Tyrosine (Tyr; Y); and Valine (Val; V).

An "amino acid substitution" refers to a polypeptide or protein including one or more substitutions of wild-type or naturally occurring amino acid with a different amino acid relative to the wild-type or naturally occurring amino acid at that amino acid residue. The substituted amino acid may be a synthetic or naturally occurring amino acid. In some embodiments, the substituted amino acid is a naturally occurring amino acid selected from the group consisting of: A, R, N, D, C, Q, E, G, H, I, L, K, M, F, P, S, T, W, Y, and V. Substitution mutants may be described using an abbreviated system. For example, a substitution mutation in which the fifth (5^{th}) amino acid residue is substituted may be abbreviated as "X5Y" wherein "X" is the wild-type or naturally occurring amino acid to be replaced, "5" is the amino acid residue position within the amino acid sequence of the protein or polypeptide, and "Y" is the substituted, or non-wild-type or non-naturally occurring, amino acid.

An "isolated" polypeptide, protein, peptide, or nucleic acid is a molecule that has been removed from its natural environment. It is also to be understood that "isolated" polypeptides, proteins, peptides, or nucleic acids may be formulated with excipients such as diluents or adjuvants and still be considered isolated.

The term "recombinant" when used in reference to a nucleic acid molecule, peptide, polypeptide, or protein means of, or resulting from, a new combination of genetic material that is not known to exist in nature. A recombinant molecule can be produced by any of the well-known techniques available in the field of recombinant technology, including, but not limited to, polymerase chain reaction (PCR), gene splicing (e.g., using restriction endonucleases), and solid-phase synthesis of nucleic acid molecules, peptides, or proteins.

The term "domain" when used in reference to a polypeptide or protein means a distinct functional and/or structural unit in a protein. Domains are sometimes responsible for a particular function or interaction, contributing to the overall role of a protein. Domains may exist in a variety of biological contexts. Similar domains may be found in proteins with different functions. Alternatively, domains with low sequence identity (i.e., less than about 50%, less than about 40%, less than about 30%, less than about 20%, less than about 10%, less than about 5%, or less than about 1% sequence identity) may have the same function. In some embodiments, a DNA-targeting domain is Cas9, or a Cas9 domain. In some embodiments, a Cas9 domain is a RuvC domain. In some embodiments, a Cas9 domain is an HNH domain. In some embodiments, a Cas9 domain is a Rec domain. In some embodiments, a DNA-editing domain is a deaminase, or a deaminase domain.

The term "motif," when used in reference to a polypeptide or protein, generally refers to a set of conserved amino acid residues, typically shorter than 20 amino acids in length, that may be important for protein function. Specific sequence motifs may mediate a common function, such as protein-binding or targeting to a particular subcellular location, in a variety of proteins. Examples of motifs include, but are not limited to, nuclear localization signals, microbody targeting motifs, motifs that prevent or facilitate secretion, and motifs that facilitate protein recognition and binding. Motif databases and/or motif searching tools are known to the skilled artisan and include, for example, PROSITE (expasy.ch/sprot/prosite.html), Pfam (pfam.wustl.edu), PRINTS (biochem.ucl.ac.uk/bsm/dbbrowser/PRINTS/PRINTS.html), and Minimotif Miner (cse-mnm.engr.uconn.edu:8080/MNM/SMSSearchServlet).

An "engineered" protein, as used herein, means a protein that includes one or more modifications in a protein to achieve a desired property. Exemplary modifications include, but are not limited to, insertion, deletion, substitution, or fusion with another domain or protein. Engineered proteins of the present disclosure include engineered Cas9 proteins.

In some embodiments, engineered protein is generated from a wild-type protein. As used herein, a "wild-type" protein or nucleic acid is a naturally-occurring, unmodified protein or nucleic acid. For example, a wild-type Cas9 protein can be isolated from the organism *Streptococcus pyogenes.* Wild-type is contrasted with "mutant," which includes one or more modifications in the amino acid and/or nucleotide sequence of the protein or nucleic acid.

As used herein, the terms "sequence similarity" or "% similarity" refers to the degree of identity or correspondence between nucleic acid sequences or amino acid sequences. As used herein, "sequence similarity" refers to nucleic acid sequences wherein changes in one or more nucleotide bases results in substitution of one or more amino acids, but do not affect the functional properties of the protein encoded by the DNA sequence. "Sequence similarity" also refers to modifications of the nucleic acid, such as deletion or insertion of one or more nucleotide bases that do not substantially affect the functional properties of the resulting transcript. It is therefore understood that the present disclosure encompasses more than the specific exemplary sequences. Methods of making nucleotide base substitutions are known, as are methods of determining the retention of biological activity of the encoded products.

Moreover, the skilled artisan recognizes that similar sequences encompassed by this disclosure are also defined by their ability to hybridize, under stringent conditions, with the sequences exemplified herein. Similar nucleic acid sequences of the present disclosure are those nucleic acids whose DNA sequences are at least 70%, at least 80%, at least 90%, at least 95%, or at least 99% identical to the DNA sequence of the nucleic acids disclosed herein. Similar nucleic acid sequences of the present disclosure are those nucleic acids whose DNA sequences are about 70%, at least about 70%, about 75%, at least about 75%, about 80%, at least about 80%, about 85%, at least about 85%, about 90%, at least about 90%, about 95%, at least about 95%, about 99%, at least about 99%, or about 100% identical to the DNA sequence of the nucleic acids disclosed herein.

As used herein, "sequence similarity" refers to two or more amino acid sequences wherein greater than about 40% of the amino acids are identical, or greater than about 60% of the amino acids are functionally identical. Functionally identical or functionally similar amino acids have chemically similar side chains. For example, amino acids can be grouped in the following manner according to functional similarity:
Positively-charged side chains: Arg, His, Lys;
Negatively-charged side chains: Asp, Glu;
Polar, uncharged side chains: Ser, Thr, Asn, Gln;
Hydrophobic side chains: Ala, Val, Ile, Leu, Met, Phe, Tyr, Trp;
Other: Cys, Gly, Pro.

In some embodiments, similar amino acid sequences of the present disclosure have at least 40%, at least 50%, at least 60%, at least 70%, at least 80%, at least 90%, or at least 99% identical amino acids.

In some embodiments, similar amino acid sequences of the present disclosure have at least 60%, at least 70%, at least 80%, at least 90%, or at least 95% functionally identical amino acids. In some embodiments, similar amino acid sequences of the present disclosure have about 40%, at least about 40%, about 45%, at least about 45%, about 50%, at least about 50%, about 55%, at least about 55%, about 60%, at least about 60%, about 65%, at least about 65%, about 70%, at least about 70%, about 75%, at least about 75%, about 80%, at least about 80%, about 85%, at least about 85%, about 90%, at least about 90%, about 95%, at least about 95%, about 97%, at least about 97%, about 98%, at least about 98%, about 99%, at least about 99%, or about 100% identical amino acids.

In some embodiments, similar amino acid sequences of the present disclosure have about 60%, at least about 60%, about 65%, at least about 65%, about 70%, at least about 70%, about 75%, at least about 75%, about 80%, at least about 80%, about 85%, at least about 85%, about 90%, at least about 90%, about 95%, at least about 95%, about 97%, at least about 97%, about 98%, at least about 98%, about 99%, at least about 99%, or about 100% functionally identical amino acids.

As used herein, the term "the same protein" refers to a protein having a substantially similar structure or amino acid sequence as a reference protein that performs the same biochemical function as the reference protein and can include proteins that differ from a reference protein by the substitution or deletion of one or more amino acids at one or more sites in the amino acid sequence, deletion of i.e., at least about 60%, at least about 60%, about 65%, at least about 65%, about 70%, at least about 70%, about 75%, at least about 75%, about 80%, at least about 80%, about 85%, at least about 85%, about 90%, at least about 90%, about 95%, at least about 95%, about 97%, at least about 97%, about 98%, at least about 98%, about 99%, at least about 99%, or about 100% identical amino acids. In one aspect, "the same protein" refers to a protein with an identical amino acid sequence as a reference protein.

Sequence similarity can be determined by sequence alignment using routine methods in the art, such as, for example, BLAST, MUSCLE, Clustal (including ClustalW and ClustalX), and T-Coffee (including variants such as, for example, M-Coffee, R-Coffee, and Expresso).

The terms "sequence identity" or "% identity" in the context of nucleic acid sequences or amino acid sequences refers to the percentage of residues in the compared sequences that are the same when the sequences are aligned over a specified comparison window. In some embodiments, only specific portions of two or more sequences are aligned to determine sequence identity. In some embodiments, only specific domains of two or more sequences are aligned to determine sequence similarity. A comparison window can be a segment of at least 10 to over 1000 residues, at least 20 to about 1000 residues, or at least 50 to 500 residues in which the sequences can be aligned and compared. Methods of alignment for determination of sequence identity are well-known and can be performed using publicly available databases such as BLAST. "Percent identity" or "% identity" when referring to amino acid sequences can be determined by methods known in the art. For example, in some embodiments, "percent identity" of two amino acid sequences is determined using the algorithm of Karlin and Altschul, Proc Nat Acad Sci USA 87:2264-2268 (1990), modified as in Karlin and Altschul, Proc Nat Acad Sci USA 90:5873-5877 (1993). Such an algorithm is incorporated into the BLAST programs, e.g., BLAST+ or the NBLAST and XBLAST programs described in Altschul et al., Journal of Molecular Biology, 215: 403-410 (1990). BLAST protein searches can be performed with programs such as, e.g., the XBLAST program, score=50, wordlength=3 to obtain amino acid sequences homologous to the protein molecules of the disclosure. Where gaps exist between two sequences, Gapped BLAST can be utilized as described in Altschul et al., Nucleic Acids Research 25(17): 3389-3402 (1997). When utilizing BLAST and Gapped BLAST programs, the default parameters of the respective programs (e.g., XBLAST and NBLAST) can be used.

In some embodiments, polypeptides or nucleic acid molecules have 70%, at least 70%, 75%, at least 75%, 80%, at least 80%, 85%, at least 85%, 90%, at least 90%, 95%, at least 95%, 97%, at least 97%, 98%, at least 98%, 99%, or at least 99% or 100% sequence identity with a reference polypeptide or nucleic acid molecule, respectively (or a fragment of the reference polypeptide or nucleic acid molecule). In some embodiments, polypeptides or nucleic acid molecules have about 70%, at least about 70%, about 75%, at least about 75%, about 80%, at least about 80%, about 85%, at least about 85%, about 90%, at least about 90%, about 95%, at least about 95%, about 97%, at least about 97%, about 98%, at least about 98%, about 99%, at least about 99% or about 100% sequence identity with a reference polypeptide or nucleic acid molecule, respectively (or a fragment of the reference polypeptide or nucleic acid molecule).

"Base edit" or "base editing", as used herein, refers to the conversion of one nucleotide base pair to another base pair. For example, base editing can convert a cytosine (C) to a thymine (T), or an adenine (A) to a guanine (G). Accordingly, base editing can swap a C-G base pair to an A-T base pair in a double-stranded polynucleotide, i.e., base editing generates a point mutation in the polynucleotide. Base editing is typically performed by a base-editing enzyme, which includes, in some embodiments, a DNA-targeting domain and a catalytic domain capable of base editing, i.e., a DNA-editing domain. In some embodiments, the DNA-targeting domain is Cas9, e.g., a catalytically inactive Cas9 (dCas9) or a Cas9 capable of generating single-stranded breaks (nCas9). In some embodiments, the DNA-editing domain is a deaminase domain. The term "deaminase" refers to an enzyme that catalyzes a deamination reaction.

Base-editing typically occurs via deamination, which refers to the removal of an amine group from a molecule, e.g., cytosine or adenosine. Deamination converts cytosine into uracil and adenosine into inosine. Exemplary cytidine deaminases include, e.g., apolipoprotein B mRNA-editing complex (APOBEC) deaminase, activation-induced cytidine deaminase (AID), and ACF1/ASE deaminase. Exemplary adenosine deaminases include, e.g., ADAR deaminase and ADAT deaminase (e.g., TadA).

In an exemplary base-editing process, the base-editing enzyme includes a modified Cas9 domain capable of generating a single-stranded DNA break (i.e., a "nick") (nCas9), a cytidine deaminase domain, and an uracil DNA-glycosylase inhibitor domain (UGI). The nCas9 is directed to the target polynucleotide, which includes a "C-G" base pair, by the guide RNA, where the cytidine deaminase converts the cytosine in "C-G" to uracil, generating a "U-G" mismatch. The nCas9 also generates a nick in the non-edited strand of the target polynucleotide. The UGI inhibits native cellular repair of the newly-converted uracil back to cytosine, and native cellular mismatch repair mechanisms, activated by the nicked DNA strand, convert the "U-G" mismatch to an "U-A" match. Further DNA replication and repair convert the uracil to thymine, and the base editing of the target polynucleotide is complete. An example of a base-editing enzyme is BE3, described in Komor et al., Nature 533(7603):420-424 (2016). Further exemplary base-editing processes are described in, e.g., Eid et al., Biochem J475:1955-1964 (2018).

Methods for generating a catalytically dead Cas9 domain (dCas9) are known *(see,* e.g., Jinek et al., Science 337:816-821 (2012); Qi et al., Cell 152(5):1173-1183 (2013)). For example, the DNA cleavage domain of Cas9 is known to include two subdomains, the HNH nuclease subdomain and the RuvC1 subdomain. The HNH subdomain cleaves the strand complementary to the gRNA, whereas the RuvC1 subdomain cleaves the non-complementary strand. Mutations within these subdomains can silence the nuclease activity of Cas9. For example, the mutations D10A and H840A completely inactivate the nuclease activity of *S. pyogenes* Cas9.

Non-limiting examples of base-editing enzymes are described in, e.g., U.S. Patent Nos. 9,068,179; 9,840,699; 10,167,457; and Eid et al., Biochem J 475(11):1955-1964 (2018); Gehrke et al., Nat Biotechnol 36:977-982 (2018); Hess et al., Mol Cell 68:26-43 (2017); Kim et al., Nat Biotechnol 35:435-437 (2017); Komor et al., Nature 533:420-424 (2016); Komor et al., Science Adv 3(8):eaao4774 (2017); Nishida et al., Science 353:aaf8729 (2016); Rees et al., Nat Commun 8:15790 (2017); Shimatani et al., Nat Biotechnol 35:441-443 (2017).

"Cytotoxic agent" or "cytotoxin" as used herein refers to any agent that results in cell death, typically by impairing or inhibiting one or more essential cellular processes. For example, cytotoxins such as, e.g., diphtheria toxin, Shiga toxin, Pseudomonas exotoxin function by impairing or inhibiting ribosome function, which halts protein synthesis and leads to cell death. Cytotoxins such as, e.g., dolastatin, auristatin, and maytansine target microtubules function, which disrupts cell division and leads to cell death. Cytotoxins such as, e.g., duocarmycin or calicheamicin directly target DNA and will kill cells at any point in the cell cycle. In many cases, the cytotoxic agent is introduced into the cell by binding to a receptor on the surface of the cell. The cytotoxic agent may be a naturally-occurring compound or derivative thereof, or the cytotoxic agent may be a synthetic molecule or peptide. In one example, a cytotoxic agent may be an antibody-drug conjugate (ADC), which includes a monoclonal antibody (mAb) attached to biologically active drug using chemical linkers with labile bonds. ADCs combine the specificity of the mAb with the potency of the drug for targeted killing of specific cells, e.g., cancer cells. ADCs (also referred to as "immune-toxins") are further described in, e.g., Srivastava et al., Biomed Res Ther 2(1):169-183 (2015), and Grawunder and Barth (Eds.), Next Generation Antibody Drug Conjugates (ADCs) and Immunotoxins, Springer, 2017; doi:10.1007/978-3-319-46877-8.

A "bi-allelic" site, as used herein, is a locus in a genome that contains two observed alleles. Accordingly, "bi-allelic" modification refers to modification of both alleles in a genome of a mammalian cell. For example, a bi-allelic mutation means that there is a mutation in both copies (i.e., the maternal copy and the paternal copy) of a particular gene.

### Methods of Introducing Site-Specific Mutations and Determining the Efficacy Thereof

In some embodiments, the present disclosure provides a method of introducing a site-specific mutation in a target polynucleotide in a target cell in a population of cells, the method comprising (a) introducing into the population of cells: (i) a base-editing enzyme; (ii) a first guide polynucleotide that (1) hybridizes to a gene encoding a cytotoxic agent (CA) receptor, and (2) forms a first complex with the base-editing enzyme, wherein the base-editing enzyme of the first complex provides a mutation in the gene encoding the CA receptor, and wherein the mutation in the gene encoding the CA receptor forms a CA-resistant cell in the population of cells; and (iii) a second guide polynucleotide that (1) hybridizes with the target polynucleotide, and (2) forms a second complex with the base-editing enzyme, wherein the base-editing enzyme of the second complex provides a mutation in the target polynucleotide; (b) contacting the population of cells with the CA; and (c) selecting the CA-resistant cell from the population of cells, thereby enriching for the target cell comprising the mutation in the target polynucleotide.

In some embodiments, the present disclosure provides a method of determining efficacy of a base-editing enzyme in a population of cells, the method comprising (a) introducing into the population of cells: (i) a base-editing enzyme; (ii) a first guide polynucleotide that (1) hybridizes to a gene encoding a cytotoxic agent (CA) receptor, and (2) forms a first complex with the base-editing enzyme, wherein the base-editing enzyme of the first complex introduces a mutation in the gene encoding the CA receptor, and wherein the mutation in the gene encoding the CA receptor forms a CA-resistant cell in the population of cells; and (iii) a second guide polynucleotide that (1) hybridizes with the target polynucleotide, and (2) forms a second complex with the base-editing enzyme, wherein the base-editing enzyme of the second complex introduces a mutation in the target polynucleotide; (b) contacting the population of cells with the CA to isolate CA-resistant cells; and (c) determining the efficacy of the base-editing enzyme by determining the ratio of the CA-resistant cells to the total population of cells.

The method of the present disclosure provides an efficient method to introduce single nucleotide mutations (e.g., C:G to T:A mutations) in various cell lines. Previous limitations of genome engineering and gene editing strategies suffered from the inability to distinguish between cells that have successfully been edited from cells that did not undergo editing, for example, because one or more of the editing components may not have been properly introduced or expressed in the cell. Therefore, a need exists in the field for increasing editing efficiency by selection and enrichment of edited cells.

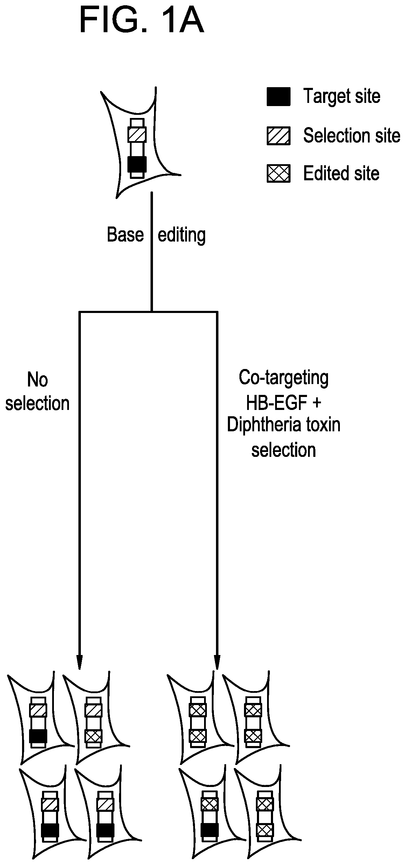

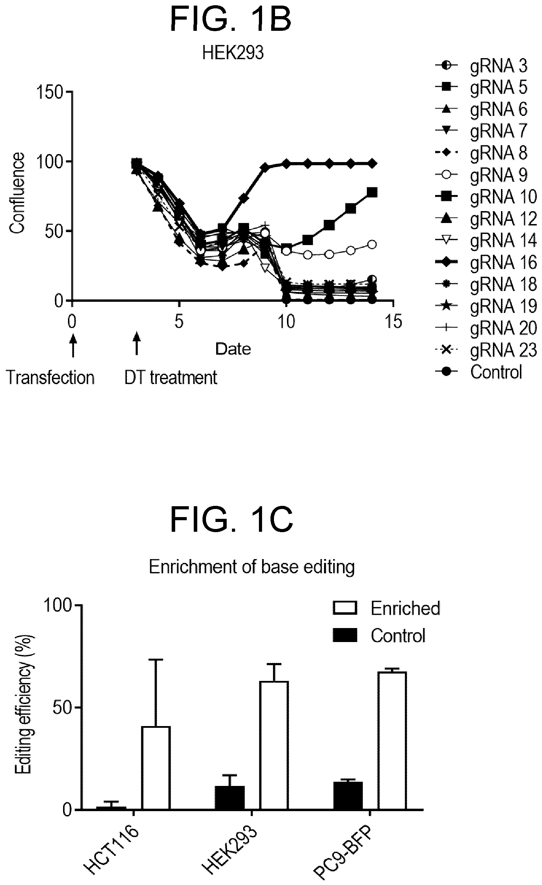

The present disclosure also provides a quick and accurate method to determine editing efficacy in a population of cells. Such a method may facilitate the determination of whether editing has occurred, without the need for extensive sequencing analysis of target cells. The method may also allow for evaluation of multiple guide polynucleotides to determine the most effective guide polynucleotide sequence for a particular purpose. The method of the present disclosure is a "co-targeting enrichment" strategy that dramatically improves the editing efficiency of a base-editing enzyme. In the "co-targeting enrichment" strategy, two guide polynucleotides are introduced into a cell: a first guide polynucleotide, e.g., a "selection" polynucleotide that guides the base-editing enzyme to a "selection" site, and a second guide polynucleotide, e.g., a "target" polynucleotide that guides the base-editing enzyme to a "target" site. In some embodiments, successful editing of the "selection" site results in cells surviving certain selection conditions (e.g., exposure to a cytotoxic agent, elevated or lowered temperature, culture media deficient in one or more nutrients, etc.). FIG. 1A illustrates embodiments of the present disclosure and shows a starting population of cells having "target" and "selection" sites. Under conditions with no selection, only a small percentage of cells have the desired "edited" site. Under the "co-targeting HB-EGF + diphtheria toxin selection," a much higher percentage of cells have the desired "edited" target site.

In some embodiments, successful editing of the "selection" site allows the edited cells to be easily separated from the non-edited cells based on a physical or chemical characteristic (e.g., change in the cell shape or size, and/or ability to generate fluorescence, chemiluminescence, etc.). In some embodiments, cells having edited "selection" sites are more likely to also have edited "target" sites (due to, e.g., successful introduction and/or expression of one or more of the editing components). Therefore, selection of the cells having the edited "selection" site enriches for the cells having the edited "target" site, increasing editing efficiency.

A "site-specific mutation" as described herein includes a single nucleotide substitution, e.g., conversion of cytosine to thymine or vice versa, or adenine to guanine or vice versa, in a polynucleotide sequence. In some embodiments, the site-specific mutation is generated by a base-editing enzyme. In some embodiments, the site-specific mutation occurs via deamination, e.g., by a deaminase, of a nucleotide in the target polynucleotide. In some embodiments, the base-editing enzyme comprises a deaminase.

In some embodiments, a site-specific mutation in a target polynucleotide results in a change in the polypeptide sequence encoded by the polynucleotide. In some embodiments, a site-specific mutation in a target polynucleotide alters expression of a downstream polynucleotide sequence in the cell. For example, expression of the downstream polynucleotide sequence can be inactivated such that the sequence is not transcribed, the encoded protein is not produced, or the sequence does not function as the wild-type sequence. For example, a protein or miRNA coding sequence may be inactivated such that the protein is not produced.

In some embodiments, a site-specific mutation in a regulatory sequence increases expression of a downstream polynucleotide. In some embodiments, a site-specific mutation inactivates a regulatory sequence such that it no longer functions as a regulatory sequence. Non-limiting examples of regulatory sequences include promoters, transcription terminators, enhancers, and other regulatory elements described herein. In some embodiments, a site-specific mutation results in a "knock-out" of the target polynucleotide.

In some embodiments, the target cell is a eukaryotic cell. In some embodiments, the eukaryotic cell is an animal or human cell. In some embodiments, the target cell is a human cell. In some embodiments, the human cell is a stem cell. The stem cell can be, for example, a pluripotent stem cell, including embryonic stem cell (ESC), adult stem cell, induced pluripotent stem cell (iPSC), tissue specific stem cell (e.g., hematopoietic stem cell), and mesenchymal stem cell (MSC). In some embodiments, the human cell is a differentiated form of any of the cells described herein. In some embodiments, the eukaryotic cell is a cell derived from a primary cell in culture. In some embodiments, the cell is a stem cell or a stem cell line.

In some embodiments, the eukaryotic cell is a hepatocyte such as a human hepatocyte, animal hepatocyte, or a non-parenchymal cell. For example, the eukaryotic cell can be a plateable metabolism qualified human hepatocyte, a plateable induction qualified human hepatocyte, plateable QUALYST TRANSPORTER CERTIFIED human hepatocyte, suspension qualified human hepatocyte (including 10-donor and 20-donor pooled hepatocytes), human hepatic kupffer cells, human hepatic stellate cells, dog hepatocytes (including single and pooled Beagle hepatocytes), mouse hepatocytes (including CD-1 and C57BI/6 hepatocytes), rat hepatocytes (including Sprague-Dawley, Wistar Han, and Wistar hepatocytes), monkey hepatocytes (including Cynomolgus or Rhesus monkey hepatocytes), cat hepatocytes (including Domestic Shorthair hepatocytes), and rabbit hepatocytes (including New Zealand White hepatocytes).

In some embodiments, the methods of the present disclosure comprising introducing into a population of cells, a base-editing enzyme. In some embodiments, the base-editing enzyme comprises a DNA-targeting domain and a DNA-editing domain. In some embodiments, the DNA-targeting domain comprises Cas9. In some embodiments, the Cas9 comprises a mutation in a catalytic domain. In some embodiments, the base-editing enzyme comprises a catalytically inactive Cas9 and a DNA-editing domain. In some embodiments, the base-editing enzyme comprises a Cas9 capable of generating single-stranded DNA breaks (nCas9) and a DNA-editing domain. In some embodiments, the nCas9 comprises a mutation at amino acid residue D10 or H840 relative to wild-type Cas9 (numbering relative to SEQ ID NO: 3). In some embodiments, the Cas9 comprises a polypeptide having at least 80%, at least 85%, at least 90%, at least 91%, at least 92%, at least 93%, at least 94%, at least 95%, at least 96%, at least 97%, at least 98%, at least 99%, or about 100% sequence identity to SEQ ID NO: 3. In some embodiments, the Cas9 comprises a polypeptide having at least 90% identical to SEQ ID NO: 3. In some embodiments, the Cas9 comprises a polypeptide having at least 80%, at least 85%, at least 90%, at least 91%, at least 92%, at least 93%, at least 94%, at least 95%, at least 96%, at least 97%, at least 98%, at least 99%, or about 100% sequence identity to SEQ ID NO: 4. In some embodiments, the Cas9 comprises a polypeptide having at least 90% identical to SEQ ID NO: 4.

The CRISPR-Cas system is a recently-discovered prokaryotic adaptive immune system that has been modified to enable robust and site-specific genome engineering in a variety of organisms and cell lines. In general, CRISPR-Cas systems are protein-RNA complexes that use an RNA molecule (e.g., a guide RNA) as a guide to localize the complex to a target DNA sequence via base-pairing of the guide RNA to the target DNA sequence. Typically, Cas9 also may require a short protospacer adjacent motif (PAM) sequence adjacent to the target DNA sequence, for binding to the DNA. Upon formation of a complex with the guide RNA, the Cas9 "searches" for the target DNA sequence by binding with sequences that match the PAM sequence. Once the Cas9 recognizes the PAM *and* the guide RNA pairs properly with the target sequence, the Cas9 protein then acts as an endonuclease to cleave the targeted DNA sequence. Cas9 proteins from different bacterial species may recognize different PAM sequences. For example, the Cas9 from *S. pyogenes* (SpCas9) recognizes the PAM sequence of 5'-NGG-3', wherein N is any nucleotide. A Cas9 protein can also be engineered to recognize a different PAM from the wild-type Cas9. *See,* e.g., Sternberg et al., Nature 507(7490): 62-67 (2014); Kleinstiver et al., Nature 523:481-485 (2015); and Hu et al., Nature 556:57-63 (2018).

Among the known Cas proteins, SpCas9 has been mostly widely used as a tool for genome engineering. The SpCas9 protein is a large, multi-domain protein containing two distinct nuclease domains. As used herein, "Cas9" encompasses any Cas9 protein and variants thereof, including codon-optimized variants and engineered Cas9, e.g., described in U.S. Pat. No. 9944912, 9512446, 10093910; and the Cas9 variant of U.S. Provisional Application 62/728,184, filed September 7, 2018. Point mutations can be introduced into Cas9 to abolish nuclease activity, resulting in a catalytically inactive Cas9, or dead Cas9 (dCas9) that still retains its ability to bind DNA in a guide RNA-programmed manner. In principle, when fused to another protein or domain, dCas9 can target that protein to virtually any DNA sequence simply by co-expression with an appropriate guide RNA. *See,* e.g., Mali et al., Nat Methods 10(10):957-963 (2013); Horvath et al., Nature 482:331-338 (2012); Qi et al., Cell 152(5):1173-1183 (2013). In embodiments, the point mutations comprise mutations at positions D10 and H840 of wild-type Cas9 (numbering relative to the amino acid sequence of wild-type SpCas9). In embodiments, the dCas9 comprises D10A and H840A mutations.

Wild-type Cas9 protein can also be modified such that the Cas9 protein has nickase activity, which are capable of only cleaving one strand of double-stranded DNA, rather than nuclease activity, which generates a double-stranded break. Cas9 nickases (nCas9) are described in, e.g., Cho et al., Genome Res 24:132-141 (2013); Ran et al., Cell 154:1380-1389 (2013); and Mali et al., Nat Biotechnol 31:833-838 (2013). In some embodiments, a Cas9 nickase comprises a single amino acid substitution relative to wild-type Cas9. In some embodiments, the single amino acid substitution is at position D10 of Cas9 (numbering relative to SEQ ID NO: 3). In some embodiments, the single amino acid substitution is H10A (numbering relative to SEQ ID NO: 3). In some embodiments, the single amino acid substitution is at position H840 of Cas9 (numbering relative to SEQ ID NO: 3). In some embodiments, the single amino acid substitution is H840A (numbering relative to SEQ ID NO: 3).

In some embodiments, the base-editing enzyme comprises a DNA-targeting domain and a DNA-editing domain. In some embodiments, the DNA-editing domain comprises a deaminase. In some embodiments, the deaminase is cytidine deaminase or adenosine deaminase. In some embodiments, the deaminase is cytidine deaminase. In some embodiments, the deaminase is adenosine deaminase. In some embodiments, the deaminase is an apolipoprotein B mRNA-editing complex (APOBEC) deaminase, an activation-induced cytidine deaminase (AID), an ACF1/ASE deaminase, an ADAT deaminase, or an ADAR deaminase. In some embodiments, the deaminase is an apolipoprotein B mRNA-editing complex (APOBEC) family deaminase. In some embodiments, the deaminase is APOBEC1.

As described herein, deaminase enzymes catalyze deamination, e.g., deamination of cytosine or adenosine. One exemplary family of cytosine deaminases is the APOBEC family, which encompasses eleven proteins that serve to initiate mutagenesis in a controlled and beneficial manner (Conticello et al., Genome Biol 9(6):229 (2008)). One family member, activation-induced cytidine deaminase (AID), is responsible for the maturation of antibodies by converting cytosines in ssDNA to uracils in a transcription-dependent, strand-biased fashion (Reynaud et al., Nat Immunol 4(7):631-638 (2003)). APOBEC3 provides protection to human cells against a certain HIV-1 strain via the deaminase of cytosines in reverse-transcribed viral ssDNA (Bhagwat et al., DNA Repair (Amst) 3(1):85-89 (2004)). These proteins all require a Zn²⁺-coordinating motif (His-X-Glu-X₂₃₋₂₆-Pro-Cys-X₂₋₄-Cys) and bound water molecule for catalytic activity. The Glu residue in the motif acts to activate the water molecule to a zinc hydroxide for nucleophilic attack in the deamination reaction. Each family member preferentially deaminates at its own particular "hotspot," ranging from WRC (W is A or T, R is A or G) for hAID, to TTC for hAPOBEC3F (Navaratnam et al., Int J Hematol 83(3):195-200 (2006)). A recently crystal structure of the catalytic domain of APOBEC3G revealed that a secondary structure comprised of a five-stranded β-sheet core flanked by six α-helices, which is believed to be conserved across the entire family (Holden et al., Nature 456:121-124 (2008)). The active center loops have been shown to be responsible for both ssDNA binding and in determining "hotspot" identity (Chelico et al., J Biol Chem 284(41):27761-27765 (2009)). Overexpression of these enzymes has been linked to genomic instability and cancer, thus highlighting the importance of sequence-specific targeting (Pham et al., Biochemistry 44(8):2703-2715 (2005)).

Another exemplary suitable type of nucleic acid-editing enzymes and domains are adenosine deaminases. Examples of adenosine deaminases include Adenosine Deaminase Acting on tRNA (ADAT) and Adenosine Deaminase Acting on RNA (ADAR) families. ADAT family deaminases include TadA, a tRNA adenosine deaminase that shares sequence similarity with the APOBEC enzyme. ADAR family deaminases include ADAR2, which converts adenosine to inosine in double-stranded RNA, thus enabling base editing of RNA. *See,* e.g., Gaudelli et al., Nature 551:464-471 (2017); Cox et al., Science 358:1019-1027 (2017).

In some embodiments, the base-editing enzyme further comprises a DNA glycosylase inhibitor domain. In some embodiments, the DNA glycosylase inhibitor is uracil DNA glycosylase inhibitor (UGI). In general, DNA glycosylases such as uracil DNA glycosylase are part of the base excision repair pathway and perform error-free repair upon detecting a U:G mismatch (wherein the "U" is generated from deamination of a cytosine), converting the U back to the wild-type sequence and effectively "undoing" the base-editing. Thus, addition of a DNA glycosylase inhibitor (e.g., uracil DNA glycosylase inhibitor) inhibits the base excision repair pathway, increasing the base-editing efficiency. Non-limiting examples of DNA glycosylases include OGG1, MAG1, and UNG. DNA glycosylase inhibitors can be small molecules or proteins. For example, protein inhibitors of uracil DNA glycosylase are described in Mol et al., Cell 82:701-708 (1995); Serrano-Heras et al., J Biol Chem 281:7068-7074 (2006); and New England Biolabs Catalog No. M0281S and M0281L (neb.com/products/m0281-uracil-glycosylase-inhibitor-ugi). Small molecule inhibitors of DNA glycosylases are described in, e.g., Huang et al., J Am Chem Soc 131(4):1344-1345 (2009); Jacobs et al., PLoS One 8(12):e81667 (2013); Donley et al., ACS Chem Biol 10(10):2334-2343 (2015); Tahara et al., J Am Chem Soc 140(6):2105-2114 (2018).

Thus, in some embodiments, the base-editing enzyme of the present disclosure comprises a Cas9 capable of making single stranded breaks and a cytidine deaminase. In some embodiments, the base-editing enzyme of the present disclosure comprises nCas9 and cytidine deaminase. In some embodiments, the base-editing enzyme of the present disclosure comprises a Cas9 capable of making single stranded breaks and an adenosine deaminase. In some embodiments, the base-editing enzyme of the present disclosure comprises nCas9 and adenosine deaminase. In some embodiments, the base-editing enzyme is at least 90% identical to SEQ ID NO: 6. In some embodiments, the base-editing enzyme comprises a polypeptide having at least 50%, at least 60%, at least 70%, at least 80%, at least 85%, or at least 90% sequence identity to SEQ ID NO: 6. In some embodiments, the base-editing enzyme comprises a polypeptide having at least 91%, at least 92%, at least 93%, at least 94%, at least 95%, at least 96%, at least 97%, at least 98%, at least 99%, or about 100% sequence identity to SEQ ID NO: 6. In some embodiments, a polynucleotide encoding the base-editing enzyme is at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or about 100% identical to SEQ ID NO: 5. In some embodiments, the base-editing enzyme is BE3.

In some embodiments, the methods of the present disclosure comprise introducing into a population of cells, a first guide polynucleotide that hybridizes to a gene encoding a cytotoxic agent (CA) receptor, and forms a first complex with the base-editing enzyme; wherein the base-editing enzyme of the first complex provides a mutation in the gene encoding the CA receptor, and wherein the mutation in the gene encoding the CA receptor forms a CA-resistant cell in the population of cells.

In some embodiments, the first guide polynucleotide is an RNA molecule. The RNA molecule that binds to CRISPR-Cas components and targets them to a specific location within the target DNA is referred to herein as "RNA guide polynucleotide," "guide RNA," "gRNA," "small guide RNA," "single-guide RNA," or "sgRNA" and may also be referred to herein as a "DNA-targeting RNA." The guide polynucleotide can be introduced into the target cell as an isolated molecule, e.g., an RNA molecule, or is introduced into the cell using an expression vector containing DNA encoding the guide polynucleotide, e.g., the RNA guide polynucleotide. In some embodiments, the guide polynucleotide is 10 to 150 nucleotides. In some embodiments, the guide polynucleotide is 20 to 120 nucleotides. In some embodiments, the guide polynucleotide is 30 to 100 nucleotides. In some embodiments, the guide polynucleotide is 40 to 80 nucleotides. In some embodiments, the guide polynucleotide is 50 to 60 nucleotides. In some embodiments, the guide polynucleotide is 10 to 35 nucleotides. In some embodiments, the guide polynucleotide is 15 to 30 nucleotides. In some embodiments, the guide polynucleotide is 20 to 25 nucleotides.

In some embodiments, an RNA guide polynucleotide comprises at least two nucleotide segments: at least one "DNA-binding segment" and at least one "polypeptide-binding segment." By "segment" is meant a part, section, or region of a molecule, e.g., a contiguous stretch of nucleotides of guide polynucleotide molecule. The definition of "segment," unless otherwise specifically defined, is not limited to a specific number of total base pairs.

In some embodiments, the guide polynucleotide includes a DNA-binding segment. In some embodiments, the DNA-binding segment of the guide polynucleotide comprises a nucleotide sequence that is complementary to a specific sequence within a target polynucleotide. In some embodiments, the DNA-binding segment of the guide polynucleotide hybridizes with a gene encoding a cytotoxic agent (CA) receptor in a target cell. In some embodiments, the DNA-binding segment of the guide polynucleotide hybridizes with a target polynucleotide sequence in a target cell. Target cells, including various types of eukaryotic cells, are described herein.

In some embodiments, the guide polynucleotide includes a polypeptide-binding segment. In some embodiments, the polypeptide-binding segment of the guide polynucleotide binds the DNA-targeting domain of a base-editing enzyme of the present disclosure. In some embodiments, the polypeptide-binding segment of the guide polynucleotide binds to Cas9 of a base-editing enzyme. In some embodiments, the polypeptide-binding segment of the guide polynucleotide binds to dCas9 of a base-editing enzyme. In some embodiments, the polypeptide-binding segment of the guide polynucleotide binds to nCas9 of a base-editing enzyme. Various RNA guide polynucleotides which bind to Cas9 proteins are described in, e.g., U.S. Patent Publication Nos. 2014/0068797, 2014/0273037, 2014/0273226, 2014/0295556, 2014/0295557, 2014/0349405, 2015/0045546, 2015/0071898, 2015/0071899, and 2015/0071906.

In some embodiments, the guide polynucleotide further comprises a tracrRNA. The "tracrRNA," or trans-activating CRISPR-RNA, forms an RNA duplex with a pre-crRNA, or pre-CRISPR-RNA, and is then cleaved by the RNA-specific ribonuclease RNase III to form a crRNA/tracrRNA hybrid. In some embodiments, the guide polynucleotide comprises the crRNA/tracrRNA hybrid. In some embodiments, the tracrRNA component of the guide polynucleotide activates the Cas9 protein. In some embodiments, activation of the Cas9 protein comprises activating the nuclease activity of Cas9. In some embodiments, activation of the Cas9 protein comprises the Cas9 protein binding to a target polynucleotide sequence.

In some embodiments, the sequence of the guide polynucleotide is designed to target the base-editing enzyme to a specific location in a target polynucleotide sequence. Various tools and programs are available to facilitate design of such guide polynucleotides, e.g., the Benchling base editor design guide (benchling.com/editor#create/crispr), and BE-Designer and BE-Analyzer from CRISPRRGEN Tools *(see* Hwang et al., bioRxiv dx.doi.org/10.1101/373944, first published July 22, 2018).

In some embodiments, the DNA-binding segment of the first guide polynucleotide hybridizes with a gene encoding a cytotoxic agent (CA) receptor, and the polypeptide-binding segment of the first guide polynucleotide forms a first complex with the base-editing enzyme by binding to the DNA-targeting domain of the base-editing enzyme. In some embodiments, the DNA-binding segment of the first guide polynucleotide hybridizes with a gene encoding a cytotoxic agent (CA) receptor, and the polypeptide-binding segment of the first guide polynucleotide forms a first complex with the base-editing enzyme by binding to Cas9 of the base-editing enzyme. In some embodiments, the DNA-binding segment of the first guide polynucleotide hybridizes with a gene encoding a cytotoxic agent (CA) receptor, and the polypeptide-binding segment of the first guide polynucleotide forms a first complex with the base-editing enzyme by binding to dCas9 of the base-editing enzyme. In some embodiments, the DNA-binding segment of the first guide polynucleotide hybridizes with a gene encoding a cytotoxic agent (CA) receptor, and the polypeptide-binding segment of the first guide polynucleotide forms a first complex with the base-editing enzyme by binding to nCas9 of the base-editing enzyme.

In some embodiments, the first complex is targeted to the gene encoding the CA receptor by the first guide polynucleotide, and the base-editing enzyme of the first complex introduces a mutation in a gene encoding the CA receptor. In some embodiments, the mutation in the gene encoding the CA receptor is introduced by the base-editing domain of the base-editing enzyme of the first complex. In some embodiments, the mutation in the gene encoding the CA receptor forms a CA-resistant cell in the population of cells. In some embodiments, the mutation is a cytidine (C) to thymine (T) point mutation. In some embodiments, the mutation is an adenine (A) to guanine (G) point mutation. The specific location of the mutation in the CA receptor may be directed by, e.g., design of the first guide polynucleotide using tools such as, e.g., the Benchling base editor design guide, BE-Designer, and BE-Analyzer described herein. In some embodiments, the first guide polynucleotide is an RNA polynucleotide. In some embodiments, the first guide polynucleotide further comprises a tracrRNA sequence.

In some embodiments, the CA is a compound that causes or promotes cell death, as described herein. In some embodiments, the CA is a toxin. In some embodiments, the CA is a naturally-occurring toxin. In some embodiments, the CA is a synthetic toxicant. In some embodiments, the CA is a small molecule, a peptide, or a protein. In some embodiments, the CA is an antibody-drug conjugate. In some embodiments, the CA is a monoclonal antibody attached a biologically active drug with a chemical linker having a labile bond. In some embodiments, the CA is a biotoxin. In some embodiments, the toxin is produced by cyanobacteria (cyanotoxin), dinoflagellates (dinotoxin), spiders, snakes, scorpions, frogs, sea creatures such as jellyfish, venomous fish, coral, or the blue-ringed octopus. Examples of toxins include, e.g., diphtheria toxin, botulinum toxin, ricin, apitoxin, Shiga toxin, Pseudomonas exotoxin, and mycotoxin. In some embodiments, the CA is diphtheria toxin. In some embodiments, the CA is an antibody-drug conjugate. In some embodiments, the antibody-drug conjugate comprises an antibody linked to a toxin. In some embodiments, the toxin is a small molecule, an RNase, or a proapoptotic protein.

In some embodiments, the CA is toxic to one organism, e.g., a human, but not to another organism, e.g., a mouse. In some embodiments, the CA is toxic to an organism in one stage of its life cycle (e.g., fetal stage) but not toxic in another life stage of the organism (e.g., adult stage). In some embodiments, the CA is toxic in one organ of an animal, but not to another organ of the same animal. In some embodiments, the CA is toxic to a subject (e.g., a human or an animal) in one condition or state (e.g., diseased), but not to the same subject in another condition or state (e.g., healthy). In some embodiments, the CA is toxic to one cell type, but not to another cell type. In some embodiments, the CA is toxic to a cell in one cellular state (e.g., differentiated), but not toxic to the same cell in another cellular state (e.g., undifferentiated). In some embodiments, the CA is toxic to the cell in one environment (e.g., low temperature), but not toxic to the same cell in another environment (e.g., high temperature). In some embodiments, the toxin is toxic to human cells, but not to mouse cells.

In some embodiments, the CA receptor is a biological receptor that binds the CA. A CA receptor is a protein molecule, typically located on the membrane of a cell, which binds to the CA. For example, diphtheria toxin binds to the human heparin binding EGF like growth factor (HB-EGF). A CA receptor can be specific for one CA, or a CA receptor can bind more than one CA. For example, monosialoganglioside (GMi) can act as a receptor for both cholera toxin and *E. coli* heat-labile enterotoxin. Or, more than one CA receptor can bind one CA. For example, the botulinum toxin is believed to bind to different receptors in nerve cells and epithelial cells. In some embodiments, the CA receptor is a receptor that binds to the CA. In some embodiments, the CA receptor is a G-protein coupled receptor. In some embodiments, the CA receptor is a receptor for an antibody, e.g., an antibody of an antibody-drug conjugate. In some embodiments, the CA receptor is a receptor for diphtheria toxin. In some embodiments, the CA receptor is HB-EGF.

In some embodiments, one or more mutations in the polynucleotide encoding the CA receptor protein confers resistance to the CA. In some embodiments, a mutation in the CA-binding region of the CA-receptor confers resistance to the CA. In some embodiments, a charge-reversal mutation of an amino acid at or near the CA-binding site of the CA receptor confers resistance to the CA. Charge-reversal mutations include, e.g., a negatively-charged amino acid such as Glu or Asp replaced with a positively-charged amino acid such as Lys or Arg, or vice versa. In some embodiments, a polarity-reversal mutation of an amino acid at or near the CA-binding site of the CA receptor confers resistance to the CA. Polarity-reversal mutations include, e.g., a polar amino acid such as Gln or Asn replaced with a non-polar amino acid such as Val or Ile, or vice versa. In some embodiments, replacement of a relatively small amino acid residue at or near the CA-binding site of the CA receptor with a "bulky" amino acid residue blocks the binding pocket and prevents the CA from binding, thus conferring resistance to the CA. Small amino acids include, e.g., Gly or Ala, while Trp is generally considered a bulky amino acid.

In some embodiments, the one or more mutations in the polynucleotide encoding the CA receptor changes one or more codons in the amino acid sequence of the CA receptor. In some embodiments, the one or more mutations in the polynucleotide encoding the CA receptor changes a single codon in the amino acid sequence of the CA receptor. In some embodiments, a single nucleotide mutation in the polynucleotide encoding the CA receptor confers resistance to the CA receptor. In some embodiments, the single nucleotide mutation is a cytidine (C) to thymine (T) point mutation in the polynucleotide sequence encoding the CA receptor. In some embodiments, the single nucleotide mutation is an adenine (A) to guanine (G) point mutation in the polynucleotide sequence encoding the CA receptor. In some embodiments, the one or more mutations in the CA receptor is provided by the base-editing enzyme described herein. The base-editing enzyme is specifically targeted to the CA receptor by the DNA-targeting domain (e.g., a Cas9 domain), and the base-editing domain (e.g., a deaminase domain) then provides the mutation in the CA receptor. In some embodiments, the one or more mutations in the CA receptor is provided by a base-editing enzyme comprising nCas9 and a cytidine deaminase. In some embodiments, the one or more mutations in the CA receptor is provided by a base-editing enzyme comprising nCas9 and an adenosine deaminase. In some embodiments, the one or more mutations in the CA receptor is provided by a base-editing enzyme comprising a polypeptide having at least 90% sequence identity to SEQ ID NO: 6. In some embodiments, the base-editing enzyme is BE3.

In some embodiments, the CA receptor is a receptor for diphtheria toxin. In some embodiments, the diphtheria toxin receptor is human HB-EGF. Unless specified otherwise, "HB-EGF," used herein without an organism modifier, refers to human HB-EGF. The HB-EGF protein from other organisms, such as mice, are described specifically as "mouse HB-EGF."

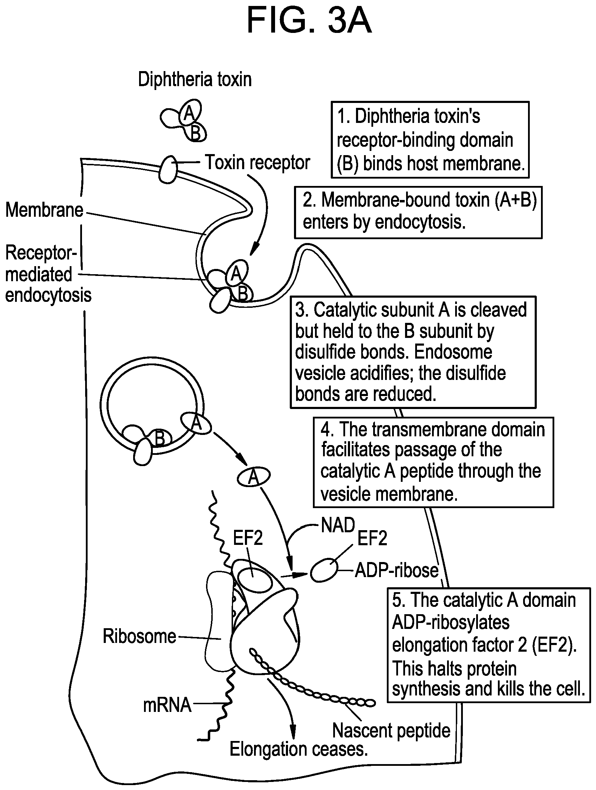

Diphtheria toxin is known as an "A-B" toxin, which are two-component protein complexes with two subunits, typically linked with a disulfide bridge: the "A" subunit is typically considered the "active" portion," while the "B" subunit is generally the "binding" portion. Diphtheria toxin is known to bind to the EGF-like domain of HB-EGF, which is widely expressed in different tissues. FIG. 3A illustrates an exemplary mechanism of action of the A-B diphtheria toxin on its receptor. As shown in FIG. 3A, diphtheria subunit B is responsible for binding HB-EGF, a membrane-bound receptor. Upon binding, the diphtheria toxin enters the cell via receptor-mediated endocytosis. The catalytic subunit A then cleaves from subunit B via reduction of the disulfide linkage between the two subunits, leaves the endocytosis vesicle, and catalyzes the addition of ADP-ribose to elongation factor 2 (EF2) of the ribosome. ADP-ribosylation of EF2 halts protein synthesis and results in cell death.

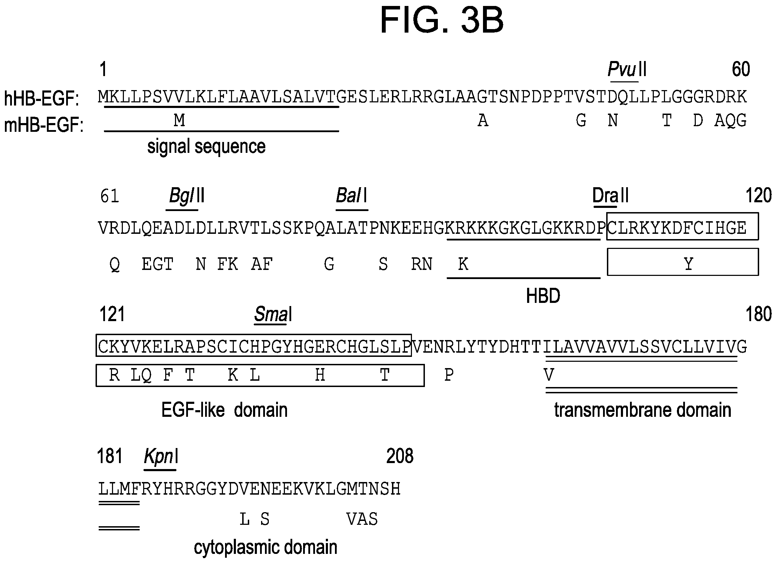

Unlike human HB-EGF, mouse HB-EGF is resistant to diphtheria toxin binding, and thus, mice are resistant to diphtheria toxin. FIG. 3B shows the significant differences in the amino acid sequences of human and mouse HB-EGF proteins. Thus, in some embodiments, one or more mutations in the polynucleotide encoding the HB-EGF protein confers resistance to diphtheria toxin. In some embodiments, the one or more mutations in the polynucleotide encoding HB-EGF changes one or more codons in the amino acid sequence of HB-EGF. In some embodiments, the one or more mutations in the polynucleotide encoding HB-EGF changes a single codon in the amino acid sequence of HB-EGF. In some embodiments, a single nucleotide mutation in the polynucleotide encoding the HB-EGF protein confers resistance to diphtheria toxin. In some embodiments, the single nucleotide mutation is a cytidine (C) to thymine (T) point mutation in the polynucleotide sequence encoding HB-EGF. In some embodiments, the single nucleotide mutation is an adenine (A) to guanine (G) point mutation in the polynucleotide sequence encoding HB-EGF.

In some embodiments, a mutation in the diphtheria toxin-binding region of HB-EGF confers resistance to diphtheria toxin. In some embodiments, a mutation in the EGF-like domain of HB-EGF confers resistance to diphtheria toxin. In some embodiments, a charge-reversal mutation of an amino acid at or near the diphtheria toxin binding site of HB-EGF confers resistance to diphtheria toxin. In some embodiments, the charge-reversal mutation is replacement of a negatively-charged residue, e.g., Glu or Asp, with a positively-charged residue, e.g., Lys or Arg. In some embodiments, the charge-reversal mutation is replacement of a positively-charged residue, e.g., Lys or Arg, with a negatively-charged residue, e.g., Glu or Asp. In some embodiments, a polarity-reversal mutation of an amino acid at or near the diphtheria toxin binding site of HB-EGF confers resistance to diphtheria toxin. In some embodiments, the polarity-reversal mutation is replacement of a polar amino acid residue, e.g., Gln or Asn, with a non-polar amino acid residue, e.g., Ala, Val, or Ile. In some embodiments, the polarity-reversal mutation is replacement of a non-polar amino acid residue, e.g., Ala, Val, or Ile, with a polar amino acid residue, e.g., Gln or Asn. In some embodiments, the mutation is replacement of a relatively small amino acid residue, e.g., Gly or Ala, at or near the diphtheria toxin binding site of HB-EGF with a "bulky" amino acid residue, e.g., Trp. In some embodiments, the mutation of a small residue to a bulky residue blocks the binding pocket and prevents diphtheria toxin from binding, thereby conferring resistance.

In some embodiments, a mutation in one or more of amino acids 100 to 160 of wild-type HB-EGF (SEQ ID NO: 8) confers resistance to diphtheria toxin. In some embodiments, a mutation in one or more of amino acids 105 to 150 of wild-type HB-EGF (SEQ ID NO: 8) confers resistance to diphtheria toxin. In some embodiments, a mutation in or more of amino acids 107 to 148 of wild-type HB-EGF (SEQ ID NO: 8) confers resistance to diphtheria toxin. In some embodiments, a mutation in one or more of amino acids 120 to 145 of wild-type HB-EGF (SEQ ID NO: 8) confers resistance to diphtheria toxin. In some embodiments, a mutation in one or more of amino acids 135 to 143 of wild-type HB-EGF (SEQ ID NO: 8) confers resistance to diphtheria toxin. In some embodiments, a mutation in or more of amino acids 138 to 144 of wild-type HB-EGF (SEQ ID NO: 8) confers resistance to diphtheria toxin. In some embodiments, a mutation in amino acid 141 of wild-type HB-EGF (SEQ ID NO: 8) confers resistance to diphtheria toxin. In some embodiments, the mutation in amino acid 141 of wild-type HB-EGF (SEQ ID NO: 8) is GLU141 to ARG141. In some embodiments, the mutation in amino acid 141 of wild-type HB-EGF (SEQ ID NO: 8) is GLU141 to HIS141. In some embodiments, the mutation in amino acid 141 of wild-type HB-EGF (SEQ ID NO: 8) is GLU141 to LYS141. In some embodiments, a mutation of GLU141 to LYS141 of wild-type HB-EGF (SEQ ID NO: 8) confers resistance to diphtheria toxin.

In some embodiments, the one or more mutations in HB-EGF is provided by the base-editing enzyme described herein. The base-editing enzyme is specifically targeted to the HB-EGF by the DNA-targeting domain (e.g., a Cas9 domain), and the base-editing domain (e.g., a deaminase domain) then provides the mutation in HB-EGF. In some embodiments, the one or more mutations in HB-EGF is provided by a base-editing enzyme comprising nCas9 and a cytidine deaminase. In some embodiments, the one or more mutations in HB-EGF is provided by a base-editing enzyme comprising nCas9 and an adenosine deaminase. In some embodiments, the one or more mutations in HB-EGF is provided by a base-editing enzyme comprising a polypeptide having at least 90% sequence identity to SEQ ID NO: 6. In some embodiments, the base-editing enzyme is BE3.

In some embodiments, the DNA-binding segment of the second guide polynucleotide hybridizes with the target polynucleotide in the target cell, and the polypeptide-binding segment of the second guide polynucleotide forms a second complex with the base-editing enzyme by binding to the DNA-targeting domain of the base-editing enzyme. In some embodiments, the DNA-binding segment of the second guide polynucleotide hybridizes with the target polynucleotide in the target cell, and the polypeptide-binding segment of the second guide polynucleotide forms a second complex with the base-editing enzyme by binding to Cas9 of the base-editing enzyme. In some embodiments, the DNA-binding segment of the second guide polynucleotide hybridizes with the target polynucleotide in the target cell, and the polypeptide-binding segment of the second guide polynucleotide forms a second complex with the base-editing enzyme by binding to dCas9 of the base-editing enzyme. In some embodiments, the DNA-binding segment of the second guide polynucleotide hybridizes with the target polynucleotide in the target cell, and the polypeptide-binding segment of the second guide polynucleotide forms a second complex with the base-editing enzyme by binding to nCas9 of the base-editing enzyme.

In some embodiments, the second complex is targeted to the target polynucleotide by the second guide polynucleotide, and the base-editing enzyme of the second complex introduces a mutation in the target polynucleotide. In some embodiments, the mutation in the target polynucleotide is introduced by the base-editing domain of the base-editing enzyme of the second complex. In some embodiments, the mutation in the target polynucleotide is a cytidine (C) to thymine (T) point mutation. In some embodiments, the mutation in the target polynucleotide is an adenine (A) to guanine (G) point mutation. The specific location of the mutation in the target polynucleotide may be directed by, e.g., design of the second guide polynucleotide using tools such as, e.g., the Benchling base editor design guide, BE-Designer, and BE-Analyzer described herein. In some embodiments, the second guide polynucleotide is an RNA polynucleotide. In some embodiments, the second guide polynucleotide further comprises a tracrRNA sequence.

In some embodiments, the C to T mutation in the target polynucleotide inactivates expression of the target polynucleotide in the target cell. In some embodiments, the A to G mutation in the target polynucleotide inactivates expression of the target polynucleotide in the target cell. In some embodiments, the target polynucleotide encodes a protein or miRNA. In some embodiments, the target polynucleotide is a regulatory sequence, and the C to T mutation changes the function of the regulatory sequence. In some embodiments, the target polynucleotide is a regulatory sequence, and the A to G mutation changes the function of the regulatory sequence.

In some embodiments, the base-editing enzyme of the present disclosure is introduced into the population of cells as a polynucleotide encoding the base-editing enzyme. In some embodiments, the first and/or second guide polynucleotides are introduced into the population of cells as one or more polynucleotides encoding the first and/or second guide polynucleotides. In some embodiments, the base-editing enzyme, the first guide polynucleotide, and the second guide polynucleotide are introduced into the population of cells via a vector. In some embodiments, the polynucleotide encoding the base-editing enzyme, the first guide polynucleotide, and the second guide polynucleotide are on a single vector. In some embodiments, the vector is a viral vector. In some embodiments, the polynucleotide encoding the base-editing enzyme, the first guide polynucleotide, and the second guide polynucleotide are on one or more vectors. In some embodiments, the one or more vectors are viral vectors. In some embodiments, the viral vector is an adenovirus, an adeno-associated virus, or a lentivirus. Viral transduction with adenovirus, adeno-associated virus (AAV), and lentiviral vectors (where administration can be local, targeted or systemic) have been used as delivery methods for *in vivo* gene therapy. Methods of introducing vectors, e.g., viral vectors, into cells (e.g., transfection) are described herein.

In some embodiments, the base-editing enzyme, the first guide polynucleotide, and/or the second guide polynucleotide are introduced into the population of cells via a delivery particle. In some embodiments, the base-editing enzyme, the first guide polynucleotide, and/or the second guide polynucleotide are introduced into the population of cells via a vesicle.

In some embodiments, the efficacy of the base-editing enzyme can be determined by calculating the ratio of the CA-resistant cells to the total population of cells. In some embodiments, the number of CA-resistant cells can be counted using techniques known in the art, for example, counting using a hematocytometer, measuring absorbance at a certain wavelength (e.g., 580 nm or 600 nm), and/or measuring the fluorescence of a fluorophore for detection of cell populations. In some embodiments, the total population of cells is determined, and the ratio of the CA-resistant cells to the total population of cells is calculated by dividing the total population of cells by the CA-resistant cells. In some embodiments, the ratio of the CA-resistant cells to the total population of cells approximates the base-editing efficacy at the target polynucleotide.

### Methods of Site-Specific Integration

As described herein, HDR-based DNA double-stranded break repair can provide site-specific integration, e.g., bi-allelic integration, of a desired sequence of interest (SOI) at a target locus. For the applications of genetic mutant correction, gene therapy, and transgenic animal generation, site specific integration, and specifically bi-allelic integration, of the gene modification of interest is highly desirable. Unfortunately, due to the low efficiency of HDR-based DNA double-stranded break repair, screening and isolation of site-specific integration, particularly bi-allelic integration, is often difficult and cumbersome, and may require costly and time-consuming sequencing and analysis. The methods of the present disclosure apply the "co-targeting enrichment" strategy described herein to generate site-specific integration of a sequence of interest, and provide a simple and efficient screening method for cells which have the desired integration. In some embodiment, the site-specific integration is a bi-allelic integration.

In some embodiments, the present disclosure includes a method of providing a bi-allelic integration of a sequence of interest (SOI) into a toxin sensitive gene (TSG) locus in a genome of a cell, the method comprising (a) introducing into a population of cells: (i) a nuclease capable of generating a double-stranded break; (ii) a guide polynucleotide that forms a complex with the nuclease and is capable of hybridizing with the TSG locus; and (iii) a donor polynucleotide comprising (1) 5' homology arm, a 3' homology arm, and a mutation in a native coding sequence of the TSG, wherein the mutation confers resistance to the toxin; and (2) the SOI, wherein introduction of (i), (ii), and (iii) results in integration of the donor polynucleotide in the TSG locus; (b) contacting the population of cells with the toxin; and selecting one or more cells resistant to the toxin, wherein the one or more cells resistant to the toxin comprise the bi-allelic integration of the SOI.

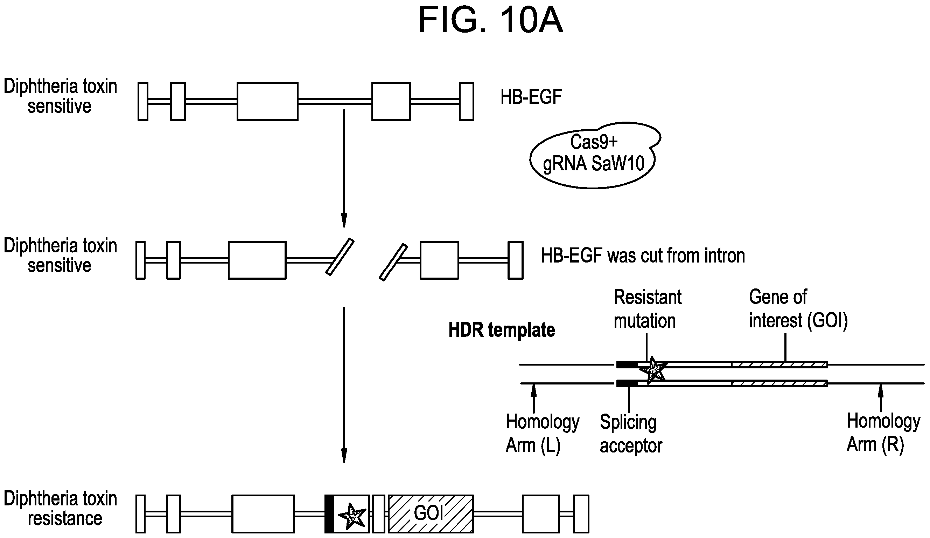

FIG. 10A illustrates an embodiment of the methods provided herein. In FIG. 10A, the wild-type sequence of HB-EGF is diphtheria toxin sensitive. The solid boxes in the sequence represent exons, while the double lines represent introns. The Cas9 nuclease is targeted to an intron of the HB-EGF by the guide polynucleotide of the CRISPR-Cas complex and generates a double-stranded break. An HDR template is introduced into the cell having a splicing acceptor sequence for joining the exon on the HDR template and the adjacent genomic exons, a diphtheria toxin-resistant mutation in the exon immediately preceding the double-stranded break, and a gene of interest (GOI). HDR repairs the double-stranded break and inserts the splicing acceptor sequence, the diphtheria toxin-resistant mutation, and the GOI at the site of the break. Thus, only cells that have bi-allelic integration of the HDR template (and thereby the GOI) are resistant to diphtheria toxin; cells that are mono-allelic or were not repaired by HDR are sensitive to the toxin. Therefore, cells that survive upon contact with the toxin have a bi-allelic integration of the GOI.

In some embodiments, the TSG locus encodes HB-EGF, and the toxin is diphtheria toxin. In some embodiments, the nuclease capable of generating a double-stranded break is Cas9. In some embodiments, the guide polynucleotide is a guide RNA. In some embodiments, the donor polynucleotide is an HDR template. In some embodiments, the SOI is a gene of interest. In some embodiments, integration of the donor polynucleotide in the TSG locus is bi-allelic integration.

In some embodiments, the present disclosure provides a method of integrating a sequence of interest (SOI) into a target locus in a genome of a cell, the method comprising (a) introducing into a population of cells: (i) a nuclease capable of generating a double-stranded break; (ii) a guide polynucleotide that forms a complex with the nuclease and is capable of hybridizing with a toxin sensitive gene (TSG) locus in the genome of the cell, wherein the TSG is an essential gene; and (iii) a donor polynucleotide comprising: (1) a functional TSG gene comprising a mutation in a native coding sequence of the TSG, wherein the mutation confers resistance to the toxin, (2) the SOI, and (3) a sequence for genome integration at the target locus; wherein introduction of (i), (ii), and (iii) results in inactivation of the TSG in the genome of the cell by the nuclease, and integration of the donor polynucleotide in the target locus; (b) contacting the population of cells with the toxin; and (c) selecting one or more cells resistant to the toxin, wherein the one or more cells resistant to the toxin comprise the SOI integrated in the target locus.

In some embodiments, the present disclosure provides a method of introducing a stable episomal vector into a cell, the method comprising (a) introducing into a population of cells: (i) a nuclease capable of generating a double-stranded break; (ii) a guide polynucleotide that forms a complex with the nuclease and is capable of hybridizing with a toxin sensitive gene (TSG) locus in the genome of the cell, wherein introduction of (i) and (ii) results in inactivation of the TSG in the genome of the cell by the nuclease; and (iii) an episomal vector comprising: (1) a functional TSG comprising a mutation in a native coding sequence of the TSG, wherein the mutation confers resistance to the toxin; (2) the SOI; and (3) an autonomous DNA replication sequence; (b) contacting the population of cells with the toxin; and (c) selecting one or more cells resistant to the toxin, wherein the one or more cells resistant to the toxin comprise the episomal vector. In some embodiments, the TSG is an essential gene.

In some embodiments, the nuclease capable of generating double-stranded breaks is Cas9. As described herein, Cas9 is a monomeric protein comprising a DNA-targeting domain (which interacts with the guide polynucleotide, e.g., guide RNA) and a nuclease domain (which cleaves the target polynucleotide, e.g., the TSG locus). Cas9 proteins generate site-specific breaks in a nucleic acid. In some embodiments, Cas9 proteins generate site-specific double-stranded breaks in DNA. The ability of Cas9 to target a specific sequence in a nucleic acid (i.e., site specificity) is achieved by the Cas9 complexing with a guide polynucleotide (e.g., guide RNA) that hybridizes with the specified sequence (e.g., the TSG locus). In some embodiments, the Cas9 is a Cas9 variant described in U.S. Provisional Application 62/728,184, filed September 7, 2018.

In some embodiments, the Cas9 is capable of generating cohesive ends. Cas9 capable of generating cohesive ends are described in, e.g., PCT/US2018/061680, filed November 16, 2018. In some embodiments, the Cas9 capable of generating cohesive ends is a dimeric Cas9 fusion protein. In some embodiments, it is advantageous to use a dimeric nuclease, i.e., a nuclease which is not active until both monomers of the dimer are present at the target sequence, in order to achieve higher targeting specificity. Binding domains and cleavage domains of naturally-occurring nucleases (such as, e.g., Cas9), as well as modular binding domains and cleavage domains that can be fused to create nucleases binding specific target sites, are well known to those of skill in the art. For example, the binding domain of RNA-programmable nucleases (e.g., Cas9), or a Cas9 protein having an inactive DNA cleavage domain, can be used as a binding domain (e.g., that binds a gRNA to direct binding to a target site) to specifically bind a desired target site, and fused or conjugated to a cleavage domain, for example, the cleavage domain of the endonuclease FokI, to create an engineered nuclease cleaving the target site. Cas9-FokI fusion proteins are further described in, e.g., U.S. Patent Publication No. 2015/0071899 and Guilinger et al., "Fusion of catalytically inactive Cas9 to FokI nuclease improves the specificity of genome modification," Nature Biotechnology 32: 577-582 (2014).

In some embodiments, the Cas9 comprises a polypeptide of SEQ ID NO: 3 or 4. In some embodiments, the Cas9 comprises a polypeptide having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence identity to SEQ ID NO: 3 or 4. In some embodiments, the Cas9 is SEQ ID NO: 3 or 4.

In some embodiments, the guide polynucleotide is an RNA polynucleotide. The RNA molecule that binds to CRISPR-Cas components and targets them to a specific location within the target DNA is referred to herein as "RNA guide polynucleotide," "guide RNA," "gRNA," "small guide RNA," "single-guide RNA," or "sgRNA" and may also be referred to herein as a "DNA-targeting RNA." The guide polynucleotide can be introduced into the target cell as an isolated molecule, e.g., an RNA molecule, or is introduced into the cell using an expression vector containing DNA encoding the guide polynucleotide, e.g., the RNA guide polynucleotide. In some embodiments, the guide polynucleotide is 10 to 150 nucleotides. In some embodiments, the guide polynucleotide is 20 to 120 nucleotides. In some embodiments, the guide polynucleotide is 30 to 100 nucleotides. In some embodiments, the guide polynucleotide is 40 to 80 nucleotides. In some embodiments, the guide polynucleotide is 50 to 60 nucleotides. In some embodiments, the guide polynucleotide is 10 to 35 nucleotides. In some embodiments, the guide polynucleotide is 15 to 30 nucleotides. In some embodiments, the guide polynucleotide is 20 to 25 nucleotides.

In some embodiments, an RNA guide polynucleotide comprises at least two nucleotide segments: at least one "DNA-binding segment" and at least one "polypeptide-binding segment." By "segment" is meant a part, section, or region of a molecule, e.g., a contiguous stretch of nucleotides of guide polynucleotide molecule. The definition of "segment," unless otherwise specifically defined, is not limited to a specific number of total base pairs.

In some embodiments, the guide polynucleotide includes a DNA-binding segment. In some embodiments, the DNA-binding segment of the guide polynucleotide comprises a nucleotide sequence that is complementary to a specific sequence within a target polynucleotide. In some embodiments, the DNA-binding segment of the guide polynucleotide hybridizes with a toxin sensitive gene (TSG) locus in a cell. Various types of cells, e.g., eukaryotic cells, are described herein.

In some embodiments, the guide polynucleotide includes a polypeptide-binding segment. In some embodiments, the polypeptide-binding segment of the guide polynucleotide binds the DNA-targeting domain of a nuclease of the present disclosure. In some embodiments, the polypeptide-binding segment of the guide polynucleotide binds to Cas9. In some embodiments, the polypeptide-binding segment of the guide polynucleotide binds to dCas9. In some embodiments, the polypeptide-binding segment of the guide polynucleotide binds to nCas9. Various RNA guide polynucleotides which bind to Cas9 proteins are described in, e.g., U.S. Patent Publication Nos. 2014/0068797, 2014/0273037, 2014/0273226, 2014/0295556, 2014/0295557, 2014/0349405, 2015/0045546, 2015/0071898, 2015/0071899, and 2015/0071906.

In some embodiments, the guide polynucleotide further comprises a tracrRNA. The "tracrRNA," or trans-activating CRISPR-RNA, forms an RNA duplex with a pre-crRNA, or pre-CRISPR-RNA, and is then cleaved by the RNA-specific ribonuclease RNase III to form a crRNA/tracrRNA hybrid. In some embodiments, the guide polynucleotide comprises the crRNA/tracrRNA hybrid. In some embodiments, the tracrRNA component of the guide polynucleotide activates the Cas9 protein. In some embodiments, activation of the Cas9 protein comprises activating the nuclease activity of Cas9. In some embodiments, activation of the Cas9 protein comprises the Cas9 protein binding to a target polynucleotide sequence, e.g., a TSG locus.

In some embodiments, the guide polynucleotide guides the nuclease to the TSG locus, and the nuclease generates a double-stranded break at the TSG locus. In some embodiments, the guide polynucleotide is a guide RNA. In some embodiments, the nuclease is Cas9. In some embodiments, the double-stranded break at TSG locus inactivates the TSG. In some embodiments, inactivation of the TSG locus confers to the cell, resistance to the toxin. In some embodiments, inactivation of the TSG locus confers to the cell, resistance to the toxin, but also disrupts a normal cellular function of the TSG locus. In some embodiments, the TSG locus encodes a gene that performs a cellular function unrelated to toxin sensitivity. For example, the TSG locus can encode a protein that promotes cell growth or division, a receptor for a signaling molecule (e.g., a molecule by the cell), or a protein that interacts with another protein, organelle, or biomolecule to perform a normal cellular function.

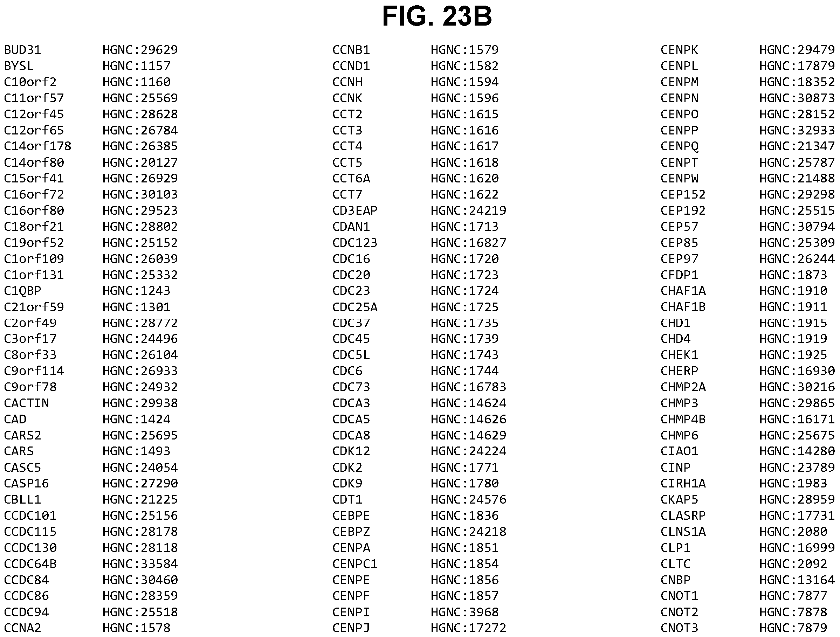

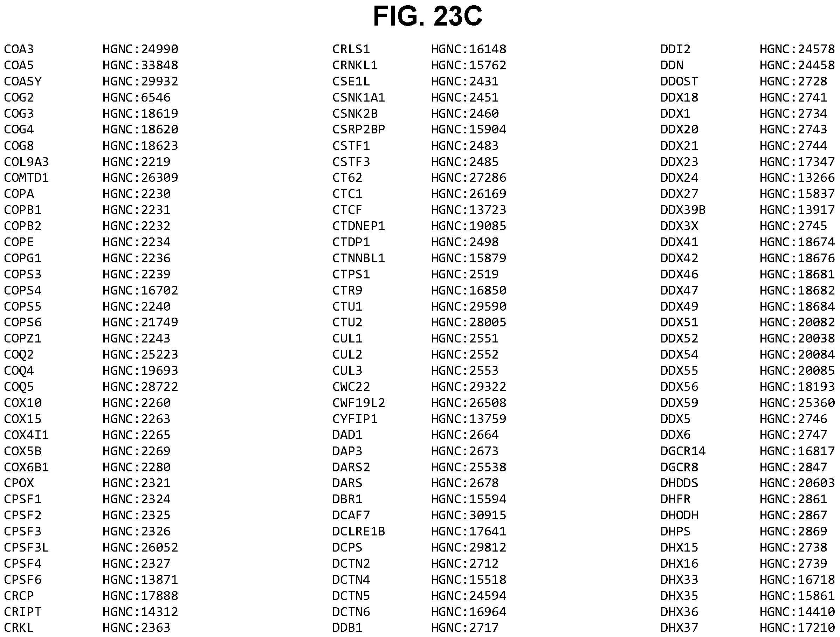

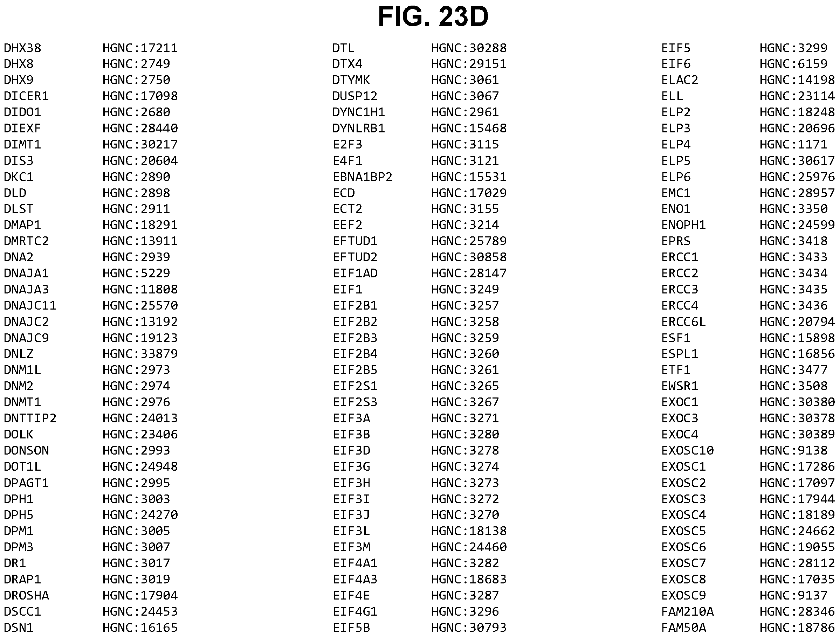

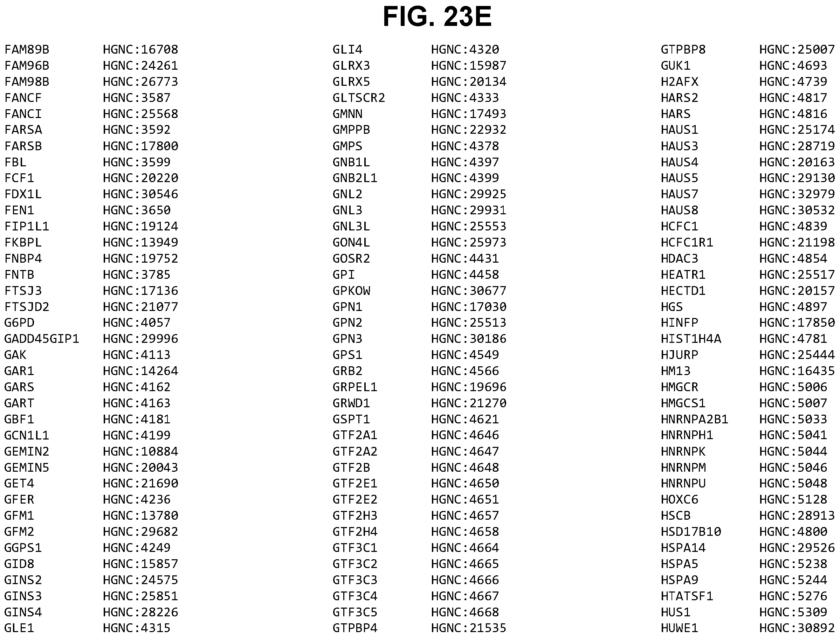

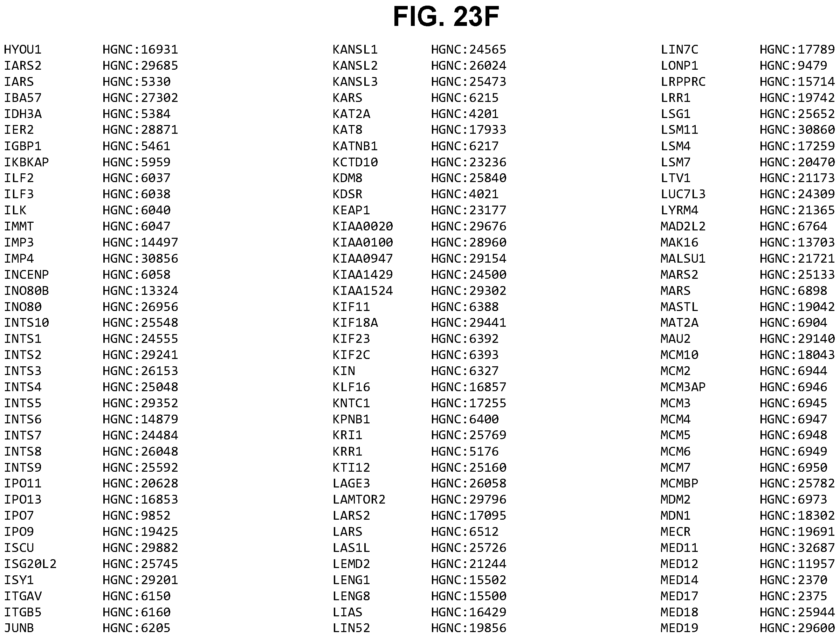

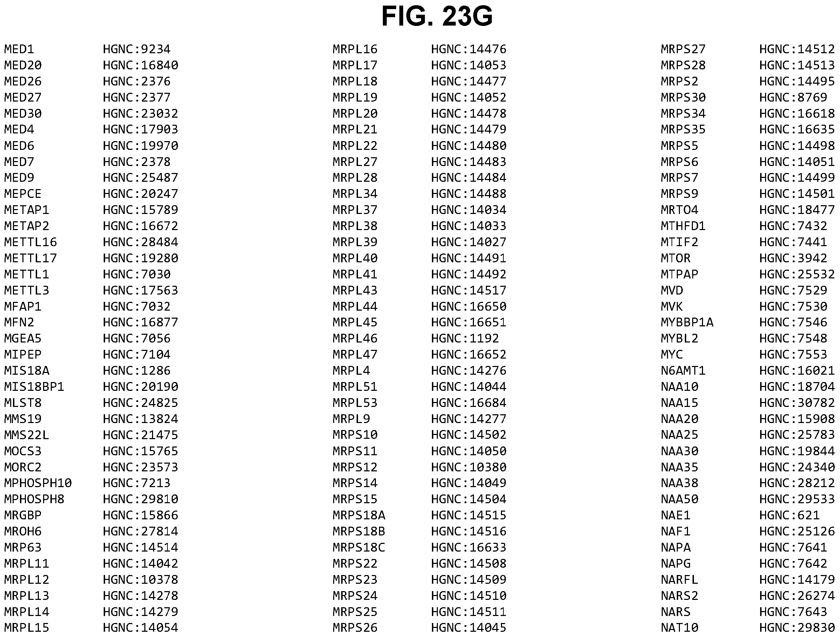

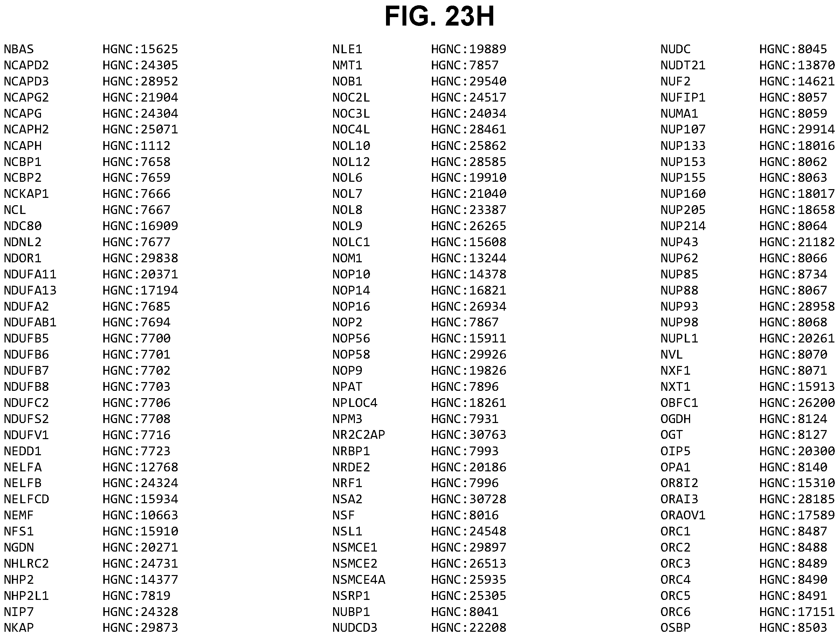

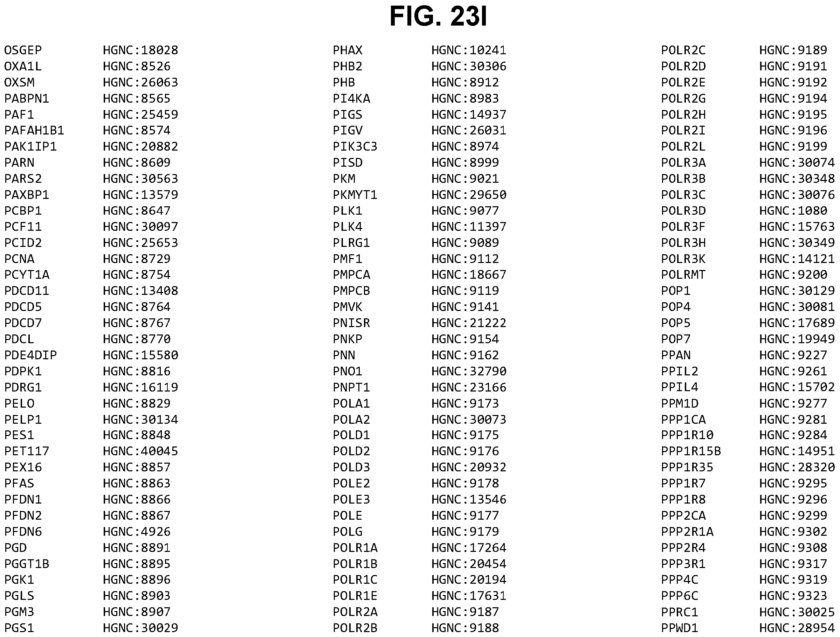

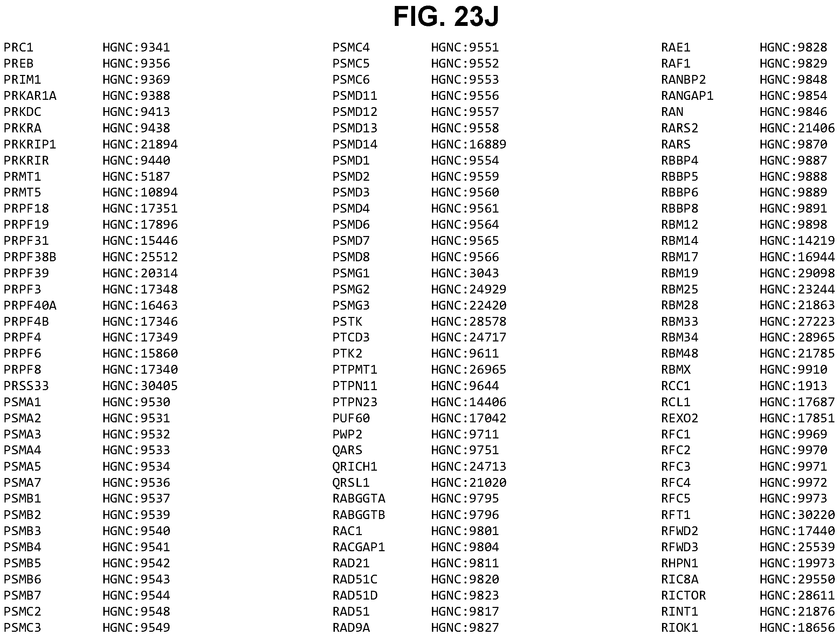

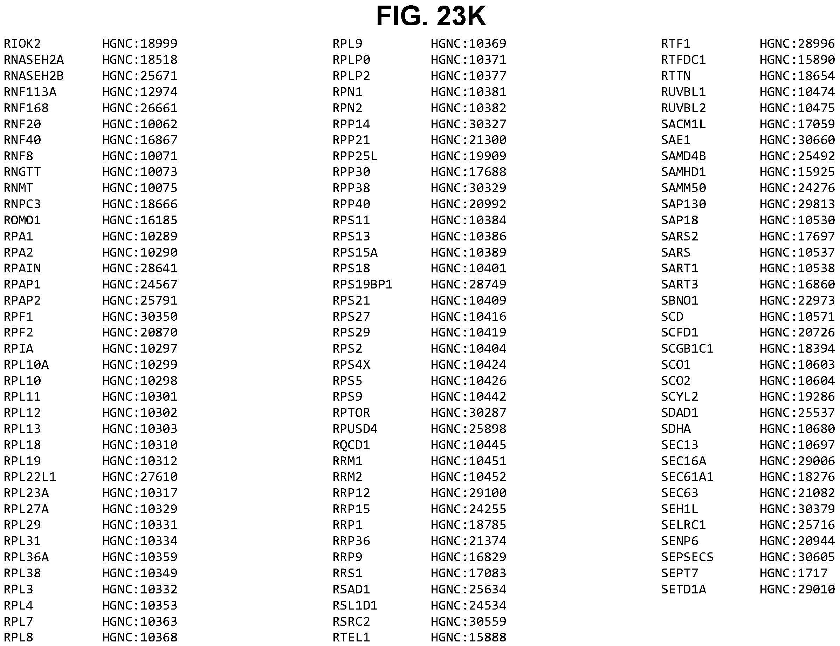

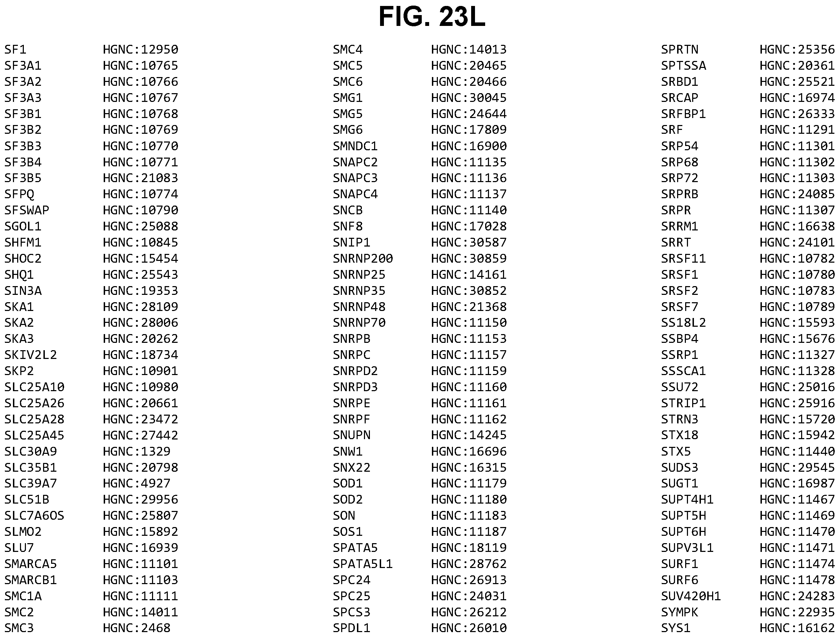

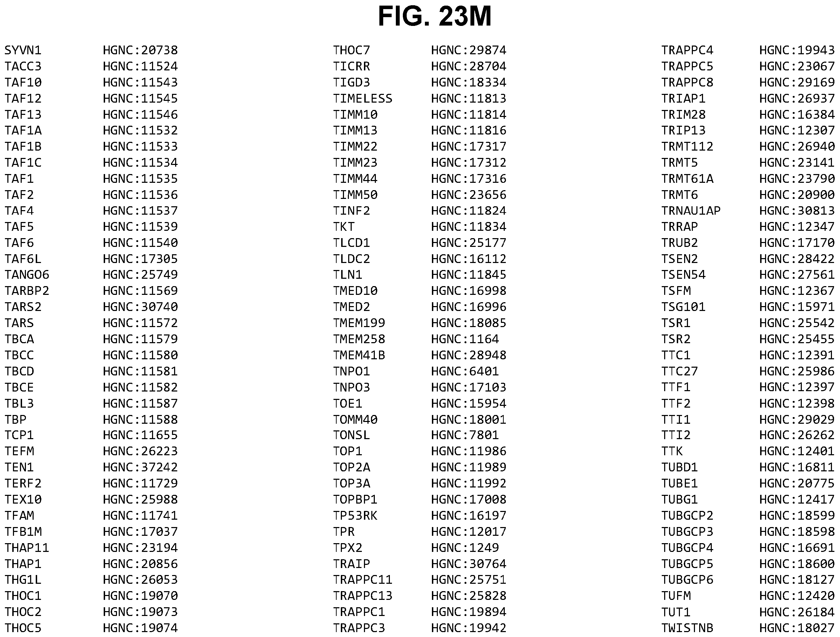

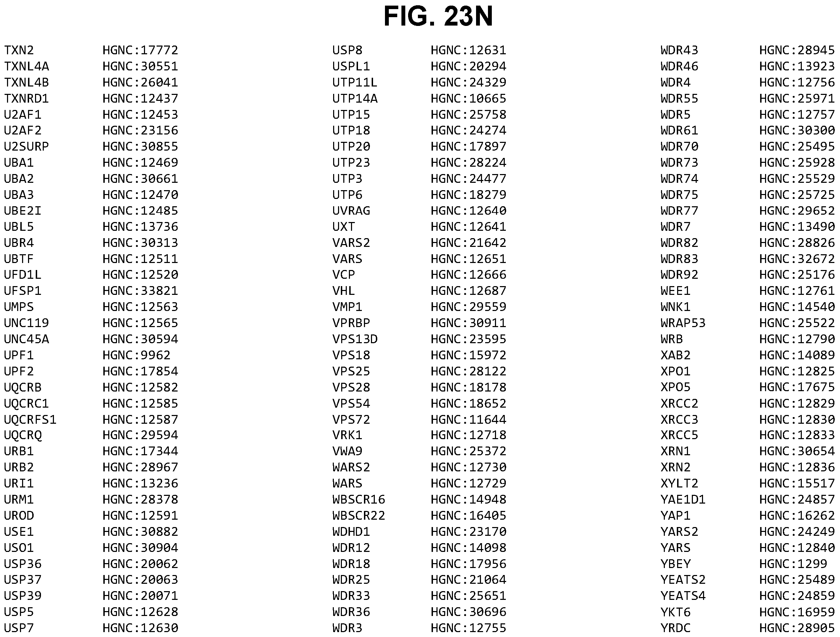

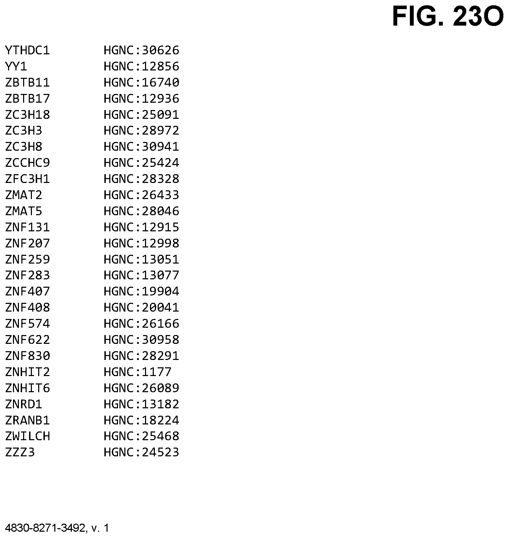

In some embodiments, the TSG is an essential gene. Essential genes are genes of an organism that are thought to be critical for survival in certain conditions. In some embodiments, disruption or deletion of the TSG causes cell death. In some embodiments, the TSG is an auxotrophic gene, i.e., a gene that produces a particular compound required for growth or survival. Examples of auxotrophic genes include genes involved in nucleotide biosynthesis such as adenine, cytosine, guanine, thymine, or uracil; or amino acid biosynthesis such as histidine, leucine, lysine, methionine, or tryptophan. In some embodiments, the TSG is a gene in a metabolic pathway. In some embodiments, the TSG is a gene in an autophagy pathway. In some embodiments, the TSG is a gene in cell division, e.g., mitosis, cytoskeleton organization, or response to stress or stimulus. In some embodiments, the TSG encodes a protein that promotes cell growth or division, a receptor for a signaling molecule (e.g., a molecule by the cell), or a protein that interacts with another protein, organelle, or biomolecule. Exemplary essential genes include, but are not limited to, the genes listed in FIG. 23. Further examples of essential genes are provided in, e.g., Hart et al., Cell 163:1515-1526 (2015); Zhang et al., Microb Cell 2(8):280-287 (2015); and Fraser, Cell Systems 1:381-382 (2015).

Thus, in some embodiments, inactivation (e.g., a double-stranded break in the sequence generated by the nuclease) of the native TSG (i.e., the TSG in the genome of the cell) creates an adverse effect on the cell. In some embodiments, inactivation of the native TSG results in cell death. In such cases, an "exogenous" TSG or portion thereof can be introduced into the cell to compensate for the inactivated native TSG. In some embodiments, a portion of the TSG encodes a polypeptide that performs substantially the same function as the native protein encoded by the TSG. In some embodiments, a portion of the TSG is introduced to complement a partially-inactivated TSG. In some embodiments, the nuclease inactivates a portion of the native TSG (e.g., by disruption of a portion of the coding sequence of the TSG), and the exogenous TSG comprises the disrupted portion of the coding sequence that can be transcribed together with the non-disrupted portion of the native sequence to form a functional TSG. In some embodiments, the exogenous TSG or portion thereof is integrated in the native TSG locus in the genome of the cell. In some embodiments, the exogenous TSG or portion thereof is integrated at a genome locus different from the TSG locus. In some embodiments, the exogenous TSG or portion thereof is integrated by a sequence for genome integration. In some embodiments, the sequence for genome integration is obtained from a retroviral vector. In some embodiments, the sequence for genome integration is obtained from a transposon. In some embodiments, the TSG encodes a CA receptor. In some embodiments, the TSG encodes HB-EGF. In some embodiments, the TSG encodes a receptor for an antibody, e.g., an antibody of an antibody-drug conjugate.

In some embodiment, the exogenous TSG is introduced into the cell in an exogenous polynucleotide. In some embodiments, the exogenous TSG is expressed from the exogenous polynucleotide. In some embodiments, the exogenous polynucleotide is a plasmid. In some embodiments, the exogenous polynucleotide is a donor polynucleotide. In some embodiments, the donor polynucleotide is a vector. Exemplary vectors are provided herein.

In some embodiments, the exogenous polynucleotide is an episomal vector. In some embodiments, the episomal vector is a stable episomal vector, i.e., an episomal vector that remains in the cell. As described herein, episomal vectors include an autonomous DNA replication sequence, which allows the episomal vector to replicate and remain in the cell. In some embodiments, the episomal vector is an artificial chromosome. In some embodiments, the episomal vector is a plasmid.

In some embodiments, the donor polynucleotide comprises 5' and 3' homology arms. In some embodiments, the donor polynucleotide is a donor plasmid. In some embodiments, the 5' and 3' homology arms of the donor polynucleotide are complementary to a portion of the TSG locus in the genome of the cell. Thus, when optimally aligned, the donor polynucleotide overlaps with one or more nucleotides of TSG (e.g., about or at least about 1, 5, 10, 15, 20, 25, 30, 35, 40, 45, 50, 60, 70, 80, 90, or 100 or more nucleotides). In some embodiments, when the donor polynucleotide and a portion of the TSG locus are optimally aligned, the nearest nucleotide of the donor polynucleotide is within about 1, 5, 10, 15, 20, 25, 50, 75, 100, 200, 300, 400, 500, 100, 1500, 2000, 2500, 5000, 10000 or more nucleotides from the TSG locus. In some embodiments, the donor polynucleotide comprising the SOI flanked by the 5' and 3' homology arms is introduced into the cell, and the 5' and 3' homology arms share sequence similarity with either side of the site of integration at the TSG locus. In some embodiments, the 5' and 3' homology arms share at least 60%, at least 70%, at least 80%, at least 85%, at least 90%, at least 91%, at least 92%, at least 93%, at least 94%, at least 95%, at least 96%, at least 97%, at least 98%, at least 99%, or about 100% sequence similarity with either side of the site of integration at the TSG locus. In some embodiments, the TSG encodes a CA receptor. In embodiments, the TSG encodes HB-EGF. In some embodiments, the TSG encodes a receptor for an antibody, e.g., an antibody of an antibody-drug conjugate.

In some embodiments, the 5' and 3' homology arms in the donor polynucleotide promote integration of the donor polynucleotide into the genome by homology-directed repair (HDR). In some embodiments, the donor polynucleotide is integrated by HDR. In some embodiments, the donor polynucleotide is an HDR template. The HDR pathway is an endogenous DNA repair pathway capable of repairing double-stranded breaks. Repairs by the HDR pathway are typically high-fidelity and rely on homologous recombination with an HDR template having homologous regions to the repair site (e.g., 5' and 3' homology arms). In some embodiments, the TSG locus is cut by the nuclease in a manner that facilitates HDR, e.g., by generating cohesive ends. In some embodiments, the TSG locus is cut by the nuclease in a manner that promotes HDR over low-fidelity repair pathways such as non-homologous end joining (NHEJ).

In some embodiments, the donor polypeptide is integrated by NHEJ. The NHEJ pathway is an endogenous DNA repair pathway capable of repairing double-stranded breaks. In general, NHEJ has higher repair efficiency compared with HDR, but with lower fidelity, although errors decrease when the double-stranded breaks in the DNA have compatible cohesive ends or overhangs. In some embodiments, the TSG locus is cut by the nuclease in a manner that decreases errors in NHEJ repair. In some embodiments, the cut in the TSG locus comprises cohesive ends.

In some embodiments, the donor polynucleotide comprises a sequence for genome integration. In some embodiments, the sequence for genome integration at the target locus is obtained from a transposon. As described herein, transposons include a transposon sequence that is recognized by transposase, which then inserts the transposon comprising the transposon sequence and sequence of interest (SOI) into the genome. In some embodiments, the target locus is any genomic locus capable of expressing the SOI without disrupting normal cellular function. Exemplary transposons are described herein. Accordingly, in some embodiments, the donor polynucleotide comprises a functional TSG comprising a mutation in a native coding sequence of the TSG, wherein the mutation confers resistance to the toxin, the SOI, and a transposon sequence for genome integration at the target locus. In some embodiments, the native TSG of the cell is inactivated by the nuclease, and the donor polynucleotide provides a functional TSG capable of compensating the native cellular function of the native TSG, while being resistant to the toxin. In some embodiments, the TSG encodes a CA receptor. In embodiments, the TSG encodes HB-EGF. In some embodiments, the TSG encodes a receptor for an antibody, e.g., an antibody of an antibody-drug conjugate.

In some embodiments, the donor polynucleotide comprises a sequence for genome integration. In some embodiments, the sequence for genome integration at the target locus is obtained from a retroviral vector. As described herein, retroviral vectors include a sequence, typically an LTR, that is recognized by integrase, which then inserts the retroviral vector comprising the LTR and SOI into the genome. In some embodiments, the target locus is any genomic locus capable of expressing the SOI without disrupting normal cellular function. Exemplary retroviral vectors are described herein. Accordingly, in some embodiments, the donor polynucleotide comprises a functional TSG comprising a mutation in a native coding sequence of the TSG, wherein the mutation confers resistance to the toxin, the SOI, and a retroviral vector for genome integration at the target locus. In some embodiments, the native TSG of the cell is inactivated by the nuclease, and the donor polynucleotide provides a functional TSG capable of compensating the native cellular function of the native TSG, while being resistant to the toxin. In some embodiments, the TSG encodes a CA receptor. In embodiments, the TSG encodes HB-EGF. In some embodiments, the TSG encodes a receptor for an antibody, e.g., an antibody of an antibody-drug conjugate.

In some embodiments, an episomal vector is introduced into the cell. In some embodiments, the episomal vector comprises a functional TSG comprising a mutation in a native coding sequence of the TSG, wherein the mutation confers resistance to the toxin, the SOI, and an autonomous DNA replication sequence. As described herein, episomal vectors are non-integrated extrachromosomal plasmids capable of autonomous replication. In some embodiments, the autonomous DNA replication sequence is derived from a viral genomic sequence. In some embodiments, the autonomous DNA replication sequence is derived from a mammalian genomic sequence. In some embodiments, the episomal vector an artificial chromosome or a plasmid. In some embodiments, the plasmid is a viral plasmid. In some embodiments, the viral plasmid is an SV40 vector, a BKV vector, a KSHV vector, or an EBV vector. Thus, in some embodiments, the native TSG of the cell is inactivated by the nuclease, and the episomal vector provides a functional TSG capable of compensating the native cellular function of the native TSG, while being resistant to the toxin. In some embodiments, the TSG encodes a CA receptor. In embodiments, the TSG encodes HB-EGF. In some embodiments, the TSG encodes a receptor for an antibody, e.g., an antibody of an antibody-drug conjugate.

In some embodiments, the toxin sensitive gene (TSG) confers toxin sensitivity to a cell, i.e., the cell is prone to adverse reaction, e.g., stunted growth or death, by the toxin. In some embodiments, the TSG encodes a receptor that binds to the toxin. In some embodiments, the receptor is a CA receptor. A CA receptor is a protein molecule, typically located on the membrane of a cell, which binds to the CA. For example, diphtheria toxin binds to the human heparin binding EGF like growth factor (HB-EGF). A CA receptor can be specific for one CA, or a CA receptor can bind more than one CA. For example, monosialoganglioside (GMi) can act as a receptor for both cholera toxin and *E. coli* heat-labile enterotoxin. Or, more than one CA receptor can bind one CA. For example, the botulinum toxin is believed to bind to different receptors in nerve cells and epithelial cells. In some embodiments, the CA receptor is a receptor that binds to the CA. In some embodiments, the CA receptor is a G-protein coupled receptor. In some embodiments, the CA receptor binds diphtheria toxin. In some embodiments, the CA receptor is a receptor for an antibody, e.g., an antibody of an antibody-drug conjugate. In some embodiments, the TSG locus comprises a gene encoding heparin binding EGF-like growth factor (HB-EGF). HB-EGF and the mechanism by which diphtheria toxin causes cell death are described herein and illustrated, e.g., in FIG. 3A.

In some embodiments, the TSG locus comprises an intron and an exon. In some embodiments, the double-stranded break is generated by the nuclease at the intron. In some embodiments, the double-stranded break is generated by the nuclease at the exon. In some embodiments, the mutation in the native coding sequence of the TSG, e.g., conferring resistance to the toxin, is in the exon. In some embodiments, the donor polynucleotide comprises a native coding sequence of the TSG that comprises a mutation conferring resistance to the toxin. In some embodiments, "native coding sequence" refers to a sequence that is substantially similar to a wild-type sequence encoding a polypeptide, e.g., having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence similarity with the wild-type sequence.

In some embodiments, the donor polynucleotide comprises an exon of a native coding sequence of the TSG, wherein the exon comprises a mutation conferring resistance to the toxin, and the donor polynucleotide additionally comprises a splicing acceptor sequence. As used herein, a "splicing acceptor" or "splicing acceptor sequence" refers to a sequence at the 3' end of an intron, which facilitates the joining of two exons flanking the intron. In some embodiments, the splicing acceptor sequence has at least about 90% sequence identity with a splicing acceptor sequence of the TSG locus in the genome of the cell. In some embodiments, the exon that is integrated at the TSG locus from the donor polynucleotide is joined with an adjacent exon in the genome of the cell when the TSG is transcribed for expression. In some embodiments, the splicing acceptor sequence that is integrated at the TSG locus from the donor polynucleotide facilitates the joining of the exon that is integrated at the TSG locus from the donor polynucleotides with an adjacent exon in the genome of the cell.

In some embodiments, the 5' and 3' homology arms of the donor polynucleotide are complementary to a portion of the TSG locus in the genome of the cell. Thus, when optimally aligned, the donor polynucleotide overlaps with one or more nucleotides of TSG (e.g., about or at least about 1, 5, 10, 15, 20, 25, 30, 35, 40, 45, 50, 60, 70, 80, 90, or 100 or more nucleotides). In some embodiments, when the donor polynucleotide and a portion of the TSG locus are optimally aligned, the nearest nucleotide of the donor polynucleotide is within about 1, 5, 10, 15, 20, 25, 50, 75, 100, 200, 300, 400, 500, 100, 1500, 2000, 2500, 5000, 10000 or more nucleotides from the TSG locus. In some embodiments, the donor polynucleotide comprising the SOI flanked by the 5' and 3' homology arms is introduced into the cell, and the 5' and 3' homology arms share sequence similarity with either side of the site of integration at the TSG locus. In some embodiments, the site of integration at the TSG locus is the nuclease cleavage site, i.e., the site of the double-stranded break. In some embodiments, the 5' and 3' homology arms share at least 60%, at least 70%, at least 80%, at least 85%, at least 90%, at least 91%, at least 92%, at least 93%, at least 94%, at least 95%, at least 96%, at least 97%, at least 98%, at least 99%, or about 100% sequence similarity with either side of the site of integration at the TSG locus. In some embodiments, the site of integration at the TSG locus is the nuclease cleavage site. In some embodiments, the TSG encodes a CA receptor. In embodiments, the TSG encodes HB-EGF.

In some embodiments, the TSG encodes HB-EGF, and the double-stranded break is generated at an intron of the HB-EGF gene. In some embodiments, the TSG encodes HB-EGF, and the double-stranded break is generated at an exon of the HB-EGF gene. In some embodiments, the double-stranded break is at an intron of the HB-EGF gene, and mutation in a native coding sequence of the HB-EGF gene is in an exon of the HB-EGF gene. In some embodiments, the double-stranded break is in an intron of the HB-EGF gene, and the mutation in the native coding sequence of the HB-EGF gene is in the exon that immediately follows the cleaved intron. In some embodiments, the double-stranded break is in an exon of the HB-EGF gene, and the mutation in a native coding sequence of the HB-EGF gene is in the same exon of the HB-EGF gene. In some embodiments, the double-stranded break is in an exon of the HB-EGF gene, and the mutation in a native coding sequence of the HB-EGF gene is in a different exon of the HB-EGF gene.

In some embodiments, the 5' and 3' homology arms of the donor polynucleotide share sequence similarity with HB-EGF at the nuclease cleavage site. In some embodiments, the double-stranded break is at an intron of the HB-EGF, and the 5' and 3' homology arms comprise homology to the sequence of the intron. In some embodiments, the double-stranded break is at an exon of the HB-EGF, and the 5' and 3' homology arms comprise homology to the sequence of the exon. In some embodiments, the 5' and 3' homology arms of the donor polynucleotide are designed to insert the donor polynucleotide at the site of the double-stranded break, e.g., by HDR. In some embodiments, the 5' and 3' homology arms have at least 60%, at least 70%, at least 80%, at least 85%, at least 90%, at least 91%, at least 92%, at least 93%, at least 94%, at least 95%, at least 96%, at least 97%, at least 98%, at least 99%, or about 100% sequence similarity with either side of the nuclease (e.g., Cas9) cleavage site in the HB-EGF.

In some embodiments, the native coding sequence includes one or more changes relative to the wild-type sequence, but the polypeptide encoded by the native coding sequence is substantially similar to the polypeptide encoded by the wild-type sequence, e.g., the amino acid sequences of the polypeptides are at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% identical. In some embodiments, the polypeptides encoded by the native coding sequence and the wild-type sequence have similar structure, e.g., a similar overall shape and fold as determined by the skilled artisan. In some embodiments, a native coding sequence comprises a portion of the wild-type sequence, e.g., the native coding sequence is substantially similar to one or more exons and/or one or more introns of the wild-type sequence encoding a protein, such that the exon and/or intron of the native coding sequence can replace the corresponding wild-type exon and/or intron to encode a polypeptide with substantial sequence identity and/or structure as the wild-type polypeptide. In some embodiments, the native coding sequence comprises a mutation relative to the wild-type sequence. In some embodiments, the mutation in the native coding sequence of the TSG is in the exon.

In some embodiments, the donor polynucleotide comprises a functional TSG comprising a mutation in a native coding sequence of the TSG, wherein the mutation confers resistance to the toxin, the SOI, and a sequence for genome integration at the target locus. The term "functional" TSG refers to a TSG that encodes a polypeptide that is substantially similar to the polypeptide encoded by the native coding sequence. In some embodiments, the functional TSG comprises a sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence similarity to the native coding sequence of the TSG, and also comprises a mutation in the native coding sequence of the TSG that confers resistance to the toxin. In some embodiments, the polypeptide encoded by the functional TSG has a substantially same structure and performs the same cellular function as the polypeptide encoded by the native coding sequence, except that the polypeptide encoded by the functional TSG is resistant to the toxin. In some embodiments, the polypeptide encoded by the functional TSG loses its ability to bind the toxin. In some embodiments, the polypeptide encoded by the functional TSG loses its ability to transport and/or translocate the toxin into the cell.

In some embodiments, the mutation in the native coding sequence of the TSG is a substitution mutation, an insertion, or a deletion. In some embodiments, the mutation is substitution of one nucleotide in the coding sequence of the TSG that changes a single amino acid in the encoded polypeptide sequence. In some embodiments, the mutation is substitution of one or more nucleotides that changes one or more amino acids in the encoded polypeptide sequence. In some embodiments, the mutation is substitution of one or more nucleotides that changes an amino acid codon to a stop codon. In some embodiments, the mutation is a nucleotide insertion in the coding sequence of the TSG that results in insertion of one or more amino acids in the encoded polypeptide sequence. In some embodiments, the mutation is a nucleotide deletion in the coding sequence of the TSG that results in deletion of one or more amino acids in the encoded polypeptide sequence.

In some embodiments, the mutation in the native coding sequence of the TSG is a mutation in a toxin-binding region of a protein encoded by the TSG. In some embodiments, the mutation in the toxin-binding region results in the protein losing its ability to bind to the toxin. In some embodiments, the protein encoded by the functional TSG has a substantially same structure and performs the same cellular function as the protein encoded by the native coding sequence, except that the protein encoded by the functional TSG comprising the mutation is resistant to the toxin. In some embodiments, the protein encoded by the functional TSG loses its ability to bind the toxin. In some embodiments, the protein encoded by the functional TSG loses its ability to transport and/or translocate the toxin into the cell.

In some embodiments, the TSG encodes a receptor that binds to the toxin. In some embodiments, the receptor is a CA receptor. In some embodiments, the TSG encodes a receptor that binds diphtheria toxin. In some embodiments, the TSG encodes heparin binding EGF-like growth factor (HB-EGF). In some embodiments, the mutation in the native coding sequence of the TSG makes the cell resistant to diphtheria toxin.

In some embodiments, the toxin is a naturally-occurring toxin. In some embodiments, the toxin is a synthetic toxicant. In some embodiments, the toxin is a small molecule, a peptide, or a protein. In some embodiments, the toxin is an antibody-drug conjugate. In some embodiments, the toxin is a monoclonal antibody attached a biologically active drug with a chemical linker having a labile bond. In some embodiments, the toxin is a biotoxin. In some embodiments, the toxin is produced by cyanobacteria (cyanotoxin), dinoflagellates (dinotoxin), spiders, snakes, scorpions, frogs, sea creatures such as jellyfish, venomous fish, coral, or the blue-ringed octopus. Examples of toxins include, e.g., diphtheria toxin, botulinum toxin, ricin, apitoxin, Shiga toxin, Pseudomonas exotoxin, and mycotoxin. In some embodiments, the toxin is diphtheria toxin. In some embodiments, the toxin is an antibody-drug conjugate.

In some embodiments, the toxin is toxic to one organism, e.g., a human, but not to another organism, e.g., a mouse. In some embodiments, the toxin is toxic to an organism in one stage of its life cycle (e.g., fetal stage) but not toxic in another life stage of the organism (e.g., adult stage). In some embodiments, the toxin is toxic in one organ of an animal, but not to another organ of the same animal. In some embodiments, the toxin is toxic to a subject (e.g., a human or an animal) in one condition or state (e.g., diseased), but not to the same subject in another condition or state (e.g., healthy). In some embodiments, the toxin is toxic to one cell type, but not to another cell type. In some embodiments, the toxin is toxic to a cell in one cellular state (e.g., differentiated), but not toxic to the same cell in another cellular state (e.g., undifferentiated). In some embodiments, the toxin is toxic to the cell in one environment (e.g., low temperature), but not toxic to the same cell in another environment (e.g., high temperature). In some embodiments, the toxin is toxic to human cells, but not to mouse cells.

In some embodiments, a mutation in one or more of amino acids 100 to 160 of wild-type HB-EGF (SEQ ID NO: 8) confers resistance to diphtheria toxin. In some embodiments, a mutation in one or more of amino acids 105 to 150 of wild-type HB-EGF (SEQ ID NO: 8) confers resistance to diphtheria toxin. In some embodiments, a mutation in or more of amino acids 107 to 148 of wild-type HB-EGF (SEQ ID NO: 8) confers resistance to diphtheria toxin. In some embodiments, a mutation in one or more of amino acids 120 to 145 of wild-type HB-EGF (SEQ ID NO: 8) confers resistance to diphtheria toxin. In some embodiments, a mutation in one or more of amino acids 135 to 143 of wild-type HB-EGF (SEQ ID NO: 8) confers resistance to diphtheria toxin. In some embodiments, a mutation in or more of amino acids 138 to 144 of wild-type HB-EGF (SEQ ID NO: 8) confers resistance to diphtheria toxin. In some embodiments, a mutation in amino acid 141 of wild-type HB-EGF (SEQ ID NO: 8) confers resistance to diphtheria toxin. In some embodiments, the mutation in amino acid 141 of wild-type HB-EGF (SEQ ID NO: 8) is GLU141 to ARG141. In some embodiments, the mutation in amino acid 141 of wild-type HB-EGF (SEQ ID NO: 8) is GLU141 to HIS141. In some embodiments, the mutation in amino acid 141 of wild-type HB-EGF (SEQ ID NO: 8) is GLU141 to LYS141. In some embodiments, a mutation of GLU141 to LYS141 of wild-type HB-EGF (SEQ ID NO: 8) confers resistance to diphtheria toxin.

Accordingly, in some embodiments, the mutation in the native coding sequence of the TSG is a mutation in one or more of amino acids 100 to 160 in HB-EGF (SEQ ID NO: 8). In some embodiments, the mutation in the native coding sequence of the TSG is a mutation in one or more of amino acids 105 to 150 in HB-EGF (SEQ ID NO: 8). In some embodiments, the mutation in the native coding sequence of the TSG is a mutation in one or more of amino acids 107 to 148 in HB-EGF (SEQ ID NO: 8). In some embodiments, the mutation in the native coding sequence of the TSG is a mutation in one or more of amino acids 120 to 145 in HB-EGF (SEQ ID NO: 8). In some embodiments, the mutation in the native coding sequence of the TSG is a mutation in one or more of amino acids 135 to 143 in HB-EGF (SEQ ID NO: 8). In some embodiments, the mutation in the native coding sequence of the TSG is a mutation in one or more of amino acids 138 to 144 of wild-type HB-EGF (SEQ ID NO: 8). In some embodiments, the mutation in the native coding sequence of the TSG is a mutation in amino acid 141 in HB-EGF (SEQ ID NO: 8). In some embodiments, the mutation in the native coding sequence of the TSG is a mutation of GLU141 to LYS141 in HB-EGF (SEQ ID NO: 8). In some embodiments, the mutation in the native coding sequence of the TSG is a mutation of GLU141 to HIS141 in HB-EGF (SEQ ID NO: 8). In some embodiments, the mutation in the native coding sequence of the TSG is a mutation of GLU141 to ARG141 in HB-EGF (SEQ ID NO: 8). In some embodiments, the mutation of GLU141 to LYS141 in HB-EGF (SEQ ID NO: 8) confers resistance to diphtheria toxin.

In some embodiments, the functional TSG in the donor polynucleotide or the episomal vector is resistant to inactivation by the nuclease. In some embodiments, the functional TSG comprises one or more mutations in the native coding sequence of the TSG, wherein the one or more mutations confers resistance to inactivation by the nuclease. In some embodiments, the functional TSG does not bind to the nuclease. In some embodiments, a TSG that does not bind to the nuclease is not prone to cleavage by the nuclease. As discussed herein, nucleases such as certain types of Cas9 may require a PAM sequence at or near the target sequence, in addition to recognition of the target sequence by the guide polynucleotide (e.g., guide RNA) via hybridization. In some embodiments, the Cas9 binds to the PAM sequence prior to initiating nuclease activity. In some embodiments, a target sequence that does not include a PAM in the target sequence or an adjacent or nearby region does not bind to the nuclease. Thus, in some embodiments, a target sequence that does not include a PAM in the target sequence or an adjacent or nearby region is not cleaved by the nuclease, and is therefore resistant to inactivation by the nuclease. In some embodiments, the functional TSG does not comprise a PAM sequence. In some embodiments, a TSG that does not comprise a PAM sequence is resistant to inactivation by the nuclease.

In some embodiments, the PAM is within from about 30 to about 1 nucleotides of the target sequence. In some embodiments, the PAM is within from about 20 to about 2 nucleotides of the target sequence. In some embodiments, the PAM is within from about 10 to about 3 nucleotides of the target sequence. In some embodiments, the PAM is within about 10, about 9, about 8, about 7, about 6, about 5, about 4, about 3, about 2, or about 1 nucleotide of the target sequence. In some embodiments, the PAM is upstream (i.e., in the 5' direction) of the target sequence. In some embodiments, the PAM is downstream (i.e., in the 3' direction) of the target sequence. In some embodiments, the PAM is located within the target sequence.

In some embodiments, the polypeptide encoded by the functional TSG is not capable of hybridizing with the guide polynucleotide. In some embodiments, a TSG that does not hybridize with the guide polynucleotide is not prone to cleavage by the nuclease such as Cas9. As described herein, the guide polynucleotide is capable of hybridizing with a target sequence, i.e., "recognized" by the guide polynucleotide for cleavage by the nuclease such as Cas9. Therefore, a sequence that does not hybridize with a guide polynucleotide is not recognized for cleavage by the nuclease such as Cas9. In some embodiments, a sequence that does not hybridize with a guide polynucleotide is resistant to inactivation by the nuclease. In some embodiments, the guide polynucleotide is capable of hybridizing with the TSG in the genome of the cell, and the functional TSG on the donor polynucleotide or the episomal vector comprises one or more mutations in the native coding sequence of the TSG, such that the guide polynucleotide is (1) capable of hybridizing to the TSG in the genome of the cell, and (2) not capable of hybridizing with the functional TSG on the donor polynucleotide or the episomal vector. In some embodiments, the functional TSG that is resistant to inactivation by the nuclease is introduced into the cell concurrently with the nuclease targeting the ExG in the genome of the cell.

In some embodiments, the SOI comprises a polynucleotide encoding a protein. In some embodiments, the SOI comprises a mutated gene. In some embodiments, the SOI comprises a non-coding sequence, e.g., a microRNA. In some embodiments, the SOI is operably linked to a regulatory element. In some embodiments, the SOI is a regulatory element. In some embodiments, the SOI comprises a resistance cassette, e.g., a gene that confers resistance to an antibiotic. In some embodiments, the SOI comprises a marker, e.g., a selection or screenable marker. In some embodiments, the SOI comprises a marker, e.g., a restriction site, a fluorescent protein, or a selectable marker.

In some embodiments, the SOI comprises a mutation of a wild-type gene in the genome of the cell. In some embodiments, the mutation is a point mutation, i.e., a single-nucleotide substitution. In some embodiments, the mutation comprises multiple-nucleotide substitutions. In some embodiments, the mutation introduces a stop codon. In some embodiments, the mutation comprises a nucleotide insertion in the wild-type sequence. In some embodiments, the mutation comprises a nucleotide deletion in the wild-type sequence. In some embodiments, the mutation comprises a frameshift mutation.

In some embodiments, the population of cells is contacted with the toxin after introduction of the nuclease, guide polynucleotide, and donor polynucleotide or episomal vector. Examples of toxins are provided herein. In some embodiments, the toxin is a naturally-occurring toxin. In some embodiments, the toxin is a synthetic toxicant. In some embodiments, the toxin is a small molecule, a peptide, or a protein. In some embodiments, the toxin is an antibody-drug conjugate. In some embodiments, the toxin is a monoclonal antibody attached a biologically active drug with a chemical linker having a labile bond. In some embodiments, the toxin is a biotoxin. In some embodiments, the toxin is produced by cyanobacteria (cyanotoxin), dinoflagellates (dinotoxin), spiders, snakes, scorpions, frogs, sea creatures such as jellyfish, venomous fish, coral, or the blue-ringed octopus. Examples of toxins include, e.g., diphtheria toxin, botulinum toxin, ricin, apitoxin, Shiga toxin, Pseudomonas exotoxin, and mycotoxin. In some embodiments, the toxin is diphtheria toxin. In some embodiments, the toxin is an antibody-drug conjugate.

In some embodiments, the toxin is toxic to one organism, e.g., a human, but not to another organism, e.g., a mouse. In some embodiments, the toxin is toxic to an organism in one stage of its life cycle (e.g., fetal stage) but not toxic in another life stage of the organism (e.g., adult stage). In some embodiments, the toxin is toxic in one organ of an animal, but not to another organ of the same animal. In some embodiments, the toxin is toxic to a subject (e.g., a human or an animal) in one condition or state (e.g., diseased), but not to the same subject in another condition or state (e.g., healthy). In some embodiments, the toxin is toxic to one cell type, but not to another cell type. In some embodiments, the toxin is toxic to a cell in one cellular state (e.g., differentiated), but not toxic to the same cell in another cellular state (e.g., undifferentiated). In some embodiments, the toxin is toxic to the cell in one environment (e.g., low temperature), but not toxic to the same cell in another environment (e.g., high temperature). In some embodiments, the toxin is toxic to human cells, but not to mouse cells. In some embodiments, the toxin is diphtheria toxin. In some embodiments, the toxin is an antibody-drug conjugate.

In some embodiments, after contacting the population of cells with the toxin, one or more cells resistant to the toxin are selected. In some embodiments, the one or more cells resistant to the toxin are surviving cells. In some embodiments, the surviving cells have (1) an inactivated native TSG (e.g., inactivated by a nuclease-generated double-stranded break), and (2) a functional TSG comprising a mutation conferring toxin resistance. Cells that meet only one of the above two conditions are subject to cell death: if the native TSG is not inactivated, the cell is sensitive to the toxin and dies upon being contacted with the toxin; if the functional TSG is not introduced, the cell lacks the normal cellular function of the TSG and dies from absence of the normal cellular function.

In embodiments comprising introduction of a donor polynucleotide comprising 5' and 3' homology arms (e.g., homologous sequences for HDR), the surviving cells comprise bi-allelic integration of the donor polynucleotide comprising the SOI at the native TSG locus, wherein the native TSG is disrupted by integration of the donor polynucleotide, and wherein the cells comprise a functional, toxin-resistant TSG. Thus, in such embodiments, the one or more cells resistant to the toxin comprise bi-allelic integration of the SOI. In embodiments comprising introduction of a donor polynucleotide comprising a sequence for genome integration (e.g., a transposon, a lentiviral vector sequence, or a retroviral vector sequence) at a target locus, the surviving cells comprise an inactivated native TSG and integration of the donor polynucleotide comprising the functional, toxin-resistant TSG and the SOI at the target locus. In such embodiments, the one or more cells resistant to the toxin comprise the SOI integrated at the target locus. In embodiments comprising introduction of an episomal vector, the surviving cells comprise an inactivated native TSG and a stable episomal vector comprising a functional, toxin-resistant TSG and the SOI. In such embodiments, the one or more cells resistant to the toxin comprise the episomal vector.

### Methods of Providing Diphtheria Toxin Resistance

In some embodiments, the present disclosure provides a method of providing resistance to diphtheria toxin in a human cell, the method comprising introducing into the cell: (i) a base-editing enzyme; and (ii) a guide polynucleotide targeting a heparin-binding EGF-like growth factor (HB-EGF) receptor in the human cell, wherein base-editing enzyme forms a complex with the guide polynucleotide, and wherein the base-editing enzyme is targeted to the HB-EGF and provides a site-specific mutation in the HB-EGF, thereby providing resistance to diphtheria toxin in the human cell.

In some embodiments, the human cell is of a human cell line. In some embodiments, the human cell is a stem cell. The stem cell can be, for example, a pluripotent stem cell, including embryonic stem cell (ESC), adult stem cell, induced pluripotent stem cell (iPSC), tissue specific stem cell (e.g., hematopoietic stem cell), and mesenchymal stem cell (MSC). In some embodiments, the human cell is a differentiated form of any of the cells described herein. In some embodiments, the eukaryotic cell is a cell derived from a primary cell in culture. In some embodiments, the cell is a stem cell or a stem cell line. In some embodiments, the human cell is a hepatocyte such as a human hepatocyte, animal hepatocyte, or a non-parenchymal cell. For example, the eukaryotic cell can be a plateable metabolism qualified human hepatocyte, a plateable induction qualified human hepatocyte, plateable QUALYST TRANSPORTER CERTIFIED human hepatocyte, suspension qualified human hepatocyte (including 10-donor and 20-donor pooled hepatocytes), human hepatic kupffer cells, or human hepatic stellate cells. In some embodiments, the human cell is an immune cell. In some embodiments, the immune cell is a granulocyte, a mast cell, a monocyte, a dendritic cell, a natural killer cell, B cell, a primary T cell, a cytotoxic T cell, a helper T cell, a CD8+ T cell, a CD4+ T cell, or a regulatory T cell.

In some embodiments, the human cell is xenografted or transplanted into a non-human animal. In some embodiments, the non-human animal is a mouse, a rat, a hamster, a guinea pig, a rabbit, or a pig. In some embodiments, the human cell is a cell in a humanized organ of a non-human animal. In some embodiments, a "humanized" organ refers to a human organ that is grown in an animal. In some embodiments, a "humanized" organ refers to an organ that is produced by an animal, depleted of its animal-specific cells, and grafted with human cells. The humanized organ can be immune-compatible with a human. In some embodiments, the humanized organ is liver, kidney, pancreas, heart, lungs, or stomach. Humanized organs are highly useful for the study and modeling of human disease. However, most genetic selection tools cannot be translated to a humanized organ in a host animal, because most selection markers are detrimental to the host animal. Humanized organs are further described in, e.g., Garry et al., Regen Med 11(7):617-619; Garry et al., Circ Res 124:23-25 (2019); and Nguyen et al., Drug Discov Today 23(11):1812-1817 (2018).

The present disclosure provides a highly advantageous selection method that can be used for humanized cells in an animal host by utilizing diphtheria toxin, which is toxic to humans but not to mice. The present methods are not limited, however, to diphtheria toxin, and can be utilized with any compound that is differentially toxic, i.e., toxic to one organism but not toxic to another organism. The present methods also provide diphtheria toxin resistance by manipulating the receptor of the toxin, which may be desirable in circumstances because no toxin enters the cell, in contrast to previous methods focusing on Diphthamide Biosynthesis Protein 2 (DPH2) (*see,* e.g., Picco et al., Sci Rep 5: 14721).

In some embodiments, the humanized organ is produced by transplanting human cells in an animal. In some embodiments, the animal is an immunodeficient mouse. In some embodiments, the animal is an immunodeficient adult mouse. In some embodiments, the humanized organ is produced by repressing one or more animal genes and expressing one or more human genes in an organ of an animal. In some embodiments, the humanized organ is a liver. In some embodiments, the humanized organ is a pancreas. In some embodiments, the humanized organ is a heart. In some embodiments, the humanized organ expresses a human gene encoding a receptor for a cytotoxic agent, i.e., a CA receptor described herein. In some embodiments, the humanized organ is sensitive to a toxin, while the rest of the animal is resistant to the toxin. In some embodiments, the humanized organ expressed human HB-EGF. In some embodiments, the humanized organ is sensitive to diphtheria toxin, while the rest of the animal is resistant to diphtheria toxin. In some embodiments, the humanized organ is a humanized liver in a mouse, wherein the humanized liver is sensitive expresses human HB-EGF and is sensitive to diphtheria toxin, while the rest of the mouse is resistant to HB-EGF. Thus, upon exposure to diphtheria toxin, only the humanized cells in the liver of the mouse would die.

In some embodiments, the base-editing enzyme comprises a DNA-targeting domain and a DNA-editing domain. In some embodiments, the DNA-targeting domain comprises Cas9. Cas9 proteins are described herein. In some embodiments, the Cas9 comprises a mutation in a catalytic domain. In some embodiments, the base-editing enzyme comprises a catalytically inactive Cas9 (dCas9) and a DNA-editing domain. In some embodiments, the nCas9 comprises a mutation at amino acid residue D10 and H840 relative to wild-type Cas9 (numbering relative to SEQ ID NO: 3). In some embodiments, the base-editing enzyme comprises a Cas9 capable of generating single-stranded DNA breaks (nCas9) and a DNA-editing domain. In some embodiments, the nCas9 comprises a mutation at amino acid residue D10 or H840 relative to wild-type Cas9 (numbering relative to SEQ ID NO: 3). In some embodiments, the Cas9 comprises a polypeptide having at least 80%, at least 85%, at least 90%, at least 91%, at least 92%, at least 93%, at least 94%, at least 95%, at least 96%, at least 97%, at least 98%, at least 99%, or about 100% sequence identity to SEQ ID NO: 3. In some embodiments, the Cas9 comprises a polypeptide having at least 90% sequence identity to SEQ ID NO: 3. In some embodiments, the Cas9 comprises a polypeptide having at least 80%, at least 85%, at least 90%, at least 91%, at least 92%, at least 93%, at least 94%, at least 95%, at least 96%, at least 97%, at least 98%, at least 99%, or about 100% sequence identity to SEQ ID NO: 4. In some embodiments, the Cas9 comprises a polypeptide having at least 90% sequence identity to SEQ ID NO: 4.

In some embodiments, the DNA-editing domain comprises a deaminase. In some embodiments, the deaminase is cytidine deaminase or adenosine deaminase. In some embodiments, the deaminase is cytidine deaminase. In some embodiments, the deaminase is adenosine deaminase. In some embodiments, the deaminase is an apolipoprotein B mRNA-editing complex (APOBEC) deaminase, an activation-induced cytidine deaminase (AID), an ACF1/ASE deaminase, an ADAT deaminase, or an ADAR deaminase. In some embodiments, the deaminase is an apolipoprotein B mRNA-editing complex (APOBEC) family deaminase. In some embodiments, the deaminase is APOBEC1.

In some embodiments, the base-editing enzyme further comprises a DNA glycosylase inhibitor domain. In some embodiments, the DNA glycosylase inhibitor is uracil DNA glycosylase inhibitor (UGI). In general, DNA glycosylases such as uracil DNA glycosylase are part of the base excision repair pathway and perform error-free repair upon detecting a U:G mismatch (wherein the "U" is generated from deamination of a cytosine), converting the U back to the wild-type sequence and effectively "undoing" the base-editing. Thus, addition of a DNA glycosylase inhibitor (e.g., uracil DNA glycosylase inhibitor) inhibits the base excision repair pathway, increasing the base-editing efficiency. Non-limiting examples of DNA glycosylases include OGG1, MAGI, and UNG. DNA glycosylase inhibitors can be small molecules or proteins. For example, protein inhibitors of uracil DNA glycosylase are described in Mol et al., Cell 82:701-708 (1995); Serrano-Heras et al., J Biol Chem 281:7068-7074 (2006); and New England Biolabs Catalog No. M0281S and M0281L (neb.com/products/m0281-uracil-glycosylase-inhibitor-ugi). Small molecule inhibitors of DNA glycosylases are described in, e.g., Huang et al., J Am Chem Soc 131(4):1344-1345 (2009); Jacobs et al., PLoS One 8(12):e81667 (2013); Donley et al., ACS Chem Biol 10(10):2334-2343 (2015); Tahara et al., J Am Chem Soc 140(6):2105-2114 (2018).

Thus, in some embodiments, the base-editing enzyme of the present disclosure comprises nCas9 and cytidine deaminase. In some embodiments, the base-editing enzyme of the present disclosure comprises nCas9 and adenosine deaminase. In some embodiments, the base-editing enzyme comprises a polypeptide having at least 90% sequence identity to SEQ ID NO: 6. In some embodiments, the base-editing enzyme comprises a polypeptide having at least 50%, at least 60%, at least 70%, at least 80%, at least 85%, or at least 90% sequence identity to SEQ ID NO: 6. In some embodiments, the base-editing enzyme is at least 91%, at least 92%, at least 93%, at least 94%, at least 95%, at least 96%, at least 97%, at least 98%, at least 99%, or about 100% identical to SEQ ID NO: 6. In some embodiments, a polynucleotide encoding the base-editing enzyme is at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or about 100% identical to SEQ ID NO: 5. In some embodiments, the base-editing enzyme is BE3.

In some embodiments, the methods of the present disclosure comprising introducing into a human cell, a guide polynucleotide targeting a HB-EGF receptor in the human cell. In some embodiments, the guide polynucleotide forms a complex with the base-editing enzyme, and the base-editing enzyme is targeted to the HB-EGF by the guide polynucleotide and provides a site-specific mutation in HB-EGF, thereby providing resistance to diphtheria toxin in the human cell.

In some embodiments, the guide polynucleotide is an RNA molecule. The guide polynucleotide can be introduced into the target cell as an isolated molecule, e.g., an RNA molecule, or is introduced into the cell using an expression vector containing DNA encoding the guide polynucleotide, e.g., the RNA guide polynucleotide. In some embodiments, the guide polynucleotide is 10 to 150 nucleotides. In some embodiments, the guide polynucleotide is 20 to 120 nucleotides. In some embodiments, the guide polynucleotide is 30 to 100 nucleotides. In some embodiments, the guide polynucleotide is 40 to 80 nucleotides. In some embodiments, the guide polynucleotide is 50 to 60 nucleotides. In some embodiments, the guide polynucleotide is 10 to 35 nucleotides. In some embodiments, the guide polynucleotide is 15 to 30 nucleotides. In some embodiments, the guide polynucleotide is 20 to 25 nucleotides.

In some embodiments, an RNA guide polynucleotide comprises at least two nucleotide segments: at least one "DNA-binding segment" and at least one "polypeptide-binding segment." By "segment" is meant a part, section, or region of a molecule, e.g., a contiguous stretch of nucleotides of guide polynucleotide molecule. The definition of "segment," unless otherwise specifically defined, is not limited to a specific number of total base pairs.

In some embodiments, the guide polynucleotide includes a DNA-binding segment. In some embodiments, the DNA-binding segment of the guide polynucleotide comprises a nucleotide sequence that is complementary to a specific sequence within a target polynucleotide. In some embodiments, the DNA-binding segment of the guide polynucleotide hybridizes with a gene encoding a cytotoxic agent (CA) receptor in a target cell. In some embodiments, the DNA-binding segment of the guide polynucleotide hybridizes with the gene encoding HB-EGF. In some embodiments, the DNA-binding segment of the guide polynucleotide hybridizes with a target polynucleotide sequence in a target cell. Target cells, including various types of eukaryotic cells, are described herein.

In some embodiments, the guide polynucleotide includes a polypeptide-binding segment. In some embodiments, the polypeptide-binding segment of the guide polynucleotide binds the DNA-targeting domain of a base-editing enzyme of the present disclosure. In some embodiments, the polypeptide-binding segment of the guide polynucleotide binds to Cas9 of a base-editing enzyme. In some embodiments, the polypeptide-binding segment of the guide polynucleotide binds to dCas9 of a base-editing enzyme. In some embodiments, the polypeptide-binding segment of the guide polynucleotide binds to nCas9 of a base-editing enzyme. Various RNA guide polynucleotides which bind to Cas9 proteins are described in, e.g., U.S. Patent Publication Nos. 2014/0068797, 2014/0273037, 2014/0273226, 2014/0295556, 2014/0295557, 2014/0349405, 2015/0045546, 2015/0071898, 2015/0071899, and 2015/0071906.

In some embodiments, the guide polynucleotide further comprises a tracrRNA. The "tracrRNA," or trans-activating CRISPR-RNA, forms an RNA duplex with a pre-crRNA, or pre-CRISPR-RNA, and is then cleaved by the RNA-specific ribonuclease RNase III to form a crRNA/tracrRNA hybrid. In some embodiments, the guide polynucleotide comprises the crRNA/tracrRNA hybrid. In some embodiments, the tracrRNA component of the guide polynucleotide activates the Cas9 protein. In some embodiments, activation of the Cas9 protein comprises activating the nuclease activity of Cas9. In some embodiments, activation of the Cas9 protein comprises the Cas9 protein binding to a target polynucleotide sequence.

In some embodiments, the sequence of the guide polynucleotide is designed to target the base-editing enzyme to a specific location in a target polynucleotide sequence. Various tools and programs are available to facilitate design of such guide polynucleotides, e.g., the Benchling base editor design guide (benchling.com/editor#create/crispr), and BE-Designer and BE-Analyzer from CRISPRRGEN Tools (*see* Hwang et al., bioRxiv dx.doi.org/10.1101/373944, first published July 22, 2018).

In some embodiments, the DNA-binding segment of the guide polynucleotide hybridizes with a gene encoding HB-EGF, and the polypeptide-binding segment of the guide polynucleotide forms a complex with the base-editing enzyme by binding to the DNA-targeting domain of the base-editing enzyme. In some embodiments, the DNA-binding segment of the guide polynucleotide hybridizes with a gene encoding HB-EGF, and the polypeptide-binding segment of the guide polynucleotide forms a complex with the base-editing enzyme by binding to Cas9 of the base-editing enzyme. In some embodiments, the DNA-binding segment of the guide polynucleotide hybridizes with a gene encoding HB-EGF, and the polypeptide-binding segment of the guide polynucleotide forms a complex with the base-editing enzyme by binding to dCas9 of the base-editing enzyme. In some embodiments, the DNA-binding segment of the guide polynucleotide hybridizes with a gene encoding HB-EGF, and the polypeptide-binding segment of the guide polynucleotide forms a complex with the base-editing enzyme by binding to nCas9 of the base-editing enzyme.

In some embodiments, the complex is targeted to HB-EGF by the guide polynucleotide, and the base-editing enzyme of the complex introduces a mutation in HB-EGF. In some embodiments, the mutation in the HB-EGF is introduced by the base-editing domain of the base-editing enzyme of the complex. In some embodiments, the mutation in HB-EGF forms a diphtheria toxin-resistant cell. In some embodiments, the mutation is a cytidine (C) to thymine (T) point mutation. In some embodiments, the mutation is an adenine (A) to guanine (G) point mutation. The specific location of the mutation in the HB-EGF may be directed by, e.g., design of the guide polynucleotide using tools such as, e.g., the Benchling base editor design guide, BE-Designer, and BE-Analyzer described herein. In some embodiments, the guide polynucleotide is an RNA polynucleotide. In some embodiments, the guide polynucleotide further comprises a tracrRNA sequence.

In some embodiments, the site-specific mutation is in a region of the HB-EGF that binds diphtheria toxin. In some embodiments, a mutation in the EGF-like domain of HB-EGF confers resistance to diphtheria toxin. In some embodiments, a charge-reversal mutation of an amino acid at or near the diphtheria toxin binding site of HB-EGF confers resistance to diphtheria toxin. In some embodiments, the charge-reversal mutation is replacement of a negatively-charged residue, e.g., Glu or Asp, with a positively-charged residue, e.g., Lys or Arg. In some embodiments, the charge-reversal mutation is replacement of a positively-charged residue, e.g., Lys or Arg, with a negatively-charged residue, e.g., Glu or Asp. In some embodiments, a polarity-reversal mutation of an amino acid at or near the diphtheria toxin binding site of HB-EGF confers resistance to diphtheria toxin. In some embodiments, the polarity-reversal mutation is replacement of a polar amino acid residue, e.g., Gln or Asn, with a non-polar amino acid residue, e.g., Ala, Val, or Ile. In some embodiments, the polarity-reversal mutation is replacement of a non-polar amino acid residue, e.g., Ala, Val, or Ile, with a polar amino acid residue, e.g., Gln or Asn. In some embodiments, the mutation is replacement of a relatively small amino acid residue, e.g., Gly or Ala, at or near the diphtheria toxin binding site of HB-EGF with a "bulky" amino acid residue, e.g., Trp. In some embodiments, the mutation of a small residue to a bulky residue blocks the binding pocket and prevents diphtheria toxin from binding, thereby conferring resistance.

In some embodiments, a mutation in one or more of amino acids 100 to 160 of wild-type HB-EGF (SEQ ID NO: 8) confers resistance to diphtheria toxin. In some embodiments, a mutation in one or more of amino acids 105 to 150 of wild-type HB-EGF (SEQ ID NO: 8) confers resistance to diphtheria toxin. In some embodiments, a mutation in or more of amino acids 107 to 148 of wild-type HB-EGF (SEQ ID NO: 8) confers resistance to diphtheria toxin. In some embodiments, a mutation in one or more of amino acids 120 to 145 of wild-type HB-EGF (SEQ ID NO: 8) confers resistance to diphtheria toxin. In some embodiments, a mutation in one or more of amino acids 135 to 143 of wild-type HB-EGF (SEQ ID NO: 8) confers resistance to diphtheria toxin. In some embodiments, a mutation in or more of amino acids 138 to 144 of wild-type HB-EGF (SEQ ID NO: 8) confers resistance to diphtheria toxin. In some embodiments, a mutation in amino acid 141 of wild-type HB-EGF (SEQ ID NO: 8) confers resistance to diphtheria toxin. In some embodiments, the mutation in amino acid 141 of wild-type HB-EGF (SEQ ID NO: 8) is GLU141 to ARG141. In some embodiments, the mutation in amino acid 141 of wild-type HB-EGF (SEQ ID NO: 8) is GLU141 to HIS141. In some embodiments, the mutation in amino acid 141 of wild-type HB-EGF (SEQ ID NO: 8) is GLU141 to LYS141. In some embodiments, a mutation of GLU141 to LYS141 of wild-type HB-EGF (SEQ ID NO: 8) confers resistance to diphtheria toxin.

Accordingly, in some embodiments, the site-specific mutation is in one or more of amino acids 100 to 160 in HB-EGF (SEQ ID NO: 8). In some embodiments, the site-specific mutation is in one or more of amino acids 105 to 150 in HB-EGF (SEQ ID NO: 8). In some embodiments, the site-specific mutation is in one or more of amino acids 107 to 148 in HB-EGF (SEQ ID NO: 8). In some embodiments, the site-specific mutation is in one or more of amino acids 120 to 145 in HB-EGF (SEQ ID NO: 8). In some embodiments, the site-specific mutation is in one or more of amino acids 135 to 143 in HB-EGF (SEQ ID NO: 8). In some embodiments, the site-specific mutation is in one or more of amino acids 138 to 144 of wild-type HB-EGF (SEQ ID NO: 8). In some embodiments, the site-specific mutation is in amino acid 141 in HB-EGF (SEQ ID NO: 8). In some embodiments, the site-specific mutation is a mutation of GLU141 to LYS141 in HB-EGF (SEQ ID NO: 8). In some embodiments, the site-specific mutation is a mutation of GLU141 to HIS141 in HB-EGF (SEQ ID NO: 8). In some embodiments, the site-specific mutation is a mutation of GLU141 to ARG141 in HB-EGF (SEQ ID NO: 8). In some embodiments, the mutation of GLU141 to LYS141 in HB-EGF (SEQ ID NO: 8) confers resistance to diphtheria toxin.

### Selection Methods Using an Essential Gene

The methods of the present disclosure are not necessarily limited to selection with a toxin-sensitive gene. Essential genes are genes of an organism that are thought to be critical for survival in certain conditions. In embodiments, an essential gene is used as the "selection" site in the co-targeting enrichment strategies described herein.

In some embodiments, the present disclosure provides a method of integrating and enriching a sequence of interest (SOI) into a mammalian genome target locus in a genome of a cell, the method comprising: (a) introducing into a population of cells: (i) a nuclease capable of generating a double-stranded break; (ii) a guide polynucleotide that forms a complex with the nuclease and is capable of hybridizing with an essential gene (ExG) locus in the genome of the cell and inactivating the same; and (iii) a donor polynucleotide comprising: (1) a functional ExG gene containing comprising a mutation in the a native coding sequence of the ExG, wherein the mutation confers resistance to inactivation by the guide polynucleotide, (2) the SOI, and (3) a sequence for genome integration at the target locus; wherein introduction of (i), (ii), and (iii) results in inactivation of the ExG in the genome of the cell by the nuclease, and integration of the donor polynucleotide in the target locus; (b) cultivating the cells; and (c) selecting one or more surviving cells, wherein the one or more surviving cells comprise the SOI integrated at the target locus.

FIG. 13 illustrates an embodiment of the present methods. In FIG. 13, a CRISPR-Cas complex is introduced into a cell targeting ExG, an essential gene for cell survival. A vector containing a gene of interest (GOI) and a modified ExG*, which is resistant to targeting by the CRISPR-Cas complex, is also introduced into the cell. As a result, cells that have the cleaved ExG (indicated by the star in the ExG sequence) and the successfully introduced vector with the ExG* are able to survive, while the cells that do not have the vector die as a result of the lacking ExG. The guide RNA of the CRISPR-Cas complex can be designed and selected such that it has a close to 100% efficiency for the ExG in the genome of the cell, and/or multiple guide RNAs can be used for targeting the same ExG. Alternatively or additionally, multiple rounds of selecting surviving cells and introducing the CRISPR-Cas complex can be performed, such that the surviving cells are more likely to lack the genomic copy of the ExG, and survive due to presence of the ExG* (and thus, the GOI). Thus, the surviving cells are enriched for the having the GOI.

In some embodiments, the essential gene is a gene that is required for an organism to survive. In some embodiments, disruption or deletion of an essential gene causes cell death. In some embodiments, the essential gene is an auxotrophic gene, i.e., a gene that produces a particular compound required for growth or survival. Examples of auxotrophic genes include genes involved in nucleotide biosynthesis such as adenine, cytosine, guanine, thymine, or uracil; or amino acid biosynthesis such as histidine, leucine, lysine, methionine, or tryptophan. In some embodiments, the essential gene is a gene in a metabolic pathway. In some embodiments, the essential gene is a gene in an autophagy pathway. In some embodiments, the essential gene is a gene in cell division, e.g., mitosis, cytoskeleton organization, or response to stress or stimulus. In some embodiments, the essential gene encodes a protein that promotes cell growth or division, a receptor for a signaling molecule (e.g., a molecule by the cell), or a protein that interacts with another protein, organelle, or biomolecule. Exemplary essential genes include, but are not limited to, the genes listed in FIG. 23. Further examples of essential genes are provided in, e.g., Hart et al., Cell 163: 1515-1526 (2015); Zhang et al., Microb Cell 2(8):280-287 (2015); and Fraser, Cell Systems 1:381-382 (2015).

In some embodiments, the nuclease capable of generating double-stranded breaks is Cas9. In some embodiments, Cas9 proteins generate site-specific breaks in a nucleic acid. In some embodiments, Cas9 proteins generate site-specific double-stranded breaks in DNA. The ability of Cas9 to target a specific sequence in a nucleic acid (i.e., site specificity) is achieved by the Cas9 complexing with a guide polynucleotide (e.g., guide RNA) that hybridizes with the specified sequence (e.g., the ExG locus). In some embodiments, the Cas9 is a Cas9 variant described in U.S. Provisional Application No. 62/728,184, filed September 7, 2018.

In some embodiments, the Cas9 is capable of generating cohesive ends. Cas9 capable of generating cohesive ends are described in, e.g., PCT/US2018/061680, filed November 16, 2018. In some embodiments, the Cas9 capable of generating cohesive ends is a dimeric Cas9 fusion protein. Binding domains and cleavage domains of naturally-occurring nucleases (such as, e.g., Cas9), as well as modular binding domains and cleavage domains that can be fused to create nucleases binding specific target sites, are well known to those of skill in the art. For example, the binding domain of RNA-programmable nucleases (e.g., Cas9), or a Cas9 protein having an inactive DNA cleavage domain, can be used as a binding domain (e.g., that binds a gRNA to direct binding to a target site) to specifically bind a desired target site, and fused or conjugated to a cleavage domain, for example, the cleavage domain of the endonuclease FokI, to create an engineered nuclease cleaving the target site. Cas9-FokI fusion proteins are further described in, e.g., U.S. Patent Publication No. 2015/0071899 and Guilinger et al., "Fusion of catalytically inactive Cas9 to FokI nuclease improves the specificity of genome modification," Nature Biotechnology 32: 577-582 (2014).

In some embodiments, the Cas9 comprises the polypeptide sequence of SEQ ID NO: 3 or 4. In some embodiments, the Cas9 comprises at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence identity to SEQ ID NO: 3 or 4. In some embodiments, the Cas9 is SEQ ID NO: 3 or 4.

In some embodiments, the guide polynucleotide is an RNA polynucleotide. The RNA molecule that binds to CRISPR-Cas components and targets them to a specific location within the target DNA is referred to herein as "RNA guide polynucleotide," "guide RNA," "gRNA," "small guide RNA," "single-guide RNA," or "sgRNA" and may also be referred to herein as a "DNA-targeting RNA." The guide polynucleotide can be introduced into the target cell as an isolated molecule, e.g., an RNA molecule, or is introduced into the cell using an expression vector containing DNA encoding the guide polynucleotide, e.g., the RNA guide polynucleotide. In some embodiments, the guide polynucleotide is 10 to 150 nucleotides. In some embodiments, the guide polynucleotide is 20 to 120 nucleotides. In some embodiments, the guide polynucleotide is 30 to 100 nucleotides. In some embodiments, the guide polynucleotide is 40 to 80 nucleotides. In some embodiments, the guide polynucleotide is 50 to 60 nucleotides. In some embodiments, the guide polynucleotide is 10 to 35 nucleotides. In some embodiments, the guide polynucleotide is 15 to 30 nucleotides. In some embodiments, the guide polynucleotide is 20 to 25 nucleotides.

In some embodiments, an RNA guide polynucleotide comprises at least two nucleotide segments: at least one "DNA-binding segment" and at least one "polypeptide-binding segment." By "segment" is meant a part, section, or region of a molecule, e.g., a contiguous stretch of nucleotides of guide polynucleotide molecule. The definition of "segment," unless otherwise specifically defined, is not limited to a specific number of total base pairs.

In some embodiments, the guide polynucleotide includes a DNA-binding segment. In some embodiments, the DNA-binding segment of the guide polynucleotide comprises a nucleotide sequence that is complementary to a specific sequence within a target polynucleotide. In some embodiments, the DNA-binding segment of the guide polynucleotide hybridizes with an essential gene locus (ExG) in a cell. Various types of cells, e.g., eukaryotic cells, are described herein.

In some embodiments, the guide polynucleotide includes a polypeptide-binding segment. In some embodiments, the polypeptide-binding segment of the guide polynucleotide binds the DNA-targeting domain of a nuclease of the present disclosure. In some embodiments, the polypeptide-binding segment of the guide polynucleotide binds to Cas9. In some embodiments, the polypeptide-binding segment of the guide polynucleotide binds to dCas9. In some embodiments, the polypeptide-binding segment of the guide polynucleotide binds to nCas9. Various RNA guide polynucleotides which bind to Cas9 proteins are described in, e.g., U.S. Patent Publication Nos. 2014/0068797, 2014/0273037, 2014/0273226, 2014/0295556, 2014/0295557, 2014/0349405, 2015/0045546, 2015/0071898, 2015/0071899, and 2015/0071906.

In some embodiments, the guide polynucleotide further comprises a tracrRNA. The "tracrRNA," or trans-activating CRISPR-RNA, forms an RNA duplex with a pre-crRNA, or pre-CRISPR-RNA, and is then cleaved by the RNA-specific ribonuclease RNase III to form a crRNA/tracrRNA hybrid. In some embodiments, the guide polynucleotide comprises the crRNA/tracrRNA hybrid. In some embodiments, the tracrRNA component of the guide polynucleotide activates the Cas9 protein. In some embodiments, activation of the Cas9 protein comprises activating the nuclease activity of Cas9. In some embodiments, activation of the Cas9 protein comprises the Cas9 protein binding to a target polynucleotide sequence, e.g., an ExG locus.

In some embodiments, the guide polynucleotide guides the nuclease to the ExG locus, and the nuclease generates a double-stranded break at the ExG locus. In some embodiments, the guide polynucleotide is a guide RNA. In some embodiments, the nuclease is Cas9. In some embodiments, the double-stranded break at ExG locus inactivates the ExG. In some embodiments, inactivation of the ExG locus disrupts an essential cellular function. In some embodiments, inactivation of the ExG locus prevents cell division. In some embodiments, inactivation of the ExG locus causes cell death.

In some embodiments, an "exogenous" ExG or portion thereof can be introduced into the cell to compensate for the inactivated native ExG. In some embodiments, the exogenous ExG is a functional ExG. The term "functional" ExG refers to an ExG that encodes a polypeptide that is substantially similar to the polypeptide encoded by the native coding sequence. In some embodiments, the functional ExG comprises a sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence similarity to the native coding sequence of the ExG, and also comprises a mutation in the native coding sequence of the ExG that confers resistance to inactivation by the nuclease. In some embodiments, the functional ExG is resistant to inactivation by the nuclease, and the polypeptide encoded by the functional ExG has a substantially same structure and performs the same cellular function as the polypeptide encoded by the native coding sequence.

In some embodiments, a portion of the ExG encodes a polypeptide that performs substantially the same function as the native protein encoded by the ExG. In some embodiments, a portion of the ExG is introduced to complement a partially-inactivated ExG. In some embodiments, the nuclease inactivates a portion of the native ExG (e.g., by disruption of a portion of the coding sequence of the ExG), and the exogenous ExG comprises the disrupted portion of the coding sequence that can be transcribed together with the non-disrupted portion of the native sequence to form a functional ExG. In some embodiments, the exogenous ExG or portion thereof is integrated in the native ExG locus in the genome of the cell. In some embodiments, the exogenous ExG or portion thereof is integrated at a genome locus different from the ExG locus.

In some embodiments, the functional ExG does not bind to the nuclease. In some embodiments, an ExG that does not bind to the nuclease is not prone to cleavage by the nuclease. As discussed herein, nucleases such as certain types of Cas9 may require a PAM sequence at or near the target sequence, in addition to recognition of the target sequence by the guide polynucleotide (e.g., guide RNA) via hybridization. In some embodiments, the Cas9 binds to the PAM sequence prior to initiating nuclease activity. In some embodiments, a target sequence that does not include a PAM in the target sequence or an adjacent or nearby region does not bind to the nuclease. Thus, in some embodiments, a target sequence that does not include a PAM in the target sequence or an adjacent or nearby region is not cleaved by the nuclease, and is therefore resistant to inactivation by the nuclease. In some embodiments, the mutation in the native coding sequence of the ExG removes a PAM sequence. In some embodiments, an ExG that does not comprise a PAM sequence is resistant to inactivation by the nuclease.

In some embodiments, the PAM is within from about 30 to about 1 nucleotides of the target sequence. In some embodiments, the PAM is within from about 20 to about 2 nucleotides of the target sequence. In some embodiments, the PAM is within from about 10 to about 3 nucleotides of the target sequence. In some embodiments, the PAM is within about 10, about 9, about 8, about 7, about 6, about 5, about 4, about 3, about 2, or about 1 nucleotide of the target sequence. In some embodiments, the PAM is upstream (i.e., in the 5' direction) of the target sequence. In some embodiments, the PAM is downstream (i.e., in the 3' direction) of the target sequence. In some embodiments, the PAM is located within the target sequence.

In some embodiments, the polypeptide encoded by the functional ExG is not capable of hybridizing with the guide polynucleotide. In some embodiments, an ExG that does not hybridize with the guide polynucleotide is not prone to cleavage by the nuclease such as Cas9. As described herein, the guide polynucleotide is capable of hybridizing with a target sequence, i.e., "recognized" by the guide polynucleotide for cleavage by the nuclease such as Cas9. Therefore, a sequence that does not hybridize with a guide polynucleotide is not recognized for cleavage by the nuclease such as Cas9. In some embodiments, a sequence that does not hybridize with a guide polynucleotide is resistant to inactivation by the nuclease. In some embodiments, the guide polynucleotide is capable of hybridizing with the ExG in the genome of the cell, and the functional ExG on the donor polynucleotide or the episomal vector comprises a mutation in the native coding sequence of the ExG, such that the guide polynucleotide is (1) capable of hybridizing to the ExG in the genome of the cell, and (2) not capable of hybridizing with the functional ExG on the donor polynucleotide or the episomal vector. In some embodiments, the functional ExG that is resistant to inactivation by the nuclease is introduced into the cell concurrently with the nuclease targeting the ExG in the genome of the cell.

In some embodiments, the functional ExG includes one or more mutations relative to the wild-type sequence, but the polypeptide encoded by the native coding sequence is substantially similar to the polypeptide encoded by the wild-type sequence, e.g., the amino acid sequences of the polypeptides are at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or about 100% identical. In some embodiments, the polypeptides encoded by the functional ExG and the wild-type ExG have similar structure, e.g., a similar overall shape and fold as determined by the skilled artisan. In some embodiments, the functional ExG comprises a portion of the wild-type sequence. In some embodiments, the functional ExG comprises a mutation relative to the wild-type sequence. In some embodiments, the functional ExG comprises a mutation in a native coding sequence of the ExG, wherein the mutation confers resistance to inactivation by the nuclease.

In some embodiments, the mutation in the native coding sequence of the ExG is a substitution mutation, an insertion, or a deletion. In some embodiments, the substitution mutation is substitution of one or more nucleotides in the polynucleotide sequence, but the encoded amino acid sequence remains unchanged. In some embodiments, the substitution mutation replaces one or more nucleotides to change a codon for an amino acid into a degenerate codon for the same amino acid. For example, the native coding sequence may comprise the sequence "CAT," which encodes for histidine, and the mutation may change the sequence to "CAC," which also encodes for histidine. In some embodiments, the substitution mutation replaces one or more nucleotides to change an amino acid into a different amino acid, but with similar properties such that the overall structure of the encoded polypeptide, or the overall function of the protein, is not affected. For example, the substitution mutation may result in a change from leucine to isoleucine, glutamine to asparagine, glutamate to aspartate, serine to threonine, etc.

In some embodiment, the exogenous ExG or portion thereof (e.g., the ExG comprising a mutation in a native coding sequence of the ExG, wherein the mutation confers resistance to the inactivation by the nuclease) is introduced into the cell in an exogenous polynucleotide. In some embodiments, the exogenous ExG is expressed from the exogenous polynucleotide. In some embodiments, the exogenous polynucleotide is a plasmid. In some embodiments, the exogenous polynucleotide is a donor polynucleotide. In some embodiments, the donor polynucleotide is a vector. Exemplary vectors are provided herein.

In some embodiments, the exogenous ExG or portion thereof on the donor polynucleotide is integrated into the genome of the cell by a sequence for genome integration. In some embodiments, the sequence for genome integration is obtained from a retroviral vector. In some embodiments, the sequence for genome integration is obtained from a transposon.

In some embodiments, the donor polynucleotide comprises a sequence for genome integration. In some embodiments, the sequence for genome integration at the target locus is obtained from a transposon. As described herein, transposons include a transposon sequence that is recognized by transposase, which then inserts the transposon comprising the transposon sequence and sequence of interest (SOI) into the genome. In some embodiments, the target locus is any genomic locus capable of expressing the SOI without disrupting normal cellular function. Exemplary transposons are described herein. Accordingly, in some embodiments, the donor polynucleotide comprises a functional ExG comprising a mutation in a native coding sequence of the ExG, wherein the mutation confers resistance to the inactivation by the nuclease, the SOI, and a transposon sequence for genome integration at the target locus. In some embodiments, the native ExG of the cell is inactivated by the nuclease, and the donor polynucleotide provides a functional ExG capable of compensating the native cellular function of the native ExG, while being resistant to inactivation by the nuclease.

In some embodiments, the donor polynucleotide comprises a sequence for genome integration. In some embodiments, the sequence for genome integration at the target locus is obtained from a retroviral vector. As described herein, retroviral vectors include a sequence, typically an LTR, that is recognized by integrase, which then inserts the retroviral vector comprising the LTR and SOI into the genome. In some embodiments, the target locus is any genomic locus capable of expressing the SOI without disrupting normal cellular function. Exemplary retroviral vectors are described herein. Accordingly, in some embodiments, the donor polynucleotide comprises a functional ExG comprising a mutation in a native coding sequence of the ExG, wherein the mutation confers resistance to the inactivation by the nuclease, the SOI, and a retroviral vector for genome integration at the target locus. In some embodiments, the native ExG of the cell is inactivated by the nuclease, and the donor polynucleotide provides a functional ExG capable of compensating the native cellular function of the native ExG, while being resistant to inactivation by the nuclease.

In some embodiments, the exogenous polynucleotide is an episomal vector. In some embodiments, the episomal vector is a stable episomal vector, i.e., an episomal vector that remains in the cell. As described herein, episomal vectors include an autonomous DNA replication sequence, which allows the episomal vector to replicate and remain in the cell. In some embodiments, the episomal vector is an artificial chromosome. In some embodiments, the episomal vector is a plasmid.

In some embodiments, an episomal vector is introduced into the cell. In some embodiments, the episomal vector comprises a functional ExG comprising a mutation in a native coding sequence of the ExG, wherein the mutation confers resistance to the inactivation by the nuclease, the SOI, and an autonomous DNA replication sequence. As described herein, episomal vectors are non-integrated extrachromosomal plasmids capable of autonomous replication. In some embodiments, the autonomous DNA replication sequence is derived from a viral genomic sequence. In some embodiments, the autonomous DNA replication sequence is derived from a mammalian genomic sequence. In some embodiments, the episomal vector an artificial chromosome or a plasmid. In some embodiments, the plasmid is a viral plasmid. In some embodiments, the viral plasmid is an SV40 vector, a BKV vector, a KSHV vector, or an EBV vector. Thus, in some embodiments, the native ExG of the cell is inactivated by the nuclease, and the episomal vector provides a functional ExG capable of compensating the native cellular function of the native ExG, while being resistant to inactivation by the nuclease.

In some embodiments, the SOI comprises a polynucleotide encoding a protein. In some embodiments, the SOI comprises a mutated gene. In some embodiments, the SOI comprises a non-coding sequence, e.g., a microRNA. In some embodiments, the SOI is operably linked to a regulatory element. In some embodiments, the SOI is a regulatory element. In some embodiments, the SOI comprises a resistance cassette, e.g., a gene that confers resistance to an antibiotic. In some embodiments, the SOI comprises a marker, e.g., a selection or screenable marker. In some embodiments, the SOI comprises a marker, e.g., a restriction site, a fluorescent protein, or a selectable marker.

In some embodiments, the SOI comprises a mutation of a wild-type gene in the genome of the cell. In some embodiments, the mutation is a point mutation, i.e., a single-nucleotide substitution. In some embodiments, the mutation comprises multiple-nucleotide substitutions. In some embodiments, the mutation introduces a stop codon. In some embodiments, the mutation comprises a nucleotide insertion in the wild-type sequence. In some embodiments, the mutation comprises a nucleotide deletion in the wild-type sequence. In some embodiments, the mutation comprises a frameshift mutation.

In some embodiments, the guide polynucleotide has a targeting efficiency of greater than 80%, greater than 85%, greater than 90%, greater than 95%, or about 100% for the ExG in the genome of the cell. Targeting efficiency may be measured by, e.g., the percentage of cells that have inactivated ExG in the population of cells. Guide polynucleotides can be designed and selected to have increased efficiency using various design tools such as, e.g., Chop Chop (chopchop.cbu.uib.no); CasFinder (arep.med.harvard.edu/CasFinder); E-CRISP (e-crisp.org/E-CRISP/designcrispr.html); CRISPR-ERA (crispr-era.stanford.edu/index.jsp); etc.

In some embodiments, more than one guide polynucleotide is introduced into the population of cells, wherein each guide polynucleotide forms a complex with the nuclease, and wherein each guide polynucleotide hybridizes to a different region of the ExG. In some embodiments, multiple guide polynucleotides are used to increase the efficiency of inactivating the ExG in the genome of the cell. For example, a first guide polynucleotide can target a 5' region of the ExG, a second guide polynucleotide can target an internal region of the ExG, and a third guide polynucleotide can target a 3' region of the ExG. The targeting efficiency of each guide polynucleotide may vary; however, nuclease cleavage at any of the 5', 3', or internal regions inactivates the ExG and thus, utilizing more than one guide polynucleotide targeting the same gene may increase the overall efficiency. In some embodiments, at least 2, at least 3, at least 4, at least 5, at least 6, at least 7, at least 8, at least 9, at least 10, at least 15, or at least 20 different guide polynucleotides are introduced into the population of cells.

In some embodiments, the surviving cells comprise a mixture of cells that comprise the ExG* and SOI integrated at the target locus or on the episomal vector, and cells that comprise ExG not inactivated by the nuclease, for example, due to inherent inefficiencies in the nuclease or unsuccessful introduction of the nuclease and/or guide polynucleotide into the cell. Thus, in some embodiments, one or more steps of the methods are repeated to enrich for surviving cells comprising the desired SOI. Repeated introduction of the nuclease and guide polynucleotide can increase the likelihood that the ExG in the genome of the cell is inactivated, thereby enriching for surviving cells comprising the ExG* and SOI integrated at the target locus or on the episomal vector.

Thus, in embodiments of methods for integrating a SOI in a target locus, the methods further comprise introducing the nuclease capable of generating a double-stranded break and the guide polynucleotide that forms with a complex and is capable of hybridizing with an ExG in the genome of the cell, into the selected one or more surviving cells, to enrich for surviving cells comprising the SOI integrated at the target locus. In embodiments of methods for introducing a stable episomal vector into a cell, the method further comprises introducing the nuclease capable of generating a double-stranded break and the guide polynucleotide that forms with a complex and is capable of hybridizing with an ExG in the genome of the cell, into the selected one or more surviving cells, to enrich for surviving cells comprising the episomal vector.

In some embodiments, the nuclease and guide polynucleotide are introduced into the surviving cells for multiple rounds of enrichment. In some embodiments, the nuclease and guide polynucleotide are introduced for 2, 3, 4, 5, 6, 7, 8, 9, 10, 15, 20, or more than 20 rounds of enrichment. Each round of targeting increases the likelihood that the surviving cells comprise the SOI, i.e., enriches for surviving cells comprising the SOI integrated at the target locus or the episomal vector.

### Sequences

Sequences of various polynucleotides and polypeptides are provided herein.
Polynucleotide sequence of the Cas9 protein from *Streptococcus pyogenes* (SpCas9; SEQ ID NO: 1): 
ATGGACTATAAGGACCACGACGGAGACTACAAGGATCATGATATTGATTACAAAGACGATGACGATAAGATGGCCCC
AAAGAAGAAGCGGAAGGTCGGTATCCACGGAGTCCCAGCAGCCGACAAGAAGTACAGCATCGGCCTGGACATCGGCA
CCAACTCTGTGGGCTGGGCCGTGATCACCGACGAGTACAAGGTGCCCAGCAAGAAATTCAAGGTGCTGGGCAACACC
GACCGGCACAGCATCAAGAAGAACCTGATCGGAGCCCTGCTGTTCGACAGCGGCGAAACAGCCGAGGCCACCCGGCT
GAAGAGAACCGCCAGAAGAAGATACACCAGACGGAAGAACCGGATCTGCTATCTGCAAGAGATCTTCAGCAACGAGA
TGGCCAAGGTGGACGACAGCTTCTTCCACAGACTGGAAGAGTCCTTCCTGGTGGAAGAGGATAAGAAGCACGAGCGG
CACCCCATCTTCGGCAACATCGTGGACGAGGTGGCCTACCACGAGAAGTACCCCACCATCTACCACCTGAGAAAGAA
ACTGGTGGACAGCACCGACAAGGCCGACCTGCGGCTGATCTATCTGGCCCTGGCCCACATGATCAAGTTCCGGGGCC
ACTTCCTGATCGAGGGCGACCTGAACCCCGACAACAGCGACGTGGACAAGCTGTTCATCCAGCTGGTGCAGACCTAC
AACCAGCTGTTCGAGGAAAACCCCATCAACGCCAGCGGCGTGGACGCCAAGGCCATCCTGTCTGCCAGACTGAGCAA
GAGCAGACGGCTGGAAAATCTGATCGCCCAGCTGCCCGGCGAGAAGAAGAATGGCCTGTTCGGAAACCTGATTGCCC
TGAGCCTGGGCCTGACCCCCAACTTCAAGAGCAACTTCGACCTGGCCGAGGATGCCAAACTGCAGCTGAGCAAGGAC
ACCTACGACGACGACCTGGACAACCTGCTGGCCCAGATCGGCGACCAGTACGCCGACCTGTTTCTGGCCGCCAAGAA
CCTGTCCGACGCCATCCTGCTGAGCGACATCCTGAGAGTGAACACCGAGATCACCAAGGCCCCCCTGAGCGCCTCTA
TGATCAAGAGATACGACGAGCACCACCAGGACCTGACCCTGCTGAAAGCTCTCGTGCGGCAGCAGCTGCCTGAGAAG
TACAAAGAGATTTTCTTCGACCAGAGCAAGAACGGCTACGCCGGCTACATTGACGGCGGAGCCAGCCAGGAAGAGTT
CTACAAGTTCATCAAGCCCATCCTGGAAAAGATGGACGGCACCGAGGAACTGCTCGTGAAGCTGAACAGAGAGGACC
TGCTGCGGAAGCAGCGGACCTTCGACAACGGCAGCATCCCCCACCAGATCCACCTGGGAGAGCTGCACGCCATTCTG
CGGCGGCAGGAAGATTTTTACCCATTCCTGAAGGACAACCGGGAAAAGATCGAGAAGATCCTGACCTTCCGCATCCC
CTACTACGTGGGCCCTCTGGCCAGGGGAAACAGCAGATTCGCCTGGATGACCAGAAAGAGCGAGGAAACCATCACCC
CCTGGAACTTCGAGGAAGTGGTGGACAAGGGCGCTTCCGCCCAGAGCTTCATCGAGCGGATGACCAACTTCGATAAG
AACCTGCCCAACGAGAAGGTGCTGCCCAAGCACAGCCTGCTGTACGAGTACTTCACCGTGTATAACGAGCTGACCAA
AGTGAAATACGTGACCGAGGGAATGAGAAAGCCCGCCTTCCTGAGCGGCGAGCAGAAAAAGGCCATCGTGGACCTGC
TGTTCAAGACCAACCGGAAAGTGACCGTGAAGCAGCTGAAAGAGGACTACTTCAAGAAAATCGAGTGCTTCGACTCC
GTGGAAATCTCCGGCGTGGAAGATCGGTTCAACGCCTCCCTGGGCACATACCACGATCTGCTGAAAATTATCAAGGA
CAAGGACTTCCTGGACAATGAGGAAAACGAGGACATTCTGGAAGATATCGTGCTGACCCTGACACTGTTTGAGGACA
GAGAGATGATCGAGGAACGGCTGAAAACCTATGCCCACCTGTTCGACGACAAAGTGATGAAGCAGCTGAAGCGGCGG
AGATACACCGGCTGGGGCAGGCTGAGCCGGAAGCTGATCAACGGCATCCGGGACAAGCAGTCCGGCAAGACAATCCT
GGATTTCCTGAAGTCCGACGGCTTCGCCAACAGAAACTTCATGCAGCTGATCCACGACGACAGCCTGACCTTTAAAG
AGGACATCCAGAAAGCCCAGGTGTCCGGCCAGGGCGATAGCCTGCACGAGCACATTGCCAATCTGGCCGGCAGCCCC
GCCATTAAGAAGGGCATCCTGCAGACAGTGAAGGTGGTGGACGAGCTCGTGAAAGTGATGGGCCGGCACAAGCCCGA
 
GAACATCGTGATCGAAATGGCCAGAGAGAACCAGACCACCCAGAAGGGACAGAAGAACAGCCGCGAGAGAATGAAGC
GGATCGAAGAGGGCATCAAAGAGCTGGGCAGCCAGATCCTGAAAGAACACCCCGTGGAAAACACCCAGCTGCAGAAC
GAGAAGCTGTACCTGTACTACCTGCAGAATGGGCGGGATATGTACGTGGACCAGGAACTGGACATCAACCGGCTGTC
CGACTACGATGTGGACCATATCGTGCCTCAGAGCTTTCTGAAGGACGACTCCATCGACAACAAGGTGCTGACCAGAA
GCGACAAGAACCGGGGCAAGAGCGACAACGTGCCCTCCGAAGAGGTCGTGAAGAAGATGAAGAACTACTGGCGGCAG
CTGCTGAACGCCAAGCTGATTACCCAGAGAAAGTTCGACAATCTGACCAAGGCCGAGAGAGGCGGCCTGAGCGAACT
GGATAAGGCCGGCTTCATCAAGAGACAGCTGGTGGAAACCCGGCAGATCACAAAGCACGTGGCACAGATCCTGGACT
CCCGGATGAACACTAAGTACGACGAGAATGACAAGCTGATCCGGGAAGTGAAAGTGATCACCCTGAAGTCCAAGCTG
GTGTCCGATTTCCGGAAGGATTTCCAGTTTTACAAAGTGCGCGAGATCAACAACTACCACCACGCCCACGACGCCTA
CCTGAACGCCGTCGTGGGAACCGCCCTGATCAAAAAGTACCCTAAGCTGGAAAGCGAGTTCGTGTACGGCGACTACA
AGGTGTACGACGTGCGGAAGATGATCGCCAAGAGCGAGCAGGAAATCGGCAAGGCTACCGCCAAGTACTTCTTCTAC
AGCAACATCATGAACTTTTTCAAGACCGAGATTACCCTGGCCAACGGCGAGATCCGGAAGCGGCCTCTGATCGAGAC
AAACGGCGAAACCGGGGAGATCGTGTGGGATAAGGGCCGGGATTTTGCCACCGTGCGGAAAGTGCTGAGCATGCCCC
AAGTGAATATCGTGAAAAAGACCGAGGTGCAGACAGGCGGCTTCAGCAAAGAGTCTATCCTGCCCAAGAGGAACAGC
GATAAGCTGATCGCCAGAAAGAAGGACTGGGACCCTAAGAAGTACGGCGGCTTCGACAGCCCCACCGTGGCCTATTC
TGTGCTGGTGGTGGCCAAAGTGGAAAAGGGCAAGTCCAAGAAACTGAAGAGTGTGAAAGAGCTGCTGGGGATCACCA
TCATGGAAAGAAGCAGCTTCGAGAAGAATCCCATCGACTTTCTGGAAGCCAAGGGCTACAAAGAAGTGAAAAAGGAC
CTGATCATCAAGCTGCCTAAGTACTCCCTGTTCGAGCTGGAAAACGGCCGGAAGAGAATGCTGGCCTCTGCCGGCGA
ACTGCAGAAGGGAAACGAACTGGCCCTGCCCTCCAAATATGTGAACTTCCTGTACCTGGCCAGCCACTATGAGAAGC
TGAAGGGCTCCCCCGAGGATAATGAGCAGAAACAGCTGTTTGTGGAACAGCACAAGCACTACCTGGACGAGATCATC
GAGCAGATCAGCGAGTTCTCCAAGAGAGTGATCCTGGCCGACGCTAATCTGGACAAAGTGCTGTCCGCCTACAACAA
GCACCGGGATAAGCCCATCAGAGAGCAGGCCGAGAATATCATCCACCTGTTTACCCTGACCAATCTGGGAGCCCCTG
CCGCCTTCAAGTACTTTGACACCACCATCGACCGGAAGAGGTACACCAGCACCAAAGAGGTGCTGGACGCCACCCTG
ATCCACCAGAGCATCACCGGCCTGTACGAGACACGGATCGACCTGTCTCAGCTGGGAGGCGACAAAAGGCCGGCGGC
CACGAAAAAGGCCGGCCAGGCAAAAAAGAAAAAGTAA
Polynucleotide sequence of the Cas9 protein from *Francisella novicida* (FnCas9; SEQ ID NO: 2): 
ATGTACCCATACGATGTTCCAGATTACGCTTCGCCGAAGAAAAAGCGCAAGGTCGAAGCGTCCAATTTTAAGATCCT
GCCTATCGCAATCGACCTGGGCGTCAAGAATACTGGCGTGTTTAGTGCTTTTTATCAGAAGGGGACCTCACTGGAGA
GACTGGACAATAAGAACGGAAAAGTGTATGAACTGTCCAAGGATTCTTACACTCTGCTGATGAACAATAGGACCGCA
CGGAGACACCAGAGGCGAGGAATTGACAGGAAACAGCTGGTGAAGCGCCTGTTCAAACTGATCTGGACAGAGCAGCT
GAACCTGGAATGGGATAAGGACACTCAGCAGGCCATCAGCTTCCTGTTTAATCGACGGGGATTCTCTTTTATTACTG
ACGGCTATAGTCCTGAGTACCTGAACATCGTGCCAGAACAGGTCAAGGCAATCCTGATGGACATTTTCGACGATTAT
AATGGCGAGGACGATCTGGATTCCTACCTGAAACTGGCCACAGAGCAAGAGAGTAAGATCAGCGAAATCTACAACAA
GCTGATGCAGAAGATCCTGGAGTTCAAGCTGATGAAACTGTGCACCGACATCAAGGACGATAAAGTGAGTACCAAGA
CACTGAAAGAGATCACAAGCTACGAGTTCGAACTGCTGGCCGATTATCTGGCTAACTACAGCGAATCCCTGAAGACC
 
CAGAAATTTTCCTACACAGACAAGCAGGGCAATCTGAAAGAGCTGTCTTACTACCACCATGATAAGTACAACATCCA
GGAGTTCCTGAAGAGACACGCCACCATCAATGACAGGATTCTGGATACACTGCTGACTGACGATCTGGACATCTGGA
ACTTCAACTTCGAGAAGTTCGATTTCGACAAGAACGAGGAAAAACTGCAGAATCAGGAAGATAAGGACCACATTCAG
GCTCATCTGCACCATTTCGTGTTTGCAGTCAATAAGATCAAAAGCGAGATGGCATCCGGCGGGCGCCATCGAAGCCA
GTACTTCCAGGAAATCACCAACGTGCTGGACGAGAACAATCACCAGGAAGGCTACCTGAAAAACTTCTGTGAGAATC
TGCATAACAAGAAGTACAGCAATCTGTCCGTGAAGAATCTGGTCAACCTGATTGGAAATCTGTCCAACCTGGAACTG
AAGCCCCTGCGCAAATACTTCAACGACAAGATCCACGCTAAAGCAGACCATTGGGATGAGCAGAAGTTTACTGAAAC
CTATTGCCACTGGATTCTGGGCGAGTGGCGGGTGGGGGTCAAGGATCAGGACAAGAAAGACGGCGCAAAGTATTCTT
ACAAGGACCTGTGTAACGAGCTGAAGCAGAAAGTGACTAAGGCCGGGCTGGTGGACTTCCTGCTGGAGCTGGACCCC
TGCCGAACCATTCCACCTTACCTGGACAACAATAACAGAAAGCCACCCAAATGTCAGAGCCTGATCCTGAATCCCAA
GTTTCTGGATAATCAGTATCCTAACTGGCAGCAGTACCTGCAGGAGCTGAAGAAACTGCAGTCAATCCAGAACTACC
TGGACAGCTTCGAAACCGATCTGAAGGTGCTGAAAAGCTCCAAGGACCAGCCTTACTTCGTCGAGTACAAGTCTAGT
AACCAGCAGATCGCTTCCGGCCAGCGGGATTACAAGGATCTGGACGCAAGAATCCTGCAGTTCATTTTTGACAGGGT
GAAGGCCTCTGATGAGCTGCTGCTGAACGAAATCTATTTCCAGGCAAAGAAACTGAAGCAGAAAGCCTCAAGCGAGC
TGGAAAAGCTGGAGTCCTCTAAGAAACTGGACGAAGTGATCGCTAACTCTCAGCTGAGTCAGATTCTGAAGTCTCAG
CACACAAATGGAATCTTCGAGCAGGGCACTTTTCTGCATCTGGTGTGCAAATACTATAAGCAGCGACAGAGAGCCAG
GGACAGCCGCCTGTACATCATGCCTGAATATCGATACGATAAGAAACTGCACAAGTACAACAACACCGGCCGCTTTG
ACGATGACAACCAGCTGCTGACATATTGTAATCATAAGCCCCGGCAGAAAAGATACCAGCTGCTGAACGACCTGGCA
GGAGTGCTGCAGGTCTCTCCTAATTTTCTGAAGGATAAAATCGGGTCCGATGACGATCTGTTCATTTCTAAGTGGCT
GGTGGAGCACATCCGGGGCTTTAAGAAGGCCTGCGAAGACAGCCTGAAAATCCAGAAGGATAACAGGGGACTGCTGA
ATCATAAGATCAACATTGCACGCAATACCAAGGGCAAATGCGAGAAAGAAATCTTCAACCTGATCTGTAAGATTGAG
GGGAGCGAAGACAAGAAAGGGAATTATAAGCACGGACTGGCCTACGAGCTGGGAGTGCTGCTGTTCGGAGAGCCAAA
CGAGGCCAGCAAGCCCGAATTTGATAGGAAAATCAAGAAATTCAATTCAATCTACAGCTTTGCCCAGATCCAGCAGA
TTGCCTTTGCTGAGAGGAAGGGGAATGCAAACACATGCGCCGTGTGTAGTGCAGACAACGCCCATCGCATGCAGCAG
ATCAAAATTACTGAGCCAGTCGAAGACAATAAGGATAAAATCATTCTGTCAGCAAAGGCACAGCGACTGCCTGCAAT
CCCAACCCGAATTGTGGATGGAGCTGTCAAGAAAATGGCTACAATTCTGGCAAAGAATATCGTGGACGATAATTGGC
AGAACATTAAGCAGGTCCTGAGCGCAAAACACCAGCTGCATATCCCAATCATTACCGAGTCCAACGCCTTCGAGTTT
GAACCCGCTCTGGCAGACGTGAAGGGCAAATCTCTGAAGGATAGAAGGAAGAAAGCCCTGGAGCGAATTAGTCCCGA
AAACATCTTCAAGGATAAGAACAACAGAATCAAGGAGTTTGCTAAGGGGATTTCCGCCTACTCTGGAGCTAACCTGA
CAGATGGGGACTTCGATGGAGCAAAGGAGGAACTGGATCACATCATTCCTCGCAGCCATAAGAAATATGGCACTCTG
AACGACGAGGCTAATCTGATTTGCGTGACCCGGGGCGATAATAAGAACAAAGGGAACCGGATCTTCTGTCTGAGAGA
CCTGGCCGATAATTACAAGCTGAAACAGTTTGAGACCACAGACGATCTGGAGATCGAAAAGAAAATTGCCGACACCA
TCTGGGATGCTAATAAGAAGGACTTCAAGTTCGGAAACTATCGGAGCTTCATCAATCTGACACCTCAGGAGCAGAAA
GCATTCAGACACGCCCTGTTTCTGGCTGATGAAAACCCAATCAAGCAGGCAGTGATCAGAGCCATTAATAACCGCAA
CCGAACCTTCGTGAATGGCACACAGAGGTATTTTGCTGAGGTCCTGGCAAATAACATCTACCTGCGCGCCAAGAAAG
AAAATCTGAACACTGACAAGATCAGCTTCGATTACTTTGGAATCCCTACCATTGGAAACGGCCGAGGGATCGCTGAG
ATTCGGCAGCTGTATGAAAAGGTGGACAGTGATATCCAGGCCTACGCTAAAGGCGACAAGCCACAGGCCTCTTATAG
TCACCTGATTGATGCTATGCTGGCATTCTGCATCGCCGCTGACGAGCATCGGAACGATGGATCTATTGGCCTGGAAA
 
TCGACAAAAACTATAGTCTGTACCCTCTGGATAAGAATACTGGCGAGGTGTTCACCAAAGACATCTTTTCACAGATC
AAGATTACCGACAACGAGTTCAGCGATAAGAAACTGGTCAGAAAGAAAGCTATTGAAGGGTTTAACACACACAGACA
GATGACTAGGGATGGAATCTATGCAGAGAATTACCTGCCTATCCTGATTCATAAGGAGCTGAACGAAGTGAGGAAGG
GGTACACATGGAAAAATTCCGAGGAAATCAAAATTTTCAAGGGAAAGAAATACGACATCCAGCAGCTGAATAACCTG
GTGTATTGTCTGAAGTTTGTGGACAAACCAATCAGTATTGATATCCAGATTTCAACCCTGGAGGAACTGAGAAACAT
CCTGACTACCAATAACATTGCAGCCACTGCCGAGTACTATTACATTAATCTGAAAACCCAGAAGCTGCACGAGTATT
ACATCGAAAATTACAACACAGCCCTGGGGTATAAGAAATACAGCAAGGAGATGGAGTTCCTGAGGTCCCTGGCTTAT
AGGTCTGAGCGCGTGAAGATCAAAAGTATTGACGATGTCAAGCAGGTCCTGGACAAGGATTCAAACTTCATCATCGG
AAAGATCACACTGCCCTTCAAGAAAGAGTGGCAGCGACTGTACCGGGAATGGCAGAACACAACTATCAAAGACGATT
ATGAGTTTCTGAAGAGCTTCTTTAATGTGAAGTCCATTACTAAACTGCACAAGAAAGTCCGGAAAGACTTCTCTCTG
CCCATCAGTACAAACGAGGGCAAGTTTCTGGTGAAGAGAAAAACTTGGGATAATAACTTCATCTACCAGATTCTGAA
TGACTCAGATAGCAGGGCAGACGGGACTAAACCCTTCATTCCTGCCTTTGATATCAGCAAGAACGAGATTGTGGAAG
CCATCATTGACAGTTTCACCTCAAAAAACATCTTTTGGCTGCCAAAGAATATTGAGCTGCAGAAGGTGGACAACAAG
AACATCTTCGCCATTGATACCAGCAAGTGGTTTGAGGTCGAAACACCATCCGACCTGCGCGATATCGGCATTGCTAC
CATTCAGTACAAGATCGACAATAACTCACGCCCCAAGGTGCGAGTCAAACTGGATTACGTGATCGACGATGACAGCA
AGATTAACTATTTCATGAATCACTCACTGCTGAAGAGCCGGTATCCCGACAAAGTCCTGGAGATCCTGAAGCAGAGC
ACAATCATTGAGTTCGAAAGTTCAGGGTTTAACAAAACTATTAAGGAGATGCTGGGAATGAAGCTGGCCGGCATCTA
CAATGAAACCTCCAATAACTAA
Polypeptide sequence of SpCas9 (SEQ ID NO: 3): 
MDYKDHDGDYKDHDIDYKDDDDKMAPKKKRKVGIHGVPAADKKYSIGLDIGTNSVGWAVITDEYKVPSKKFKVLGNT
DRHSIKKNLIGALLFDSGETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFDNGSIPHQIHLGELHAIL
RRQEDFYPFLKDNREKIEKILTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTNRKVTVKQLKEDYFKKIECFDS
VEISGVEDRFNASLGTYHDLLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKAQVSGQGDSLHEHIANLAGSP
AIKKGILQTVKVVDELVKVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDENDKLIREVKVITLKSKL
VSDFRKDFQFYKVREINNYHHAHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITIMERSSFEKNPIDFLEAKGYKEVKKD
LIIKLPKYSLFELENGRKRMLASAGELQKGNELALPSKYVNFLYLASHYEKLKGSPEDNEQKQLFVEQHKHYLDEII
 
EQISEFSKRVILADANLDKVLSAYNKHRDKPIREQAENIIHLFTLTNLGAPAAFKYFDTTIDRKRYTSTKEVLDATL
IHQSITGLYETRIDLSQLGGDKRPAATKKAGQAKKKK
Polypeptide sequence of FnCas9 (SEQ ID NO: 4): 
MYPYDVPDYASPKKKRKVEASNFKILPIAIDLGVKNTGVFSAFYQKGTSLERLDNKNGKVYELSKDSYTLLMNNRTA
RRHQRRGIDRKQLVKRLFKLIWTEQLNLEWDKDTQQAISFLFNRRGFSFITDGYSPEYLNIVPEQVKAILMDIFDDY
NGEDDLDSYLKLATEQESKISEIYNKLMQKILEFKLMKLCTDIKDDKVSTKTLKEITSYEFELLADYLANYSESLKT
QKFSYTDKQGNLKELSYYHHDKYNIQEFLKRHATINDRILDTLLTDDLDIWNFNFEKFDFDKNEEKLQNQEDKDHIQ
AHLHHFVFAVNKIKSEMASGGRHRSQYFQEITNVLDENNHQEGYLKNFCENLHNKKYSNLSVKNLVNLIGNLSNLEL
KPLRKYFNDKIHAKADHWDEQKFTETYCHWILGEWRVGVKDQDKKDGAKYSYKDLCNELKQKVTKAGLVDFLLELDP
CRTIPPYLDNNNRKPPKCQSLILNPKFLDNQYPNWQQYLQELKKLQSIQNYLDSFETDLKVLKSSKDQPYFVEYKSS
NQQIASGQRDYKDLDARILQFIFDRVKASDELLLNEIYFQAKKLKQKASSELEKLESSKKLDEVIANSQLSQILKSQ
HTNGIFEQGTFLHLVCKYYKQRQRARDSRLYIMPEYRYDKKLHKYNNTGRFDDDNQLLTYCNHKPRQKRYQLLNDLA
GVLQVSPNFLKDKIGSDDDLFISKWLVEHIRGFKKACEDSLKIQKDNRGLLNHKINIARNTKGKCEKEIFNLICKIE
GSEDKKGNYKHGLAYELGVLLFGEPNEASKPEFDRKIKKFNSIYSFAQIQQIAFAERKGNANTCAVCSADNAHRMQQ
IKITEPVEDNKDKIILSAKAQRLPAIPTRIVDGAVKKMATILAKNIVDDNWQNIKQVLSAKHQLHIPIITESNAFEF
EPALADVKGKSLKDRRKKALERISPENIFKDKNNRIKEFAKGISAYSGANLTDGDFDGAKEELDHIIPRSHKKYGTL
NDEANLICVTRGDNKNKGNRIFCLRDLADNYKLKQFETTDDLEIEKKIADTIWDANKKDFKFGNYRSFINLTPQEQK
AFRHALFLADENPIKQAVIRAINNRNRTFVNGTQRYFAEVLANNIYLRAKKENLNTDKISFDYFGIPTIGNGRGIAE
IRQLYEKVDSDIQAYAKGDKPQASYSHLIDAMLAFCIAADEHRNDGSIGLEIDKNYSLYPLDKNTGEVFTKDIFSQI
KITDNEFSDKKLVRKKAIEGFNTHRQMTRDGIYAENYLPILIHKELNEVRKGYTWKNSEEIKIFKGKKYDIQQLNNL
VYCLKFVDKPISIDIQISTLEELRNILTTNNIAATAEYYYINLKTQKLHEYYIENYNTALGYKKYSKEMEFLRSLAY
RSERVKIKSIDDVKQVLDKDSNFIIGKITLPFKKEWQRLYREWQNTTIKDDYEFLKSFFNVKSITKLHKKVRKDFSL
PISTNEGKFLVKRKTWDNNFIYQILNDSDSRADGTKPFIPAFDISKNEIVEAIIDSFTSKNIFWLPKNIELQKVDNK
NIFAIDTSKWFEVETPSDLRDIGIATIQYKIDNNSRPKVRVKLDYVIDDDSKINYFMNHSLLKSRYPDKVLEILKQS
TIIEFESSGFNKTIKEMLGMKLAGIYNETSNN
Polynucleotide sequence of BE3 (SEQ ID NO: 5): 
ATGAGCTCAGAGACTGGCCCAGTGGCTGTGGACCCCACATTGAGACGGCGGATCGAGCCCCATGAGTTTGAGGTATT
CTTCGATCCGAGAGAGCTCCGCAAGGAGACCTGCCTGCTTTACGAAATTAATTGGGGGGGCCGGCACTCCATTTGGC
GACATACATCACAGAACACTAACAAGCACGTCGAAGTCAACTTCATCGAGAAGTTCACGACAGAAAGATATTTCTGT
CCGAACACAAGGTGCAGCATTACCTGGTTTCTCAGCTGGAGCCCATGCGGCGAATGTAGTAGGGCCATCACTGAATT
CCTGTCAAGGTATCCCCACGTCACTCTGTTTATTTACATCGCAAGGCTGTACCACCACGCTGACCCCCGCAATCGAC
AAGGCCTGCGGGATTTGATCTCTTCAGGTGTGACTATCCAAATTATGACTGAGCAGGAGTCAGGATACTGCTGGAGA
AACTTTGTGAATTATAGCCCGAGTAATGAAGCCCACTGGCCTAGGTATCCCCATCTGTGGGTACGACTGTACGTTCT
TGAACTGTACTGCATCATACTGGGCCTGCCTCCTTGTCTCAACATTCTGAGAAGGAAGCAGCCACAGCTGACATTCT
TTACCATCGCTCTTCAGTCTTGTCATTACCAGCGACTGCCCCCACACATTCTCTGGGCCACCGGGTTGAAAAGCGGC
 
AGCGAGACTCCCGGGACCTCAGAGTCCGCCACACCCGAAAGTGATAAAAAGTATTCTATTGGTTTAGCCATCGGCAC
TAATTCCGTTGGATGGGCTGTCATAACCGATGAATACAAAGTACCTTCAAAGAAATTTAAGGTGTTGGGGAACACAG
ACCGTCATTCGATTAAAAAGAATCTTATCGGTGCCCTCCTATTCGATAGTGGCGAAACGGCAGAGGCGACTCGCCTG
AAACGAACCGCTCGGAGAAGGTATACACGTCGCAAGAACCGAATATGTTACTTACAAGAAATTTTTAGCAATGAGAT
GGCCAAAGTTGACGATTCTTTCTTTCACCGTTTGGAAGAGTCCTTCCTTGTCGAAGAGGACAAGAAACATGAACGGC
ACCCCATCTTTGGAAACATAGTAGATGAGGTGGCATATCATGAAAAGTACCCAACGATTTATCACCTCAGAAAAAAG
CTAGTTGACTCAACTGATAAAGCGGACCTGAGGTTAATCTACTTGGCTCTTGCCCATATGATAAAGTTCCGTGGGCA
CTTTCTCATTGAGGGTGATCTAAATCCGGACAACTCGGATGTCGACAAACTGTTCATCCAGTTAGTACAAACCTATA
ATCAGTTGTTTGAAGAGAACCCTATAAATGCAAGTGGCGTGGATGCGAAGGCTATTCTTAGCGCCCGCCTCTCTAAA
TCCCGACGGCTAGAAAACCTGATCGCACAATTACCCGGAGAGAAGAAAAATGGGTTGTTCGGTAACCTTATAGCGCT
CTCACTAGGCCTGACACCAAATTTTAAGTCGAACTTCGACTTAGCTGAAGATGCCAAATTGCAGCTTAGTAAGGACA
CGTACGATGACGATCTCGACAATCTACTGGCACAAATTGGAGATCAGTATGCGGACTTATTTTTGGCTGCCAAAAAC
CTTAGCGATGCAATCCTCCTATCTGACATACTGAGAGTTAATACTGAGATTACCAAGGCGCCGTTATCCGCTTCAAT
GATCAAAAGGTACGATGAACATCACCAAGACTTGACACTTCTCAAGGCCCTAGTCCGTCAGCAACTGCCTGAGAAAT
ATAAGGAAATATTCTTTGATCAGTCGAAAAACGGGTACGCAGGTTATATTGACGGCGGAGCGAGTCAAGAGGAATTC
TACAAGTTTATCAAACCCATATTAGAGAAGATGGATGGGACGGAAGAGTTGCTTGTAAAACTCAATCGCGAAGATCT
ACTGCGAAAGCAGCGGACTTTCGACAACGGTAGCATTCCACATCAAATCCACTTAGGCGAATTGCATGCTATACTTA
GAAGGCAGGAGGATTTTTATCCGTTCCTCAAAGACAATCGTGAAAAGATTGAGAAAATCCTAACCTTTCGCATACCT
TACTATGTGGGACCCCTGGCCCGAGGGAACTCTCGGTTCGCATGGATGACAAGAAAGTCCGAAGAAACGATTACTCC
ATGGAATTTTGAGGAAGTTGTCGATAAAGGTGCGTCAGCTCAATCGTTCATCGAGAGGATGACCAACTTTGACAAGA
ATTTACCGAACGAAAAAGTATTGCCTAAGCACAGTTTACTTTACGAGTATTTCACAGTGTACAATGAACTCACGAAA
GTTAAGTATGTCACTGAGGGCATGCGTAAACCCGCCTTTCTAAGCGGAGAACAGAAGAAAGCAATAGTAGATCTGTT
ATTCAAGACCAACCGCAAAGTGACAGTTAAGCAATTGAAAGAGGACTACTTTAAGAAAATTGAATGCTTCGATTCTG
TCGAGATCTCCGGGGTAGAAGATCGATTTAATGCGTCACTTGGTACGTATCATGACCTCCTAAAGATAATTAAAGAT
AAGGACTTCCTGGATAACGAAGAGAATGAAGATATCTTAGAAGATATAGTGTTGACTCTTACCCTCTTTGAAGATCG
GGAAATGATTGAGGAAAGACTAAAAACATACGCTCACCTGTTCGACGATAAGGTTATGAAACAGTTAAAGAGGCGTC
GCTATACGGGCTGGGGACGATTGTCGCGGAAACTTATCAACGGGATAAGAGACAAGCAAAGTGGTAAAACTATTCTC
GATTTTCTAAAGAGCGACGGCTTCGCCAATAGGAACTTTATGCAGCTGATCCATGATGACTCTTTAACCTTCAAAGA
GGATATACAAAAGGCACAGGTTTCCGGACAAGGGGACTCATTGCACGAACATATTGCGAATCTTGCTGGTTCGCCAG
CCATCAAAAAGGGCATACTCCAGACAGTCAAAGTAGTGGATGAGCTAGTTAAGGTCATGGGACGTCACAAACCGGAA
AACATTGTAATCGAGATGGCACGCGAAAATCAAACGACTCAGAAGGGGCAAAAAAACAGTCGAGAGCGGATGAAGAG
AATAGAAGAGGGTATTAAAGAACTGGGCAGCCAGATCTTAAAGGAGCATCCTGTGGAAAATACCCAATTGCAGAACG
AGAAACTTTACCTCTATTACCTACAAAATGGAAGGGACATGTATGTTGATCAGGAACTGGACATAAACCGTTTATCT
GATTACGACGTCGATCACATTGTACCCCAATCCTTTTTGAAGGACGATTCAATCGACAATAAAGTGCTTACACGCTC
GGATAAGAACCGAGGGAAAAGTGACAATGTTCCAAGCGAGGAAGTCGTAAAGAAAATGAAGAACTATTGGCGGCAGC
TCCTAAATGCGAAACTGATAACGCAAAGAAAGTTCGATAACTTAACTAAAGCTGAGAGGGGTGGCTTGTCTGAACTT
GACAAGGCCGGATTTATTAAACGTCAGCTCGTGGAAACCCGCCAAATCACAAAGCATGTTGCACAGATACTAGATTC
CCGAATGAATACGAAATACGACGAGAACGATAAGCTGATTCGGGAAGTCAAAGTAATCACTTTAAAGTCAAAATTGG
 
TGTCGGACTTCAGAAAGGATTTTCAATTCTATAAAGTTAGGGAGATAAATAACTACCACCATGCGCACGACGCTTAT
CTTAATGCCGTCGTAGGGACCGCACTCATTAAGAAATACCCGAAGCTAGAAAGTGAGTTTGTGTATGGTGATTACAA
AGTTTATGACGTCCGTAAGATGATCGCGAAAAGCGAACAGGAGATAGGCAAGGCTACAGCCAAATACTTCTTTTATT
CTAACATTATGAATTTCTTTAAGACGGAAATCACTCTGGCAAACGGAGAGATACGCAAACGACCTTTAATTGAAACC
AATGGGGAGACAGGTGAAATCGTATGGGATAAGGGCCGGGACTTCGCGACGGTGAGAAAAGTTTTGTCCATGCCCCA
AGTCAACATAGTAAAGAAAACTGAGGTGCAGACCGGAGGGTTTTCAAAGGAATCGATTCTTCCAAAAAGGAATAGTG
ATAAGCTCATCGCTCGTAAAAAGGACTGGGACCCGAAAAAGTACGGTGGCTTCGATAGCCCTACAGTTGCCTATTCT
GTCCTAGTAGTGGCAAAAGTTGAGAAGGGAAAATCCAAGAAACTGAAGTCAGTCAAAGAATTATTGGGGATAACGAT
TATGGAGCGCTCGTCTTTTGAAAAGAACCCCATCGACTTCCTTGAGGCGAAAGGTTACAAGGAAGTAAAAAAGGATC
TCATAATTAAACTACCAAAGTATAGTCTGTTTGAGTTAGAAAATGGCCGAAAACGGATGTTGGCTAGCGCCGGAGAG
CTTCAAAAGGGGAACGAACTCGCACTACCGTCTAAATACGTGAATTTCCTGTATTTAGCGTCCCATTACGAGAAGTT
GAAAGGTTCACCTGAAGATAACGAACAGAAGCAACTTTTTGTTGAGCAGCACAAACATTATCTCGACGAAATCATAG
AGCAAATTTCGGAATTCAGTAAGAGAGTCATCCTAGCTGATGCCAATCTGGACAAAGTATTAAGCGCATACAACAAG
CACAGGGATAAACCCATACGTGAGCAGGCGGAAAATATTATCCATTTGTTTACTCTTACCAACCTCGGCGCTCCAGC
CGCATTCAAGTATTTTGACACAACGATAGATCGCAAACGATACACTTCTACCAAGGAGGTGCTAGACGCGACACTGA
TTCACCAATCCATCACGGGATTATATGAAACTCGGATAGATTTGTCACAGCTTGGGGGTGACTCTGGTGGTTCTACT
AATCTGTCAGATATTATTGAAAAGGAGACCGGTAAGCAACTGGTTATCCAGGAATCCATCCTCATGCTCCCAGAGGA
GGTGGAAGAAGTCATTGGGAACAAGCCGGAAAGCGATATACTCGTGCACACCGCCTACGACGAGAGCACCGACGAGA
ATGTCATGCTTCTGACTAGCGACGCCCCTGAATACAAGCCTTGGGCTCTGGTCATACAGGATAGCAACGGTGAGAAC
AAGATTAAGATGCTCTCTGGTGGTTCTCCCAAGAAGAAGAGGAAAGTCTAA
Polypeptide sequence of BE3 (SEQ ID NO: 6): 
MSSETGPVAVDPTLRRRIEPHEFEVFFDPRELRKETCLLYEINWGGRHSIWRHTSQNTNKHVEVNFIEKFTTERYFC
PNTRCSITWFLSWSPCGECSRAITEFLSRYPHVTLFIYIARLYHHADPRNRQGLRDLISSGVTIQIMTEQESGYCWR
NFVNYSPSNEAHWPRYPHLWVRLYVLELYCIILGLPPCLNILRRKQPQLTFFTIALQSCHYQRLPPHILWATGLKSG
SETPGTSESATPESDKKYSIGLAIGTNSVGWAVITDEYKVPSKKFKVLGNTDRHSIKKNLIGALLFDSGETAEATRL
KRTARRRYTRRKNRICYLQEIFSNEMAKVDDSFFHRLEESFLVEEDKKHERHPIFGNIVDEVAYHEKYPTIYHLRKK
LVDSTDKADLRLIYLALAHMIKFRGHFLIEGDLNPDNSDVDKLFIQLVQTYNQLFEENPINASGVDAKAILSARLSK
SRRLENLIAQLPGEKKNGLFGNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQSKNGYAGYIDGGASQEEF
YKFIKPILEKMDGTEELLVKLNREDLLRKQRTFDNGSIPHQIHLGELHAILRRQEDFYPFLKDNREKIEKILTFRIP
YYVGPLARGNSRFAWMTRKSEETITPWNFEEVVDKGASAQSFIERMTNFDKNLPNEKVLPKHSLLYEYFTVYNELTK
VKYVTEGMRKPAFLSGEQKKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGRLSRKLINGIRDKQSGKTIL
DFLKSDGFANRNFMQLIHDDSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYLQNGRDMYVDQELDINRLS
DYDVDHIVPQSFLKDDSIDNKVLTRSDKNRGKSDNVPSEEVVKKMKNYWRQLLNAKLITQRKFDNLTKAERGGLSEL
 
DKAGFIKRQLVETRQITKHVAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGKATAKYFFYSNIMNFFKTEITLANGEIRKRPLIET
NGETGEIVWDKGRDFATVRKVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYSLFELENGRKRMLASAGE
LQKGNELALPSKYVNFLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRIDLSQLGGDSGGST
NLSDIIEKETGKQLVIQESILMLPEEVEEVIGNKPESDILVHTAYDESTDENVMLLTSDAPEYKPWALVIQDSNGEN
KIKMLSGGSPKKKRKV
Polynucleotide sequence of HB-EGF locus (SEQ ID NO: 7): 
ATTCGGCCGAAGGAGCTACGCGGGCCACGCTGCTGGCTGGCCTGACCTAGGCGCGCGGGGTCGGGCGGCCGCGCGGG
CGGGCTGAGTGAGCAAGACAAGACACTCAAGAAGAGCGAGCTGCGCCTGGGTCCCGGCCAGGCTTGCACGCAGAGGC
GGGCGGCAGACGGTGCCCGGCGGAATCTCCTGAGCTCCGCCGCCCAGCTCTGGTGCCAGCGCCCAGTGGCCGCCGCT
TCGAAAGTGACTGGTGCCTCGCCGCCTCCTCTCGGTGCGGGACCATGAAGCTGCTGCCGTCGGTGGTGCTGAAGCTC
TTTCTGGCTGCAGGTAAGAGGGCTGCCGACGCCCCCGGAGATCGGGGGGATGGGGGCGTTGTGCTGGGGGCATGGGG
GAAGGTCGCCGCAGCGCACCCGGCACGGGCCACTTGGTGGGGCCCTTGCGCTCTGGCGGACGGGCGTCGGCATCGGT
GCGTGTTGGTCAGGGGTCTGGGCGGGTGTCTGATGCGGCCTGGCCTCTCGCCCGCAGTTCTCTCGGCACTGGTGACT
GGCGAGAGCCTGGAGCGGCTTCGGAGAGGGCTAGCTGCTGGAACCAGCAACCCGGACCCTCCCACTGTATCCACGGA
CCAGCTGCTACCCCTAGGAGGCGGCCGGGACCGGAAAGTCCGTGACTTGCAAGAGGCAGATCTGGACCTTTTGAGAG
GTGGGTGTGGAGGCCCCCCATCCTTGGACCTTGGTGGGCTGTTGAAGAATAAGCAGATCCAAGATTCTTGCTGTTTG
GGCAATACTGTGGGTTGAGGGTATTCATGGAGAACCTCGGGGAAAAGCTGATCGGCCTGATGGGCACTGGGGGATCC
TGGAATATAGGTCCCACTCTCTCTCTCTTGTCATTGCCTCACCTGCTGGGTTGCTGCCCTTCTGGGTACTCCGGGGC
AAATTGAATCAGACGTGTTGTCTGGGGTTGTTACGTTCTTCTTAGGTAAGCTGGGTGATAGGAACAAGGAATGGTTG
AGATGCTTTCCCTAGAGCTACTATGTAAAAATGGGCGCCAGTTCTAATTCCCATATCAAATGACTATTATATATAAA
ATAGAGGTAACACATGCGGAGATGCCCAGGCACATCTCTAGAAAGTGTGCAGTGTTGGCCTCCTCCATCCACCTGTC
TCCAGATTGGGGAAACAGAGGGGAATGAGGAGCTCTTGGCCGCCCTAGATGAGGCTGTGAATGGTGAGCACTGAGCC
CCTAGGGGGCTGTATTAAAATGCTGGATATCTGTGAATGCTACCGGAAACCTGCAGCTTACTGAGCACCTTGCATTC
CTGAGGAGACTCCAAATGGGGAGGGCTGTGTAGGATCCTCCAACCAGCCTCTTTGGCTGTGGCCAAGTACAGGTACA
GGGCAGAGTCCAGAGCCTGCCAGCTCTCCTGCCTCCAAACCTGAGGAGATTATCCAGAGTAGAGCAAGGACTCAGCA
CTGTACCCTGGAATGACTATATTTGGTTGGACAGATGCCCACCTGTTCTAGTTCCACCTGCTCCTCAGCTGCCCTTC
TCCCTCATTCCCAGGAGCTTTCCTTGGATACTCTCTCTACTTTGTATAAATCAAGCACATACTCCAAAACTGAGCCT
GGGCTCCCATACTTCATCCTCTCCCAGTGGCCCTCTGGGGTTGCCCATGACCTGAACAGCCTGGATTCTCCTGGCCC
TCTCCTCCTAGGCTGGGCAGGGCTGGGCTGTGACTCACCCCACCCCCACCCCCCACCCACACGGCTGCTCCTCTTAC
CTCTGCAGACCTGACTCACTGCTCCCTGTCCATGGCAGGAGCCTGGCTGTCACCCTGCACCTTCTCCCTCCCCTTTC
TGATTGGCTTGGCCCCCCTGCCTTGCTCTCCCCGAAGCTCTGGTCACTGGGTTCCTCTGACCACCTGTATCACCTTC
TGAGCTCTGAGGGGGCCTGGGACTGGATGAGAGGAAATGAAAGACTGTGGGGGCTGCTGGCACCTACTTCTCTTCCC
TTCTTTTGGCTTTGCTGGGCAAGGACTATTTTTCAGGTCTGGGGATCCTACCACCTAAAATAAATGACTGCTACCAT
 
TTATTAAATTCCTACTGTGTTCTAGGCACTTGATATGTTATCCTGGCTAATGTAACACTTATAGCAACCTTTTGAGA
TAGTTACTTTGGCTATCCACATTTTACTGAGAACCTGAGGTTCAGAGGAGTTAAGTGACTGCCCACAGTAAATAGCT
GAAATTGGAGCACAGGTCTATGGACTTCAGAGCCCATTCATGCCTGGATCAGCATCTCAGGTGCTCTAGACTTGTGA
GAGGGAGGAGATGGGAGTGTGTGAGGCAGCTTGGTGTGGTGAGGAAGGACATTGGAGTGAAGTCCAGAGAACACAGT
TCTAATCCCAATCCTGCATGACCTTGAGTAAGTCACTCTGCCTGCCATGAGTTTTTTCTTTTTTTCTTTTTTTTTTT
TTTAAACATAGTCTCACTCTGTCACCCAGGCTGGAGTGCAATGGCACGATCTCAGCTCACTGCAATCTCTGCCTCCC
AGGTTCAAGTGATTCTCCTGCCTCAGCCTCCTGAGTAGCTGCGATAACAGGCACACACCACCACGCCCGGCTAATTT
TTGTATTTTTTGTAGAGATGAGATTTTTGCCATGTTGGCAAGGCTGGTCTCGAACTCCCGACCTCAGGTGATCCACC
TGCCTCAGCCTCCCAAAGTGTTGGGATTACAGGCGTGAGCCACCGTGCCTGGCCACATGGTATTCTTTGAAGTCCCT
CTAGCTTGAGACTCTAAGTCTCTAGTCTAACGTATCATGCTTACCCTTCTGTAAGACACATGGCTGTAGCCATGGAT
GTGGGCACCTTTTTCCTGATGGGGGATAAAAGGGTGGGATTGGGCTGATAGGCATAGTCCCTGGTCAATCCCAGCTG
GATATCTGGGTGAGGCTGTTTTTCCCCCAGTCTCTCTGAAGCATGGAAAGAAGGAGGGAGTCATCATTGTTCCAGTT
CCTTCTGGACAGTTCCTTACTTTCCATTTTTCTATCCCTTGTACACCCTGTACCCCCCAATCCAGAGAGCTATAAAC
AGGACATTGGGGGTTAAATATGAATGAATCTTTGAGAAAGTGGGTGAGCTGTAAAGGGTATGCAAGTTAAATATTTT
GCTTGAAGTTGAAAAAGCAAGGCCGTGACCAGGGCTGGCCTGCTTGCTGTTCCTGAGCCAGGCTCTGCCCTGGGCTC
ATAGTACTAAGGGGTGCCCCAGAAGAGACCACCTGAACACATGGACACTGTTCTTATATTAGGAGCCCTCCAACCCC
AGAACCTCCAAGTACCTTCTCTAGAAGCAATTTTTGTGTGTGACACTGTCTTTCTGCAAGTGGTTCACTGAGTACAG
CATCAGGAAATGAGGCTGATTGAAGGCCAAAATAGAATGAAGTGGGTGTGGGGGAGTAGGAGATGGGGGTGTAAGGT
GGACAGTGGGGTGGAGGTGAGGTTGGTAGAATTGCCCAGTTACTCAACAAAAGCATTCTGAGAATGAGGCTCTTACA
CAGAGACTGTGAAATGCCTTCCTTGGGACCCACCCTAGCTTCTACTTCCTACCGAGGTTCCCTCTTTCTGGTGGTTC
TGCCCAATCTTCCTGCTCTTCCTTCTGCCTCTTAGGAGGCACTGAGCTAAGGGGCCTTCCCAGATCTCTGACTTCAG
GTGGAATCAAAGCATATATACTCCTTTCAAGCACTATGCTCTTCTGATTTTCTTCCCAAAGAGTCAGACTTTAACAG
AGTGCTTTTCTCCTACAGTCACTTTATCCTCCAAGCCACAAGCACTGGCCACACCAAACAAGGAGGAGCACGGGAAA
AGAAAGAAGAAAGGCAAGGGGCTAGGGAAGAAGAGGGACCCATGTCTTCGGAAATACAAGGACTTCTGCATCCATGG
AGAATGCAAATATGTGAAGGAGCTCCGGGCTCCCTCCTGCATGTAAGTGCCCCTTCCCCAGGGCTGAATCTCATCAG
CACACTTTGTCAGCCACGTGGCTGTTCCTCGTTGTCACTGTTCCTTGAATTCATAATTTCACCCAGTTTCTTCTCAA
CCTCTGGGCGGAAGTTGGGAGGAGGGGAAATATATTTTTAGTCAGCGGAAGCCCCCTCCCCCCTATAGGATGCAATT
TCCTGTGGTATGGTTTTGTGACGTGCTTTAATCCTTGGGGACATTTCCTGCTTGCCCAGAAATGAGCATGTGGCTAG
GACAGCTGGCACCTGAAGGCAGGCCCTTAATTCTTGCCTGATGCCCTACTCTGGGAGGGAGAAGCCAGTAGGAAACA
TGGCAGAGTGGGCTTCCAGGGCAGAGTAGAGCTCCTGTGGGAAGGTAGGAAGTGCATTTGGATGCATGATGTATAGG
TATGTGTGTATTTGGGTTTATGTGCATGTAAGTGTGCAAATGTGGATTGACTGTGAGGCATGGCAGGACTGTACAGA
GAGGGATCATCATGGCGGCAGGTTGAGGCCTCTCTTTCTTCTTCCTTATCCCAGCAAGGACGAGGAGGTGGGAGACA
TGGAGAGTACTGGCCTTTGGCCACGTTGTGAGAGAACAATTCCTTTGTGCAGGGTTCACAGGAAATGGAACCTGACC
CATTAGGCATCAGCCCCCGGTCAGGCAACATCACCCCTTCCCTGGGTAGGTGTGTGGGTGGAGGGGCTGTGGGTTCC
TTAGCCTCTCTCCTAAGCCAAACCCAGCAAACGGCTGCCTTGGCAACCCCTCAGGGATGACAGCACTGCCATGCTCT
CTGGCAGGCATAATGTTGCCACTGTGCCTGAGGCCAACACCCTGCGTCAGGCTGCAAACATCCATTCCCTTCCCTGT
GGGGAGGGAGGCTCTGGGGGCCTTAGTGGGAGACTCTGGACAGGGCCAAGAGACTGTTGTATGCACACTGCCTCCAG
CCTGTCAAGAAGGCGGCGTGCCTGGCATCCCTTCTACTGGTGATTGGTGCAGATCCCTTAGCTTTTTAAAGCTTCCT
 
TGTTTTGTCTGATCACACACAGCAGAGCTGCCCTGTATTTGGCAGTTGGCAGACAGACCCATCACTCCCCACCATGT
CCACAGTCACTTGTGCATCCTTTCCTATAACATCCTTGTCAGGAGCTTGGTATTAGAGGGAGTTGTTTAAGAGTGGC
ATAGAAAGCCCCCATATTATCCTTCCCAAGGTCTTGGGACAGGGTGGGAAATGTTCATCTTAAATTTGTAAAATGGC
ATCATTAGTACAGGGTGAAGAAGGTGACTCAAGTAGTCAAGGTGGATTGAGGTCAGGAATCTGTCTATACCAGATTG
GTCCTGGGCATTTTGGTGGATGGATGTGGGGCTTGCACTGTGTGGTTGAGAGGCCTTATAAGGTTGCCCTCCTGGAG
AGCTGGACTCGGATGACCACCTAAACCCAGAGAACCTGATATGGGTGCCCAGGCCACCTTCCCAGTGGTCCCTAGGG
ATAGTGATAACTATAATGATGTCATATCTCCTTTGTCCCAGAGTTTCAGTGTTTATATATAATATGAGTTGAGCCCA
AGTATGTTGAGCCCCTATTTGGTGGCAGACACTACTTTAGGAGCTGGAGAGATATAGTTTCCTGGGATTTTTCAAAA
GCCCTCTGCTGAGTAGGCAGGACTTGGTACCTCTACTTGAAAGGTGATGAAACTGGAGCCAGAAAATAGGAAGTAAT
TTGCCTGAGGTCAATAGCTAAATAAGTAGTTGGAAATAAGACAGAGTCTCAGTACCTGACTCCTAGTCCAACATGCT
TTTCATGCCCTCAAGCTGTACTGGGTGTTGGCTTTCATCTTTCTTTCCTGTATCTGTCCTTATAGAGTTGGAGCAGC
ATTTTATAGAGGGCAGAGGGCAGCTGTTGTCCTAGAGGTCTCTTATTCTTTTACTAGTCTAACAGCACAGCAATCTG
ATTTGAAAACTTTACATTAACTTCTTGGGCAGAATTTTCTTTTTCTTTGTTCTTTTCTTTCTTTCTTTCTTTTTTTT
TTTTTTTTTTTTTTTGAGACAGAGTCTCACTCTGTCTCCCATGCTGGGGTGCAGTGGTGTGATCTCAGCTCACTGCA
ACCTCTGCCTCCTGGGTTCAAGCAATTCTCCTGCCTCAGCCTCCTAAGTGGCTGGGACTACAGGCACCTGCCACCAT
GCCGAATTAATAATTTTTATATTTTTAGTAGAGACGTAGTTTTGCCGTGTTGGCCAGGCTGGTCTTGAACTCTTGAC
CTCAGGTGATCCGCCTGCCTCAGCCTCCCAAAGTGCTGGGATTACAGGCATGAGCCACCATATCTAGCCTTTTTTTT
TTTTGAGATGGAATCTCGCTCTGTCACCCAGGCTGGAGTGCAGTGACACAATCTCGGCTCTCTGCAGCCTCCGCCTC
CCAGATTAAAGTGATTTTCCTGCTTCAGCCTCCTGAGCAGCTGGTATTACAGGCACATGCCCCCACATCTGGCTAAT
TTTTAAATTTTTGTGGAGATGGGGTTTCACCATGTTGGCCAGGCTGGTCTTGAACTCCTAACCTCAAGTAATCAGCC
TGCCTTGGACTCCCAAAGTGCTGGGATTACAGGCGTGGGCCACCACTTCCTGGGCAGATTTTCAGGGGGTTGATTGC
ATGTCTGGACTGGCCCCCTACTGCCTCCTGCCCTTGCTACTCAGGGCAGAAAGCAGCAAGAAGACAGAAATCCTGGT
TTGGGGGAATGTGACATCTGTGCACGTTCATCTGGGGATCTTTGTGGCTCTTGTTTGACTCCAGACCCAGGAACCAC
TAGCCAGGGTGTGTCCAGGCTGCTGTGGTGAGCCTGAGGCTAGCTGGCTTCCTAAACTAGCCCTCTGCAGCCACCAT
GAACAGGAAAACCCTTTTTGTGTCACCAGCCAAAAGTTGCCCTCAAAGAGTAGTTTCTGCTGGGCACAGTGGCTCAC
ACCTGTAATCACAGCACTTTGGGAGGCCGAGGCACGTGGGTCGCCTGAGGTCAGGAGTTCGAGACCAGCCTGGCCAA
CATAGAGAAACCCCCGTCTCTACTAAAAATACAAAAATTAGCTGGGTGTTGTGGCGGGCGCCTGTAATCTCAGCTAC
TAGAGAGGCTGAGGCAGGAGAATCTCTCAAACCCAGGAGGCAGAACTTGCAGTGAGCCGAGATAGTGCCATTGCACT
CCAGCCTAGGCAACAAGAGCAAAACTCCATCTCAAAAAAATAATAATAAATAAATAAAAGAGTAGTTTCCTGGG
ATTCCTGACTAGTTGCCTACCCAGAAATTGGCTGCAGAGTTTCCTGTGGCTGGAGGAAAACTGGGGACACTTGGGCT
GAGGAGGACTCAGAGCTGGAGGAGAGACAGGCTAGGGGGCTCTACTTGGCCTCACTGCCCAGGTGCTAAGAAGGAAT
GGTGATCCCGCTTCTCTTGTCTCCATCTGACTTGGGTGCCCCATTCCTCAGGCCATGGGCAGTAACCTCTGGAGTCT
GATTATGTAATAACTCACACAATGTGGGACTTGGCCTTTATAAAGCCCTTTCATTTGTATTACCTCATTTTATCTTT
TCACAATACTCTAGTGAAGTAGGCATTTCTTATCCCTGTGTTTTACATGAGGAAACCAATGTTTAGAAAGGTAACGT
GACTTGCCCAAAATTACCTGGCTAGAAATAGCAGCAGAACCAGTCTGGAACTCATGCACTCAGTCTCCTCCATCCAG
ACGTGTCCCCTCCACCTCCTGGGGTAAAGGTGGAGAAATCCAGTTTGGAAGATGTCTCTGGACCCTAGAGGGTTCTT
GCATCTGTTGTAATACAAGTTCTGAAATGGGTCACAGACGTGGGTGGGAAGAATGTGTCCTAGTCTGGTGGGTGGCT
GGCTCTGGACAAGACACAAAATTTTGCCCCTACCCTGGGATGCTTGGAATGTACTCATCCCCCCTCCTTCTCTGGGG
 
AAGCCAGGAGTTGTCTGCAAAGGGAGGGGGAGGTAGGTAATATTAGGATGTTTACATTATTATCCTTTTGACTCAGG
GTGGGGGTGGAGGGATTATGTAACTGAATTGCGGGACTCTGAGGCCAAACTTTATTTCTATCTTCTGAGTAACTACC
TGTGGAGTTTGAATGATGGACTGGAAGTGAAAAACAGACTCAACTTCAGCTTCCCTCCTCCCAGGAAAGCAAAGTCT
CTGAAGTCATCCAGACTGCTGTTGAATCCTGGCTCTACGACTCACTAGCTTTGTAACCTTGGGCGAGGTGTTTAACA
AAAGCTAAGCCTCAGTCCATCTTTAAAATGGGGCTAGTAACTTCTCCTTCACAGAGCTGGCTTTAAATGAAATAATT
CTTGTAAAGCAGTTAGCACAAAGTACTTGGCTCATGGTAAGCCTTCAATGATTGCTAATTATTATTCTTTATTATTC
AAGTTATGAGTAATAAATAATAATAACATAGTCAGAGAGAAGGGTCAGACTGCCCCCCAGGAGCCTATCAGATATGC
TTCCTTGGAGTTACCTGCGCTATCCTGCATTGTTCAAAGTGGAAGGAATGATGAATTTGGAATCTGCCAAGACTTGT
TCCTAGTCTTAGCCCTGCTGCTTCCTAGTTGTGCCACTTTTGGTGAATCACTTAATTTCTCTGACCCTTAATCTTAG
CTTTTCCATCTGTAATATGGGGTTGTACCTGCCTACCAGAATGTTAGGAGGCTCAGTTGAGCTAGTAGATAAGGCTA
GTGGCTTGTGAATGGTAAACTGCTGTGCACAAGTGATTTTCCAGGGGTGCTTGTGCAAGTGTCCTCTATGTCCTGGC
AGGATAGGGGTCGCTTTTAGGCCTACATGGGCTGATGGGACAGATACATGGAGAGGCTGGGCAAGGAACTGTGGACT
GTGCTATACGTATAGTGGGCCTGACCTACATTTATCCTGCTGTGAGGTGGTTTCTCGAAGTACCCAGGAGGAACTAG
GGCAGGGAGAGGCTCAGGGCAGGAAAGCAAGAATGCAGTACCACCCAGCCTGGCCCCTCTGCCACTGCTGGTTGTGG
ACAAGTCTGTCTCTTGGAGCTTCCCTGGTGCTCTGTCCGCAGGAAGAAGGGATTCCTTGTTCTGAGGTACCAGAGAA
AGCACCTCCTTCCCAGAGAAAGCACAGCTCAGAAAAGAGGGCCACCAGGTTCTTGGTGCTTCCTTCAGCAGCTGGTG
GTCTAAAGTCCTCAGGCAGACAGTGCCACTGTGCCCCCTGGCTGGATGGTAGGCAGTTGTCAGGTGTGAGTGGGCAG
CACACTGAGCTCAGAGTCAGACAATCTACATCTACATCTTCATTTCTGTCTTACTGTGTGACCTTGGGAAAACCACT
CCACCTTTCTGTAAAACAGGGCTCCTACTTATATCAAAGGATCTCTGGGATGCTCAGATAAAGGAAAGGATGTGAAT
GTGCTTCTTCAACTGTAAGCACGTCTGAGTCTTTCTAAGAGCTTCAAGGAAATGCTTTGTGTTAGAAAAGGCAGTTG
CCAGCCCGGTGTGGTGGCTCATGCCTGTAATCCTTGCACATTGGGAGGCAGAGGCGGGTGGATCACCTGAGGTCAGG
AGTTTGAGACCAGCCTAGTTAACATGGTGAAACTCCGTCTCTTCTAAAAAATTACAAAAATTAGCTGGGCGTGGTGG
CGGGCACCTGTAATCCCAGCTACTTGGGAGGCTGGGGCAGGAGAATCACTTGAATCCGGAGGTAGGGGTTGCAGTGA
GCCAAGATTGCGCCACTGCACTCCAGCCTGGGAGACAGAGCAAGACTCTGTCTCAAAAAAAAAAAAAAAAAAAGAAA
AAGAAAAAGAAAAGGCAGTTGCCATGTGATTTATTTCTTGAGTGAGAAGAGCCAAGGGATTGTTTCTGACAGTCTTC
CATGCTCTGGCAGGGCAGCTGGGCAGAAAGATGTTTCTTGATTTGTTTGGTTTGTCCTGTGATGAAAGAGGCCTGGT
AGCTCAGCGTGCAGAGGCCAAAGGCCAGAGTTGAGCTCCCAAGTTGGGCCCTGCACCCAGGGGGAGCTGGAGTTAAA
TGAAGGAAACTTGAGAAAAACGACTCCTGGCAGAGGCACAGGGCCTATTAATAGGCTGGACAGCAGTGGAGAGGGAC
TGGACGCTGGAAGCACGATGGGGAAGGCTGGGTTTATTTCTGGGTCAGAATGTTGAGGGGCCTCACTGGAGGGAGTG
ATACGAATTCCCTCAATTTAGCCTACCAGCTCTTGTGCCCAAGCCCTCATAAGTGGCTTAAACAGAACGCCTGAACA
CACATGTCATAAATCAGCCACACGTGGAACATATCTAGCTGAGGCCTTCAAGTCCTCCCTTGCTTTTTCCATGCCTA
GAACAGGATTCTCAGCCCAGAGAACCAGAGGAAATGGAAAAGGGGAGGGTGTCAAGTGAGAGAGGAATGCTACAGAG
CTTTCAGAGGGGCTTTAAAGAGTTTTCTACTAGAGGAGAAGGATGGAGGATGGGCAGGGATCGTGGTCAGGGATTGA
CAGGCTGAGGGTATGAGGAATGGGGTTTGGCTTATGCAGGTGGGCCATTGCCAAGAGAGGCCAAAGCACTAACTCCA
TCTCCTTCTTGTTCTGTCTTGAACTAGCTGCCACCCGGGTTACCATGGAGAGAGGTGTCATGGGCTGAGCCTCCCAG
TGGAAAATCGCTTATATACCTATGACCACACAACCATCCTGGCCGTGGTGGCTGTGGTGCTGTCATCTGTCTGTCTG
CTGGTCATCGTGGGGCTTCTCATGTTTAGGTGAGTGTTGGGGTCCCCTGCAGGCTGTTTCTGCAAATCACTCCCTTT
CTTCCTCCTCCTGGGCCCTCTCCTTGATGGTCACATGCACTTCCCTCAATCTTTCCAAATCATGGGCTAGCTCCGGG
 
GTGTAGATTCTCCAAAAACCTGGTATTTCTGGCATGACATGAGTCCTGTGTCTAGAGCCCAGGGTCAAATTTGCGAG
GCCATAGCAGGTTCTGCTCCTCACAGGAGTTCTTTTCCTGCCTCCATGACCCAGCTACCCACTCATGGAGTCACTTT
GTCACACATTTCTTTCTCCTGGCTGTTCTTTGATGGCATTAGTATGTGGTTTGGTAGTCAAGGTGTGGGTGGTGCTA
GTGGTATATCCTTCCACTTCTGAGGCGTCTGGACCTCAGGCCCTGCTTTCTAATCCAGGTATGCTCTAGCTTGGGAG
ACCCACCAAGCACTCTATGCCTGTTTTCTTTCTTTCTTTTTTTTTTTTTTTTTTTGAGACAGAGTCTTGCTCTGTCG
CCCAGGCTGGAGTGCAGTGGTGTGATCTCGGCTCACTGCAAACTCCGCCTCCTGGGTTCACGCCATTCTCCTGCCTC
AGCCTCCTGAGTAGCTGGGACTACAGGCACCCGCCACCACACCCAGCTAATTTTTTCTATTTTTTAGTAGAGACGGG
GTTTCACCATGTTAGCCAGGATGGTCTCGATCTCCTGACCTCGTGATCTGCCCGCCTCGGCCTCCCAAAGTGCTGGG
ATTACAGGCATGAGCCACCGTGCCTAGCTCTATGCCTGTTTTCAAGCAGTGTAACTCATCTGTCATGAGACCTGGAA
CAAGTTACTGTCTTTCTGAGGATTGTAACCTTGTAGTGATTGTAATGTTTGTCCATCTACCTCATAAGGATGTTGTG
AGGATCACGTAAATGAGGTGAAAGCTATTTGTAAATTGCATCCTGCTATTAGAGACAGGAGTTCCTCGGGGCAGTTG
GGCCTTTGACCAGAGTTTGGGCTGCCCTACTGCCTGGGCTTTTCCAAGTAGTAGAGGAAACCACCATGGCAGAGTTC
TTTGGAAGGACCTGCTCTGGACCTGCACTTTGTCATAGCAGGCAGGGCTTATTCACAAAACTTATCTTCCTCAGGTA
CCATAGGAGAGGAGGTTATGATGTGGAAAATGAAGAGAAAGTGAAGTTGGGCATGACTAATTCCCACTGAGAGAGAC
TTGTGCTCAAGGTAACGCTCCATCCTTTGCCCCATGACATGATTATCCTTTGTCCCCTTTCCTGGCTGTGCTTCAGT
GGGTGCTGAATTCTTCATATAGGGGTTGGGGGCCAGGCTACTGTGACATTAATATCCCATTGCAGAATTATTTTCAA
AAAGACTCAGTGCTTCACTTAAGGTAAAAGTTGCTAGAGAGACACCTAAGAGAGATGCCTGAGAGGACAGCTTCTCC
CACCCTCATCCCCTCCCTTCCCCTCCCCTCTCCTCCCCTGGGAGACAGAGTGAAACCCTGTCTCAAAAAGTTTAAAA
ATAAAAAAGACTGGACCAGGAAAATCTTAAGACTTCTTTAGACTGGACCTGGCTTTACATGCCTTCCTTTTGTGCTT
TAGGAATCGGCTGGGGACTGCTACCTCTGAGAAGACACAAGGTGATTTCAGACTGCAGAGGGGAAAGACTTCCATCT
AGTCACAAAGACTCCTTCGTCCCCAGTTGCCGTCTAGGATTGGGCCTCCCATAATTGCTTTGCCAAAATACCAGAGC
CTTCAAGTGCCAAACAGAGTATGTCCGATGGTATCTGGGTAAGAAGAAAGCAAAAGCAAGGGACCTTCATGCCCTTC
TGATTCCCCTCCACCAAACCCCACTTCCCCTCATAAGTTTGTTTAAACACTTATCTTCTGGATTAGAATGCCGGTTA
AATTCCATATGCTCCAGGATCTTTGACTGAAAAAAAAAAAGAAGAAGAAGAAGGAGAGCAAGAAGGAAAGATTTGTG
AACTGGAAGAAAGCAACAAAGATTGAGAAGCCATGTACTCAAGTACCACCAAGGGATCTGCCATTGGGACCCTCCAG
TGCTGGATTTGATGAGTTAACTGTGAAATACCACAAGCCTGAGAACTGAATTTTGGGACTTCTACCCAGATGGAAAA
ATAACAACTATTTTTGTTGTTGTTGTTTGTAAATGCCTCTTAAATTATATATTTATTTTATTCTATGTATGTTAATT
TATTTAGTTTTTAACAATCTAACAATAATATTTCAAGTGCCTAGACTGTTACTTTGGCAATTTCCTGGCCCTCCACT
CCTCATCCCCACAATCTGGCTTAGTGCCACCCACCTTTGCCACAAAGCTAGGATGGTTCTGTGACCCATCTGTAGTA
ATTTATTGTCTGTCTACATTTCTGCAGATCTTCCGTGGTCAGAGTGCCACTGCGGGAGCTCTGTATGGTCAGGATGT
AGGGGTTAACTTGGTCAGAGCCACTCTATGAGTTGGACTTCAGTCTTGCCTAGGCGATTTTGTCTACCATTTGTGTT
TTGAAAGCCCAAGGTGCTGATGTCAAAGTGTAACAGATATCAGTGTCTCCCCGTGTCCTCTCCCTGCCAAGTCTCAG
AAGAGGTTGGGCTTCCATGCCTGTAGCTTTCCTGGTCCCTCACCCCCATGGCCCCAGGCCCACAGCGTGGGAACTCA
CTTTCCCTTGTGTCAAGACATTTCTCTAACTCCTGCCATTCTTCTGGTGCTACTCCATGCAGGGGTCAGTGCAGCAG
AGGACAGTCTGGAGAAGGTATTAGCAAAGCAAAAGGCTGAGAAGGAACAGGGAACATTGGAGCTGACTGTTCTTGGT
AACTGATTACCTGCCAATTGCTACCGAGAAGGTTGGAGGTGGGGAAGGCTTTGTATAATCCCACCCACCTCACCAAA
ACGATGAAGTTATGCTGTCATGGTCCTTTCTGGAAGTTTCTGGTGCCATTTCTGAACTGTTACAACTTGTATTTCCA
AACCTGGTTCATATTTATACTTTGCAATCCAAATAAAGATAACCCTTATTCCATA
Polypeptide sequence of HB-EGF protein (SEQ ID NO: 8):

MKLLPSVVLKLFLAAVLSALVTGESLERLRRGLAAGTSNPDPPTVSTDQLLPLGGGRDRKVRDLQEADLDLLRVTLS
SKPQALATPNKEEHGKRKKKGKGLGKKRDPCLRKYKDFCIHGECKYVKELRAPSCICHPGYHGERCHGLSLPVENRL
YTYDHTTILAVVAVVLSSVCLLVIVGLLMFRYHRRGGYDVENEEKVKLGMTNSH

All references cited herein, including patents, patent applications, papers, text books and the like, and the references cited therein, to the extent that they are not already, are hereby incorporated herein by reference in their entirety.

### EXAMPLES

### Example 1. Experimental Protocol

In this Example, a protocol for co-targeting enrichment is provided.

Maintain cell lines expressing the heparin-binding EGF-receptor in culture and subculture every 2-3 days until transfection. Cells should be >80% confluent on the day of transfection.

Transfect cells with plasmids coding for a base editor or Cas9, and/or together with a plasmid encoding for the guide RNAs targeting HB-EGF and the gene of interest. DNA-lipid complexes for transfection are prepared according to manufacturer's protocols. Alternatively, mRNA and RNP complexes can also be used.

Add complexes to the plates with freshly trypsinized cells seeded the previous day.

Remove culture media 72 hours after transfection, trypsinize cells and re-seed in a new plate with double the surface area of the previous plate.

On the following day, add diphtheria toxin at a concentration of 20 ng/mL to the wells. After 2 days, perform a new diphtheria toxin treatment.

Monitor cell growth, and when necessary, pass cells to bigger plates or flasks until all cells of the negative selection have died.

Analyze the cells after 1-2 weeks by next-generating sequence to determine the efficiency of editing.

### Example 2. Screening of Guide RNA

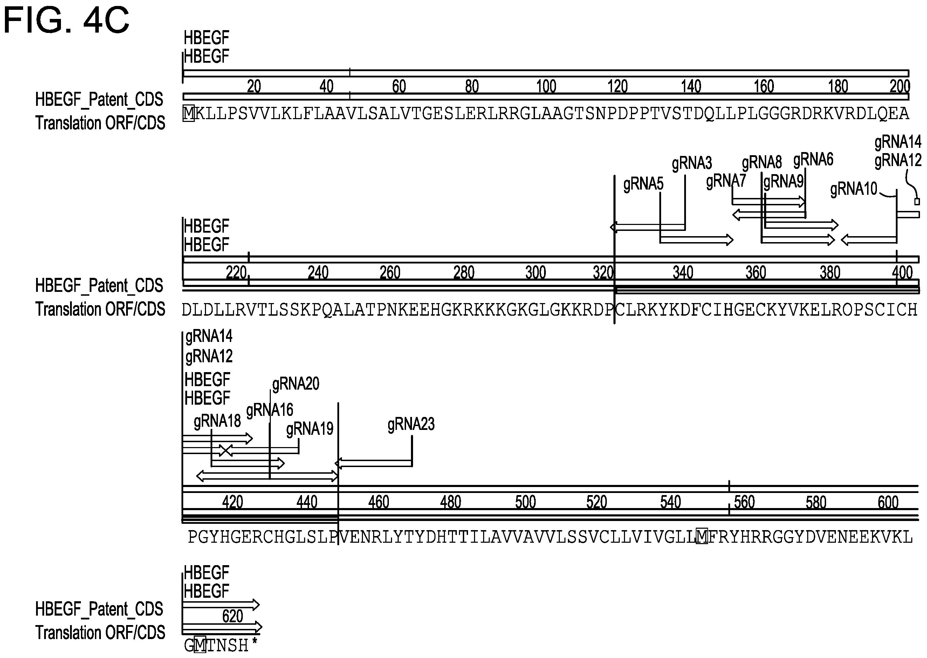

In this Example, guide RNAs (gRNA) were screened to identify a gRNA that, when co-transfected with BE3, will result in resistance to diphtheria toxin. A panel of gRNAs were designed to tile through the EGF-like domain of HB-EGF (see FIG. 4C). Each gRNA was co-transfected with BE3 at a transfection weight ratio of 1:4 into HEK293 or HCT116 cells.

The cells were treated with 20 ng/mL of diphtheria toxin at day 3 after transfection, then treated again at day 5 after transfection. Cell growth was measured by confluence using INCUCYTE ZOOM.

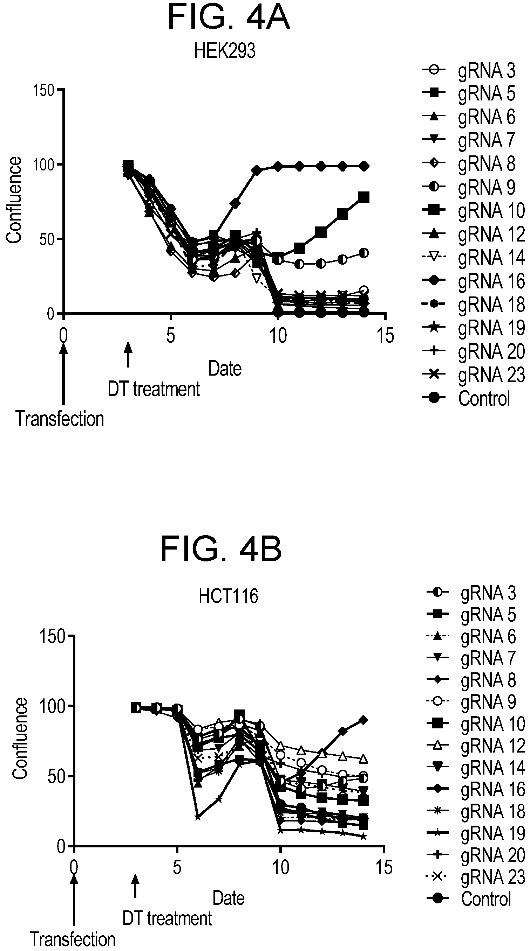

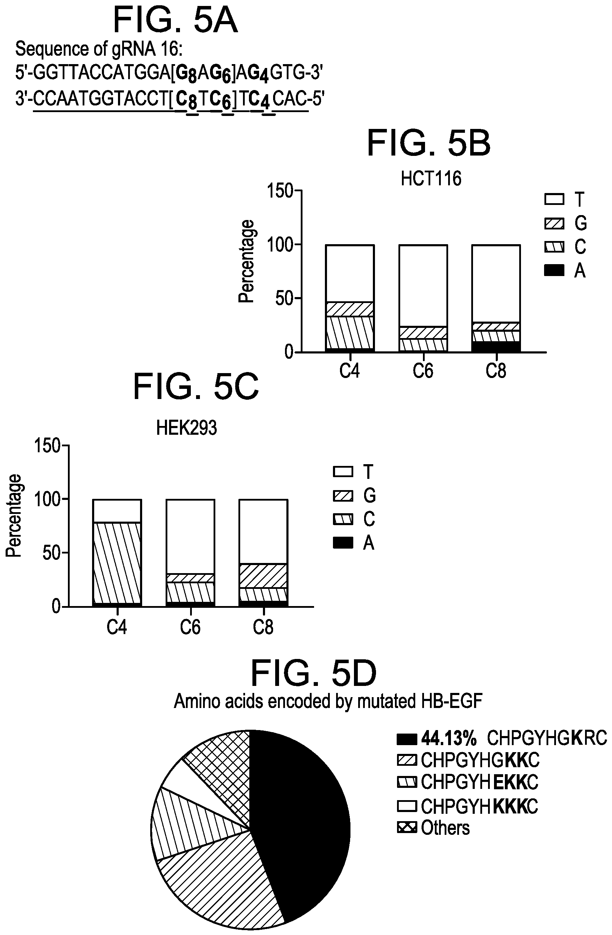

Results shown in FIGS. 4A and 4B respectively show that HEK293 and HCT116 cells co-transfected with HB-EGF gRNA 16 and BE3 had the highest level of growth among all the transfected cells. The results of sanger sequencing and next-generation sequencing analysis, shown in FIGS. 5B-5D, revealed that resistance to diphtheria toxin in gRNA 16-transfected cells was a result of the E141K mutation introduced by BE3 base-editing. The sequence of gRNA 16 is shown in FIG. 5A.

### Example 3. Co-Targeting Enrichment with BE3 and Cas9

In this Example, the co-targeting enrichment using diphtheria toxin selection was tested using BE3 and Cas9, with co-transfection of a targeting gRNA and gRNA 16 identified in Example 2 to generate diphtheria toxin-resistant cells.

### Plasmid Construction

Cas9 plasmid: DNA sequence encoding SpCas9, T2A self-cleavage peptide, and puromycin N-acetyltransferase was synthesized by GeneArt and cloned into an expression vector with a CMV promoter and a BGH polyA tail. See FIG. 15 for the plasmid map.

BE3 plasmid. DNA sequence of Base editor 3 was synthesized and cloned into pcDNA3.1(+) by GeneArt using restriction site BamHI and XhoI. See FIG. 14 for the plasmid map.

gRNA plasmid. Target sequences of gRNAs were introduced into a template plasmid at AarI cutting site using complementary primer pairs (5'-AAAC-N20-3' and 5'-ACCG-N20-3'). The template plasmid was synthesized by GeneArt. It contains a U6 promoter driving gRNA expression cassette, in which a rpsL-BSD selection cassette was cloned in the region of gRNA target sequence with two AarI restriction sites flanking. Primers can be found in Table 1. Plasmids for gRNA targeting BFP and EGFR are described in Coelho et al., BMC Biology 16:150 (2018) and shown in FIGS. 17-23.

**Table 1. Primers**

| | |
|---|---|
| gRNA DPM2_F | ACCGAATCACCCAGGCGGTGTAGT (SEQ ID NO: 9) |
| gRNA DPM2_R | AAACACTACACCGCCTGGGTGATT (SEQ ID NO: 10) |
| gRNA PCSK9_F | ACCGCAGGTTCCACGGGATGCTCT (SEQ ID NO: 11) |
| gRNA PCSK9_R | AAACAGAGCATCCCGTGGAACCTG (SEQ ID NO: 12) |
| gRNA Yas85_F | ACCGGCACTGCGGCTGGAGGTGG (SEQ ID NO: 13) |
| gRNA Yas85_R | AAACCCACCTCCAGCCGCAGTGC (SEQ ID NO: 14) |
| HBEGF gRNA16_F | ACCGCACCTCTCTCCATGGTAACC (SEQ ID NO: 15) |
| HBEGF gRNA16_R | AAACGGTTACCATGGAGAGAGGTG (SEQ ID NO: 16) |
| gRNA CTR_F | ACCGGCGTCGTCGGTCGCGATTAA (SEQ ID NO: 17) |
| gRNA CTR_R | AAACTTAATCGCGACCGACGACGC (SEQ ID NO: 18) |
| gRNA SaW10_F | ACCGGGGTGATGTTGCCTGACCGG (SEQ ID NO: 19) |
| gRNA SaW10_R | AAACCCGGTCAGGCAACATCACCC (SEQ ID NO: 20) |
| PCR2_F primer | CTTTGGCCACGTTGTGAGAGA (SEQ ID NO: 21) |
| PCR2_R primer | GGATGTTTGCAGCCTGACG (SEQ ID NO: 22) |
| PCR1_F primer | GAGTGCTTTTCTCCTACAGTCAC (SEQ ID NO: 23) |
| PCR1_R primer | TTCAAGTAGTCGGGGATGTC (SEQ ID NO: 24) |
| HBEGF_gRNA16_N GS_F | TCGTCGGCAGCGTCAGATGTGTATAAGAGACAGAAAGCACTAACTCCATCTCC (SEQ ID NO: 25) |
| HBEGF_gRNA16_N GS_R | GTCTCGTGGGCTCGGAGATGTGTATAAGAGACAGACAGCCACCACGGCCAGGAT (SEQ ID NO: 26) |
| EGFR_NGS_F | TCGTCGGCAGCGTCAGATGTGTATAAGAGACAGCATTCATGCGTCTTCACCT (SEQ ID NO: 27) |
| EGFR_NGS_R | GTCTCGTGGGCTCGGAGATGTGTATAAGAGACAGATATTGTCTTTGTGTTCCCG (SEQ ID NO: 28) |
| EMX1_NGS_F | TCGTCGGCAGCGTCAGATGTGTATAAGAGACAGTTCCAGAACCGGAGGACAAAG (SEQ ID NO: 29) |
| EMX1_NGS_R | GTCTCGTGGGCTCGGAGATGTGTATAAGAGACAGCCACCCTAGTCATTGGAGGT (SEQ ID NO: 30) |
| Yas85_NGS_F | TCGTCGGCAGCGTCAGATGTGTATAAGAGACAGAGGCAGAGGGTCCAAAGCAG (SEQ ID NO: 31) |
| Yas85_NGS_R | GTCTCGTGGGCTCGGAGATGTGTATAAGAGACAGATCAGAAGCCCTAAGCGGGA (SEQ ID NO: 32) |
| DPM2_NGS_F | TCGTCGGCAGCGTCAGATGTGTATAAGAGACAGCTCCCTTTTCTCCAGGCCAC (SEQ ID NO: 33) |
| DPM2_NGS_R | GTCTCGTGGGCTCGGAGATGTGTATAAGAGACAGATAGTAGTTGCTCTGGCGGT (SEQ ID NO: 34) |

### Cell Culture and Transfection

HEK293T and HCT116 cells, obtained from ATCC, were maintained in Dulbecco's modified Eagle's medium (DMEM) supplemented with 10% fetal bovine serum (FBS). PC9-BFP cells were maintained in DMEM medium with 10% FBS.

Transfection were performed using FUGENE HD Transfection Reagent (Promega), using a 3:1 ratio of transfection reagent to DNA according to instructions. Transfections in this study were performed in 24 well plate and 48 well plate. 1.25×10⁵ and 6.75 ×10⁴ cells were seeded in 24 well and 48 well plates, 24 hours before transfection, respectively. Transfection were performed using 500 ng and 250 ng total DNA for 24 well and 48 well plate, respectively

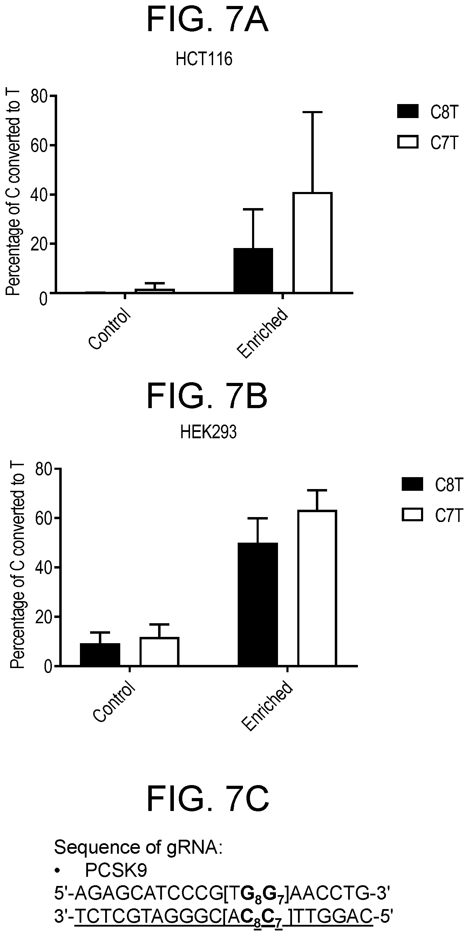

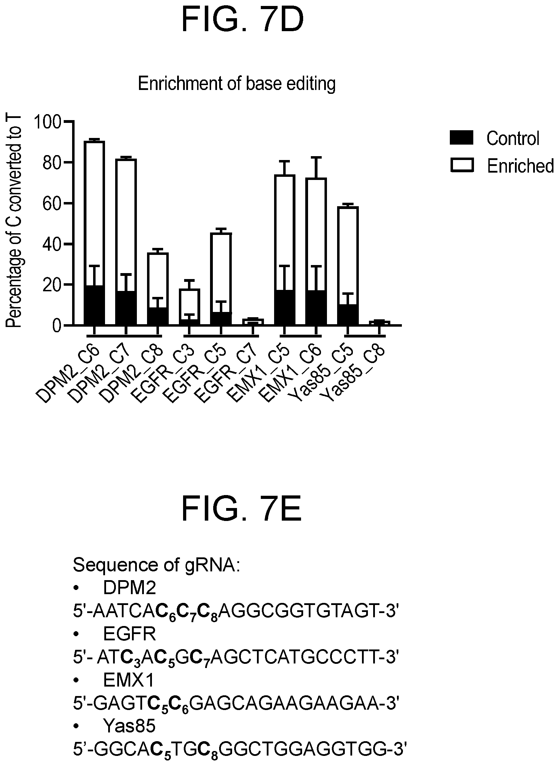

For co-targeting enrichment, Cas9 or BE3 plasmid DNA, targeting gRNA plasmid DNA and selection gRNA plasmid DNA were transfected at a weight ratio of 8:1:1. The sequence of the targeting gRNA for the PCKS9 site is shown in FIG. 7C, and the sequences of the targeting gRNAs for the DPM2, EGFR, EMX1, and Yas85 sites are shown in FIG. 7E. Cells were treated with 20 ng/ml diphtheria toxin 3 days after transfection, and then treated again 5 days after transfection. Harvest cells for downstream application when cells grow to >80% confluence. For all the cell types used in this study, cells were harvested 7 days after transfection for genomic extraction. For other different cell lines or primary cells, different dose of diphtheria toxin and treatment time can be applied to kill all wild type cells.

### Next-Generation Sequencing and Data Analysis

Genomic DNA were extracted from cells 72 hours after transfection or after treatment using QUICKEXTRACT DNA Extraction Solution (Lucigen) according to instructions. NGS libraries were prepared via two steps of PCR. First PCR were performed using NEBNEXT Q5 Hot Start HiFi PCR Master Mix (New England Biolabs) according to instructions. Second PCR was performed using 1 ng product from first PCR using KAPA HiFi PCR Kit (KAPABIOSYSTEMS). PCR products were purified using Agencourt AMPure XP (Beckman Coulter) and analyzed by Fragment analyzer.

Results in FIGS. 7A and 7B show the BE3 base-editing efficiency of different cytosines in the PCSK9 target site in HCT116 and HEK293 cells, respectively. The "control" condition shows a relatively low base-editing efficiency without diphtheria toxin selection, while the "enriched" condition shows drastically higher base-editing efficiency when diphtheria toxin selection was utilized. Results in FIG. 7D shows an increase in base-editing efficiency at different cytosines in the DPM2, EGFR, EMX1, and Yas85 target sites when diphtheria toxin selection was utilized ("enriched") compared to the "control" condition without diphtheria toxin.

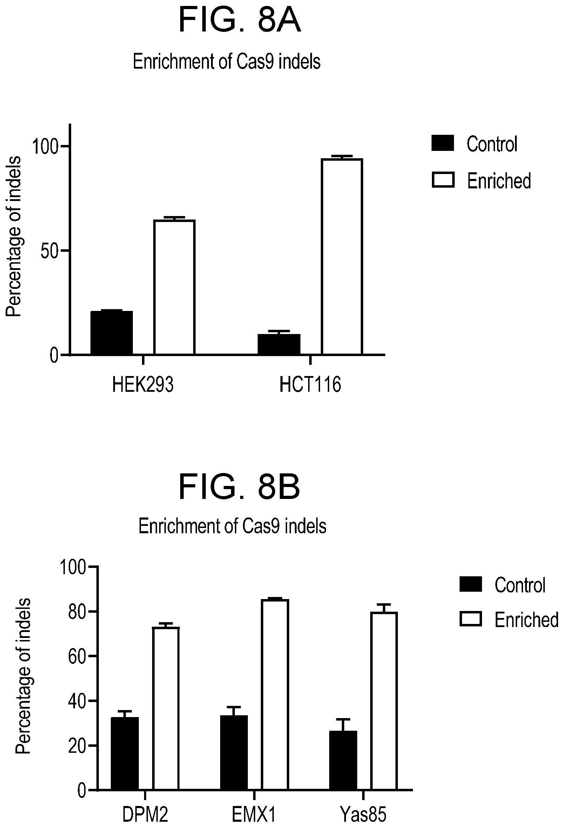

Results in FIG. 8A show the Cas9 editing efficiency by measuring the percentage of indels generated at the PCSK9 target site in HEK293 and HCT116 cells. As with base-editing, Cas9 editing efficiency increased significantly in the "enriched" condition, which used diphtheria toxin selection, over the "control" condition that did not use diphtheria toxin selection. Results in FIG. 8B show similar increases in Cas9 editing efficiency at the DPM2, EXM1, and Yas85 target sites.

### Example 4. Bi-Allelic Integration

In this Example, diphtheria toxin selection was tested to improve knock-in (insertion) efficiency of a gene of interest to achieve bi-allelic integration.

Donor plasmid for knock-in. Knock-in plasmid for mCherry was synthesized by Genescripts. See FIG. 23 for the plasmid map, and FIG. 10A for the experimental design.

For knock-in experiment, transfection was performed in 24 well plate format. Cas9 plasmid DNA, gRNA plasmid DNA and an mCherry knock-in (KI) or control plasmid DNA were transfected at different weight ratios in different conditions as shown in Table 2. Cells were treated with 20 ng/ml diphtheria toxin 3 days after transfection, then treated again 5 days after transfection. Afterwards, cells were maintained in fresh medium without diphtheria toxin. 13 days after transfection, genomes for all samples were harvested for PCR analysis. 22 days after transfection, cells with transfection condition 3, transfection negative control 1 and 2, and a mCherry positive control cell line were resuspended and analyzed by FACS.

**Table 2**

| | Cas9 or BE3 plasmid (ng) | gRNA plasmid (ng) | mCherry Knock-in template plasmid (ng) |
|---|---|---|---|
| Cas9 + gSaW10 + KI (Condition 1) | 320 | 80 | 200 |
| Cas9 + gSaW10 + KI (Condition 2) | 240 | 60 | 300 |
| Cas9 + gSaW10 + KI (Condition 3) | 160 | 40 | 400 |
| Cas9 + gRNA16 (Negative control 1) | 480 | 120 | |
| BE3 + gRNA16 (Negative control 2) | 480 | 120 | |

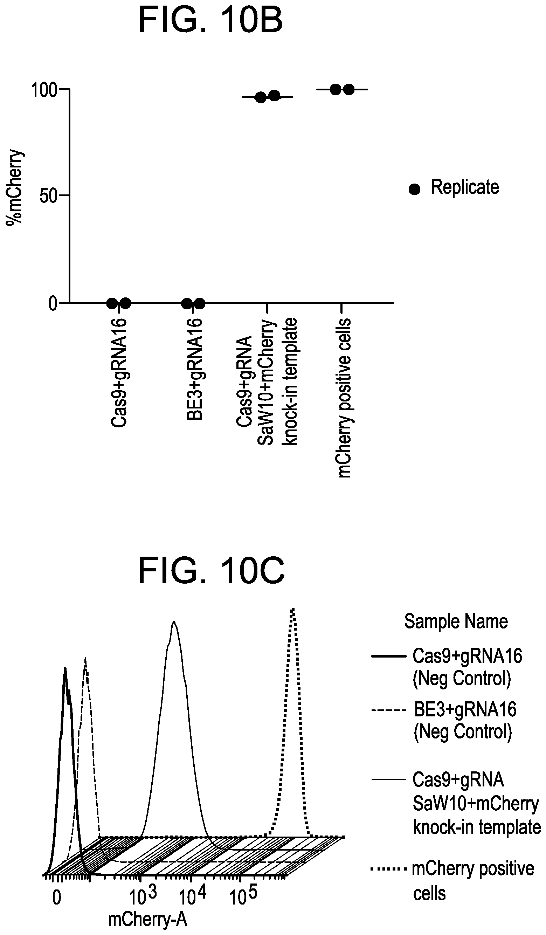

Cells with successful insertions would translate mCherry with the mutated HB-EGF gene, and the cells would show mCherry fluorescence. As shown in FIG. 10B, after diphtheria toxin selection, almost all cells transfected with Cas9, gRNA SaW10, and mCherry HDR template are mCherry positive, while cells without the mCherry donor plasmid did not show any mCherry fluorescence. FIG. 10C shows expression of mCherry is homogenous across the whole population (FIG. 10C).

FIGS. 10E and 10F show the PCR analysis results using the strategy outlined in FIG. 10D. A first PCR reaction (PCR1) amplifies the junction region with forward primer (PCR1_F primer) binding a sequence in the genome and reverse primer (PCR1_R primer) binding a sequence in the GOI. Thus, only cells with GOI integrated would show a positive band with PCR1. A second PCR reaction (PCR2) amplifies the insertion region with forward primer (PCR2_F primer) binding a sequence in the 5' end of the insertion and reverse primer (PCR2_R primer) binding a sequence at the 3' end of the insertion. Thus, PCR2 amplification only occurs if all alleles in the cells were inserted sucessfully with the GOI, and the amplified product would be shown as a single integrant band. If any wild type allele exists, a WT band would be shown.

FIG. 10E shows positive bands for all conditions tested that included introduction of the Cas9, gRNA, mCherry donor plasmids, indicating that insertions were successfully achieved. The single integrant bands for all three conditions in FIG. 10F indicate that no wild-type alleles exist in the tested cells, i.e., bi-allelic integration was achieved.

### Example 5. Detailed Experimental Protocol

An experimental protocol relating to the subsequent Examples is provided.

### Plasmids and Template DNA Construction

Plasmids expressing *S. pyogenes* Cas9 (SpCas9) were constructed by cloning GeneArt-synthesized sequence encoding a codon-optimized SpCas9 fused to a nuclear localization signal (NLS) and a self-cleaving puromycin-resistant protein (T2A-Puro) into a pVAX1 vector. Two vesions of the SpCas9 plasmids were constructed to drive expression of the SpCas9 under control of the CMV promoter (CMV-SpCas9) or the EF1α promoter (EF1α-SpCas9). Cytidine base editor 3 (CBE3) was synthesized using its published sequence and cloned into pcDNA3.1(+) vector by GeneArt. Two versions of the plasmid were constructed to control CBE3 expression under CMV promoter (CMV-CBE3) or EF1α promoter (EF1α-CBE3). Likewise, adenine base editor 7.10 (ABE7.10) was synthesized using its published sequence and cloned into pcDNA3.1(+) vector. Two versions of the plasmid were constructed to control ABE7.10 expression under CMV promoter (CMV- ABE7.10) or EF1α promoter (EF1α- ABE7.10). Individual sequence components were ordered from a Integrated DNA Technolgies (IDT) and assembled using Gibson assembly (New England Biolabs).

Plasmids expressing different sgRNAs were cloned by replacing the target sequence of the template plasmid. Complementary primer pairs containing the target sequence (5'-AAAC-N20-3' and 5'-ACCG-N20-3') were annealed (95 °C 5 min, then ramp down to 25 °C at 1 °C/min) and assembled with *Aar*I-digested template using T4 ligase. All primer pairs are listed in Table 3A. The plasmid expressing sgRNA targeting BFP and the plasmid expressing sgRNA targeting EGFR and CBE3 are described in a previous publication.

The plasmids acting as repair templates for *HBEGF* or *HIST2BC* loci were ordered from GenScript or modified using Gibson assembly. Individual sequence components were ordered from IDT. Template plasmids for *HBEGF* locus were designed to contain a strong splicing acceptor sequence, followed by the mutated CDS sequence of *HBEGF* starting from exon 4 and a self-cleaving mCherry coding sequence, encoded by a polyA sequence. Template plasmids for *HIST2BC* were designed to contain a GFP coding sequence followed by a self-cleaving blasticidin-resistance protein coding sequence. For both loci, pHMEJ and pHR were designed to contain left and right hmology arms flanking the insertion sequence, while pNHEJ was designed to contain no homology arms. pHMEJ was designed to contain one sgRNA cutting site flanking each homology arm, while pHR did not contain the site. For comparing puromycin selection with DT selection, a self-cleaving puromycin-resistant protein coding sequence was inserted between the *HBEGF* exon sequence and the self-cleaving mCherry coding sequence (pHMEJ_PuroR).

Double-stranded DNA (dsDNA) templates were prepared by PCR amplification of the plasmid pHMEJ with primers listed in Table 3B, followed by purification with MAGBIO magnetic SPRI beads. PCR amplification was performed using high-fidelity PHUSION polymerase. ssDNA templates were prepared using the GUIDE-IT^{™} Long ssDNA Production System (Takara Bio) with primers listed in Tables 3A-3E. Final products were purifed by MAGBIO magnetic SPRI beads and analyzed by Fragment Analyzer (Agilent). The template for the *CD34* locus was ordered from IDT as a PAGE-purified oligonucleotide.

**Table 3A. sgRNA Cloning Primers**

| **sgRNA cloning primers** | **Sequence** | **SEQ ID NO:** |
|---|---|---|
| HBEGF_sgRNA1_fwd | ACCG CCTTGTATTTCCGAAGACAT | 35 |
| HBEGF_sgRNA2_fwd | ACCG TACAAGGACTTCTGCATCCA | 36 |
| HBEGF_sgRNA3_fwd | ACCG TCACATATTTGCATTCTCCA | 37 |
| HBEGF_sgRNA4_fwd | ACCG TGGAGAATGCAAATATGTGA | 38 |
| HBEGF_sgRNA5_fwd | ACCG GCAAATATGTGAAGGAGCTC | 39 |
| HBEGF_sgRNA6_fwd | ACCG CAAATATGTGAAGGAGCTCC | 40 |
| HBEGF_sgRNA7_fwd | ACCG CTTACATGCAGGAGGGAGCC | 41 |
| HBEGF_sgRNA8_fwd | ACCG AGCTGCCACCCGGGTTACCA | 42 |
| HBEGF_sgRNA9_fwd | ACCG ACCCGGGTTACCATGGAGAG | 43 |
| HBEGF_sgRNA10_fwd | ACCG CACCTCTCTCCATGGTAACC | 44 |
| HBEGF_sgRNA11_fwd | ACCG ACCATGGAGAGAGGTGTCAT | 45 |
| HBEGF_sgRNA12_fwd | ACCG GCCCATGACACCTCTCTCCA | 46 |
| HBEGF_sgRNA13_fwd | ACCG TCATGGGCTGAGCCTCCCAG | 47 |
| HBEGF_sgRNA14_fwd | ACCG GTATATAAGCGATTTTCCAC | 48 |
| HBEGF_sgRNA1_rev | AAAC ATGTCTTCGGAAATACAAGG | 49 |
| HBEGF_sgRNA2_rev | AAAC TGGATGCAGAAGTCCTTGTA | 50 |
| HBEGF_sgRNA3_rev | AAAC TGGAGAATGCAAATATGTGA | 51 |
| HBEGF_sgRNA4_rev | AAAC TCACATATTTGCATTCTCCA | 52 |
| HBEGF_sgRNA5_rev | AAAC GAGCTCCTTCACATATTTGC | 53 |
| HBEGF_sgRNA6_rev | AAAC GGAGCTCCTTCACATATTTG | 54 |
| HBEGF_sgRNA7_rev | AAAC GGCTCCCTCCTGCATGTAAG | 55 |
| HBEGF_sgRNA8_rev | AAAC TGGTAACCCGGGTGGCAGCT | 56 |
| HBEGF_sgRNA9_rev | AAAC CTCTCCATGGTAACCCGGGT | 57 |
| HBEGF_sgRNA10_rev | AAAC GGTTACCATGGAGAGAGGTG | 58 |
| HBEGF_sgRNA11_rev | AAAC ATGACACCTCTCTCCATGGT | 59 |
| HBEGF_sgRNA12_rev | AAAC TGGAGAGAGGTGTCATGGGC | 60 |
| HBEGF_sgRNA13_rev | AAAC CTGGGAGGCTCAGCCCATGA | 61 |
| HBEGF_sgRNA14_rev | AAAC GTGGAAAATCGCTTATATAC | 62 |
| PCSK9_sgRNA_fwd | ACCG CAGGTTCCACGGGATGCTCT | 63 |
| PCSK9_sgRNA_rev | AAAC AGAGCATCCCGTGGAACCTG | 64 |
| EMX1_sgRNA_fwd | ACCG GAGTCCGAGCAGAAGAAGAA | 65 |
| EMX1_sgRNA_rev | AAAC TTCTTCTTCTGCTCGGACTC | 66 |
| DPM2_sgRNA_fwd | ACCG AATCACCCAGGCGGTGTAGT | 67 |
| DPM2_sgRNA_rev | AAAC ACTACACCGCCTGGGTGATT | 68 |
| DNMT3B_sgRNA_fwd | ACCG GCACTGCGGCTGGAGGTGG | 69 |
| DNMT3B_sgRNA_rev | AAAC CCACCTCCAGCCGCAGTGC | 70 |
| Neg Control_sgRNA_fwd | ACCG GCGTCGTCGGTCGCGATTAA | 71 |
| Neg Control_sgRNA_rev | AAAC TTAATCGCGACCGACGACGC | 72 |
| PDCD1_sgRNA_fwd | ACCG GGGGTTCCAGGGCCTGTCTG | 73 |
| PDCD1_sgRNA_rev | AAAC CAGACAGGCCCTGGAACCCC | 74 |
| CTLA4_sgRNA_fwd | ACCG GGCCCAGCCTGCTGTGGTAC | 75 |
| CTLA4_sgRNA_rev | AAAC GTACCACAGCAGGCTGGGCC | 76 |
| IL2RA_sgRNA1_fwd | ACCG CAATGTCAATGCACAAGCTC | 77 |
| IL2RA_sgRNA1_rev | AAAC GAGCTTGTGCATTGACATTG | 78 |
| IL2RA_sgRNA2_fwd | ACCG GTGGACCAAGCGAGCCTTCC | 79 |
| IL2RA_sgRNA2_rev | AAAC GGAAGGCTCGCTTGGTCCAC | 80 |
| HIST2BC_sgRNA_fwd | ACCG GCTTACTTGGAATGTTTACT | 81 |
| HIST2BC_sgRNA_rev | AAAC AGTAAACATTCCAAGTAAGC | 82 |
| CD34_sgRNA_fwd | ACCG TTCATGAGTCTTGACAACAA | 83 |
| CD34_sgRNA_rev | AAAC TTGTTGTCAAGACTCATGAA | 84 |
| HBEGF_sgRNAIn3_fwd | ACCG GGGTGATGTTGCCTGACCGG | 85 |
| HBEGF_sgRNAIn3_rev | AAAC CCGGTCAGGCAACATCACCC | 86 |

**Table 3B. Primers for dsDNA and ssDNA Template Generation**

| **Primers for dsDNA and ssDNA template generation** | **Sequence** | **SEQ ID NO:** | **Size (bp)** | **Anneali ng temp (°C)** | **Elongati on time (s)** |
|---|---|---|---|---|---|
| dsHMEJ_fwd | GACCGAGATAGGGTTGAGTG | 87 | 3925 | 62.3 | 150 |
| dsHMEJ_rev | CACCCCAGGCTTTACCCGAA | 88 | | | |
| dsHR_fwd | GCGTCCATGTCTTCGGAA | 89 | 3436 | 62.6 | 150 |
| dsHR_rev | ATAAGGCCTCTCAACCACAC | 90 | | | |
| dsHR2_fwd | CGTTGTAAAACGACGGCCAG TCCCCCGGTCAGGCAACAGA ACCCGAGCGCGACGTAATA | 91 | 3580 | 62.6 | 150 |
| dsHR2_rev | CATGTTAATGCAGCTGGCAC ATGTTGCCTGACCGGGGGAT AAGGCCTCTCAACCACAC | 92 | | | |
| ssHR_fwd | GCGTCCATGTCTTCGGAA | 93 | 3436 | 62.6 | 150 |
| ssHR_rev | ATAAGGCCTCTCAACCACAC (5′-Phosphorylated) | 94 | | | |

**Table 3C. Next Generation Sequencing Primers**

| **NGS primers** | **Sequence** | **SEQ ID NO:** | **Amplico n Size (bp)** | **Anneali ng temp (°C)** | **Elongati on time (s)** |
|---|---|---|---|---|---|
| HBEGFg5_ _NGS_F | TCGTCGGCAGCGTCAGATGTGTATAAGAGACAG CGGGAAAAGAAAGAAGAAAG | 95 | 171 | 59 | 10 |
| HBEGFg5_ NGS_R | GTCTCGTGGGCTCGGAGATGTGTATAAGAGACAG ACAAAGTGTGCTGATGAGAT | 96 | | | |
| HBFGFg10 _NGS_F | TCGTCGGCAGCGTCAGATGTGTATAAGAGACAG AAAGCACTAACTCCATCTCC | 97 | 147 | 62 | 10 |
| HBFGFg10 _NGS_R | GTCTCGTGGGCTCGGAGATGTGTATAAGAGACAG ACAGCCACCACGGCCAGGAT | 98 | | | |
| PCSK9_N GS_F | TCGTCGGCAGCGTCAGATGTGTATAAGAGACAG ATGTGGGGACAGGTTTGATC | 99 | 216 | 66 | 10 |
| PCSK9_N GS_R | GTCTCGTGGGCTCGGAGATGTGTATAAGAGACAG TGGTATTCATCCGCCCGGTA | 100 | | | |
| EGFR_NG S_F | TCGTCGGCAGCGTCAGATGTGTATAAGAGACAG CATTCATGCGTCTTCACCT | 101 | 234 | 61 | 10 |
| EGFR_NG S_R | GTCTCGTGGGCTCGGAGATGTGTATAAGAGACAG ATATTGTCTTTGTGTTCCCG | 102 | | | |
| EMX1_NG S_F | TCGTCGGCAGCGTCAGATGTGTATAAGAGACAG TTCCAGAACCGGAGGACAAAG | 103 | 161 | 67 | 10 |
| EMX1_NG S_R | GTCTCGTGGGCTCGGAGATGTGTATAAGAGACAG CCACCCTAGTCATTGGAGGT | 104 | | | |
| DNMT3B_ NGS_F | TCGTCGGCAGCGTCAGATGTGTATAAGAGACAG AGGCAGAGGGTCCAAAGCAG | 105 | 252 | 69 | 10 |
| DNMT3B_ NGS_R | GTCTCGTGGGCTCGGAGATGTGTATAAGAGACAG ATCAGAAGCCCTAAGCGGGA | 106 | | | |
| DPM2_NG S_F | TCGTCGGCAGCGTCAGATGTGTATAAGAGACAG CTCCCTTTTCTCCAGGCCAC | 107 | 171 | 67 | 10 |
| DPM2_NG S_R | GTCTCGTGGGCTCGGAGATGTGTATAAGAGACAG ATAGTAGTTGCTCTGGCGGT | 108 | | | |
| AAVS1_N GS_F | TCGTCGGCAGCGTCAGATGTGTATAAGAGACAG GCCCCCTGTCATGGCATCTT | 109 | 293 | 68 | 10 |
| AAVS1_N GS_R | GTCTCGTGGGCTCGGAGATGTGTATAAGAGACAG GTGGGGGTTAGACCCAATATCAG | 110 | | | |
| PDCD1_N GS_F | TCGTCGGCAGCGTCAGATGTGTATAAGAGACAG CCCTTCCTCACCTCTCTCCA | 111 | 144 | 68 | 10 |
| PDCD1_N GS_R | GTCTCGTGGGCTCGGAGATGTGTATAAGAGACAG CACGAAGCTCTCCGATGTGT | 112 | | | |
| CTLA4_N GS_F | TCGTCGGCAGCGTCAGATGTGTATAAGAGACAG TAGAAGGCAGAAGGGCTTGC | 113 | 172 | 68 | 10 |
| CTLA4_N GS_R | GTCTCGTGGGCTCGGAGATGTGTATAAGAGACAG AGTGGCTTTGCCTGGAGATG | 114 | | | |
| CD25g1_N GS_F | TCGTCGGCAGCGTCAGATGTGTATAAGAGACAG AGCGGGTCACTCTATATGCTCT | 115 | 104 | 66 | 10 |
| CD25g1_N GS_R | GTCTCGTGGGCTCGGAGATGTGTATAAGAGACAG TGGTAGTCACAGAAGGACAC | 116 | | | |
| CD25g2_N GS_F | TCGTCGGCAGCGTCAGATGTGTATAAGAGACAG AAACAAGTGACACCTCAACCTG | 117 | 134 | 66 | 10 |
| CD25g2_N GS_R | GTCTCGTGGGCTCGGAGATGTGTATAAGAGACAG CGCTAGCAGGAGTTAGCTGGA | 118 | | | |
| mPCSK9_ NGS_F | GTCTCGTGGGCTCGGAGATGTGTATAAGAGACAG AGTGCAGACTCTGGAGCCCTGA | 119 | 218 | 72 | 10 |
| mPCSK9_ NGS_R | TCGTCGGCAGCGTCAGATGTGTATAAGAGACAG CTGTAGGCCCTGAAGTTGCCCC | 120 | | | |

**Table 3D. Primers for Knock-In Analysis**

| **Primers for knock-in analysis** | **Sequence** | **SEQ ID NO:** | **Amplicon Size (bp)** | **Annealing temp (°C)** | **Elongatio n time (s)** |
|---|---|---|---|---|---|
| PCR1_fwd | GAGTGCTTTTCTCCTACAGTCAC | 121 | 1509 | 62 | 60 |
| PCR1_rev | TTCAAGTAGTCGGGGATGTC | 122 | | | |
| PCR2_fwd | CTTTGGCCACGTTGTGAGAGA | 123 | 280 | 64.5 | 5 |
| PCR2_rev | GGATGTTTGCAGCCTGACG | 124 | | | |

**Table 3E. Oligonucleotide Template and Neon Enhancer**

| **Oligo template and neon enhancer** | **Sequence** | **SEQ ID NO:** | **Modification** |
|---|---|---|---|
| Oligo_CD34 | T*T*TGTAGAAACATTTGAAAATGTTCCCTGGGTA GGTAACTCTGGGGTAGCAGTACCGTTGGTTTAATT GAGTTGCAATTGGTTAATAACGGTATTTGTCAAGA CTCATGAACCCAGAAGCTATAGGGAAACGAGGAGG AAGAATCAGAACCT*A*A | 125 | * Phosphorothioate Bond |
| Electroporation enhancer oligos | TTATTAGGATATTTTTATTTTTTATTTTTTTTTTT TTTTTTTGGATAATTATTATTTTATTATTTATTTT TTTTTTATTAAATATTTTAAGGATA | 126 | |

### Cell Culture

HEK293 (ATCC, CRL-1573), HCT116 (ATCC, CCL-247), and PC9-BFP cells were maintained in Dulbecco's modified Eagle medium (DMEM) supplemented with 10% fetal bovine serum. Human induced pluripotent stem cells (iPSCs) were maintained in the Cellartis DEF-CS 500 Culture System (Takara Bio) according to manufacturer instructions. All cell lines were cultured at 37 °C with 5% CO₂. Cell lines were authenticated by STR profiling and tested negative for mycoplasma.

### T-Cell Isolation, Activation, and Propagation

Blood from healthy donors was obtained from AstraZeneca's blood donation center (Mölndal, Sweden). Peripheral blood mononuclear cells were isolated from fresh blood using Lympoprep (STEMCELL Technologies) density gradient centrifugation and total CD4+ T cells were enriched by negative selection with the EasySep Human CD4+ T Cell Enrichment Kit (STEMCELL Technologies. Enriched CD4+ T cells were further purified by fluorescence-activated cell sorting (FACSAria III, BD Biosciences) based on exclusion of CD8+ CD14+ CD16+ CD19+ CD25+ cell surface markers to an average purity of 98%. The following antibodies were purchased from BD Biosciences: CD4-PECF594 (RPA-T4), CD25-PECy7 (M-A251), CD8-APCCy7 (RPA-T8), CD14-APCCy7 (MϕP-9), CD16-APCCy7 (3G8), CD19-APCCy7 (SJ25-C1), CD45RO-BV510. (UCHL1). Cell sorting was performed using a FACSAria III (BD Biosciences).

CD4+ T cells were propagated in RPMI-1640 medium containing the following supplements: 1% (v/v) GlutaMAX-I, 1% (v/v) non-essential amino acids, 1 mM sodium pyruvate, 1% (v/v) L-glutamine, 50 U/mL penicillin and streptomycin and 10% heat-inactivated FBS (all from Gibco, life Technologies). T cells were activated using the T Cell Activation/Expansion kit (130-091-441, Miltenyi). 1×10⁶ cells/mL were activated at bead-to-cell ratio of 1:2 and 2×10⁵ cells per well were seeded into round-bottom tissue culture-treated 96-well plates for 24 hours. Cells were pooled prior to electroporation.

### Cell Transfection

24 hours prior to transfection, 1.25×10⁵ or 6.75×10⁴ HEK293, HCT116, and PC9-BFP cells were seeded in 24-well or 48-well plates, respectively. Transfections were performed with FuGENE HD Transfection Reagent (Promega) using a 3:1 transfection reagent to plasmid DNA ratio. For 24-well plate formats, the amount and weight ratios of transfected DNA are listed in Tables 4 and 5. For 48-well plate formats, the amount of DNA was reduced by half.

**Table 4. Transfection Amounts**

| | Genome editor/ sgRNA | Genome editor/ sgRNA1/ sgRNA2 | Genome editor/ sgRNA1/ *HBEGF* repair template | Genome editor/ sgRNA1/ *HBEGF* repair template/ sgRNA2 | Genome editor/ sgRNA2/ target repair template |
|---|---|---|---|---|---|
| Genome editor (SpCas9/CBE 3/ABE7.10) | 400 ng | 400 ng | 160 ng | 160 ng | 160 ng |
| sgRNA 1 (Selection sgRNA) | 100 ng | 50 ng | 40 ng | 20 ng | |
| sgRNA2 (Target sgRNA) | | 50 ng | | 20 ng | 40 ng |
| *HBEGF* repair template | | | 400 ng | 400 ng | |
| Target repair template | | | | | 400 ng |

**Table 5. Transfection Amounts for Co-Selection**

| | Target pHR: *HBEGF* pHR 2:1 | Target pHMEJ: *HBEGF* pHMEJ 1:1 | Target pHMEJ: *HBEGF* pHMEJ 3:1 | Target pHMEJ: *HBEGF* pHMEJ 4:1 | Target oligos: *HBEGF* pHR 2:1 |
|---|---|---|---|---|---|
| Genome editor (SpCas9/CBE3/ABE7.10) | 160 ng | 160 ng | 160 ng | 160 ng | 160 ng |
| sgRNA1 (Selection sgRNA) | 13.3 ng | 20 ng | 10 ng | 8 ng | 13.3 ng |
| sgRNA2 (Target sgRNA) | 26.7 ng | 20 ng | 30 ng | 32 ng | 26.7 ng |
| *HBEGF* repair template | 133 ng | 200 ng | 100 ng | 80 ng | 133 ng |
| Target repair template | 267 ng | 200 ng | 300 ng | 320 ng | |
| Target oligo | | | | | 267 ng |

iPSCs were transfected with FuGENE HD using a 2.5:1 transfection reagent to DNA ratio and a reverse transfection protocol. For transfections, 4.2×10⁴ cells were seeded per well in 48-well format directly onto prepared transfection complexes as described in Table 6.

**Table 6. Transfection of iPSCs**

| | Genome editor/ sgRNA | Genome editor/ sgRNA1/ sgRNA2 | Genome editor/ sgRNA1/ *HBEGF* repair template |
|---|---|---|---|
| Genome editor (SpCas9/CBE3/ABE7.10) | 200 ng | 200 ng | 66 ng |
| sgRNA1 (Selection sgRNA) | 50 ng | 25 ng | 17 ng |
| sgRNA2 (Target sgRNA) | | 25 ng | |
| *HBEGF* repair template | | | 167 ng |

CD4+ T cells were electroporated with ribonucleoprotein complexes (RNPs) using a 10 µL Neon transfection kit (MPK1096, ThermoFisher). CD3 proteins were produced using a previously described method. An extra purification step was performed on a HiLoad 26/600 Superdex 200 pg column (GE Healthcare) with a mobile phase including: 20 mM Tris-Cl pH 8.0, 200 mM NaCl, 10% glycerol, and 1 mM TCEP. Purified CBE3 protein was concentrated to 5 mg/mL in a Vivaspin protein concentrator spin column (GE Healthcare) at 4 °C, before flash freezing in small aliquots in liquid nitrogen. RNPs were prepared as follows: 20 µg CBE3 protein, 2 µg of target sgRNA, and 2 µg of selection sgRNA (TrueGuide Synthetic gRNA, Life Technologies), and 2.4 µg electroporation enhancer oligonucleotides (Sigma) (Table 3E) were mixed and incubated for 15 minutes. Cells were washed with PBS and resuspended in buffer R at a concentration of 5×10⁷ cells/mL. 5×10⁵ cells were electroporated with RNPs using the following settings: voltage: 1600 V, width: 10 ms, pulse number: 3. After electroporation, cells were incubated overnight in 1 mL of RPMI medium complemented with 10% heat-inactivated FBS in a 24-well plate. The next day, cells were collected, centrifuged at 300×*g* for 5 minutes, resuspended in 1 mL of complete growth medium containing 500 U/mL IL-2 (Prepotech), and split in to 5 wells of a round-bottom 96-well plate.

### Diphtheria Toxin (DT) Treatment

Transfected HEK293, HCT116, and PC9-BFP cells were selected with 20 ng/mL DT at day 3 and day 5 after transfection. iPSCs were treated with 20 ng/mL DT from day 3 after transfection. DT-supplemented growth medium was exchanged daily until negative control cells died. Transfected CD4+ T cells were treated with 1000 ng/mL DT at days 1, 4, and 7 after electroporation.

### Alamar Blue Assay

Cell viability was analyzed using the AlamarBlue cell viability reagent (ThermoFisher) according to manufacturer instructions.

### PCR Analysis

PCR analysis was performed to discriminate between successful knock-in into *HBEGF* intron 3 (PCR1) and wild-type sequence (PCR2). PCR reactions were performed in 20 µL volume using 1.5 µL of extracted genomic DNA as template. PHUSION (ThermoFisher) was used according to the manufacturer's recommended protocol with a primer concentrations of 0.5 µM. Primer pair PCR1_fwd and PCR1_rev was used for PCR1 to detect knock-injunctions (annealing temperature: 62 °C, elongation time: 1 min) and primer pair PCR2_fwd and PCR2_rev was used for PCR2 to detect wild-type *HBEGF* intron (annealing temperature: 64.5 °C, elongation time: 5 sec). Sequences of primer pairs are provided in Table 3D. For PCR2, the elongation time was set to 5 seconds to favor amplification of the wild-type *HBEGF* intron 3 product (280 bp) over the integrant PCT product (2229 bp).

### Flow Cytometry Analysis

The frequency of cells expressing mCherry and GFP was assessed with a BD Fortessa flow cytometer (BD Biosciences), and flow cytometry data were analyzed with the FlowJo software (Three Star).

### Genomic DNA Extractions and Next-Generation Amplicon Sequencing

Genomic DNA was extracted from cells three days after transfections or after completed DT selection using QuickExtract DNA extraction solution (Lucigen) according to manufacturer instruction. Amplicons of interest were analyzed from genomic DNA samples on a NextSeq platform (Illumina). Genomic sites of interest were amplified in a first round of PCR using primers that contained NGS forward and reverse adapters (Table 3C). The first PCR was set up using NEBNext Q5 Hot Start HiFi PCR Master Mix (New England Biolabs) in 15 µL reactions, with 0.5 µM of primers and 1.5 µL of genomic DNA. PCR was performed with the following cycling conditions: 98 °C for 2 min, 5 cycles of 98 °C for 10 s, annealing temperature for each pair of primers for 20 s (calculated using NEB Tm Calculator), and 65 °C for 10 s, then 25 cycles of 98 °C for 10 s, 98 °C for 20 s, and 65 °C for 10 s, followed by a final 65 °C extension for 5 min. PCR products were purified using HighPre PCR Clean-up System (MAGBIO Genomics), and correct PCR product size and DNA concentration were analyzed on a Fragment Analyzer (Agilent). Unique Illumina indexes were added to PCR products in a second round of PCR using KAPA HiFi HotStart Ready Mix (Roche). Indexing primers were added in a second PCR step, and 1 ng of purified PCR product from the first PCR was used as template in a 50 µL reaction. PCR was performed with the following cycling conditions: 72 °C for 3 min, 98 °C for 30 s, then 10 cycles of 98 °C for 10 s, 63 °C for 30 s, and 72 °C for 3 min, followed by a final 72 °C extension for 5 min. Final PCR products were purified using HighPre PCR Clean-up System (MAGBIO Genomics) and analyzed by Fragment analyzer (Agilent). Libraries were quantified using Qubit 4 Fluorometer (Life Technologies), pooled, and sequenced on a NextSeq instrument (Illumina).

### Bioinformatics

NGS sequencing data were demultiplexed using bcl2fastq software, and individual FASTQ files were analyzed using a Perl implementation of the Matlab script described in a previous publication. For the quantification of indel or base edit frequencies, sequencing reads were scanned for matches to two 10 bp sequences that flank both sides of an intervening window in which indels or base edits might occur. If no matches were located (allowing maximum 1 bp mismatch on each side), the read was excluded from the analysis. If the length of the intervening window was longer or shorter than the reference sequence, the sequencing read was classified as an insertion or deletion, respectively. The frequency of insertion or deletion was calculated as the percentage of reads classified as insertion or deletion within total analyzed reads. If the length of this intervening window exactly matched the reference sequence the read was classified as not containing an indel. For these reads, the frequencies of each base at each locus was calculated in the intervening window and was used as the frequencies of base edits.

### Cytidine Base Editing and DT Treatment of Mice Humanized for hHBEGF Expression

All mouse experiments were approved by the AstraZeneca internal committee for animal studies and the Gothenburg Ethics Committee for Experimental Animals (license number: 162-2015+) compliant with EU directives on the protection of animals used for scientific purposes. Experimental mice were generated as double heterozygotes by breeding Alb-Cre mice (The Jackson Laboratory) to iDTR mice (Expression of transgene, human HBEGF, is blocked by loxP-flanked STOP sequence) on the C57BL/6NCrl genetic background. Mice were housed in negative pressure IVC caging, in a temperature controlled room (21 °C) with a 12:12 h light-dark cycle (dawn: 5.30 am, lights on: 6.00 am, dusk: 5.30pm, lights off: 6pm) and with controlled humidity (45-55%). Mice had access to a normal chow diet (R36, Lactamin AB) and water *ad libitum.*

For base editing, 6-month-old mice, 6 male and 6 female, were randomized into 2 groups with equal male and female mice in each group. Adenoviral vectors expressing CBE3, sgRNA10 and sgRNA targeting mouse Pcsk9 (1 × 10⁹ IFU particles per mouse) were intravenously injected. Two weeks after virus administration, all mice received DT (200ng/kg) intraperitoneally. Control mice were terminated 24 h after DT injection. Experimental mice were terminated 11 days after DT injection. Four mice were terminated prior to experimental endpoint as the humane endpoint of the ethics license was reached. At necropsy, liver tissues were collected for morphological and molecular analyses.

### Example 6. Amino Acid Substitution in HBEGF

In this Example, base editing was used to scan for mutations in the human EGF-like domain that render cells resistant to diphtheria toxin (DT).

Detailed experimental protocols are described in Example 5. Briefly, for screening sgRNAs, each sgRNA was co-transfected together with CBE3 or ABE7.10 at a weight ratio of 1:4. Transfection was performed using FuGENE HD transfection reagent (Promega) according to the manufacturer's instructions using a 3:1 transfection reagent to plasmid DNA ratio. Cells were treated with 20 ng/mL diphtheria toxin 3 days after transfection, then treated again 5 days after transfection. Cell viability was analyzed using the AlamarBlue cell viability reagent (Thermo Fisher) according to manufacturer's instructions. Genomic DNA was extracted from surviving cells and analyzed by Amplicon-Seq using Next Generation Sequencing (NGS).

Fourteen single-guide RNAs (sgRNAs) tiling through the exon sequences encoding the human EGF-like domain, covering all regions that encode amino acids different from the mouse EGF-like domain (FIG. 24A). Each sgRNA was transiently expressed in HEK293 cells together with either cytidine base editor 3 (CBE3) or adenosine base editor 7.10 (ABE7.10). Corresponding mutations, C to T (by CBE3) or A to G (by ABE7.10), were introduced into the editing window of each sgRNA. Edited cells were treated with a lethal dose of DT (20 ng/µl for HEK293 cells) 72 hours after transfection, and cell proliferation was monitored. Results in FIG. 24B show that CBE3 in combination with sgRNA7 or sgRNA10 induced effective resistant mutations to DT in HBEGF, while ABE7.10 induced resistance in combination with sgRNA5 or sgRNA10.

The ABE7.10/sgRNA5 or CBE3/sgRNA10 combinations were selected for further analysis. Genomic DNA from resistant cells were harvested, and their corresponding targeted loci were analyzed by Amplicon-Seq using Next Generation Sequencing (NGS). The majority of mutations introduced by the combination of CBE3 and sgRNA10 in resistant cells resulted in the Glu141Lys substitution in HBEGF. Around 90% of variants introduced by the ABE7.10/sgRNA5 combination resulted in Tyr123Cys conversion in HBEGF (see FIG. 24C and FIGS. 25A-C). Compromised proliferation in edited cells as compared to wild-type cells was not observed, indicating no detrimental effect was introduced by the edited HBEGF variants (FIG. 25D).

Collectively, these data showed that resistance to DT can be introduced by modifying a single amino acid in the HBEGF protein using base-editing without altering cell proliferation. Thus, the DT-HBEGF system can be applied effectively to select for genome editing events in cells.

### Example 7. Enrichment of Cytidine and Adenosine Base Editing

In this Example, the DT-HBEGF selection system was tested for enrichment of base editing events at a second, unrelated genomic locus. FIG. 26A provides a schematic of the DT-HBEGF co-selection strategy.

Detailed experimental protocols are described in Example 5. Briefly, for co-targeting enrichment, Cas9/CBE3/ABE7.10 plasmid DNA, targeting sgRNA plasmid DNA, and selection sgRNA plasmid DNA were transfected at a weight ratio of 8:1:1. Transfection was performed using FuGENE HD transfection reagent (Promega) according to manufacturer's instructions using a 3:1 transfection reagent to plasmid DNA ratio. Cells were treated with 20 ng/mL diphtheria toxin 3 days after transfection, and then treated again 5 days after transfection. Genomic DNA was extracted from surviving cells and analyzed by Amplicon-Seq using Next Generation Sequencing (NGS).

First, CBE co-selection in HEK293 cells was performed. sgRNAs targeting five different genomic loci were tested: *DPM2* (Dolichyl-Phosphate Mannosyltransferase Subunit 2), *EGFR* (Epidermal growth factor receptor), *EMX1* (Empty Spiracles Homeobox 1), *PCSK9* (Proprotein convertase subtilisin/kexin type 9), and *DNMT3B* (DNA Methyltransferase 3 Beta). Each of these sgRNAs was co-transfected into cells with CBE3 and sgRNA10 as described in Example 6, and the selected cells were enriched with DT (20 ng/µl) starting from 72 hours after transfection. Afterwards, genomic DNA was harvested from cells with or without selection and analyzed by NGS.

Remarkably, a significant increase of the C-T conversion rate was observed across all tested sites in DT-selected cells compared to non-selected cells, and the fold change ranged from 4.1-fold to 7.0-fold (FIG. 26B). For the *DPM2* site, the total conversion rate increased from 20% to 94% by DT selection (FIG. 26B). Similar improvement in editing efficiency was observed when the method was applied to other cell lines. A 12.8-fold increase in C-T conversion rate at the *PCSK9* locus in HCT116 cells, and a 4.9-fold increase at the integrated *BFP* locus in DT-treated PC9 cells when compared to non-treated cells (FIG. 26C).

A similar co-selection experiment was performed for enriching ABE editing events. Five sgRNAs, including one targeting *EMX1* and four others targeting new genomic loci (*CTLA4* (cytotoxic T-lymphocyte-associated protein 4), *IL2RA* (Interleukin 2 Receptor Subunit Alpha), and two different sites of *MVS1* (Adeno-Associated Virus Integration Site 1)), were tested. Each of these sgRNAs was co-transfected with ABE7.10 and sgRNA5 into HEK293 cells, as described in Example 6. After 72 hours, the selected cells were treated with DT (20 ng/µl). Genomic DNA was extracted from both selected and non-selected cells and analyzed by Amplicon-Seq. Compared to non-selected cells, a dramatic increase of A-G conversion rate across all tested targets in selected cells was observed, ranging from 5.7-fold to 12.7-fold. At the targeted loci *CTLA4* and *IL2RA,* the total conversion rate was increased from 4.6% to 39% and from 11.5% to 77.4%, respectively (Fig. 26D).

In addition to co-selecting for base editing events, the possibility of co-selecting indels generated by SpCas9 was also tested. Four sgRNAs (targeting *DPM2, EMX1*, *PCSK9* and *DNMT3B*, respectively) used in CBE co-selection were tested in an experiment for genomic editing co-selection. Each sgRNA was co-transfected with the SpCas9/sgRNA10 combination (as described above in Example 6) into HEK293 cells to generate indels and performed Amplicon-Seq following selection. It was observed that indel rates across all four targets (*DPM2, EMX1*, *PCSK9* and *DNMT3B*) increased to above 90%. In particular, the editing efficiency at the *PCKS9* site increased from 30% to 98% through DT selection (FIG. 26E).

### Example 8. Efficient Enrichment of Bi-Allelic Knock-In Events at HBEGF Locus

In this Example, experiments were performed to enhance the knock-in efficiency of a gene of interest or to achieve bi-allelic knock-in of a gene of interest.

Detailed experimental protocols are described in Example 5. Briefly, for the knock-in experiment, Cas9 plasmid DNA, sgRNAIn3 plasmid DNA and template DNA were transfected at a weight ratio of 4:1:10. Transfection was performed using FuGENE HD transfection reagent (Promega) according to the manufacturer's instructions using a 3:1 transfection reagent to plasmid DNA ratio. 22 days after transfection, cells were assessed with a BD Fortessa (BD Biosciences) and flow cytometry data were analyzed with the FlowJo software (Three Star). Genomic DNA was also extracted from cells and PCR analysis was performed to discriminate between successful knock-in into HBEGF intron 3 (PCR1) and wild-type sequence (PCR2).

It was hypothesized that cells could be rendered resistant to DT by knock-in, at intron 3 of *HBEGF,* a cassette containing a strong splicing acceptor combined with a cDNA sequence containing all of the remaining exons downstream of exon 3 and containing a mutation that prevents binding of DT. The Glu141Lys amino acid substitution was inserted based on the base editing screening described in Example 6 and the presence of a similar substitution in mouse Hbegf (see FIG. 25A). To further exclude the possibility of any detrimental effect of this substitution to cell fitness, a recombinant Glu141Lys-substituted HBEGF protein and showed that it was still functional in inducing p44/p42 MAPK phosphorylation with no significant difference observed compared to wild-type HBEGF, indicating that its major function in EGFR activation is maintained (FIG. 27A).

Subsequently, a knock-in strategy was designed to introduce a DT-resistant HBEGF coupled to a gene of interest. First, a sgRNA (sgRNAIn3) targeting the middle region of intron 3 of *HBEGF* was selected, which has low predicted off-target sites and is efficient in inducing indels at the target site. Repair templates were also designed to contain a splice acceptor and the rest of mutated *HBEGF* exon sequences encoding the Glu141Lys substitution and linked by a T2A self-cleaving peptide to a gene of interest (e.g., mCherry or GFP) (FIG. 27B). In this design, wild-type cells or edited cells presenting small indels in intron 3 will not obtain resistance to DT, while cells with the desired knock-in will become resistant to DT.

Repair templates were tested in different forms, including plasmid, double-stranded DNA (dsDNA), and single-stranded DNA (ssDNA) to determine knock-in efficiency. Templates were designed with or without homology arms or flanking sgRNAs and were expected to be incorporated into the *HBEGF* locus by non-homologous end joining (NHEJ), homologous recombination (HR), or homology-mediated end-joining (HMEJ) (FIG. 27C). Each template was co-transfected with SpCas9 and sgRNAIn3 into HEK293 cells to generate knock-in cells. The selection was performed as described above. Since the expression of the mCherry or GFP gene is coupled with the mutated *HBEGF* gene, only cells with correct insertions were expected to express functional fluorescent proteins. The percentage of knock-in cells (fluorescent cells) were quantified by flow cytometry analysis.

Remarkably, it was observed that mCherry or GFP positive cells occurred independent of templates applied, and the percentage of knock-in cells increased dramatically after selection in all conditions (FIG. 27C). In particular, cells repaired with the plasmid template containing homology arms and sgRNAs (pHMEJ) or the plasmid template containing only homology arms (pHR) achieved nearly 100% of knock-in after selection (FIG. 27C). Among all templates tested, pHMEJ was shown to be most efficient, and only 34.8% of knock-in cells were obtained without selection (FIG. 27C). These observations aligned with additional results showing that bi-allelic mutations in base-editing selection (FIG. 24B), suggesting that cells may require bi-allelic knock-in to survive DT treatment. Two pairs of primers were designed to check the genomic status of edited cells, one pair amplifying the 5' junction of the knock-in sequence (PCR1) and another pair amplifying the wild type sequence of *HBEGF* intron (PCR2). PCR analysis was performed on cells repaired with pHMEJ template with or without selection, respectively. Despite both samples showing a band for homologous knock-in (PCR1), only wild type band was detected in the non-selected sample (FIG. 27E), indicating all cells obtained bi-allelic knock-in after DT selection.

The DT selection method was further compared against the traditional antibiotic-dependent selection method for enriching knock-in events. A new pHMEJ template was designed to include both DT resistant mutation and puromycin resistant gene, and the expression of these two selection markers was coupled by a P2A self-cleaving peptide (FIG. 27D). This new template for knock-in was tested, and knock-in cells were enriched with either DT or puromycin, followed by flow cytometry analysis. Interestingly, nearly 100% of mCherry positive cells in both populations was observed, but DT enriched cells showed a dramatically higher mean fluorescence intensity compared to puromycin enriched cells (FIG. 27D). This observation, together with PCR analysis (FIG. 27E), suggested DT selection enriched cells with bi-allelic knock-in while puromycin selection did not.

This genetic engineering strategy is referred to herein as "Xential" (recombination **(X)** in a locus ess**ential** for cell survival).

### Example 9. Enrichment of Knock-Out and Knock-In Events by Xential Co-Selection

In this Example, Xential knock-in for enrichment of knock-out or knock-in events at second, unrelated locus was tested.

Detailed experimental protocols are described in Example 5. Briefly, for the Xential co-selection experiment, the amount of each transfected plasmid are listed in Table 7 below. Transfection was performed using FuGENE HD transfection reagent (Promega) according to the manufacturer's instructions using a 3:1 transfection reagent to plasmid DNA ratio. Cells were treated with 20 ng/ml diphtheria toxin 3 days after transfection, and then treated again 5 days after transfection. At 22 days after transfection, cells were assessed with a BD Fortessa (BD Biosciences), and flow cytometry data were analyzed with the FlowJo software (Three Star). Genomic DNA was also extracted from cells and same PCR analysis and Amplicon-Seq analysis was performed as described for the previous Examples.

**Table 7. Transfection Amounts for Xential Co-Selection**

| **Xential co-selection of knock-out events** | | | | | |
|---|---|---|---|---|---|
| | Genome editor/ sgRNA 1/ *HBEGF* repair template/ sgRNA2 | | | | |
| Genome editor (SpCas9) | 160 ng | | | | |
| sgRNA1 (Selection sgRNA) | 20 ng | | | | |
| sgRNA2 (Target sgRNA) | 20 ng | | | | |
| *HBEGF* repair template | 400 ng | | | | |

| **Xential co-selection of knock-in events** | | | | | |
|---|---|---|---|---|---|
| | Target pHR: *HBEGF* pHR | Target pHMEJ: *HBEGF* pHMEJ | Target pHMEJ: *HBEGF* pHMEJ | Target pHMEJ: *HBEGF* pHMEJ | Target oligos: *HBEGF* pHR |
| | 2:1 | 1:1 | 3:1 | 4:1 | 2:1 |
| Genome editor (SpCas9) | 160 ng | 160 ng | 160 ng | 160 ng | 160 ng |
| sgRNA1 (Selection sgRNA) | 13.3 ng | 20 ng | 10 ng | 8 ng | 13.3 ng |
| sgRNA2 (Target sgRNA) | 26.7 ng | 20 ng | 30 ng | 32 ng | 26.7 ng |
| *HBEGF* repair template | 133 ng | 200 ng | 100 ng | 80 ng | 133 ng |
| Target repair template | 267 ng | 200 ng | 300 ng | 320 ng | |
| Target oligo | | | | | 267 ng |

First, enrichment of knock-out events was tested. The same four sgRNAs (targeting *DPM2, EMX1, PCSK9,* and *DNMT3B*, respectively) tested in the previous indel enrichment experiment described in Example 7 (FIG. 26E) were utilized. Each sgRNA was co-delivered with SpCas9, sgRNAIn3, and the pHMEJ template into HEK293 cells, and DT selection was performed as described in FIG. 28A. Genomic DNA was extracted from these cells and analyzed by Amplicon-Seq. Significant improvement in editing efficiency was observed for all targets in selected cells compared to non-selected cells, ranging from 4.4-fold to 14.3-fold of improvement. In particular, the editing efficiency at EMX1 locus was increased from 22% to 88% with DT selection (FIG. 28B). All surviving cells maintained mCherry expression indicating edited cells maintained precise knock-in at HBEGF locus (FIG. 28D).

Next, Xential was tested for co-selection of knock-in events. Two forms of repair template plasmids were designed, one pHR and one pHMEJ, to introduce a C-terminal GFP tag to histone protein H2B (HIST2BC) using the same sgRNA. SpCas9, sgRNAs, and two templates targeting HIST2BC and HBEGF were co-delivered into HEK293 cells, and the knock-in efficiency was analyzed by the percentage of GFP (HIST2BC) or mCherry (HBEGF). With either form of templates provided, significantly improved knock-in efficiency was obtained after DT selection. For the pHR template, the efficiency was improved up to 6.4-fold and for the pHMEJ template, the efficiency was improved up to 5.3-fold, reaching 48% (FIG. 28C). By reducing the ratios of the amount of sgRNA and template for HBEGF locus to that for HIST2BC locus, the knock-in efficiency at HIST2BC locus could be increased in selected cells, indicating the fold of enrichment is tunable (FIG. 28C). The percentage of GFP positive cells in enriched cells was increased from 23%, to 42%, to 48% applying a increasing weight ratios of repair plasmids for HIST2BC locus to these for HBEGF locus from 1:1, to 3:1, to 4:1, respectively, while the percentage of mCherry positive cells maintained nearly 100% (FIG. 28E). This method was also demonstrated to enrich the efficiency of oligo mediated knock-in at CD34 locus. A 26-fold increase of the percentage of knock-in cells was observed when co-selection was applied, suggesting the flexibility of template usage in knock-in mediated co-selection (FIG. 28F).

### Example 10. Enrichment of Base Editing and Knock-In Events in iPSCs

In this Example, experiments were performed using the DT-HBEGF selection to enrich base editing events and precise knock-in events in iPSCs.

Detailed experimental protocols are described in Example 5. Briefly, for CBE/ABE co-selection of iPSCs, CBE3/ABE7.10 plasmid DNA, targeting sgRNA plasmid DNA, and selection sgRNA plasmid DNA were transfected at a weight ratio of 8:1:1. For Xential knock-in in iPSCs, Cas9 plasmid DNA, sgRNAIn3 plasmid DNA, and template plasmid DNA were transfected at a weight ratio of 4:1:10. Transfection was performed using FuGENE HD transfection reagent (Promega) according to the manufacturer's instructions using a 2.5:1 transfection reagent to plasmid DNA ratio and a reverse transfection protocol. Cells were treated with 20 ng/ml diphtheria toxin 3 days after transfection. DT-supplemented growth medium was exchanged daily until negative control cells died. Xential knock-in cells were assessed with a BD Fortessa (BD Biosciences), and flow cytometry data were analyzed with the FlowJo software (Three Star). Genomic DNA was also extracted from cells and same PCR analysis and Amplicon-Seq analysis was performed as described for the previous Examples.

Two sgRNAs were selected for CBE and ABE co-selection, one targeting EMX1, a locus widely tested in other genome editing research, and another targeting CTLA4, a gene studied extensively for its role in immune signaling. Each sgRNA was co-transfected together with CBE3/sgRNA10 or with ABE7.10/ sgRNA5 pairs into iPSCs. The selection was performed by DT treatment (20 ng/µl) starting from 72 hours after transfection. Genomic DNA was extracted at confluence and target loci analyzed by Amplicon-Seq using NGS. Notably, a dramatic increase of editing efficiency upon DT selection was observed at all tested sites for both CBE and ABE. The increase of CBE editing efficiency ranged from 19-fold to 60-fold across those two sites, and the increase of ABE editing efficiency is about 24-fold for both sites. The C-T conversion rate at EMX1 site was increased from 5% to 91%, and the A-G conversion rate at CTLA4 site was increased from 0.8% to 19% through DT selection (FIGS. 29A, B).

Next, Xential was tested in iPSCs. iPSCs were provided with the pHMEJ template, together with SpCas9 and sgRNAIn3, and knock-in efficiency was 25.6% without selection. The knock-in efficiency increased to nearly 100% after DT selection (FIG. 29C). The same PCR analyses were performed as in Example 8 to detect the correct insertion and the wild-type HBEGF intron. No residual wild-type band was detected in the targeted HBEGF after DT selection, suggesting full bi-allelic knock-in in the selected pool of iPSCs (FIG. 29D).

### Example 11. Enrichment of Base Editing Events in Primary T Cells

In this Example, experiments were performed using the DT-HBEGF selection to enrich cytidine base editing events in primary T cells at a second, unrelated genomic locus. Further, experiments were performed using DT-HBEGF selection system for enrichment of knock-in events at HBEGF locus.

Detailed experimental protocols are described in Example 5. Briefly, for CBE co-selection in primary T cells, 20 µg CBE3 protein, 2 µg of target sgRNA and 2 µg of selection sgRNA (TrueGuide Synthetic gRNA, Life Technologies), and 2.4 µg electroporation enhancer oligonucleotides (HPLC-purified, Sigma) (Table 3E) were mixed and incubated for 15 minutes, then electroporated into primary T cells. Transfected CD4+ T cells were treated with 1000 ng/mL DT at days 1, 4 and 7 after electroporation. Genomic DNA was also extracted from cells, and Amplicon-Seq analysis was performed as described for previous Examples. For Xential experiment in primary T cells, 5 µg SpCas9 protein (Life Technologies), 1.2 µg of dual gRNAIn3 ( Alt-R CRISPR-Cas9 crRNA, Alt-R CRISPR-Cas9 tracrRNA, IDT) were mixed and incubated for 15 minutes, and then electroporated together with 1 µg dsDNA template into primary T cells. Transfected CD4+ T cells, were treated with 1000 ng/mL DT at day 1, 4, 6 and 8 after electroporation. Cells were analyzed by flow cytometry at day 10 after electroporation.

Three sgRNAs were designed to introduce premature stop codons in PCDC1 (Programmed cell death protein 1), CTLA4, and IL2RA, respectively, due to their important roles in immune regulation. Each sgRNA was co-electroporated with purified CBE3 proteins and synthetic sgRNA10 into isolated CD4+ T cells. Primary T cells were selected with 1000 ng/µL DT starting from 24 hours after electroporation, and genomic DNA from unselected and selected cells were analyzed 9 days after transfection. A 1.7 to 1.8-fold increase in base editing efficiency was observed for all three loci compared to non-selected cells (FIG. 30). Three different forms of dsDNA (dsHR, dsHMEJ, dsHR2) described in Figure 3 were applied as repair templates. Each template was electroporated with pre-mixed SpCas9 protein and synthetic dual gRNAIn3 complex into primary CD4+ T cells. Primary T cells with 1000 ng/µl DT were selected starting from 24 hours after electroporation, and analyzed knock-in efficiency of unselected and selected cells 10 days after transfection. A 3-8 fold of increase in knock-in efficiency for all three versions of templates in selected cells was observedcompared to non-selected cells

### Example 12. Enrichment of Base Editing Events In Vivo By Co-Selection

In this Example, experiments were performed using the DT-HBEGF selection to enrich cytidine base editing events in humanized mice models at a second, unrelated genomic locus.

Detailed experimental protocols are described in Example 5 (see section for "Cytidine Base Editing and DT Treatment of Mice Humanized for hHBEGF Expression").

Co-selection of cytidine base editing events was tested in a humanized mouse model expressing human HBEGF (hHBEGF) under the liver cell-specific albumin promoter. Mouse *Pcsk9* gene was chosen as the target locus, and an sgRNA was designed to introduce a premature stop codon with CBE3 into *Pcsk9* by adenovirus (AdV8) delivering CBE3, the sgRNA targeting *Pcsk9,* and the sgRNA targeting human *HBEGF.* Two weeks after AdV8 injection, mice were treated with DT (200 ng/kg, intraperitoneal). Mice were divided into two groups, the control non-enriched terminated at 24 hours, before DT could exert toxicity. The enriched group was terminated 11 days after DT treatment (FIG. 31A). Amplicon-Seq analysis of genomes from mouse livers indicated a 2.8-fold increase of base editing efficiency at the selection locus as a result of DT selection (FIG. 31B). Remarkably, a 2.5-fold improvement of *Pcsk9* editing was also identified in the enriched group compared to the control group (FIG. 31C), demonstrating for the first time that genome editing events can be co-selected *in vivo* using a toxin mediated selection.

### Example 13 Enrichment of prime editing events by co-selection

In this experiment DT-HBEGF selection system were used for enrichment of prime editing events at a second, unrelated genomic locus.

For co-targeting enrichment, PE2 plasmid DNA, targeting pegRNA plasmid DNA and selection pegRNA_HBEGF12 plasmid DNA were transfected at a weight ratio of 8:1:1. Transfection was performed using FuGENE HD transfection reagent (Promega) using a 3:1 transfection reagent to plasmid DNA ratio. Cells were treated with 20 ng/ml diphtheria toxin 3 days after transfection, and then treated again 5 days after transfection. Genomic DNA was extracted from surviving cells and analyzed by Amplicon-Seq using Next Generation Sequencing (NGS).

Prime editing co-selection in HEK293 cells were tested. 4 prime editing guide RNAs (pegRNA) were used for targeting 3 different genomic loci: EMX1 (Empty Spiracles Homeobox 1), FANCF (FA complementation group F), and HEK3. Each of these pegRNAs was co-transfected into cells with Prime Editor 2 (PE2) and pegRNA_HBEGF12 (Designed to introduce E141H resistant mutation at HBEGF locus), and the selected cells were enriched with DT (20 ng/mL) starting from 72 hours after transfection. Afterwards, genomic DNA was harvested from cells with or without selection and analyzed by NGS. A significant increase of prime editing efficiency at HBEGF locus, from ~1% to above 99% was observed. For all co-selected target loci, higher than average editing efficiencies in DT selected cells were observed compared to non-selected cells, and the fold of increase ranged from 1.5-fold to 44-fold.

### Example 14 Enrichment of Cas9-editing events by co-selection with anti-CD52 antibody-Drug maytansinoid (DM1) conjugates (anti-CD52-DM1)

In this experiment anti-CD52-DM1 antibody conjugated drug were used for selection of SpCas9 editing events at a second, unrelated genomic locus.

SpCas9 editing co-selection in primary CD4+ T cells was tested. 3 sgRNAs were used targeting 3 different genomic loci: PDCD1, CTLA4 and IL2RA, respectively.

For SpCas9 co-selection in primary T cells, 5 µg TrueCut Cas9 Protein v2 (Life Technologies), 0.6 µg of target sgRNA and 0.6 µg of selection sgRNA ( TrueGuide Synthetic gRNA, Life Technologies) and 0.8 µg electroportation enhancer oligos for Cas9 (HPLC-purified, Sigma) (Table S1) were mixed and incubated for 15 minutes, and then electroporated into primary T cells. Transfected CD4+ T cells were treated with 2.5ug/ml anti-CD52-DM1, 2.5ug/ml NIP228-DM1 and PBS separately, at day 2, 4 and 6 after electroporation. Genomic DNA was also extracted from cells and Amplicon-Seq analysis was performed.

The anti-CD52, Alemtuzumab, (Campath-1) antibody sequence was retrieved from the Drugbank database (https://www.drugbank.ca/drugs/DB00087) and the antibody variable light and heavy gene segments were designed and ordered from Thermofisher for cloning into the in-house pOE IgG1 antibody expression vector. The cloned pOE-anti-CD52.IgG1 expression construct was transfected into CHO-G22 cells and cultured for fourteen days. The conditioned media was collected, filtered (0.2 uM filter) and purified via protein A using an Aligent Pure FPLC instrument. The antibody was dialyzed into 1X PBS pH 7.2 and the binding to human CD52 antigen (Abeam) was confirmed via SPR using the Octet and compared to commercially available Campath-1. Additionally, mass spectrometry was used to verify the molecular weight and the monomer content was determined by size exclusion chromatography. The anti-CD52 and a negative control (NIP228) mAb was buffer exchanged in to 1X borate buffer pH 8.5 and 40 mgs of each antibody was incubated with 4.5 molar equivalencies of SMCC-DM1 payload. The degree of drug conjugation was determined by reduced reverse phase mass spectrometry and the reaction was terminated by the addition of 10% v/v 1M Tris-HCl. The free or un-conjugated SMCC-DM-1 payload and the protein aggregates were simultaneously removed using ceramic hydroxyapatite chromatography. The ADCs were then dialyzed into PBS pH 7.2. The concentration and endotoxin level were measured using a nanodrop (Thermofisher) and Endosafe (Charles Rivers) instrument, respectively.

Each synthetic sgRNA was co-electroporated with SpCas9 proteins and synthetic sgRNA targeting CD52 into isolated CD4+ T cells. Electroporated T cells were treated with 2.5ug/ml anti-CD52-DM1, 2.5ug/ml NIP228-DM1 (Negative control antibody drug conjugates) and PBS (untreated) separately, starting from 48 hours after electroporation, and analyzed genomic DNA from treated cells 7 days after the first treatment. Afterwards, genomic DNA was harvested from cells with or without selection and analyzed by NGS. An increase of indels rates in samples treated with anti-CD52-DM1 was observed compared to samples treated with Nip228-DM1 or PBS (untreated). A two-tailed paired t test was performed to compare the difference between the indels rates of anti-CD52-DM1 treated cells and that of Nip228-DM1 treated cells, which showed that the increase of indel rates at targeted loci (IL2RA, CTLA4, PDCD1) is significant (P=0.0044). The same analysis comparing indels rates of anti-CD52-DM1 treated cells and that of untreated cells showed the increase of indel rates at targeted loci is also significant (P=0.0008).

### Clauses

1. A method of introducing a site-specific mutation in a target polynucleotide in a target cell in a population of cells, the method comprising:
   (a) introducing into the population of cells:
      (i) a base-editing enzyme;
      (ii) a first guide polynucleotide that
         (1) hybridizes to a gene encoding a cytotoxic agent (CA) receptor, and
         (2) forms a first complex with the base-editing enzyme,
         wherein the base-editing enzyme of the first complex provides a mutation in the gene encoding the CA receptor, and wherein the mutation in the gene encoding the CA receptor forms a CA-resistant cell in the population of cells; and
      (iii) a second guide polynucleotide that
         (1) hybridizes with the target polynucleotide, and
         (2) forms a second complex with the base-editing enzyme,
         wherein the base-editing enzyme of the second complex provides a mutation in the target polynucleotide;
   (b) contacting the population of cells with the CA; and selecting the CA-resistant cell from the population of cells, thereby enriching for the target cell comprising the mutation in the target polynucleotide.
2. A method of determining efficacy of a base-editing enzyme in a population of cells, the method comprising:
   (a) introducing into the population of cells:
      (i) a base-editing enzyme;
      (ii) a first guide polynucleotide that
         (1) hybridizes to a gene encoding a cytotoxic agent (CA) receptor, and
         (2) forms a first complex with the base-editing enzyme,
         wherein the base-editing enzyme of the first complex introduces a mutation in the gene encoding the CA receptor, and wherein the mutation in the gene encoding the CA receptor forms a CA-resistant cell in the population of cells; and
      (iii) a second guide polynucleotide that
         (1) hybridizes with the target polynucleotide, and
         (2) forms a second complex with the base-editing enzyme,
         wherein the base-editing enzyme of the second complex introduces a mutation in the target polynucleotide;
   (b) contacting the population of cells with the CA to isolate CA-resistant cells; and
   (c) determining the efficacy of the base-editing enzyme by determining the ratio of the CA-resistant cells to the total population of cells.
3. The method of clause 1 or 2, wherein the base-editing enzyme comprises a DNA-targeting domain and a DNA-editing domain.
4. The method of clause 3, wherein the DNA-targeting domain comprises Cas9.
5. The method of clause 4, wherein the Cas9 comprises a mutation in a catalytic domain.
6. The method of any one of clauses 1-5, wherein the base-editing enzyme comprises a catalytically inactive Cas9 and a DNA-editing domain.
7. The method of any one of clauses 1-5, wherein the base-editing enzyme comprises a Cas9 capable of generating single-stranded DNA breaks (nCas9) and a DNA-editing domain.
8. The method of clause 7, wherein the nCas9 comprises a mutation at amino acid residue D10 or H840 relative to wild-type Cas9 (numbering relative to SEQ ID NO: 3).
9. The method of any one of clauses 4-8, wherein the Cas9 is at least 90% identical to SEQ ID NO: 3 or 4.
10. The method of any one of clauses 3-9, wherein the DNA-editing domain comprises a deaminase.
11. The method of clause 10, wherein the deaminase is cytidine deaminase or adenosine deaminase.
12. The method of clause 11, wherein the deaminase is cytidine deaminase.
13. The method of clause 11, wherein the deaminase is adenosine deaminase.
14. The method of any one of clauses 10-13, wherein the deaminase is an apolipoprotein B mRNA-editing complex (APOBEC) deaminase, an activation-induced cytidine deaminase (AID), an ACF1/ASE deaminase, an ADAT deaminase, or an ADAR deaminase.
15. The method of clause 14, wherein the deaminase is an apolipoprotein B mRNA-editing complex (APOBEC) family deaminase.
16. The method of clause 15, wherein the deaminase is APOBEC1.
17. The method of any one of clauses 3-16, wherein the base-editing enzyme further comprises a DNA glycosylase inhibitor domain.
18. The method of clause 17, wherein the DNA glycosylase inhibitor is uracil DNA glycosylase inhibitor (UGI).
19. The method of any one of clauses 1-4 or 6-18, wherein the base-editing enzyme comprises nCas9 and cytidine deaminase.
20. The method of any one of clauses 1-4 or 6-18, wherein the base-editing enzyme comprises nCas9 and adenosine deaminase.
21. The method of any one of clauses 1-12 or 13-19, wherein the base-editing enzyme comprises a polypeptide sequence at least 90% identical to SEQ ID NO: 6.
22. The method of any one of clauses 1-12 or 13-19, wherein the base-editing enzyme is BE3.
23. The method of any one of clauses 1-22, wherein the first and/or second guide polynucleotide is an RNA polynucleotide.
24. The method of any one of clauses 1-23, wherein the first and/or second guide polynucleotide further comprises a tracrRNA sequence.
25. The method of any one of clauses 1-24, wherein the population of cells are human cells.
26. The method of any one of clauses 1-25, wherein the mutation in the gene encoding the CA receptor is a cytidine (C) to thymine (T) point mutation.
27. The method of any one of clauses 1-25, wherein the mutation in the gene encoding the CA receptor is an adenine (A) to guanine (G) point mutation.
28. The method of any one of clauses 1-27, wherein the CA is diphtheria toxin.
29. The method of clause 28, wherein the cytotoxic agent (CA) receptor is a receptor for diphtheria toxin.
30. The method of clause 29, wherein the CA receptor is a heparin binding EGF like growth factor (HB-EGF).
31. The method of clause 30, wherein the HB-EGF comprises a polypeptide sequence of SEQ ID NO: 8.
32. The method of clause 31, wherein the base-editing enzyme of the first complex provides a mutation in one of more of amino acids 107 to 148 in HB-EGF (SEQ ID NO: 8).
33. The method of clause 32, wherein the base-editing enzyme of the first complex provides a mutation in one of more of amino acids 138 to 144 in HB-EGF (SEQ ID NO: 8).
34. The method of clause 33, wherein the base-editing enzyme of the first complex provides a mutation in amino acid 141 in HB-EGF (SEQ ID NO: 8).
35. The method of clause 34, wherein the base-editing enzyme of the first complex provides a GLU141 to LYS141 mutation in the amino acid sequence of HB-EGF (SEQ ID NO: 8).
36. The method of any one of clauses 1-35, wherein the base-editing enzyme of the first complex provides a mutation in a region of HB-EGF that binds diphtheria toxin.
37. The method of any one of clauses 1-36, wherein the base-editing enzyme of the first complex provides a mutation in HB-EGF which makes the target cell resistant to diphtheria toxin.
38. The method of any one of clauses 1-37, wherein the mutation in the target polynucleotide is a cytidine (C) to thymine (T) point mutation in the target polynucleotide.
39. The method of any one of clauses 1-37, wherein the mutation in the target polynucleotide is an adenine (A) to guanine (G) point mutation in the target polynucleotide.
40. The method of any one of clauses 1-39, wherein the base-editing enzyme is introduced into the population of cells as a polynucleotide encoding the base-editing enzyme.
41. The method of clause 40, wherein the polynucleotide encoding the base-editing enzyme, the first guide polynucleotide of (ii), and the second guide polynucleotide of (iii) are on a single vector.
42. The method of clause 40, wherein the polynucleotide encoding the base-editing enzyme, the first guide polynucleotide of (ii), and the second guide polynucleotide of (iii) are on one or more vectors.
43. The method of clause 41 or 42, wherein the vector is a viral vector.
44. The method of clause 43, wherein the viral vector is an adenovirus, a lentivirus, or an adeno-associated virus.
45. A method of providing a bi-allelic integration of a sequence of interest (SOI) into a toxin sensitive gene (TSG) locus in a genome of a cell, the method comprising:
   (a) introducing into a population of cells:
      (i) a nuclease capable of generating a double-stranded break;
      (ii) a guide polynucleotide that forms a complex with the nuclease and is capable of hybridizing with the TSG locus; and
      (iii) a donor polynucleotide comprising:
         (1) a 5' homology arm, a 3' homology arm, and a mutation in a native coding sequence of the TSG, wherein the mutation confers resistance to the toxin; and
         (2) the SOI;
         wherein introduction of (i), (ii), and (iii) results in integration of the donor polynucleotide in the TSG locus;
   (b) contacting the population of cells with the toxin; and
   (c) selecting one or more cells resistant to the toxin,
   wherein the one or more cells resistant to the toxin comprise the bi-allelic integration of the SOI.
46. The method of clause 45, wherein the donor polynucleotide is integrated by homology-directed repair (HDR).
47. The method of clause 45, wherein the donor polynucleotide is integrated by Non-Homologous End Joining (NHEJ).
48. The method of any one of clauses 45-47, wherein the TSG locus comprises an intron and an exon.
49. The method of clause 48, wherein the donor polynucleotide further comprises a splicing acceptor sequence.
50. The method of clause 48 or 49, wherein the nuclease capable of generating a double-stranded break generates a break in the intron.
51. The method of any one of clauses 48-50, wherein the mutation in the native coding sequence of the TSG is in an exon of the TSG locus.
52. A method of integrating a sequence of interest (SOI) into a target locus in a genome of a cell, the method comprising:
   (a) introducing into a population of cells:
      (i) a nuclease capable of generating a double-stranded break;
      (ii) a guide polynucleotide that forms a complex with the nuclease and is capable of hybridizing with a toxin sensitive gene (TSG) locus in the genome of the cell, wherein the TSG is an essential gene; and
      (iii) a donor polynucleotide comprising:
         (1) a functional TSG gene comprising a mutation in a native coding sequence of the TSG, wherein the mutation confers resistance to the toxin,
         (2) the SOI, and
         (3) a sequence for genome integration at the target locus;
         wherein introduction of (i), (ii), and (iii) results in:
         inactivation of the TSG in the genome of the cell by the nuclease, and
         integration of the donor polynucleotide in the target locus;
   (b) contacting the population of cells with the toxin; and
   (c) selecting one or more cells resistant to the toxin,
   wherein the one or more cells resistant to the toxin comprise the SOI integrated in the target locus.
53. The method of clause 52, wherein the sequence for genome integration is obtained from a transposon or a retroviral vector.
54. The method of any one of clauses 45-53, wherein the functional TSG of the donor polynucleotide is resistant to inactivation by the nuclease.
55. The method of any one of clauses 45-54, wherein the mutation in the native coding sequence of the TSG removes a protospacer adjacent motif from the native coding sequence.
56. The method of any one of clauses 45-55, wherein the guide polynucleotide is not capable of hybridizing to the functional TSG of the donor polynucleotide.
57. The method of any one of clauses 45-56, wherein the nuclease capable of generating a double-stranded break is Cas9.
58. The method of clause 57, wherein the Cas9 is capable of generating cohesive ends.
59. The method of clause 57 or 58, wherein the Cas9 comprises a polypeptide sequence of SEQ ID NO: 3 or 4.
60. The method of any one of clauses 45-59, wherein the guide polynucleotide is an RNA polynucleotide.
61. The method of any one of clauses 45-60, wherein the guide polynucleotide further comprises a tracrRNA sequence.
62. The method of any one of clauses 45-61, wherein the donor polynucleotide is a vector.
63. The method of any one of clauses 45-62, wherein the mutation in the native coding sequence of the TSG is a substitution mutation, an insertion, or a deletion.
64. The method of any one of clauses 45-63, wherein the mutation in the native coding sequence of the TSG is a mutation in a toxin-binding region of a protein encoded by the TSG.
65. The method of any one of clauses 45-64, wherein the TSG locus comprises a gene encoding heparin binding EGF-like growth factor (HB-EGF).
66. The method of clause 45-65, wherein the TSG encodes HB-EGF (SEQ ID NO: 8).
67. The method of any one of clauses 45-66, wherein the mutation in the native coding sequence of the TSG is a mutation in one or more of amino acids 107 to 148 in HB-EGF (SEQ ID NO: 8).
68. The method of clause 67, wherein the mutation in the native coding sequence of the TSG is a mutation in one or more of amino acids 138 to 144 in HB-EGF (SEQ ID NO: 8).
69. The method of clause 68, wherein the mutation in the native coding sequence of the TSG is a mutation in amino acid 141 in HB-EGF (SEQ ID NO: 8).
70. The method of clause 69, wherein the mutation in the native coding sequence of the TSG is a mutation of GLU141 to LYS141 in HB-EGF (SEQ ID NO: 8).
71. The method of any one of clauses 65-70, wherein the toxin is diphtheria toxin.
72. The method of any one of clauses 65-71, wherein the mutation in the native coding sequence of the TSG makes the cell resistant to diphtheria toxin.
73. The method of any one of clauses 45-72, wherein the toxin is an antibody-drug conjugate, wherein the TSG encodes a receptor for the antibody-drug conjugate.
74. A method of providing resistance to diphtheria toxin in a human cell, the method comprising introducing into the cell:
   (i) a base-editing enzyme; and
   (ii) a guide polynucleotide targeting a heparin-binding EGF-like growth factor (HB-EGF) receptor in the human cell,

   wherein the base-editing enzyme forms a complex with the guide polynucleotide, and
   wherein the base-editing enzyme is targeted to the HB-EGF and provides a site-specific mutation in the HB-EGF, thereby providing resistance to diphtheria toxin in the human cell.
75. The method of clause 74, wherein the base-editing enzyme comprises a DNA-targeting domain and a DNA-editing domain.
76. The method of clause 75, wherein the DNA-targeting domain comprises Cas9.
77. The method of clause 76, wherein the Cas9 comprises a mutation in a catalytic domain.
78. The method of any one of clauses 74-77, wherein the base-editing enzyme comprises a catalytically inactive Cas9 and a DNA-editing domain.
79. The method of any one of clauses 74-77, wherein the base-editing enzyme comprises a Cas9 capable of generating single-stranded DNA breaks (nCas9) and a DNA-editing domain.
80. The method of clause 79, wherein the nCas9 comprises a mutation at amino acid residue D10 or H840 relative to wild-type Cas9 (numbering relative to SEQ ID NO: 3).
81. The method of any one of clauses 76-80, wherein the Cas9 is at least 90% identical to SEQ ID NO: 3 or 4.
82. The method of any one of clauses 75-81, wherein the DNA-editing domain comprises a deaminase.
83. The method of clause 82, wherein the deaminase is selected from cytidine deaminase and adenosine deaminase.
84. The method of clause 83, wherein the deaminase is cytidine deaminase.
85. The method of clause 83, wherein the deaminase is adenosine deaminase.
86. The method of any one of clauses 82-85, wherein the deaminase is selected from an apolipoprotein B mRNA-editing complex (APOBEC) deaminase, an activation-induced cytidine deaminase (AID), an ACF1/ASE deaminase, an ADAT deaminase, and a TadA deaminase.
87. The method of clause 86, wherein the deaminase is an apolipoprotein B mRNA-editing complex (APOBEC) family deaminase.
88. The method of clause 87, wherein the cytidine deaminase is APOBEC1.
89. The method of any one of clauses 74-88, wherein the base-editing enzyme further comprises a DNA glycosylase inhibitor domain.
90. The method of clause 89, wherein the DNA glycosylase inhibitor is uracil DNA glycosylase inhibitor (UGI).
91. The method of clause 74-84 or 86-90, wherein the base-editing enzyme comprises nCas9 and a cytidine deaminase.
92. The method of clause 74-83 or 85-90, wherein the base-editing enzyme comprises nCas9 and an adenosine deaminase.
93. The method of any one of clauses 74-83 or 86-91, wherein the base-editing enzyme comprises a polypeptide sequence at least 90% identical to SEQ ID NO: 6.
94. The method of any one of clauses 74-83 or 86-93, wherein the base-editing enzyme is BE3.
95. The method of any one of clauses 74-94, wherein the guide polynucleotide is an RNA polynucleotide.
96. The method of any one of clauses 74-95, wherein the guide polynucleotide further comprises a tracrRNA sequence.
97. The method of any one of clauses 74-96, wherein the site-specific mutation is in one or more of amino acids 107 to 148 in the HB-EGF (SEQ ID NO: 8).
98. The method of clause 97, wherein the site-specific mutation is in one or more of amino acids 138 to 144 in the HB-EGF (SEQ ID NO: 8).
99. The method of clause 98, wherein the site-specific mutation is in amino acid 141 in the HB-EGF (SEQ ID NO: 8).
100. The method of clause 99, wherein the site-specific mutation is a GLU141 to LYS141 mutation in the HB-EGF (SEQ ID NO: 8).
101. The method of any one of clauses 74-100, wherein the site-specific mutation is in a region of the HB-EGF that binds diphtheria toxin.
102. A method of integrating and enriching a sequence of interest (SOI) into a target locus in a genome of a cell, the method comprising:
   (a) introducing into a population of cells:
      (i) a nuclease capable of generating a double-stranded break;
      (ii) a guide polynucleotide that forms a complex with the nuclease and is capable of hybridizing with an essential gene (ExG) locus in the genome of the cell; and
      (iii) a donor polynucleotide comprising:
         (1) a functional ExG gene comprising a mutation in a native coding sequence of the ExG, wherein the mutation confers resistance to inactivation by the guide polynucleotide,
         (2) the SOI, and
         (3) a sequence for genome integration at the target locus;
         wherein introduction of (i), (ii), and (iii) results in
         inactivation of the ExG in the genome of the cell by the nuclease, and integration of the donor polynucleotide in the target locus;
   (b) cultivating the cells; and
   (c) selecting one or more surviving cells,
   wherein the one or more surviving cells comprise the SOI integrated at the target locus.
103. A method of introducing a stable episomal vector into a cell, the method comprising:
   (a) introducing into a population of cells:
      (i) a nuclease capable of generating a double-stranded break;
      (ii) a guide polynucleotide that forms a complex with the nuclease and is capable of hybridizing with an essential gene (ExG) locus in the genome of the cell;
         wherein introduction of (i) and (ii) results in inactivation of the ExG in the genome of the cell by the nuclease; and
      (iii) an episomal vector comprising:
         (1) a functional ExG comprising a mutation in a native coding sequence of the ExG, wherein the mutation confers resistance to the inactivation by the nuclease;
         (2) an autonomous DNA replication sequence;
   (b) cultivating the cells; and
   (c) selecting one or more surviving cells,
   wherein the one or more surviving cells comprise the episomal vector.
104. The method of clause 102 or 103, wherein mutation in the native coding sequence of the ExG removes a protospacer adjacent motif from the native coding sequence.
105. The method of any one of clauses 102-104, wherein the guide polynucleotide is not capable of hybridizing to the functional ExG of the donor polynucleotide or the episomal vector.
106. The method of any one of clauses 102-105, wherein the nuclease capable of generating a double-stranded break is Cas9.
107. The method of clause 106, wherein the Cas9 is capable of generating cohesive ends.
108. The method of clause 104 or 107, wherein the Cas9 comprises a polypeptide sequence of SEQ ID NO: 3 or 4.
109. The method of any one of clauses 102-108, wherein the guide polynucleotide is an RNA polynucleotide.
110. The method of any one of clauses 102-109, wherein the guide polynucleotide further comprises a tracrRNA sequence.
111. The method of any one of clauses 102-110, wherein the donor polynucleotide is a vector.
112. The method of any one of clauses 102-111, wherein the mutation in the native coding sequence of the ExG is a substitution mutation, an insertion, or a deletion.
113. The method of any one of clauses 102 or 104-112, wherein the sequence for genome integration is obtained from a transposon or a retroviral vector.
114. The method of any one of clauses 103-112, wherein the episomal vector is an artificial chromosome or a plasmid.
115. The method of any one of clauses 102-114, wherein more than one guide polynucleotide is introduced into the population of cells, wherein each guide polynucleotide forms a complex with the nuclease, and wherein each guide polynucleotide hybridizes to a different region of the ExG.
116. The method of any one of clauses 102, 104-113, or 115, further comprising introducing the nuclease of (a)(i) and the guide polynucleotide of (a)(ii) into the surviving cells to enrich for surviving cells comprising the SOI integrated at the target locus.
117. The method of any one of clauses 103-112, 114, or 115, further comprising introducing the nuclease of (a)(i) and the guide polynucleotide of (a)(ii) into the surviving cells to enrich for surviving cells comprising the episomal vector.
118. The method of clause 116 or 117, wherein the nuclease of (a)(i) and the guide polynucleotide of (a)(ii) are introduced into the surviving cells for multiple rounds of enrichment.

## Claims

1. A method of providing a bi-allelic integration of a sequence of interest (SOI) into a toxin sensitive gene (TSG) locus in a genome of a cell, the method comprising:
(d) introducing into a population of cells:
(iv) a nuclease capable of generating a double-stranded break;
(v) a guide polynucleotide that forms a complex with the nuclease and is capable of hybridizing with the TSG locus; and
(vi) a donor polynucleotide comprising:
(3) a 5' homology arm, a 3' homology arm, and a mutation in a native coding sequence of the TSG, wherein the mutation confers resistance to the toxin; and
(4) the SOI;
wherein introduction of (i), (ii), and (iii) results in integration of the donor polynucleotide in the TSG locus;
(e) contacting the population of cells with the toxin; and
(f) selecting one or more cells resistant to the toxin,
wherein the one or more cells resistant to the toxin comprise the bi-allelic integration of the SOI.

2. The method of claim 1, wherein the donor polynucleotide is integrated by homology-directed repair (HDR) and/or by Non-Homologous End Joining (NHEJ),.

3. The method of claim 1 or 2, wherein the TSG locus comprises an intron and an exon, optionally wherein the donor polynucleotide further comprises a splicing acceptor sequence , optionally wherein the nuclease capable of generating a double-stranded break generates a break in the intron.

4. The method of claim 3, wherein the mutation in the native coding sequence of the TSG is in an exon of the TSG locus.

5. A method of integrating a sequence of interest (SOI) into a target locus in a genome of a cell, the method comprising:
(a) introducing into a population of cells:
(iv) a nuclease capable of generating a double-stranded break;
(v) a guide polynucleotide that forms a complex with the nuclease and is capable of hybridizing with a toxin sensitive gene (TSG) locus in the genome of the cell, wherein the TSG is an essential gene; and
(vi) a donor polynucleotide comprising:
(1) a functional TSG gene comprising a mutation in a native coding sequence of the TSG, wherein the mutation confers resistance to the toxin,
(2) the SOI, and
(3) a sequence for genome integration at the target locus;
wherein introduction of (i), (ii), and (iii) results in:
inactivation of the TSG in the genome of the cell by the nuclease, and
integration of the donor polynucleotide in the target locus;
(b) contacting the population of cells with the toxin; and
(c) selecting one or more cells resistant to the toxin,
wherein the one or more cells resistant to the toxin comprise the SOI integrated in the target locus.

6. The method of claim 5, wherein:
the sequence for genome integration is obtained from a transposon or a retroviral vector;
the functional TSG of the donor polynucleotide is resistant to inactivation by the nuclease;
the mutation in the native coding sequence of the TSG removes a protospacer adjacent motif from the native coding sequence;
the guide polynucleotide is not capable of hybridizing to the functional TSG of the donor polynucleotide; and/or
the nuclease capable of generating a double-stranded break is Cas9, optionally wherein the Cas9 is capable of generating cohesive ends, optionally wherein the Cas9 comprises a polypeptide sequence of SEQ ID NO: 3 or 4

7. The method of any one of claims 1-6, wherein the guide polynucleotide is an RNA polynucleotide, optionally wherein the guide polynucleotide further comprises a tracrRNA sequence, optionally wherein the donor polynucleotide is a vector.

8. The method of any one of claims 1-7, wherein the mutation in the native coding sequence of the TSG is a substitution mutation, an insertion, or a deletion, optionally wherein the mutation in the native coding sequence of the TSG is a mutation in a toxin-binding region of a protein encoded by the TSG.

9. The method of any one of claims 1-8, wherein the TSG locus comprises a gene encoding heparin binding EGF-like growth factor (HB-EGF), optionally wherein the TSG encodes HB-EGF (SEQ ID NO: 8).

10. The method of any one of claims 1-9, wherein the mutation in the native coding sequence of the TSG is a mutation in one or more of amino acids 107 to 148 in HB-EGF (SEQ ID NO: 8), optionally wherein the mutation in the native coding sequence of the TSG is a mutation in one or more of amino acids 138 to 144 in HB-EGF (SEQ ID NO: 8), optionally wherein the mutation in the native coding sequence of the TSG is a mutation in amino acid 141 in HB-EGF (SEQ ID NO: 8), optionally wherein the mutation in the native coding sequence of the TSG is a mutation of GLU141 to LYS141 in HB-EGF (SEQ ID NO: 8).

11. The method of claim 9 or 10, wherein the toxin is diphtheria toxin, optionally wherein the mutation in the native coding sequence of the TSG makes the cell resistant to diphtheria toxin.

12. The method of any one of claims 1-11, wherein the toxin is an antibody-drug conjugate, wherein the TSG encodes a receptor for the antibody-drug conjugate.

13. A method of integrating and enriching a sequence of interest (SOI) into a target locus in a genome of a cell, the method comprising:
(a) introducing into a population of cells:
(i) a nuclease capable of generating a double-stranded break;
(ii) a guide polynucleotide that forms a complex with the nuclease and is capable of hybridizing with an essential gene (ExG) locus in the genome of the cell; and
(iii) a donor polynucleotide comprising:
(4) a functional ExG gene comprising a mutation in a native coding sequence of the ExG, wherein the mutation confers resistance to inactivation by the guide polynucleotide,
(5) the SOI, and
(6) a sequence for genome integration at the target locus;
wherein introduction of (i), (ii), and (iii) results in inactivation of the ExG in the genome of the cell by the nuclease, and integration of the donor polynucleotide in the target locus;
(b) cultivating the cells; and
(c) selecting one or more surviving cells,
wherein the one or more surviving cells comprise the SOI integrated at the target locus.

14. A method of introducing a stable episomal vector into a cell, the method comprising:
(a) introducing into a population of cells:
(i) a nuclease capable of generating a double-stranded break;
(ii) a guide polynucleotide that forms a complex with the nuclease and is capable of hybridizing with an essential gene (ExG) locus in the genome of the cell;
wherein introduction of (i) and (ii) results in inactivation of the ExG in the genome of the cell by the nuclease; and
(iii) an episomal vector comprising:
(3) a functional ExG comprising a mutation in a native coding sequence of the ExG, wherein the mutation confers resistance to the inactivation by the nuclease;
(4) an autonomous DNA replication sequence;
(b) cultivating the cells; and
(c) selecting one or more surviving cells,
wherein the one or more surviving cells comprise the episomal vector.

15. The method of claim 13 or 14, wherein mutation in the native coding sequence of the ExG removes a protospacer adjacent motif from the native coding sequence, optionally wherein the guide polynucleotide is not capable of hybridizing to the functional ExG of the donor polynucleotide or the episomal vector, optionally wherein the nuclease capable of generating a double-stranded break is Cas9, optionally wherein the Cas9 is capable of generating cohesive ends, optionally wherein the Cas9 comprises a polypeptide sequence of SEQ ID NO: 3 or 4.

16. The method of any one of claims 13-15, wherein the guide polynucleotide is an RNA polynucleotide, optionally wherein the guide polynucleotide further comprises a tracrRNA sequence, optionally wherein the donor polynucleotide is a vector, optionally wherein the mutation in the native coding sequence of the ExG is a substitution mutation, an insertion, or a deletion.

17. The method of any one of claims 13 or 15-16, wherein the sequence for genome integration is obtained from a transposon or a retroviral vector, optionally wherein more than one guide polynucleotide is introduced into the population of cells, wherein each guide polynucleotide forms a complex with the nuclease, and wherein each guide polynucleotide hybridizes to a different region of the ExG.

18. The method of any one of claims 14-16, wherein the episomal vector is an artificial chromosome or a plasmid, optionally wherein more than one guide polynucleotide is introduced into the population of cells, wherein each guide polynucleotide forms a complex with the nuclease, and wherein each guide polynucleotide hybridizes to a different region of the ExG.

19. The method of any one of claims 13, 15-18, , further comprising introducing the nuclease of (a)(i) and the guide polynucleotide of (a)(ii) into the surviving cells to enrich for surviving cells comprising the SOI integrated at the target locus, optionally wherein the nuclease of (a)(i) and the guide polynucleotide of (a)(ii) are introduced into the surviving cells for multiple rounds of enrichment.

20. The method of any one of claims 14-16, or 18, further comprising introducing the nuclease of (a)(i) and the guide polynucleotide of (a)(ii) into the surviving cells to enrich for surviving cells comprising the episomal vector, optionally wherein the nuclease of (a)(i) and the guide polynucleotide of (a)(ii) are introduced into the surviving cells for multiple rounds of enrichment.
